# EUROPEAN PATENT APPLICATION

(11) **EP 3 502 256 A2**
(43) Date of publication of application: **26.06.2019**
(21) Application number: 18198570.6
(22) Date of filing: 26.09.2009
(51) Int. Cl.: C12N 15/52, C12N 5/10, C12N 15/867, C07K 14/39, C12N 9/10, C07K 14/55, C07K 14/57, C12N 9/78

(54) **RECOMBINANT VECTORS**

(30) Priority: 26.09.2008 US 100666 P; 08.12.2008 US 120618 P; 13.06.2009 US 186823 P
(62) Divisional of application: 09816967.5
(71) Applicant: Tocagen Inc., San Diego, CA 92109 (US)
(72) Inventor: GRUBER, Harry, E., San Diego, CA 92130 (US); JOLLY, Douglas, Encinitas, CA 92024 (US); PEREZ, Omar, San Francisco, CA 94107 (US); LOGG, Christopher, R., South Pasadena, CA 91030 (US)
(74) Representative: Daniels, Jeffrey Nicholas

(57) **Abstract**

This disclosure provides modified cytosine deaminases (CDs). The disclosure further relates to cells and vector expressing or comprising such modified CDs and methods of using such modified CDs in the treatment of disease and disorders.

SEQUENCE LISTING

<110>  Tocagen Inc.
       Gruber, Harry E.
       Jolly, Douglas
       Perez, Omar

<120>  RECOMBINANT VECTORS

<130>  00014-007WO2

<140>  Not yet known
<141>  2009-09-26

<150>  US 61/100,666
<151>  2008-09-26

<150>  US 61/120,618
<151>  2008-12-08

<150>  61/186,823
<151>  2009-06-13

<160>  30

<170>  PatentIn version 3.5

<210>  1
<211>  477
<212>  DNA
<213>  Saccharomyces cerevisiae


<220>
<221>  CDS
<222>  (1)..(477)

<400>  1
atg gtg aca ggg gga atg gca agc aag tgg gat cag aag ggt atg gac      48
Met Val Thr Gly Gly Met Ala Ser Lys Trp Asp Gln Lys Gly Met Asp
1               5                   10                  15

att gcc tat gag gag gcg gcc tta ggt tac aaa gag ggt ggt gtt cct      96
Ile Ala Tyr Glu Glu Ala Ala Leu Gly Tyr Lys Glu Gly Gly Val Pro
            20                  25                  30

att ggc gga tgt ctt atc aat aac aaa gac gga agt gtt ctc ggt cgt      144
Ile Gly Gly Cys Leu Ile Asn Asn Lys Asp Gly Ser Val Leu Gly Arg
        35                  40                  45

ggt cac aac atg aga ttt caa aag gga tcc gcc aca cta cat ggt gag      192
Gly His Asn Met Arg Phe Gln Lys Gly Ser Ala Thr Leu His Gly Glu
    50                  55                  60

atc tcc act ttg gaa aac tgt ggg aga tta gag ggc aaa gtg tac aaa      240
Ile Ser Thr Leu Glu Asn Cys Gly Arg Leu Glu Gly Lys Val Tyr Lys
65                  70                  75                  80

gat acc act ttg tat acg acg ctg tct cca tgc gac atg tgt aca ggt      288
Asp Thr Thr Leu Tyr Thr Thr Leu Ser Pro Cys Asp Met Cys Thr Gly
                85                  90                  95

gcc atc atc atg tat ggt att cca cgc tgt gtt gtc ggt gag aac gtt      336

Ala Ile Ile Met Tyr Gly Ile Pro Arg Cys Val Val Gly Glu Asn Val
            100                 105                 110

aat ttc aaa agt aag ggc gag aaa tat tta caa act aga ggt cac gag 384
Asn Phe Lys Ser Lys Gly Glu Lys Tyr Leu Gln Thr Arg Gly His Glu
        115                 120                 125

gtt gtt gtt gtt gac gat gag agg tgt aaa aag atc atg aaa caa ttt 432
Val Val Val Val Asp Asp Glu Arg Cys Lys Lys Ile Met Lys Gln Phe
    130                 135                 140

atc gat gaa aga cct cag gat tgg ttt gaa gat att ggt gag tag 477
Ile Asp Glu Arg Pro Gln Asp Trp Phe Glu Asp Ile Gly Glu
145                 150                 155

<210> 2
<211> 158
<212> PRT
<213> Saccharomyces cerevisiae

<400> 2

Met Val Thr Gly Gly Met Ala Ser Lys Trp Asp Gln Lys Gly Met Asp
1               5                   10                  15

Ile Ala Tyr Glu Glu Ala Ala Leu Gly Tyr Lys Glu Gly Gly Val Pro
            20                  25                  30

Ile Gly Gly Cys Leu Ile Asn Asn Lys Asp Gly Ser Val Leu Gly Arg
        35                  40                  45

Gly His Asn Met Arg Phe Gln Lys Gly Ser Ala Thr Leu His Gly Glu
    50                  55                  60

Ile Ser Thr Leu Glu Asn Cys Gly Arg Leu Glu Gly Lys Val Tyr Lys
65                  70                  75                  80

Asp Thr Thr Leu Tyr Thr Thr Leu Ser Pro Cys Asp Met Cys Thr Gly
                85                  90                  95

Ala Ile Ile Met Tyr Gly Ile Pro Arg Cys Val Val Gly Glu Asn Val
            100                 105                 110

Asn Phe Lys Ser Lys Gly Glu Lys Tyr Leu Gln Thr Arg Gly His Glu
        115                 120                 125

Val Val Val Val Asp Asp Glu Arg Cys Lys Lys Ile Met Lys Gln Phe
    130                 135                 140

Ile Asp Glu Arg Pro Gln Asp Trp Phe Glu Asp Ile Gly Glu
145                 150                 155

<210>  3
<211>  477
<212>  DNA
<213>  Artificial Sequence

<220>
<223>  Engineered cytosine deaminase


<220>
<221>  CDS
<222>  (1)..(477)

<400>  3
atg gtg aca ggg gga atg gca agc aag tgg gat cag aag ggt atg gac      48
Met Val Thr Gly Gly Met Ala Ser Lys Trp Asp Gln Lys Gly Met Asp
1               5                   10                  15

att gcc tat gag gag gcg tta tta ggt tac aaa gag ggt ggt gtt cct      96
Ile Ala Tyr Glu Glu Ala Leu Leu Gly Tyr Lys Glu Gly Gly Val Pro
            20                  25                  30

att ggc gga tgt ctt atc aat aac aaa gac gga agt gtt ctc ggt cgt      144
Ile Gly Gly Cys Leu Ile Asn Asn Lys Asp Gly Ser Val Leu Gly Arg
        35                  40                  45

ggt cac aac atg aga ttt caa aag gga tcc gcc aca cta cat ggt gag      192
Gly His Asn Met Arg Phe Gln Lys Gly Ser Ala Thr Leu His Gly Glu
    50                  55                  60

atc tcc act ttg gaa aac tgt ggg aga tta gag ggc aaa gtg tac aaa      240
Ile Ser Thr Leu Glu Asn Cys Gly Arg Leu Glu Gly Lys Val Tyr Lys
65                  70                  75                  80

gat acc act ttg tat acg acg ctg tct cca tgc gac atg tgt aca ggt      288
Asp Thr Thr Leu Tyr Thr Thr Leu Ser Pro Cys Asp Met Cys Thr Gly
                85                  90                  95

gcc atc atc atg tat ggt att cca cgc tgt gtc atc ggt gag aac gtt      336
Ala Ile Ile Met Tyr Gly Ile Pro Arg Cys Val Ile Gly Glu Asn Val
            100                 105                 110

aat ttc aaa agt aag ggc gag aaa tat tta caa act aga ggt cac gag      384
Asn Phe Lys Ser Lys Gly Glu Lys Tyr Leu Gln Thr Arg Gly His Glu
        115                 120                 125

gtt gtt gtt gtt gac gat gag agg tgt aaa aag tta atg aaa caa ttt      432
Val Val Val Val Asp Asp Glu Arg Cys Lys Lys Leu Met Lys Gln Phe
    130                 135                 140

atc gat gaa aga cct cag gat tgg ttt gaa gat att ggt gag tag          477
Ile Asp Glu Arg Pro Gln Asp Trp Phe Glu Asp Ile Gly Glu
145                 150                 155


<210>  4
<211>  158
<212>  PRT
<213>  Artificial Sequence

<220>
<223>  Synthetic Construct

<400>  4

Met Val Thr Gly Gly Met Ala Ser Lys Trp Asp Gln Lys Gly Met Asp
1               5                   10                  15

Ile Ala Tyr Glu Glu Ala Leu Leu Gly Tyr Lys Glu Gly Gly Val Pro
            20                  25                  30

Ile Gly Gly Cys Leu Ile Asn Asn Lys Asp Gly Ser Val Leu Gly Arg
        35                  40                  45

Gly His Asn Met Arg Phe Gln Lys Gly Ser Ala Thr Leu His Gly Glu
    50                  55                  60

Ile Ser Thr Leu Glu Asn Cys Gly Arg Leu Glu Gly Lys Val Tyr Lys
65                  70                  75                  80

Asp Thr Thr Leu Tyr Thr Thr Leu Ser Pro Cys Asp Met Cys Thr Gly
                85                  90                  95

Ala Ile Ile Met Tyr Gly Ile Pro Arg Cys Val Ile Gly Glu Asn Val
            100                 105                 110

Asn Phe Lys Ser Lys Gly Glu Lys Tyr Leu Gln Thr Arg Gly His Glu
        115                 120                 125

Val Val Val Val Asp Asp Glu Arg Cys Lys Lys Leu Met Lys Gln Phe
    130                 135                 140

Ile Asp Glu Arg Pro Gln Asp Trp Phe Glu Asp Ile Gly Glu
145                 150                 155

<210>  5
<211>  480
<212>  DNA
<213>  Artificial Sequence

<220>
<223>  Human codon optimized cytosine deaminase

<220>
<221>  CDS
<222>  (1)..(480)

<400>  5
atg gtg acc ggc ggc atg gcc tcc aag tgg gat caa aag ggc atg gat        48
Met Val Thr Gly Gly Met Ala Ser Lys Trp Asp Gln Lys Gly Met Asp
1               5                   10                  15

atc gct tac gag gag gcc gca ctg ggc tac aag gag ggc ggc gtg cct        96
Ile Ala Tyr Glu Glu Ala Ala Leu Gly Tyr Lys Glu Gly Gly Val Pro
            20                  25                  30

atc ggc ggc tgt ctg atc aac aac aag gac ggc agt gtg ctg ggc agg     144
Ile Gly Gly Cys Leu Ile Asn Asn Lys Asp Gly Ser Val Leu Gly Arg
        35                  40                  45

ggc cac aac atg agg ttc cag aag ggc tcc gcc acc ctg cac ggc gag     192
Gly His Asn Met Arg Phe Gln Lys Gly Ser Ala Thr Leu His Gly Glu
    50                  55                  60

atc tcc acc ctg gag aac tgt ggc agg ctg gag ggc aag gtg tac aag     240
Ile Ser Thr Leu Glu Asn Cys Gly Arg Leu Glu Gly Lys Val Tyr Lys
65                  70                  75                  80

gac acc acc ctg tac acc acc ctg tcc cct tgt gac atg tgt acc ggc     288
Asp Thr Thr Leu Tyr Thr Thr Leu Ser Pro Cys Asp Met Cys Thr Gly
                85                  90                  95

gct atc atc atg tac ggc atc cct agg tgt gtg gtc ggc gag aac gtg     336
Ala Ile Ile Met Tyr Gly Ile Pro Arg Cys Val Val Gly Glu Asn Val
            100                 105                 110

aac ttc aag tcc aag ggc gag aag tac ctg caa acc agg ggc cac gag     384
Asn Phe Lys Ser Lys Gly Glu Lys Tyr Leu Gln Thr Arg Gly His Glu
        115                 120                 125

gtg gtg gtt gtt gac gat gag agg tgt aag aag atc atg aag cag ttc     432
Val Val Val Val Asp Asp Glu Arg Cys Lys Lys Ile Met Lys Gln Phe
    130                 135                 140

atc gac gag agg cct cag gac tgg ttc gag gat atc ggc gag tga taa     480
Ile Asp Glu Arg Pro Gln Asp Trp Phe Glu Asp Ile Gly Glu
145                 150                 155


<210>  6
<211>  158
<212>  PRT
<213>  Artificial Sequence

<220>
<223>  Synthetic Construct

<400>  6

Met Val Thr Gly Gly Met Ala Ser Lys Trp Asp Gln Lys Gly Met Asp
1               5                   10                  15


Ile Ala Tyr Glu Glu Ala Ala Leu Gly Tyr Lys Glu Gly Gly Val Pro
            20                  25                  30


Ile Gly Gly Cys Leu Ile Asn Asn Lys Asp Gly Ser Val Leu Gly Arg
        35                  40                  45


Gly His Asn Met Arg Phe Gln Lys Gly Ser Ala Thr Leu His Gly Glu
    50                  55                  60


Ile Ser Thr Leu Glu Asn Cys Gly Arg Leu Glu Gly Lys Val Tyr Lys
65                  70                  75                  80

Asp Thr Thr Leu Tyr Thr Thr Leu Ser Pro Cys Asp Met Cys Thr Gly
                85                  90                  95

Ala Ile Ile Met Tyr Gly Ile Pro Arg Cys Val Val Gly Glu Asn Val
            100                 105                 110

Asn Phe Lys Ser Lys Gly Glu Lys Tyr Leu Gln Thr Arg Gly His Glu
        115                 120                 125

Val Val Val Val Asp Asp Glu Arg Cys Lys Lys Ile Met Lys Gln Phe
    130                 135                 140

Ile Asp Glu Arg Pro Gln Asp Trp Phe Glu Asp Ile Gly Glu
145                 150                 155

<210>  7
<211>  756
<212>  DNA
<213>  Saccharomyces cerevisiae

<220>
<221>  CDS
<222>  (1)..(756)

<400>  7
atg aac ccg tta ttc ttt ttg gct tct cca ttc ttg tac ctt aca tat      48
Met Asn Pro Leu Phe Phe Leu Ala Ser Pro Phe Leu Tyr Leu Thr Tyr
1               5                   10                  15

ctt ata tat tat cca aac aaa ggg tct ttc gtt agc aaa cct aga aat      96
Leu Ile Tyr Tyr Pro Asn Lys Gly Ser Phe Val Ser Lys Pro Arg Asn
            20                  25                  30

ctg caa aaa atg tct tcg gaa cca ttt aag aac gtc tac ttg cta cct      144
Leu Gln Lys Met Ser Ser Glu Pro Phe Lys Asn Val Tyr Leu Leu Pro
        35                  40                  45

caa aca aac caa ttg ctg ggt ttg tac acc atc atc aga aat aag aat      192
Gln Thr Asn Gln Leu Leu Gly Leu Tyr Thr Ile Ile Arg Asn Lys Asn
    50                  55                  60

aca act aga cct gat ttc att ttc tac tcc gat aga atc atc aga ttg      240
Thr Thr Arg Pro Asp Phe Ile Phe Tyr Ser Asp Arg Ile Ile Arg Leu
65                  70                  75                  80

ttg gtt gaa gaa ggt ttg aac cat cta cct gtg caa aag caa att gtg      288
Leu Val Glu Glu Gly Leu Asn His Leu Pro Val Gln Lys Gln Ile Val
                85                  90                  95

gaa act gac acc aac gaa aac ttc gaa ggt gtc tca ttc atg ggt aaa      336
Glu Thr Asp Thr Asn Glu Asn Phe Glu Gly Val Ser Phe Met Gly Lys
            100                 105                 110

atc tgt ggt gtt tcc att gtc aga gct ggt gaa tcg atg gag caa gga      384
Ile Cys Gly Val Ser Ile Val Arg Ala Gly Glu Ser Met Glu Gln Gly
        115                 120                 125

tta aga gac tgt tgt agg tct gtg cgt atc ggt aaa att tta att caa 432
Leu Arg Asp Cys Cys Arg Ser Val Arg Ile Gly Lys Ile Leu Ile Gln
    130                 135                 140

agg gac gag gag act gct tta cca aag tta ttc tac gaa aaa tta cca 480
Arg Asp Glu Glu Thr Ala Leu Pro Lys Leu Phe Tyr Glu Lys Leu Pro
145                 150                 155                 160

gag gat ata tct gaa agg tat gtc ttc cta tta gac cca atg ctg gcc 528
Glu Asp Ile Ser Glu Arg Tyr Val Phe Leu Leu Asp Pro Met Leu Ala
                165                 170                 175

acc ggt ggt agt gct atc atg gct aca gaa gtc ttg att aag aga ggt 576
Thr Gly Gly Ser Ala Ile Met Ala Thr Glu Val Leu Ile Lys Arg Gly
            180                 185                 190

gtt aag cca gag aga att tac ttc tta aac cta atc tgt agt aag gaa 624
Val Lys Pro Glu Arg Ile Tyr Phe Leu Asn Leu Ile Cys Ser Lys Glu
        195                 200                 205

ggg att gaa aaa tac cat gcc gcc ttc cca gag gtc aga att gtt act 672
Gly Ile Glu Lys Tyr His Ala Ala Phe Pro Glu Val Arg Ile Val Thr
    210                 215                 220

ggt gcc ctc gac aga ggt cta gat gaa aac aag tat cta gtt cca ggg 720
Gly Ala Leu Asp Arg Gly Leu Asp Glu Asn Lys Tyr Leu Val Pro Gly
225                 230                 235                 240

ttg ggt gac ttt ggt gac aga tac tac tgt gtt taa 756
Leu Gly Asp Phe Gly Asp Arg Tyr Tyr Cys Val
                245                 250

<210> 8
<211> 251
<212> PRT
<213> Saccharomyces cerevisiae

<400> 8

Met Asn Pro Leu Phe Phe Leu Ala Ser Pro Phe Leu Tyr Leu Thr Tyr
1               5                   10                  15

Leu Ile Tyr Tyr Pro Asn Lys Gly Ser Phe Val Ser Lys Pro Arg Asn
            20                  25                  30

Leu Gln Lys Met Ser Ser Glu Pro Phe Lys Asn Val Tyr Leu Leu Pro
        35                  40                  45

Gln Thr Asn Gln Leu Leu Gly Leu Tyr Thr Ile Ile Arg Asn Lys Asn
    50                  55                  60

Thr Thr Arg Pro Asp Phe Ile Phe Tyr Ser Asp Arg Ile Ile Arg Leu
65                  70                  75                  80

Leu Val Glu Glu Gly Leu Asn His Leu Pro Val Gln Lys Gln Ile Val
                85                  90                  95

Glu Thr Asp Thr Asn Glu Asn Phe Glu Gly Val Ser Phe Met Gly Lys
            100                 105                 110

Ile Cys Gly Val Ser Ile Val Arg Ala Gly Glu Ser Met Glu Gln Gly
        115                 120                 125

Leu Arg Asp Cys Cys Arg Ser Val Arg Ile Gly Lys Ile Leu Ile Gln
    130                 135                 140

Arg Asp Glu Glu Thr Ala Leu Pro Lys Leu Phe Tyr Glu Lys Leu Pro
145                 150                 155                 160

Glu Asp Ile Ser Glu Arg Tyr Val Phe Leu Leu Asp Pro Met Leu Ala
                165                 170                 175

Thr Gly Gly Ser Ala Ile Met Ala Thr Glu Val Leu Ile Lys Arg Gly
            180                 185                 190

Val Lys Pro Glu Arg Ile Tyr Phe Leu Asn Leu Ile Cys Ser Lys Glu
        195                 200                 205

Gly Ile Glu Lys Tyr His Ala Ala Phe Pro Glu Val Arg Ile Val Thr
    210                 215                 220

Gly Ala Leu Asp Arg Gly Leu Asp Glu Asn Lys Tyr Leu Val Pro Gly
225                 230                 235                 240

Leu Gly Asp Phe Gly Asp Arg Tyr Tyr Cys Val
                245                 250

<210>  9
<211>  1443
<212>  DNA
<213>  homo sapiens

<220>
<221>  CDS
<222>  (1)..(1443)

<400>  9
atg gct gtt gct cgt gct gct ctt ggt cct ctt gtt act ggt ctt tat      48
gat gtt caa gct ttt aaa ttt ggt gat ttt gtt ctt aaa tct ggt ctt      96
tct tct cct att tat att gat ctt cgt ggt att gtt tct cgt cct cgt      144
ctt ctt tct caa gtt gct gat att ctt ttt caa act gct caa aat gct      192
ggt att tct ttt gat act gtt tgt ggt gtt cct tat act gct ctt cct      240
ctt gct act gtt att tgt tct act aat caa att cct atg ctt att cgt      288
cgt aaa gaa act aaa gat tat ggt act aaa cgt ctt gtt gaa ggt act      336
att aat cct ggt gaa act tgt ctt att att gaa gat gtt gtt act tct      384
ggt tct tct gtt ctt gaa act gtt gaa gtt ctt caa aaa gaa ggt ctt      432
aaa gtt act gat gct att gtt ctt ctt gat cgt gaa caa ggt ggt aaa      480
gat aaa ctt caa gct cat ggt att cgt ctt cat tct gtt tgt act ctt      528
tct aaa atg ctt gaa att ctt gaa caa caa aaa aaa gtt gat gct gaa      576
act gtt ggt cgt gtt aaa cgt ttt att caa gaa aat gtt ttt gtt gct      624
gct aat cat aat ggt tct cct ctt tct att aaa gaa gct cct aaa gaa      672
ctt tct ttt ggt gct cgt gct gaa ctt cct cgt att cat cct gtt gct      720
tct aaa ctt ctt cgt ctt atg caa aaa aaa gaa act aat ctt tgt ctt      768
tct gct gat gtt tct ctt gct cgt gaa ctt ctt caa ctt gct gat gct      816
ctt ggt cct tct att tgt atg ctt aaa act cat gtt gat att ctt aat      864
gat ttt act ctt gat gtt atg aaa gaa ctt att act ctt gct aaa tgt      912
cat gaa ttt ctt att ttt gaa gat cgt aaa ttt gct gat att ggt aat         960
act gtt aaa aaa caa tat gaa ggt ggt att ttt aaa att gct tct tgg         1008
gct gat ctt gtt aat gct cat gtt gtt cct ggt tct ggt gtt gtt aaa         1056
ggt ctt caa gaa gtt ggt ctt cct ctt cat cgt ggt tgt ctt ctt att         1104
gct gaa atg tct tct act ggt tct ctt gct act ggt gat tat act cgt         1152
gct gct gtt cgt atg gct gaa gaa cat tct gaa ttt gtt gtt ggt ttt         1200
att tct ggt tct cgt gtt tct atg aaa cct gaa ttt ctt cat ctt act         1248
cct ggt gtt caa ctt gaa gct ggt ggt gat aat ctt ggt caa caa tat         1296
aat tct cct caa gaa gtt att ggt aaa cgt ggt tct gat att att att         1344
gtt ggt cgt ggt att att tct gct gct gat cgt ctt gaa gct gct gaa         1392
atg tat cgt aaa gct gct tgg gaa gct tat ctt tct cgt ctt ggt gtt         1440
taa                                                                     1443


<210>  10
<211>  480
<212>  PRT
<213>  homo sapiens

<400>  10

<210>  11
<211>  1227
<212>  DNA
<213>  Artificial Sequence

<220>
<223>  Fusion construct CDopt-UPRT

<220>
<221>  CDS
<222>  (1)..(1227)

<400>  11
atg gtg acc ggc ggc atg gcc tcc aag tgg gat caa aag ggc atg gat      48
Met Val Thr Gly Gly Met Ala Ser Lys Trp Asp Gln Lys Gly Met Asp
1               5                   10                  15

atc gct tac gag gag gcc ctg ctg ggc tac aag gag ggc ggc gtg cct      96
Ile Ala Tyr Glu Glu Ala Leu Leu Gly Tyr Lys Glu Gly Gly Val Pro
            20                  25                  30

atc ggc ggc tgt ctg atc aac aac aag gac ggc agt gtg ctg ggc agg      144
Ile Gly Gly Cys Leu Ile Asn Asn Lys Asp Gly Ser Val Leu Gly Arg
        35                  40                  45

ggc cac aac atg agg ttc cag aag ggc tcc gcc acc ctg cac ggc gag      192
Gly His Asn Met Arg Phe Gln Lys Gly Ser Ala Thr Leu His Gly Glu
    50                  55                  60

atc tcc acc ctg gag aac tgt ggc agg ctg gag ggc aag gtg tac aag      240
Ile Ser Thr Leu Glu Asn Cys Gly Arg Leu Glu Gly Lys Val Tyr Lys
65                  70                  75                  80

gac acc acc ctg tac acc acc ctg tcc cct tgt gac atg tgt acc ggc      288
Asp Thr Thr Leu Tyr Thr Thr Leu Ser Pro Cys Asp Met Cys Thr Gly
                85                  90                  95

gct atc atc atg tac ggc atc cct agg tgt gtg atc ggc gag aac gtg      336
Ala Ile Ile Met Tyr Gly Ile Pro Arg Cys Val Ile Gly Glu Asn Val
            100                 105                 110

aac ttc aag tcc aag ggc gag aag tac ctg caa acc agg ggc cac gag      384
Asn Phe Lys Ser Lys Gly Glu Lys Tyr Leu Gln Thr Arg Gly His Glu
        115                 120                 125

gtg gtg gtt gtt gac gat gag agg tgt aag aag ctg atg aag cag ttc      432
Val Val Val Val Asp Asp Glu Arg Cys Lys Lys Leu Met Lys Gln Phe
    130                 135                 140

atc gac gag agg cct cag gac tgg ttc gag gat atc ggc gag aac ccg      480
Ile Asp Glu Arg Pro Gln Asp Trp Phe Glu Asp Ile Gly Glu Asn Pro
145                 150                 155                 160

tta ttc ttt ttg gct tct cca ttc ttg tac ctt aca tat ctt ata tat      528
Leu Phe Phe Leu Ala Ser Pro Phe Leu Tyr Leu Thr Tyr Leu Ile Tyr
                165                 170                 175

tat cca aac aaa ggg tct ttc gtt agc aaa cct aga aat ctg caa aaa      576
Tyr Pro Asn Lys Gly Ser Phe Val Ser Lys Pro Arg Asn Leu Gln Lys
            180                 185                 190

atg tct tcg gaa cca ttt aag aac gtc tac ttg cta cct caa aca aac      624
Met Ser Ser Glu Pro Phe Lys Asn Val Tyr Leu Leu Pro Gln Thr Asn
        195                 200                 205

caa ttg ctg ggt ttg tac acc atc atc aga aat aag aat aca act aga      672
Gln Leu Leu Gly Leu Tyr Thr Ile Ile Arg Asn Lys Asn Thr Thr Arg
    210                 215                 220

cct gat ttc att ttc tac tcc gat aga atc atc aga ttg ttg gtt gaa      720
Pro Asp Phe Ile Phe Tyr Ser Asp Arg Ile Ile Arg Leu Leu Val Glu
225                 230                 235                 240

gaa ggt ttg aac cat cta cct gtg caa aag caa att gtg gaa act gac      768

Glu Gly Leu Asn His Leu Pro Val Gln Lys Gln Ile Val Glu Thr Asp
                245                 250                 255

acc aac gaa aac ttc gaa ggt gtc tca ttc atg ggt aaa atc tgt ggt      816
Thr Asn Glu Asn Phe Glu Gly Val Ser Phe Met Gly Lys Ile Cys Gly
            260                 265                 270

gtt tcc att gtc aga gct ggt gaa tcg atg gag caa gga tta aga gac      864
Val Ser Ile Val Arg Ala Gly Glu Ser Met Glu Gln Gly Leu Arg Asp
        275                 280                 285

tgt tgt agg tct gtg cgt atc ggt aaa att tta att caa agg gac gag      912
Cys Cys Arg Ser Val Arg Ile Gly Lys Ile Leu Ile Gln Arg Asp Glu
    290                 295                 300

gag act gct tta cca aag tta ttc tac gaa aaa tta cca gag gat ata      960
Glu Thr Ala Leu Pro Lys Leu Phe Tyr Glu Lys Leu Pro Glu Asp Ile
305                 310                 315                 320

tct gaa agg tat gtc ttc cta tta gac cca atg ctg gcc acc ggt ggt     1008
Ser Glu Arg Tyr Val Phe Leu Leu Asp Pro Met Leu Ala Thr Gly Gly
                325                 330                 335

agt gct atc atg gct aca gaa gtc ttg att aag aga ggt gtt aag cca     1056
Ser Ala Ile Met Ala Thr Glu Val Leu Ile Lys Arg Gly Val Lys Pro
            340                 345                 350

gag aga att tac ttc tta aac cta atc tgt agt aag gaa ggg att gaa     1104
Glu Arg Ile Tyr Phe Leu Asn Leu Ile Cys Ser Lys Glu Gly Ile Glu
        355                 360                 365

aaa tac cat gcc gcc ttc cca gag gtc aga att gtt act ggt gcc ctc     1152
Lys Tyr His Ala Ala Phe Pro Glu Val Arg Ile Val Thr Gly Ala Leu
    370                 375                 380

gac aga ggt cta gat gaa aac aag tat cta gtt cca ggg ttg ggt gac     1200
Asp Arg Gly Leu Asp Glu Asn Lys Tyr Leu Val Pro Gly Leu Gly Asp
385                 390                 395                 400

ttt ggt gac aga tac tac tgt gtt taa                                 1227
Phe Gly Asp Arg Tyr Tyr Cys Val
                405


<210>  12
<211>  408
<212>  PRT
<213>  Artificial Sequence

<220>
<223>  Synthetic Construct

<400>  12

Met Val Thr Gly Gly Met Ala Ser Lys Trp Asp Gln Lys Gly Met Asp
1               5                   10                  15


Ile Ala Tyr Glu Glu Ala Leu Leu Gly Tyr Lys Glu Gly Gly Val Pro
            20                  25                  30


Ile Gly Gly Cys Leu Ile Asn Asn Lys Asp Gly Ser Val Leu Gly Arg

35                  40                  45

Gly His Asn Met Arg Phe Gln Lys Gly Ser Ala Thr Leu His Gly Glu
    50                  55                  60

Ile Ser Thr Leu Glu Asn Cys Gly Arg Leu Glu Gly Lys Val Tyr Lys
65                  70                  75                  80

Asp Thr Thr Leu Tyr Thr Thr Leu Ser Pro Cys Asp Met Cys Thr Gly
                85                  90                  95

Ala Ile Ile Met Tyr Gly Ile Pro Arg Cys Val Ile Gly Glu Asn Val
            100                 105                 110

Asn Phe Lys Ser Lys Gly Glu Lys Tyr Leu Gln Thr Arg Gly His Glu
        115                 120                 125

Val Val Val Val Asp Asp Glu Arg Cys Lys Lys Leu Met Lys Gln Phe
    130                 135                 140

Ile Asp Glu Arg Pro Gln Asp Trp Phe Glu Asp Ile Gly Glu Asn Pro
145                 150                 155                 160

Leu Phe Phe Leu Ala Ser Pro Phe Leu Tyr Leu Thr Tyr Leu Ile Tyr
                165                 170                 175

Tyr Pro Asn Lys Gly Ser Phe Val Ser Lys Pro Arg Asn Leu Gln Lys
            180                 185                 190

Met Ser Ser Glu Pro Phe Lys Asn Val Tyr Leu Leu Pro Gln Thr Asn
        195                 200                 205

Gln Leu Leu Gly Leu Tyr Thr Ile Ile Arg Asn Lys Asn Thr Thr Arg
    210                 215                 220

Pro Asp Phe Ile Phe Tyr Ser Asp Arg Ile Ile Arg Leu Leu Val Glu
225                 230                 235                 240

Glu Gly Leu Asn His Leu Pro Val Gln Lys Gln Ile Val Glu Thr Asp
                245                 250                 255

Thr Asn Glu Asn Phe Glu Gly Val Ser Phe Met Gly Lys Ile Cys Gly
            260                 265                 270

Val Ser Ile Val Arg Ala Gly Glu Ser Met Glu Gln Gly Leu Arg Asp
        275                 280                 285

Cys Cys Arg Ser Val Arg Ile Gly Lys Ile Leu Ile Gln Arg Asp Glu
    290                 295                 300

Glu Thr Ala Leu Pro Lys Leu Phe Tyr Glu Lys Leu Pro Glu Asp Ile
305                 310                 315                 320

Ser Glu Arg Tyr Val Phe Leu Leu Asp Pro Met Leu Ala Thr Gly Gly
                325                 330                 335

Ser Ala Ile Met Ala Thr Glu Val Leu Ile Lys Arg Gly Val Lys Pro
            340                 345                 350

Glu Arg Ile Tyr Phe Leu Asn Leu Ile Cys Ser Lys Glu Gly Ile Glu
        355                 360                 365

Lys Tyr His Ala Ala Phe Pro Glu Val Arg Ile Val Thr Gly Ala Leu
    370                 375                 380

Asp Arg Gly Leu Asp Glu Asn Lys Tyr Leu Val Pro Gly Leu Gly Asp
385                 390                 395                 400

Phe Gly Asp Arg Tyr Tyr Cys Val
                405

<210>  13
<211>  1287
<212>  DNA
<213>  Artificial Sequence

<220>
<223>  Fusion construction - CDopt - linker - UPRT

<220>
<221>  CDS
<222>  (1)..(1287)

<400>  13
atg gtg acc ggc ggc atg gcc tcc aag tgg gat caa aag ggc atg gat      48
Met Val Thr Gly Gly Met Ala Ser Lys Trp Asp Gln Lys Gly Met Asp
1               5                   10                  15

atc gct tac gag gag gcc ctg ctg ggc tac aag gag ggc ggc gtg cct      96
Ile Ala Tyr Glu Glu Ala Leu Leu Gly Tyr Lys Glu Gly Gly Val Pro
            20                  25                  30

atc ggc ggc tgt ctg atc aac aac aag gac ggc agt gtg ctg ggc agg      144
Ile Gly Gly Cys Leu Ile Asn Asn Lys Asp Gly Ser Val Leu Gly Arg
        35                  40                  45

ggc cac aac atg agg ttc cag aag ggc tcc gcc acc ctg cac ggc gag      192
Gly His Asn Met Arg Phe Gln Lys Gly Ser Ala Thr Leu His Gly Glu
    50                  55                  60

atc tcc acc ctg gag aac tgt ggc agg ctg gag ggc aag gtg tac aag      240

Ile Ser Thr Leu Glu Asn Cys Gly Arg Leu Glu Gly Lys Val Tyr Lys
65                  70                  75                  80

gac acc acc ctg tac acc acc ctg tcc cct tgt gac atg tgt acc ggc      288
Asp Thr Thr Leu Tyr Thr Thr Leu Ser Pro Cys Asp Met Cys Thr Gly
                85                  90                  95

gct atc atc atg tac ggc atc cct agg tgt gtg atc ggc gag aac gtg      336
Ala Ile Ile Met Tyr Gly Ile Pro Arg Cys Val Ile Gly Glu Asn Val
            100                 105                 110

aac ttc aag tcc aag ggc gag aag tac ctg caa acc agg ggc cac gag      384
Asn Phe Lys Ser Lys Gly Glu Lys Tyr Leu Gln Thr Arg Gly His Glu
        115                 120                 125

gtg gtg gtt gtt gac gat gag agg tgt aag aag ctg atg aag cag ttc      432
Val Val Val Val Asp Asp Glu Arg Cys Lys Lys Leu Met Lys Gln Phe
    130                 135                 140

atc gac gag agg cct cag gac tgg ttc gag gat atc ggc gag tcc ggc      480
Ile Asp Glu Arg Pro Gln Asp Trp Phe Glu Asp Ile Gly Glu Ser Gly
145                 150                 155                 160

ggc ggc gcc tcc ggc ggc ggc gcc tcc ggc ggc ggc gcc tcc ggc ggc      528
Gly Gly Ala Ser Gly Gly Gly Ala Ser Gly Gly Gly Ala Ser Gly Gly
                165                 170                 175

ggc gcc aac ccg tta ttc ttt ttg gct tct cca ttc ttg tac ctt aca      576
Gly Ala Asn Pro Leu Phe Phe Leu Ala Ser Pro Phe Leu Tyr Leu Thr
            180                 185                 190

tat ctt ata tat tat cca aac aaa ggg tct ttc gtt agc aaa cct aga      624
Tyr Leu Ile Tyr Tyr Pro Asn Lys Gly Ser Phe Val Ser Lys Pro Arg
        195                 200                 205

aat ctg caa aaa atg tct tcg gaa cca ttt aag aac gtc tac ttg cta      672
Asn Leu Gln Lys Met Ser Ser Glu Pro Phe Lys Asn Val Tyr Leu Leu
    210                 215                 220

cct caa aca aac caa ttg ctg ggt ttg tac acc atc atc aga aat aag      720
Pro Gln Thr Asn Gln Leu Leu Gly Leu Tyr Thr Ile Ile Arg Asn Lys
225                 230                 235                 240

aat aca act aga cct gat ttc att ttc tac tcc gat aga atc atc aga      768
Asn Thr Thr Arg Pro Asp Phe Ile Phe Tyr Ser Asp Arg Ile Ile Arg
                245                 250                 255

ttg ttg gtt gaa gaa ggt ttg aac cat cta cct gtg caa aag caa att      816
Leu Leu Val Glu Glu Gly Leu Asn His Leu Pro Val Gln Lys Gln Ile
            260                 265                 270

gtg gaa act gac acc aac gaa aac ttc gaa ggt gtc tca ttc atg ggt      864
Val Glu Thr Asp Thr Asn Glu Asn Phe Glu Gly Val Ser Phe Met Gly
        275                 280                 285

aaa atc tgt ggt gtt tcc att gtc aga gct ggt gaa tcg atg gag caa      912
Lys Ile Cys Gly Val Ser Ile Val Arg Ala Gly Glu Ser Met Glu Gln
    290                 295                 300

gga tta aga gac tgt tgt agg tct gtg cgt atc ggt aaa att tta att      960
Gly Leu Arg Asp Cys Cys Arg Ser Val Arg Ile Gly Lys Ile Leu Ile
305                 310                 315                 320

caa agg gac gag gag act gct tta cca aag tta ttc tac gaa aaa tta 1008
Gln Arg Asp Glu Glu Thr Ala Leu Pro Lys Leu Phe Tyr Glu Lys Leu
                325                 330                 335

cca gag gat ata tct gaa agg tat gtc ttc cta tta gac cca atg ctg 1056
Pro Glu Asp Ile Ser Glu Arg Tyr Val Phe Leu Leu Asp Pro Met Leu
            340                 345                 350

gcc acc ggt ggt agt gct atc atg gct aca gaa gtc ttg att aag aga 1104
Ala Thr Gly Gly Ser Ala Ile Met Ala Thr Glu Val Leu Ile Lys Arg
        355                 360                 365

ggt gtt aag cca gag aga att tac ttc tta aac cta atc tgt agt aag 1152
Gly Val Lys Pro Glu Arg Ile Tyr Phe Leu Asn Leu Ile Cys Ser Lys
    370                 375                 380

gaa ggg att gaa aaa tac cat gcc gcc ttc cca gag gtc aga att gtt 1200
Glu Gly Ile Glu Lys Tyr His Ala Ala Phe Pro Glu Val Arg Ile Val
385                 390                 395                 400

act ggt gcc ctc gac aga ggt cta gat gaa aac aag tat cta gtt cca 1248
Thr Gly Ala Leu Asp Arg Gly Leu Asp Glu Asn Lys Tyr Leu Val Pro
                405                 410                 415

ggg ttg ggt gac ttt ggt gac aga tac tac tgt gtt taa 1287
Gly Leu Gly Asp Phe Gly Asp Arg Tyr Tyr Cys Val
            420                 425

<210> 14
<211> 428
<212> PRT
<213> Artificial Sequence

<220>
<223> Synthetic Construct

<400> 14

Met Val Thr Gly Gly Met Ala Ser Lys Trp Asp Gln Lys Gly Met Asp
1               5                   10                  15

Ile Ala Tyr Glu Glu Ala Leu Leu Gly Tyr Lys Glu Gly Gly Val Pro
            20                  25                  30

Ile Gly Gly Cys Leu Ile Asn Asn Lys Asp Gly Ser Val Leu Gly Arg
        35                  40                  45

Gly His Asn Met Arg Phe Gln Lys Gly Ser Ala Thr Leu His Gly Glu
    50                  55                  60

Ile Ser Thr Leu Glu Asn Cys Gly Arg Leu Glu Gly Lys Val Tyr Lys
65                  70                  75                  80

Asp Thr Thr Leu Tyr Thr Thr Leu Ser Pro Cys Asp Met Cys Thr Gly
                85                  90                  95

Ala Ile Ile Met Tyr Gly Ile Pro Arg Cys Val Ile Gly Glu Asn Val
            100                 105                 110

Asn Phe Lys Ser Lys Gly Glu Lys Tyr Leu Gln Thr Arg Gly His Glu
        115                 120                 125

Val Val Val Val Asp Asp Glu Arg Cys Lys Lys Leu Met Lys Gln Phe
    130                 135                 140

Ile Asp Glu Arg Pro Gln Asp Trp Phe Glu Asp Ile Gly Glu Ser Gly
145                 150                 155                 160

Gly Gly Ala Ser Gly Gly Gly Ala Ser Gly Gly Gly Ala Ser Gly Gly
                165                 170                 175

Gly Ala Asn Pro Leu Phe Phe Leu Ala Ser Pro Phe Leu Tyr Leu Thr
            180                 185                 190

Tyr Leu Ile Tyr Tyr Pro Asn Lys Gly Ser Phe Val Ser Lys Pro Arg
        195                 200                 205

Asn Leu Gln Lys Met Ser Ser Glu Pro Phe Lys Asn Val Tyr Leu Leu
    210                 215                 220

Pro Gln Thr Asn Gln Leu Leu Gly Leu Tyr Thr Ile Ile Arg Asn Lys
225                 230                 235                 240

Asn Thr Thr Arg Pro Asp Phe Ile Phe Tyr Ser Asp Arg Ile Ile Arg
                245                 250                 255

Leu Leu Val Glu Glu Gly Leu Asn His Leu Pro Val Gln Lys Gln Ile
            260                 265                 270

Val Glu Thr Asp Thr Asn Glu Asn Phe Glu Gly Val Ser Phe Met Gly
        275                 280                 285

Lys Ile Cys Gly Val Ser Ile Val Arg Ala Gly Glu Ser Met Glu Gln
    290                 295                 300

Gly Leu Arg Asp Cys Cys Arg Ser Val Arg Ile Gly Lys Ile Leu Ile
305                 310                 315                 320

Gln Arg Asp Glu Glu Thr Ala Leu Pro Lys Leu Phe Tyr Glu Lys Leu
                325                 330                 335

Pro Glu Asp Ile Ser Glu Arg Tyr Val Phe Leu Leu Asp Pro Met Leu
            340                 345                 350

Ala Thr Gly Gly Ser Ala Ile Met Ala Thr Glu Val Leu Ile Lys Arg
        355                 360                 365

Gly Val Lys Pro Glu Arg Ile Tyr Phe Leu Asn Leu Ile Cys Ser Lys
    370                 375                 380

Glu Gly Ile Glu Lys Tyr His Ala Ala Phe Pro Glu Val Arg Ile Val
385                 390                 395                 400

Thr Gly Ala Leu Asp Arg Gly Leu Asp Glu Asn Lys Tyr Leu Val Pro
                405                 410                 415

Gly Leu Gly Asp Phe Gly Asp Arg Tyr Tyr Cys Val
            420                 425

<210>  15
<211>  1200
<212>  DNA
<213>  Artificial Sequence

<220>
<223>  Fusion Construct - CDopt3 - OPRT

<220>
<221>  CDS
<222>  (1)..(1200)

<400>  15
atg gtg acc ggc ggc atg gcc tcc aag tgg gat caa aag ggc atg gat      48
Met Val Thr Gly Gly Met Ala Ser Lys Trp Asp Gln Lys Gly Met Asp
1               5                   10                  15

atc gct tac gag gag gcc ctg ctg ggc tac aag gag ggc ggc gtg cct      96
Ile Ala Tyr Glu Glu Ala Leu Leu Gly Tyr Lys Glu Gly Gly Val Pro
            20                  25                  30

atc ggc ggc tgt ctg atc aac aac aag gac ggc agt gtg ctg ggc agg      144
Ile Gly Gly Cys Leu Ile Asn Asn Lys Asp Gly Ser Val Leu Gly Arg
        35                  40                  45

ggc cac aac atg agg ttc cag aag ggc tcc gcc acc ctg cac ggc gag      192
Gly His Asn Met Arg Phe Gln Lys Gly Ser Ala Thr Leu His Gly Glu
    50                  55                  60

atc tcc acc ctg gag aac tgt ggc agg ctg gag ggc aag gtg tac aag      240
Ile Ser Thr Leu Glu Asn Cys Gly Arg Leu Glu Gly Lys Val Tyr Lys
65                  70                  75                  80

gac acc acc ctg tac acc acc ctg tcc cct tgt gac atg tgt acc ggc      288
Asp Thr Thr Leu Tyr Thr Thr Leu Ser Pro Cys Asp Met Cys Thr Gly
                85                  90                  95

gct atc atc atg tac ggc atc cct agg tgt gtg atc ggc gag aac gtg      336
Ala Ile Ile Met Tyr Gly Ile Pro Arg Cys Val Ile Gly Glu Asn Val
            100                 105                 110

aac ttc aag tcc aag ggc gag aag tac ctg caa acc agg ggc cac gag 384
Asn Phe Lys Ser Lys Gly Glu Lys Tyr Leu Gln Thr Arg Gly His Glu
        115                 120                 125

gtg gtg gtt gtt gac gat gag agg tgt aag aag ctg atg aag cag ttc 432
Val Val Val Val Asp Asp Glu Arg Cys Lys Lys Leu Met Lys Gln Phe
    130                 135                 140

atc gac gag agg cct cag gac tgg ttc gag gat atc ggc gag gcg gtc 480
Ile Asp Glu Arg Pro Gln Asp Trp Phe Glu Asp Ile Gly Glu Ala Val
145                 150                 155                 160

gct cgt gca gct ttg ggg cca ttg gtg acg ggt ctg tac gac gtg cag 528
Ala Arg Ala Ala Leu Gly Pro Leu Val Thr Gly Leu Tyr Asp Val Gln
                165                 170                 175

gct ttc aag ttt ggg gac ttc gtg ctg aag agc ggg ctt tcc tcc ccc 576
Ala Phe Lys Phe Gly Asp Phe Val Leu Lys Ser Gly Leu Ser Ser Pro
            180                 185                 190

atc tac atc gat ctg cgg ggc atc gtg tct cga ccg cgt ctt ctg agt 624
Ile Tyr Ile Asp Leu Arg Gly Ile Val Ser Arg Pro Arg Leu Leu Ser
        195                 200                 205

cag gtt gca gat att tta ttc caa act gcc caa aat gca ggc atc agt 672
Gln Val Ala Asp Ile Leu Phe Gln Thr Ala Gln Asn Ala Gly Ile Ser
    210                 215                 220

ttt gac acc gtg tgt gga gtg cct tat aca gct ttg cca ttg gct aca 720
Phe Asp Thr Val Cys Gly Val Pro Tyr Thr Ala Leu Pro Leu Ala Thr
225                 230                 235                 240

gtt atc tgt tca acc aat caa att cca atg ctt att aga agg aaa gaa 768
Val Ile Cys Ser Thr Asn Gln Ile Pro Met Leu Ile Arg Arg Lys Glu
                245                 250                 255

aca aag gat tat gga act aag cgt ctt gta gaa gga act att aat cca 816
Thr Lys Asp Tyr Gly Thr Lys Arg Leu Val Glu Gly Thr Ile Asn Pro
            260                 265                 270

gga gaa acc tgt tta atc att gaa gat gtt gtc acc agt gga tct agt 864
Gly Glu Thr Cys Leu Ile Ile Glu Asp Val Val Thr Ser Gly Ser Ser
        275                 280                 285

gtt ttg gaa act gtt gag gtt ctt cag aag gag ggc ttg aag gtc act 912
Val Leu Glu Thr Val Glu Val Leu Gln Lys Glu Gly Leu Lys Val Thr
    290                 295                 300

gat gcc ata gtg ctg ttg gac aga gag cag gga ggc aag gac aag ttg 960
Asp Ala Ile Val Leu Leu Asp Arg Glu Gln Gly Gly Lys Asp Lys Leu
305                 310                 315                 320

cag gcg cac ggg atc cgc ctc cac tca gtg tgt aca ttg tcc aaa atg 1008
Gln Ala His Gly Ile Arg Leu His Ser Val Cys Thr Leu Ser Lys Met
                325                 330                 335

ctg gag att ctc gag cag cag aaa aaa gtt gat gct gag aca gtt ggg 1056
Leu Glu Ile Leu Glu Gln Gln Lys Lys Val Asp Ala Glu Thr Val Gly
            340                 345                 350

aga gtg aag agg ttt att cag gag aat gtc ttt gtg gca gcg aat cat 1104
Arg Val Lys Arg Phe Ile Gln Glu Asn Val Phe Val Ala Ala Asn His
        355                 360                 365

aat ggt tct ccc ctt tct ata aag gaa gca ccc aaa gaa ctc agc ttc 1152
Asn Gly Ser Pro Leu Ser Ile Lys Glu Ala Pro Lys Glu Leu Ser Phe
    370                 375                 380

ggt gca cgt gca gag ctg ccc agg atc cac cca gtt gca tcg aag taa 1200
Gly Ala Arg Ala Glu Leu Pro Arg Ile His Pro Val Ala Ser Lys
385                 390                 395

<210> 16
<211> 399
<212> PRT
<213> Artificial Sequence

<220>
<223> Synthetic Construct

<400> 16

Met Val Thr Gly Gly Met Ala Ser Lys Trp Asp Gln Lys Gly Met Asp
1               5                   10                  15

Ile Ala Tyr Glu Glu Ala Leu Leu Gly Tyr Lys Glu Gly Gly Val Pro
            20                  25                  30

Ile Gly Gly Cys Leu Ile Asn Asn Lys Asp Gly Ser Val Leu Gly Arg
        35                  40                  45

Gly His Asn Met Arg Phe Gln Lys Gly Ser Ala Thr Leu His Gly Glu
    50                  55                  60

Ile Ser Thr Leu Glu Asn Cys Gly Arg Leu Glu Gly Lys Val Tyr Lys
65                  70                  75                  80

Asp Thr Thr Leu Tyr Thr Thr Leu Ser Pro Cys Asp Met Cys Thr Gly
                85                  90                  95

Ala Ile Ile Met Tyr Gly Ile Pro Arg Cys Val Ile Gly Glu Asn Val
            100                 105                 110

Asn Phe Lys Ser Lys Gly Glu Lys Tyr Leu Gln Thr Arg Gly His Glu
        115                 120                 125

Val Val Val Val Asp Asp Glu Arg Cys Lys Lys Leu Met Lys Gln Phe
    130                 135                 140

Ile Asp Glu Arg Pro Gln Asp Trp Phe Glu Asp Ile Gly Glu Ala Val
145                 150                 155                 160

Ala Arg Ala Ala Leu Gly Pro Leu Val Thr Gly Leu Tyr Asp Val Gln
                165                 170                 175

Ala Phe Lys Phe Gly Asp Phe Val Leu Lys Ser Gly Leu Ser Ser Pro
            180                 185                 190

Ile Tyr Ile Asp Leu Arg Gly Ile Val Ser Arg Pro Arg Leu Leu Ser
        195                 200                 205

Gln Val Ala Asp Ile Leu Phe Gln Thr Ala Gln Asn Ala Gly Ile Ser
    210                 215                 220

Phe Asp Thr Val Cys Gly Val Pro Tyr Thr Ala Leu Pro Leu Ala Thr
225                 230                 235                 240

Val Ile Cys Ser Thr Asn Gln Ile Pro Met Leu Ile Arg Arg Lys Glu
                245                 250                 255

Thr Lys Asp Tyr Gly Thr Lys Arg Leu Val Glu Gly Thr Ile Asn Pro
            260                 265                 270

Gly Glu Thr Cys Leu Ile Ile Glu Asp Val Val Thr Ser Gly Ser Ser
        275                 280                 285

Val Leu Glu Thr Val Glu Val Leu Gln Lys Glu Gly Leu Lys Val Thr
    290                 295                 300

Asp Ala Ile Val Leu Leu Asp Arg Glu Gln Gly Gly Lys Asp Lys Leu
305                 310                 315                 320

Gln Ala His Gly Ile Arg Leu His Ser Val Cys Thr Leu Ser Lys Met
                325                 330                 335

Leu Glu Ile Leu Glu Gln Gln Lys Lys Val Asp Ala Glu Thr Val Gly
            340                 345                 350

Arg Val Lys Arg Phe Ile Gln Glu Asn Val Phe Val Ala Ala Asn His
        355                 360                 365

Asn Gly Ser Pro Leu Ser Ile Lys Glu Ala Pro Lys Glu Leu Ser Phe
    370                 375                 380

Gly Ala Arg Ala Glu Leu Pro Arg Ile His Pro Val Ala Ser Lys
385                 390                 395

<210>  17
<211>  1260
<212>  DNA
<213>  Artificial Sequence

<220>

<223>  Fusion Construct - CDopt3 - linker - OPRT


<220>
<221>  CDS
<222>  (1)..(1260)

<400>  17
atg gtg acc ggc ggc atg gcc tcc aag tgg gat caa aag ggc atg gat        48
Met Val Thr Gly Gly Met Ala Ser Lys Trp Asp Gln Lys Gly Met Asp
1               5                   10                  15

atc gct tac gag gag gcc ctg ctg ggc tac aag gag ggc ggc gtg cct        96
Ile Ala Tyr Glu Glu Ala Leu Leu Gly Tyr Lys Glu Gly Gly Val Pro
            20                  25                  30

atc ggc ggc tgt ctg atc aac aac aag gac ggc agt gtg ctg ggc agg       144
Ile Gly Gly Cys Leu Ile Asn Asn Lys Asp Gly Ser Val Leu Gly Arg
        35                  40                  45

ggc cac aac atg agg ttc cag aag ggc tcc gcc acc ctg cac ggc gag       192
Gly His Asn Met Arg Phe Gln Lys Gly Ser Ala Thr Leu His Gly Glu
    50                  55                  60

atc tcc acc ctg gag aac tgt ggc agg ctg gag ggc aag gtg tac aag       240
Ile Ser Thr Leu Glu Asn Cys Gly Arg Leu Glu Gly Lys Val Tyr Lys
65                  70                  75                  80

gac acc acc ctg tac acc acc ctg tcc cct tgt gac atg tgt acc ggc       288
Asp Thr Thr Leu Tyr Thr Thr Leu Ser Pro Cys Asp Met Cys Thr Gly
                85                  90                  95

gct atc atc atg tac ggc atc cct agg tgt gtg atc ggc gag aac gtg       336
Ala Ile Ile Met Tyr Gly Ile Pro Arg Cys Val Ile Gly Glu Asn Val
            100                 105                 110

aac ttc aag tcc aag ggc gag aag tac ctg caa acc agg ggc cac gag       384
Asn Phe Lys Ser Lys Gly Glu Lys Tyr Leu Gln Thr Arg Gly His Glu
        115                 120                 125

gtg gtg gtt gtt gac gat gag agg tgt aag aag ctg atg aag cag ttc       432
Val Val Val Val Asp Asp Glu Arg Cys Lys Lys Leu Met Lys Gln Phe
    130                 135                 140

atc gac gag agg cct cag gac tgg ttc gag gat atc ggc gag tcc ggc       480
Ile Asp Glu Arg Pro Gln Asp Trp Phe Glu Asp Ile Gly Glu Ser Gly
145                 150                 155                 160

ggc ggc gcc tcc ggc ggc ggc gcc tcc ggc ggc ggc gcc tcc ggc ggc       528
Gly Gly Ala Ser Gly Gly Gly Ala Ser Gly Gly Gly Ala Ser Gly Gly
                165                 170                 175

ggc gcc gcg gtc gct cgt gca gct ttg ggg cca ttg gtg acg ggt ctg       576
Gly Ala Ala Val Ala Arg Ala Ala Leu Gly Pro Leu Val Thr Gly Leu
            180                 185                 190

tac gac gtg cag gct ttc aag ttt ggg gac ttc gtg ctg aag agc ggg       624
Tyr Asp Val Gln Ala Phe Lys Phe Gly Asp Phe Val Leu Lys Ser Gly
        195                 200                 205

ctt tcc tcc ccc atc tac atc gat ctg cgg ggc atc gtg tct cga ccg       672
Leu Ser Ser Pro Ile Tyr Ile Asp Leu Arg Gly Ile Val Ser Arg Pro
    210                 215                 220

cgt ctt ctg agt cag gtt gca gat att tta ttc caa act gcc caa aat     720
Arg Leu Leu Ser Gln Val Ala Asp Ile Leu Phe Gln Thr Ala Gln Asn
225                 230                 235                 240

gca ggc atc agt ttt gac acc gtg tgt gga gtg cct tat aca gct ttg     768
Ala Gly Ile Ser Phe Asp Thr Val Cys Gly Val Pro Tyr Thr Ala Leu
                245                 250                 255

cca ttg gct aca gtt atc tgt tca acc aat caa att cca atg ctt att     816
Pro Leu Ala Thr Val Ile Cys Ser Thr Asn Gln Ile Pro Met Leu Ile
            260                 265                 270

aga agg aaa gaa aca aag gat tat gga act aag cgt ctt gta gaa gga     864
Arg Arg Lys Glu Thr Lys Asp Tyr Gly Thr Lys Arg Leu Val Glu Gly
        275                 280                 285

act att aat cca gga gaa acc tgt tta atc att gaa gat gtt gtc acc     912
Thr Ile Asn Pro Gly Glu Thr Cys Leu Ile Ile Glu Asp Val Val Thr
    290                 295                 300

agt gga tct agt gtt ttg gaa act gtt gag gtt ctt cag aag gag ggc     960
Ser Gly Ser Ser Val Leu Glu Thr Val Glu Val Leu Gln Lys Glu Gly
305                 310                 315                 320

ttg aag gtc act gat gcc ata gtg ctg ttg gac aga gag cag gga ggc     1008
Leu Lys Val Thr Asp Ala Ile Val Leu Leu Asp Arg Glu Gln Gly Gly
                325                 330                 335

aag gac aag ttg cag gcg cac ggg atc cgc ctc cac tca gtg tgt aca     1056
Lys Asp Lys Leu Gln Ala His Gly Ile Arg Leu His Ser Val Cys Thr
            340                 345                 350

ttg tcc aaa atg ctg gag att ctc gag cag cag aaa aaa gtt gat gct     1104
Leu Ser Lys Met Leu Glu Ile Leu Glu Gln Gln Lys Lys Val Asp Ala
        355                 360                 365

gag aca gtt ggg aga gtg aag agg ttt att cag gag aat gtc ttt gtg     1152
Glu Thr Val Gly Arg Val Lys Arg Phe Ile Gln Glu Asn Val Phe Val
    370                 375                 380

gca gcg aat cat aat ggt tct ccc ctt tct ata aag gaa gca ccc aaa     1200
Ala Ala Asn His Asn Gly Ser Pro Leu Ser Ile Lys Glu Ala Pro Lys
385                 390                 395                 400

gaa ctc agc ttc ggt gca cgt gca gag ctg ccc agg atc cac cca gtt     1248
Glu Leu Ser Phe Gly Ala Arg Ala Glu Leu Pro Arg Ile His Pro Val
                405                 410                 415

gca tcg aag taa                                                     1260
Ala Ser Lys

<210>  18
<211>  419
<212>  PRT
<213>  Artificial Sequence

<220>
<223>  Synthetic Construct

<400>  18

Met Val Thr Gly Gly Met Ala Ser Lys Trp Asp Gln Lys Gly Met Asp
1               5                   10                  15

Ile Ala Tyr Glu Glu Ala Leu Leu Gly Tyr Lys Glu Gly Gly Val Pro
            20                  25                  30

Ile Gly Gly Cys Leu Ile Asn Asn Lys Asp Gly Ser Val Leu Gly Arg
        35                  40                  45

Gly His Asn Met Arg Phe Gln Lys Gly Ser Ala Thr Leu His Gly Glu
    50                  55                  60

Ile Ser Thr Leu Glu Asn Cys Gly Arg Leu Glu Gly Lys Val Tyr Lys
65                  70                  75                  80

Asp Thr Thr Leu Tyr Thr Thr Leu Ser Pro Cys Asp Met Cys Thr Gly
                85                  90                  95

Ala Ile Ile Met Tyr Gly Ile Pro Arg Cys Val Ile Gly Glu Asn Val
            100                 105                 110

Asn Phe Lys Ser Lys Gly Glu Lys Tyr Leu Gln Thr Arg Gly His Glu
        115                 120                 125

Val Val Val Val Asp Asp Glu Arg Cys Lys Lys Leu Met Lys Gln Phe
    130                 135                 140

Ile Asp Glu Arg Pro Gln Asp Trp Phe Glu Asp Ile Gly Glu Ser Gly
145                 150                 155                 160

Gly Gly Ala Ser Gly Gly Gly Ala Ser Gly Gly Gly Ala Ser Gly Gly
                165                 170                 175

Gly Ala Ala Val Ala Arg Ala Ala Leu Gly Pro Leu Val Thr Gly Leu
            180                 185                 190

Tyr Asp Val Gln Ala Phe Lys Phe Gly Asp Phe Val Leu Lys Ser Gly
        195                 200                 205

Leu Ser Ser Pro Ile Tyr Ile Asp Leu Arg Gly Ile Val Ser Arg Pro
    210                 215                 220

Arg Leu Leu Ser Gln Val Ala Asp Ile Leu Phe Gln Thr Ala Gln Asn
225                 230                 235                 240

Ala Gly Ile Ser Phe Asp Thr Val Cys Gly Val Pro Tyr Thr Ala Leu
                245                 250                 255

Pro Leu Ala Thr Val Ile Cys Ser Thr Asn Gln Ile Pro Met Leu Ile
            260                 265                 270

Arg Arg Lys Glu Thr Lys Asp Tyr Gly Thr Lys Arg Leu Val Glu Gly
        275                 280                 285

Thr Ile Asn Pro Gly Glu Thr Cys Leu Ile Ile Glu Asp Val Val Thr
    290                 295                 300

Ser Gly Ser Ser Val Leu Glu Thr Val Glu Val Leu Gln Lys Glu Gly
305                 310                 315                 320

Leu Lys Val Thr Asp Ala Ile Val Leu Leu Asp Arg Glu Gln Gly Gly
                325                 330                 335

Lys Asp Lys Leu Gln Ala His Gly Ile Arg Leu His Ser Val Cys Thr
            340                 345                 350

Leu Ser Lys Met Leu Glu Ile Leu Glu Gln Gln Lys Lys Val Asp Ala
        355                 360                 365

Glu Thr Val Gly Arg Val Lys Arg Phe Ile Gln Glu Asn Val Phe Val
    370                 375                 380

Ala Ala Asn His Asn Gly Ser Pro Leu Ser Ile Lys Glu Ala Pro Lys
385                 390                 395                 400

Glu Leu Ser Phe Gly Ala Arg Ala Glu Leu Pro Arg Ile His Pro Val
                405                 410                 415

Ala Ser Lys

<210>  19
<211>  11892
<212>  DNA
<213>  Artificial Sequence

<220>
<223>  RCR Vector - pAC3-yCD2

<400>  19
tagttattaa tagtaatcaa ttacggggtc attagttcat agcccatata tggagttccg        60

cgttacataa cttacggtaa atggcccgcc tggctgaccg cccaacgacc cccgcccatt       120

gacgtcaata atgacgtatg ttcccatagt aacgccaata gggactttcc attgacgtca       180

atgggtggag tatttacggt aaactgccca cttggcagta catcaagtgt atcatatgcc       240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cggcgccagt cctccgattg 600

actgagtcgc ccgggtaccc gtgtatccaa taaaccctct tgcagttgca tccgacttgt 660

ggtctcgctg ttccttggga gggtctcctc tgagtgattg actacccgtc agcgggggtc 720

tttcatttgg gggctcgtcc gggatcggga gacccctgcc cagggaccac cgacccacca 780

ccgggaggta agctggccag caacttatct gtgtctgtcc gattgtctag tgtctatgac 840

tgattttatg cgcctgcgtc ggtactagtt agctaactag ctctgtatct ggcggacccg 900

tggtggaact gacgagttcg gaacacccgg ccgcaaccct gggagacgtc ccagggactt 960

cgggggccgt ttttgtggcc cgacctgagt ccaaaaatcc cgatcgtttt ggactctttg 1020

gtgcaccccc cttagaggag ggatatgtgg ttctggtagg agacgagaac ctaaaacagt 1080

tcccgcctcc gtctgaattt ttgctttcgg tttgggaccg aagccgcgcc gcgcgtcttg 1140

tctgctgcag catcgttctg tgttgtctct gtctgactgt gtttctgtat ttgtctgaga 1200

atatgggcca gactgttacc actcccttaa gtttgacctt aggtcactgg aaagatgtcg 1260

agcggatcgc tcacaaccag tcggtagatg tcaagaagag acgttgggtt accttctgct 1320

ctgcagaatg gccaaccttt aacgtcggat ggccgcgaga cggcaccttt aaccgagacc 1380

tcatcaccca ggttaagatc aaggtctttt cacctggccc gcatggacac ccagaccagg 1440

tcccctacat cgtgacctgg gaagccttgg cttttgaccc ccctccctgg gtcaagccct 1500

ttgtacaccc taagcctccg cctcctcttc ctccatccgc cccgtctctc ccccttgaac 1560

ctcctcgttc gaccccgcct cgatcctccc tttatccagc cctcactcct tctctaggcg 1620

ccaaacctaa acctcaagtt ctttctgaca gtggggggcc gctcatcgac ctacttacag 1680

aagacccccc gccttatagg gacccaagac cacccccttc cgacagggac ggaaatggtg 1740

gagaagcgac ccctgcggga gaggcaccgg acccctcccc aatggcatct cgcctacgtg 1800

ggagacggga gccccctgtg gccgactcca ctacctcgca ggcattcccc ctccgcgcag 1860

gaggaaacgg acagcttcaa tactggccgt tctcctcttc tgacctttac aactggaaaa 1920

ataataaccc ttctttttct gaagatccag gtaaactgac agctctgatc gagtctgttc 1980

tcatcaccca tcagcccacc tgggacgact gtcagcagct gttggggact ctgctgaccg 2040

gagaagaaaa acaacgggtg ctcttagagg ctagaaaggc ggtgcggggc gatgatgggc 2100

gccccactca actgcccaat gaagtcgatg ccgcttttcc cctcgagcgc ccagactggg 2160

attacaccac ccaggcaggt aggaaccacc tagtccacta tcgccagttg ctcctagcgg 2220

gtctccaaaa cgcgggcaga agccccacca atttggccaa ggtaaaagga ataacacaag 2280

ggcccaatga gtctccctcg gccttcctag agagacttaa ggaagcctat cgcaggtaca 2340

ctccttatga ccctgaggac ccagggcaag aaactaatgt gtctatgtct ttcatttggc 2400

agtctgcccc agacattggg agaaagttag agaggttaga agatttaaaa aacaagacgc 2460

ttggagattt ggttagagag gcagaaaaga tctttaataa acgagaaacc ccggaagaaa 2520

gagaggaacg tatcaggaga gaaacagagg aaaaagaaga acgccgtagg acagaggatg 2580

agcagaaaga gaaagaaaga gatcgtagga gacatagaga gatgagcaag ctattggcca 2640

ctgtcgttag tggacagaaa caggatagac agggaggaga acgaaggagg tcccaactcg 2700

atcgcgacca gtgtgcctac tgcaaagaaa aggggcactg ggctaaagat tgtcccaaga 2760

aaccacgagg acctcgggga ccaagacccc agacctccct cctgacccta gatgactagg 2820

gaggtcaggg tcaggagccc ccccctgaac ccaggataac cctcaaagtc ggggggcaac 2880

ccgtcacctt cctggtagat actggggccc aacactccgt gctgacccaa aatcctggac 2940

ccctaagtga taagtctgcc tgggtccaag gggctactgg aggaaagcgg tatcgctgga 3000

ccacggatcg caaagtacat ctagctaccg gtaaggtcac ccactctttc ctccatgtac 3060

cagactgtcc ctatcctctg ttaggaagag atttgctgac taaactaaaa gcccaaatcc 3120

actttgaggg atcaggagcc caggttatgg gaccaatggg gcagcccctg caagtgttga 3180

ccctaaatat agaagatgag catcggctac atgagacctc aaaagagcca gatgtttctc 3240

tagggtccac atggctgtct gattttcctc aggcctgggc ggaaaccggg ggcatgggac 3300

tggcagttcg ccaagctcct ctgatcatac ctctgaaagc aacctctacc cccgtgtcca 3360

taaaacaata ccccatgtca caagaagcca gactggggat caagccccac atacagagac 3420

tgttggacca gggaatactg gtaccctgcc agtccccctg gaacacgccc ctgctacccg 3480

ttaagaaacc agggactaat gattataggc ctgtccagga tctgagagaa gtcaacaagc 3540

gggtggaaga catccacccc accgtgccca acccttacaa cctcttgagc gggctcccac 3600

cgtcccacca gtggtacact gtgcttgatt taaaggatgc ctttttctgc ctgagactcc 3660

accccaccag tcagcctctc ttcgcctttg agtggagaga tccagagatg ggaatctcag 3720

gacaattgac ctggaccaga ctcccacagg gtttcaaaaa cagtcccacc ctgtttgatg 3780

aggcactgca cagagaccta gcagacttcc ggatccagca cccagacttg atcctgctac 3840

agtacgtgga tgacttactg ctggccgcca cttctgagct agactgccaa caaggtactc 3900

gggccctgtt acaaacccta gggaacctcg ggtatcgggc ctcggccaag aaagcccaaa 3960

tttgccagaa acaggtcaag tatctggggt atcttctaaa agagggtcag agatggctga 4020

ctgaggccag aaaagagact gtgatggggc agcctactcc gaagacccct cgacaactaa 4080

gggagttcct agggacggca ggcttctgtc gcctctggat ccctgggttt gcagaaatgg 4140

cagccccctt gtaccctctc accaaaacgg ggactctgtt taattggggc ccagaccaac 4200

aaaaggccta tcaagaaatc aagcaagctc ttctaactgc cccagccctg gggttgccag 4260

atttgactaa gccctttgaa ctctttgtcg acgagaagca gggctacgcc aaaggtgtcc 4320

taacgcaaaa actgggacct tggcgtcggc cggtggccta cctgtccaaa aagctagacc 4380

cagtagcagc tgggtggccc ccttgcctac ggatggtagc agccattgcc gtactgacaa 4440

aggatgcagg caagctaacc atgggacagc cactagtcat tctggccccc catgcagtag 4500

aggcactagt caaacaaccc cccgaccgct ggctttccaa cgcccggatg actcactatc 4560

aggccttgct tttggacacg gaccgggtcc agttcggacc ggtggtagcc ctgaacccgg 4620

ctacgctgct cccactgcct gaggaagggc tgcaacacaa ctgccttgat atcctggccg 4680

aagcccacgg aacccgaccc gacctaacgg accagccgct cccagacgcc gaccacacct 4740

ggtacacgga tggaagcagt ctcttacaag agggacagcg taaggcggga gctgcggtga 4800

ccaccgagac cgaggtaatc tgggctaaag ccctgccagc cgggacatcc gctcagcggg 4860

ctgaactgat agcactcacc caggccctaa agatggcaga aggtaagaag ctaaatgttt 4920

atactgatag ccgttatgct tttgctactg cccatatcca tggagaaata tacagaaggc 4980

gtgggttgct cacatcagaa ggcaaagaga tcaaaaataa agacgagatc ttggccctac 5040

taaaagccct ctttctgccc aaaagactta gcataatcca ttgtccagga catcaaaagg 5100

gacacagcgc cgaggctaga ggcaaccgga tggctgacca agcggcccga aaggcagcca 5160

tcacagagac tccagacacc tctaccctcc tcatagaaaa ttcatcaccc tacacctcag 5220

aacattttca ttacacagtg actgatataa aggacctaac caagttgggg gccatttatg 5280

ataaaacaaa gaagtattgg gtctaccaag gaaaacctgt gatgcctgac cagtttactt 5340

ttgaattatt agactttctt catcagctga ctcacctcag cttctcaaaa atgaaggctc 5400

tcctagagag aagccacagt ccctactaca tgctgaaccg ggatcgaaca ctcaaaaata 5460

tcactgagac ctgcaaagct tgtgcacaag tcaacgccag caagtctgcc gttaaacagg 5520

gaactagggt ccgcgggcat cggcccggca ctcattggga gatcgatttc accgagataa 5580

agcccggatt gtatggctat aaatatcttc tagtttttat agataccttt tctggctgga 5640

tagaagcctt cccaaccaag aaagaaaccg ccaaggtcgt aaccaagaag ctactagagg 5700

agatcttccc caggttcggc atgcctcagg tattgggaac tgacaatggg cctgccttcg 5760

tctccaaggt gagtcagaca gtggccgatc tgttggggat tgattggaaa ttacattgtg 5820

catacagacc ccaaagctca ggccaggtag aaagaatgaa tagaaccatc aaggagactt 5880

taactaaatt aacgcttgca actggctcta gagactgggt gctcctactc cccttagccc 5940

tgtaccgagc ccgcaacacg ccgggccccc atggcctcac cccatatgag atcttatatg 6000

gggcaccccc gccccttgta aacttccctg accctgacat gacaagagtt actaacagcc 6060

cctctctcca agctcactta caggctctct acttagtcca gcacgaagtc tggagacctc 6120

tggcggcagc ctaccaagaa caactggacc gaccggtggt acctcaccct taccgagtcg 6180

gcgacacagt gtgggtccgc cgacaccaga ctaagaacct agaacctcgc tggaaaggac 6240

cttacacagt cctgctgacc acccccaccg ccctcaaagt agacggcatc gcagcttgga 6300

tacacgccgc ccacgtgaag gctgccgacc ccgggggtgg accatcctct agactgacat 6360

ggcgcgttca acgctctcaa aaccccctca agataagatt aacccgtgga agcccttaat 6420

agtcatggga gtcctgttag gagtagggat ggcagagagc ccccatcagg tctttaatgt 6480

aacctggaga gtcaccaacc tgatgactgg gcgtaccgcc aatgccacct ccctcctggg 6540

aactgtacaa gatgccttcc caaaattata ttttgatcta tgtgatctgg tcggagagga 6600

gtgggaccct tcagaccagg aaccgtatgt cgggtatggc tgcaagtacc ccgcagggag 6660

acagcggacc cggacttttg acttttacgt gtgccctggg cataccgtaa agtcggggtg 6720

tgggggacca ggagagggct actgtggtaa atgggggtgt gaaaccaccg gacaggctta 6780

ctggaagccc acatcatcgt gggacctaat ctcccttaag cgcggtaaca ccccctggga 6840

cacgggatgc tctaaagttg cctgtggccc ctgctacgac ctctccaaag tatccaattc 6900

cttccaaggg gctactcgag ggggcagatg caaccctcta gtcctagaat tcactgatgc 6960

aggaaaaaag gctaactggg acgggcccaa atcgtgggga ctgagactgt accggacagg 7020

aacagatcct attaccatgt tctccctgac ccggcaggtc cttaatgtgg gaccccgagt 7080

ccccataggg cccaacccag tattacccga ccaaagactc ccttcctcac caatagagat 7140

tgtaccggct ccacagccac ctagccccct caataccagt tacccccctt ccactaccag 7200

tacaccctca acctccccta caagtccaag tgtcccacag ccacccccag gaactggaga 7260

tagactacta gctctagtca aaggagccta tcaggcgctt aacctcacca atcccgacaa 7320

gacccaagaa tgttggctgt gcttagtgtc gggacctcct tattacgaag gagtagcggt 7380

cgtgggcact tataccaatc attccaccgc tccggccaac tgtacggcca cttcccaaca 7440

taagcttacc ctatctgaag tgacaggaca gggcctatgc atgggggcag tacctaaaac 7500

tcaccaggcc ttatgtaaca ccacccaaag cgccggctca ggatcctact accttgcagc 7560

acccgccgga acaatgtggg cttgcagcac tggattgact ccctgcttgt ccaccacggt 7620

gctcaatcta accacagatt attgtgtatt agttgaactc tggcccagag taatttacca 7680

ctcccccgat tatatgtatg gtcagcttga acagcgtacc aaatataaaa gagagccagt 7740

atcattgacc ctggcccttc tactaggagg attaaccatg ggagggattg cagctggaat 7800

agggacgggg accactgcct taattaaaac ccagcagttt gagcagcttc atgccgctat 7860

ccagacagac ctcaacgaag tcgaaaagtc aattaccaac ctagaaaagt cactgacctc 7920

gttgtctgaa gtagtcctac agaaccgcag aggcctagat ttgctattcc taaaggaggg 7980

aggtctctgc gcagccctaa aagaagaatg ttgtttttat gcagaccaca cggggctagt 8040

gagagacagc atggccaaat taagagaaag gcttaatcag agacaaaaac tatttgagac 8100

aggccaagga tggttcgaag ggctgtttaa tagatccccc tggtttacca ccttaatctc 8160

caccatcatg ggacctctaa tagtactctt actgatctta ctctttggac cttgcattct 8220

caatcgattg gtccaatttg ttaaagacag gatctcagtg gtccaggctc tggttttgac 8280

tcagcaatat caccagctaa aacccataga gtacgagcca tgaacgcgtt actggccgaa 8340

gccgcttgga ataaggccgg tgtgcgtttg tctatatgtt attttccacc atattgccgt 8400

cttttggcaa tgtgagggcc cggaaacctg gccctgtctt cttgacgagc attcctaggg 8460

gtctttcccc tctcgccaaa ggaatgcaag gtctgttgaa tgtcgtgaag gaagcagttc 8520

ctctggaagc ttcttgaaga caaacaacgt ctgtagcgac cctttgcagg cagcggaacc 8580

ccccacctgg cgacaggtgc ctctgcggcc aaaagccacg tgtataagat acacctgcaa 8640

aggcggcaca accccagtgc cacgttgtga gttggatagt tgtggaaaga gtcaaatggc 8700

tctcctcaag cgtattcaac aaggggctga aggatgccca gaaggtaccc cattgtatgg 8760

gatctgatct ggggcctcgg tgcacatgct ttacatgtgt ttagtcgagg ttaaaaaaac 8820

gtctaggccc cccgaaccac ggggacgtgg ttttcctttg aaaaacacga ttataaatgg 8880

tgaccggcgg catggcctcc aagtgggatc aaaagggcat ggatatcgct tacgaggagg 8940

ccctgctggg ctacaaggag ggcggcgtgc ctatcggcgg ctgtctgatc aacaacaagg 9000

acggcagtgt gctgggcagg ggccacaaca tgaggttcca gaagggctcc gccaccctgc 9060

acggcgagat ctccaccctg gagaactgtg gcaggctgga gggcaaggtg tacaaggaca 9120

ccaccctgta caccaccctg tccccttgtg acatgtgtac cggcgctatc atcatgtacg 9180

gcatccctag gtgtgtgatc ggcgagaacg tgaacttcaa gtccaagggc gagaagtacc 9240

tgcaaaccag gggccacgag gtggtggttg ttgacgatga gaggtgtaag aagctgatga 9300

agcagttcat cgacgagagg cctcaggact ggttcgagga tatcggcgag taagcggccg 9360

cagataaaat aaaagatttt atttagtctc cagaaaaagg ggggaatgaa agaccccacc 9420

tgtaggtttg gcaagctagc ttaagtaacg ccattttgca aggcatggaa aaatacataa 9480

ctgagaatag agaagttcag atcaaggtca ggaacagatg gaacagctga atatgggcca 9540

aacaggatat ctgtggtaag cagttcctgc cccggctcag ggccaagaac agatggaaca 9600

gctgaatatg ggccaaacag gatatctgtg gtaagcagtt cctgccccgg ctcagggcca 9660

agaacagatg gtccccagat gcggtccagc cctcagcagt ttctagagaa ccatcagatg 9720

tttccagggt gccccaagga cctgaaatga ccctgtgcct tatttgaact aaccaatcag 9780

ttcgcttctc gcttctgttc gcgcgcttct gctccccgag ctcaataaaa gagcccacaa 9840

cccctcactc ggggcgccag tcctccgatt gactgagtcg cccgggtacc cgtgtatcca 9900

ataaaccctc ttgcagttgc atccgacttg tggtctcgct gttccttggg agggtctcct 9960

ctgagtgatt gactacccgt cagcgggggt ctttcattac atgtgagcaa aaggccagca 10020

aaaggccagg aaccgtaaaa aggccgcgtt gctggcgttt ttccataggc tccgcccccc 10080

tgacgagcat cacaaaaatc gacgctcaag tcagaggtgg cgaaacccga caggactata 10140

aagataccag gcgtttcccc ctggaagctc cctcgtgcgc tctcctgttc cgaccctgcc 10200

gcttaccgga tacctgtccg cctttctccc ttcgggaagc gtggcgcttt ctcaatgctc 10260

acgctgtagg tatctcagtt cggtgtaggt cgttcgctcc aagctgggct gtgtgcacga 10320

accccccgtt cagcccgacc gctgcgcctt atccggtaac tatcgtcttg agtccaaccc 10380

ggtaagacac gacttatcgc cactggcagc agccactggt aacaggatta gcagagcgag 10440

gtatgtaggc ggtgctacag agttcttgaa gtggtggcct aactacggct acactagaag 10500

gacagtattt ggtatctgcg ctctgctgaa gccagttacc ttcggaaaaa gagttggtag 10560

ctcttgatcc ggcaaacaaa ccaccgctgg tagcggtggt ttttttgttt gcaagcagca 10620

gattacgcgc agaaaaaaag gatctcaaga agatcctttg atcttttcta cggggtctga 10680

cgctcagtgg aacgaaaact cacgttaagg gattttggtc atgagattat caaaaaggat 10740

cttcacctag atccttttaa attaaaaatg aagttttaaa tcaatctaaa gtatatatga 10800

gtaaacttgg tctgacagtt accaatgctt aatcagtgag gcacctatct cagcgatctg 10860

tctatttcgt tcatccatag ttgcctgact ccccgtcgtg tagataacta cgatacggga 10920

gggcttacca tctggcccca gtgctgcaat gataccgcga gacccacgct caccggctcc 10980

agatttatca gcaataaacc agccagccgg aagggccgag cgcagaagtg gtcctgcaac 11040

tttatccgcc tccatccagt ctattaattg ttgccgggaa gctagagtaa gtagttcgcc 11100

agttaatagt ttgcgcaacg ttgttgccat tgctgcaggc atcgtggtgt cacgctcgtc 11160

gtttggtatg gcttcattca gctccggttc ccaacgatca aggcgagtta catgatcccc 11220

catgttgtgc aaaaaagcgg ttagctcctt cggtcctccg atcgttgtca gaagtaagtt 11280

ggccgcagtg ttatcactca tggttatggc agcactgcat aattctctta ctgtcatgcc 11340

atccgtaaga tgcttttctg tgactggtga gtactcaacc aagtcattct gagaatagtg 11400

tatgcggcga ccgagttgct cttgcccggc gtcaacacgg gataataccg cgccacatag 11460

cagaacttta aaagtgctca tcattggaaa acgttcttcg gggcgaaaac tctcaaggat 11520

cttaccgctg ttgagatcca gttcgatgta acccactcgt gcacccaact gatcttcagc 11580

atcttttact ttcaccagcg tttctgggtg agcaaaaaca ggaaggcaaa atgccgcaaa 11640

aaagggaata agggcgacac ggaaatgttg aatactcata ctcttccttt ttcaatatta 11700

ttgaagcatt tatcagggtt attgtctcat gagcggatac atatttgaat gtatttagaa 11760

aaataaacaa ataggggttc cgcgcacatt tccccgaaaa gtgccacctg acgtctaaga 11820

aaccattatt atcatgacat taacctataa aaataggcgt atcacgaggc cctttcgtct 11880

tcaagaattc at 11892

<210> 20
<211> 11892
<212> DNA
<213> Artificial Sequence

<220>
<223> RCR Vector - pAC3-yCD

<400> 20
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cggcgccagt cctccgattg 600

actgagtcgc ccgggtaccc gtgtatccaa taaaccctct tgcagttgca tccgacttgt 660

ggtctcgctg ttccttggga gggtctcctc tgagtgattg actacccgtc agcgggggtc 720

tttcatttgg gggctcgtcc gggatcggga gacccctgcc cagggaccac cgacccacca 780

ccgggaggta agctggccag caacttatct gtgtctgtcc gattgtctag tgtctatgac 840

tgattttatg cgcctgcgtc ggtactagtt agctaactag ctctgtatct ggcggacccg 900

tggtggaact gacgagttcg gaacacccgg ccgcaaccct gggagacgtc ccagggactt 960

cgggggccgt ttttgtggcc cgacctgagt ccaaaaatcc cgatcgtttt ggactctttg 1020

gtgcaccccc cttagaggag ggatatgtgg ttctggtagg agacgagaac ctaaaacagt 1080

tcccgcctcc gtctgaattt ttgctttcgg tttgggaccg aagccgcgcc gcgcgtcttg 1140

tctgctgcag catcgttctg tgttgtctct gtctgactgt gtttctgtat ttgtctgaga 1200

atatgggcca gactgttacc actcccttaa gtttgacctt aggtcactgg aaagatgtcg 1260

agcggatcgc tcacaaccag tcggtagatg tcaagaagag acgttgggtt accttctgct 1320

ctgcagaatg gccaaccttt aacgtcggat ggccgcgaga cggcaccttt aaccgagacc 1380

tcatcaccca ggttaagatc aaggtctttt cacctggccc gcatggacac ccagaccagg 1440

tcccctacat cgtgacctgg gaagccttgg cttttgaccc ccctccctgg gtcaagccct 1500

ttgtacaccc taagcctccg cctcctcttc ctccatccgc cccgtctctc ccccttgaac 1560

ctcctcgttc gaccccgcct cgatcctccc tttatccagc cctcactcct tctctaggcg 1620

ccaaacctaa acctcaagtt ctttctgaca gtggggggcc gctcatcgac ctacttacag 1680

aagacccccc gccttatagg gacccaagac cacccccttc cgacagggac ggaaatggtg 1740

gagaagcgac ccctgcggga gaggcaccgg acccctcccc aatggcatct cgcctacgtg 1800

ggagacggga gccccctgtg gccgactcca ctacctcgca ggcattcccc ctccgcgcag 1860

gaggaaacgg acagcttcaa tactggccgt tctcctcttc tgacctttac aactggaaaa 1920

ataataaccc ttctttttct gaagatccag gtaaactgac agctctgatc gagtctgttc 1980

tcatcaccca tcagcccacc tgggacgact gtcagcagct gttggggact ctgctgaccg 2040

gagaagaaaa acaacgggtg ctcttagagg ctagaaaggc ggtgcggggc gatgatgggc 2100

gccccactca actgcccaat gaagtcgatg ccgcttttcc cctcgagcgc ccagactggg 2160

attacaccac ccaggcaggt aggaaccacc tagtccacta tcgccagttg ctcctagcgg 2220

gtctccaaaa cgcgggcaga agccccacca atttggccaa ggtaaaagga ataacacaag 2280

ggcccaatga gtctccctcg gccttcctag agagacttaa ggaagcctat cgcaggtaca 2340

ctccttatga ccctgaggac ccagggcaag aaactaatgt gtctatgtct ttcatttggc 2400

agtctgcccc agacattggg agaaagttag agaggttaga agatttaaaa aacaagacgc 2460

ttggagattt ggttagagag gcagaaaaga tctttaataa acgagaaacc ccggaagaaa 2520

gagaggaacg tatcaggaga gaaacagagg aaaaagaaga acgccgtagg acagaggatg 2580

agcagaaaga gaaagaaaga gatcgtagga gacatagaga gatgagcaag ctattggcca 2640

ctgtcgttag tggacagaaa caggatagac agggaggaga acgaaggagg tcccaactcg 2700

atcgcgacca gtgtgcctac tgcaaagaaa aggggcactg ggctaaagat tgtcccaaga 2760

aaccacgagg acctcgggga ccaagacccc agacctccct cctgacccta gatgactagg 2820

gaggtcaggg tcaggagccc ccccctgaac ccaggataac cctcaaagtc ggggggcaac 2880

ccgtcacctt cctggtagat actggggccc aacactccgt gctgacccaa aatcctggac 2940

ccctaagtga taagtctgcc tgggtccaag gggctactgg aggaaagcgg tatcgctgga 3000

ccacggatcg caaagtacat ctagctaccg gtaaggtcac ccactctttc ctccatgtac 3060

cagactgtcc ctatcctctg ttaggaagag atttgctgac taaactaaaa gcccaaatcc 3120

actttgaggg atcaggagcc caggttatgg gaccaatggg gcagcccctg caagtgttga 3180

ccctaaatat agaagatgag catcggctac atgagacctc aaaagagcca gatgtttctc 3240

tagggtccac atggctgtct gattttcctc aggcctgggc ggaaaccggg ggcatgggac 3300

tggcagttcg ccaagctcct ctgatcatac ctctgaaagc aacctctacc cccgtgtcca 3360

taaaacaata ccccatgtca caagaagcca gactggggat caagccccac atacagagac 3420

tgttggacca gggaatactg gtaccctgcc agtccccctg gaacacgccc ctgctacccg 3480

ttaagaaacc agggactaat gattataggc ctgtccagga tctgagagaa gtcaacaagc 3540

gggtggaaga catccacccc accgtgccca acccttacaa cctcttgagc gggctcccac 3600

cgtcccacca gtggtacact gtgcttgatt taaaggatgc ctttttctgc ctgagactcc 3660

accccaccag tcagcctctc ttcgcctttg agtggagaga tccagagatg ggaatctcag 3720

gacaattgac ctggaccaga ctcccacagg gtttcaaaaa cagtcccacc ctgtttgatg 3780

aggcactgca cagagaccta gcagacttcc ggatccagca cccagacttg atcctgctac 3840

agtacgtgga tgacttactg ctggccgcca cttctgagct agactgccaa caaggtactc 3900

gggccctgtt acaaacccta gggaacctcg ggtatcgggc ctcggccaag aaagcccaaa 3960

tttgccagaa acaggtcaag tatctggggt atcttctaaa agagggtcag agatggctga 4020

ctgaggccag aaaagagact gtgatggggc agcctactcc gaagacccct cgacaactaa 4080

gggagttcct agggacggca ggcttctgtc gcctctggat ccctgggttt gcagaaatgg 4140

cagccccctt gtaccctctc accaaaacgg ggactctgtt taattggggc ccagaccaac 4200

aaaaggccta tcaagaaatc aagcaagctc ttctaactgc cccagccctg gggttgccag 4260

atttgactaa gccctttgaa ctctttgtcg acgagaagca gggctacgcc aaaggtgtcc 4320

taacgcaaaa actgggacct tggcgtcggc cggtggccta cctgtccaaa aagctagacc 4380

cagtagcagc tgggtggccc ccttgcctac ggatggtagc agccattgcc gtactgacaa 4440

aggatgcagg caagctaacc atgggacagc cactagtcat tctggccccc catgcagtag 4500

aggcactagt caaacaaccc cccgaccgct ggctttccaa cgcccggatg actcactatc 4560

aggccttgct tttggacacg gaccgggtcc agttcggacc ggtggtagcc ctgaacccgg 4620

ctacgctgct cccactgcct gaggaagggc tgcaacacaa ctgccttgat atcctggccg 4680

aagcccacgg aacccgaccc gacctaacgg accagccgct cccagacgcc gaccacacct 4740

ggtacacgga tggaagcagt ctcttacaag agggacagcg taaggcggga gctgcggtga 4800

ccaccgagac cgaggtaatc tgggctaaag ccctgccagc cgggacatcc gctcagcggg 4860

ctgaactgat agcactcacc caggccctaa agatggcaga aggtaagaag ctaaatgttt 4920

atactgatag ccgttatgct tttgctactg cccatatcca tggagaaata tacagaaggc 4980

gtgggttgct cacatcagaa ggcaaagaga tcaaaaataa agacgagatc ttggccctac 5040

taaaagccct ctttctgccc aaaagactta gcataatcca ttgtccagga catcaaaagg 5100

gacacagcgc cgaggctaga ggcaaccgga tggctgacca agcggcccga aaggcagcca 5160

tcacagagac tccagacacc tctaccctcc tcatagaaaa ttcatcaccc tacacctcag 5220

aacattttca ttacacagtg actgatataa aggacctaac caagttgggg gccatttatg 5280

ataaaacaaa gaagtattgg gtctaccaag gaaaacctgt gatgcctgac cagtttactt 5340

ttgaattatt agactttctt catcagctga ctcacctcag cttctcaaaa atgaaggctc 5400

tcctagagag aagccacagt ccctactaca tgctgaaccg ggatcgaaca ctcaaaaata 5460

tcactgagac ctgcaaagct tgtgcacaag tcaacgccag caagtctgcc gttaaacagg 5520

gaactagggt ccgcgggcat cggcccggca ctcattggga gatcgatttc accgagataa 5580

agcccggatt gtatggctat aaatatcttc tagtttttat agataccttt tctggctgga 5640

tagaagcctt cccaaccaag aaagaaaccg ccaaggtcgt aaccaagaag ctactagagg 5700

agatcttccc caggttcggc atgcctcagg tattgggaac tgacaatggg cctgccttcg 5760

tctccaaggt gagtcagaca gtggccgatc tgttggggat tgattggaaa ttacattgtg 5820

catacagacc ccaaagctca ggccaggtag aaagaatgaa tagaaccatc aaggagactt 5880

taactaaatt aacgcttgca actggctcta gagactgggt gctcctactc cccttagccc 5940

tgtaccgagc ccgcaacacg ccgggccccc atggcctcac cccatatgag atcttatatg 6000

gggcaccccc gccccttgta aacttccctg accctgacat gacaagagtt actaacagcc 6060

cctctctcca agctcactta caggctctct acttagtcca gcacgaagtc tggagacctc 6120

tggcggcagc ctaccaagaa caactggacc gaccggtggt acctcaccct taccgagtcg 6180

gcgacacagt gtgggtccgc cgacaccaga ctaagaacct agaacctcgc tggaaaggac 6240

cttacacagt cctgctgacc acccccaccg ccctcaaagt agacggcatc gcagcttgga 6300

tacacgccgc ccacgtgaag gctgccgacc ccgggggtgg accatcctct agactgacat 6360

ggcgcgttca acgctctcaa aaccccctca agataagatt aacccgtgga agcccttaat 6420

agtcatggga gtcctgttag gagtagggat ggcagagagc ccccatcagg tctttaatgt 6480

aacctggaga gtcaccaacc tgatgactgg gcgtaccgcc aatgccacct ccctcctggg 6540

aactgtacaa gatgccttcc caaaattata ttttgatcta tgtgatctgg tcggagagga 6600

gtgggaccct tcagaccagg aaccgtatgt cgggtatggc tgcaagtacc ccgcagggag 6660

acagcggacc cggacttttg acttttacgt gtgccctggg cataccgtaa agtcggggtg 6720

tgggggacca ggagagggct actgtggtaa atgggggtgt gaaaccaccg gacaggctta 6780

ctggaagccc acatcatcgt gggacctaat ctcccttaag cgcggtaaca ccccctggga 6840

cacgggatgc tctaaagttg cctgtggccc ctgctacgac ctctccaaag tatccaattc 6900

cttccaaggg gctactcgag ggggcagatg caaccctcta gtcctagaat tcactgatgc 6960

aggaaaaaag gctaactggg acgggcccaa atcgtgggga ctgagactgt accggacagg 7020

aacagatcct attaccatgt tctccctgac ccggcaggtc cttaatgtgg gaccccgagt 7080

ccccataggg cccaacccag tattacccga ccaaagactc ccttcctcac caatagagat 7140

tgtaccggct ccacagccac ctagccccct caataccagt tacccccctt ccactaccag 7200

tacaccctca acctccccta caagtccaag tgtcccacag ccacccccag gaactggaga 7260

tagactacta gctctagtca aaggagccta tcaggcgctt aacctcacca atcccgacaa 7320

gacccaagaa tgttggctgt gcttagtgtc gggacctcct tattacgaag gagtagcggt 7380

cgtgggcact tataccaatc attccaccgc tccggccaac tgtacggcca cttcccaaca 7440

taagcttacc ctatctgaag tgacaggaca gggcctatgc atgggggcag tacctaaaac 7500

tcaccaggcc ttatgtaaca ccacccaaag cgccggctca ggatcctact accttgcagc 7560

acccgccgga acaatgtggg cttgcagcac tggattgact ccctgcttgt ccaccacggt 7620

gctcaatcta accacagatt attgtgtatt agttgaactc tggcccagag taatttacca 7680

ctcccccgat tatatgtatg gtcagcttga acagcgtacc aaatataaaa gagagccagt 7740

atcattgacc ctggcccttc tactaggagg attaaccatg ggagggattg cagctggaat 7800

agggacgggg accactgcct taattaaaac ccagcagttt gagcagcttc atgccgctat 7860

ccagacagac ctcaacgaag tcgaaaagtc aattaccaac ctagaaaagt cactgacctc 7920

gttgtctgaa gtagtcctac agaaccgcag aggcctagat ttgctattcc taaaggaggg 7980

aggtctctgc gcagccctaa aagaagaatg ttgtttttat gcagaccaca cggggctagt 8040

gagagacagc atggccaaat taagagaaag gcttaatcag agacaaaaac tatttgagac 8100

aggccaagga tggttcgaag ggctgtttaa tagatccccc tggtttacca ccttaatctc 8160

caccatcatg ggacctctaa tagtactctt actgatctta ctctttggac cttgcattct 8220

caatcgattg gtccaatttg ttaaagacag gatctcagtg gtccaggctc tggttttgac 8280

tcagcaatat caccagctaa aacccataga gtacgagcca tgaacgcgtt actggccgaa 8340

gccgcttgga ataaggccgg tgtgcgtttg tctatatgtt attttccacc atattgccgt 8400

cttttggcaa tgtgagggcc cggaaacctg gccctgtctt cttgacgagc attcctaggg 8460

gtctttcccc tctcgccaaa ggaatgcaag gtctgttgaa tgtcgtgaag gaagcagttc 8520

ctctggaagc ttcttgaaga caaacaacgt ctgtagcgac cctttgcagg cagcggaacc 8580

ccccacctgg cgacaggtgc ctctgcggcc aaaagccacg tgtataagat acacctgcaa 8640

aggcggcaca accccagtgc cacgttgtga gttggatagt tgtggaaaga gtcaaatggc 8700

tctcctcaag cgtattcaac aaggggctga aggatgccca gaaggtaccc cattgtatgg 8760

gatctgatct ggggcctcgg tgcacatgct ttacatgtgt ttagtcgagg ttaaaaaaac 8820

gtctaggccc cccgaaccac ggggacgtgg ttttcctttg aaaaacacga ttataaatgg 8880

tgacaggggg aatggcaagc aagtgggatc agaagggtat ggacattgcc tatgaggagg 8940

cggccttagg ttacaaagag ggtggtgttc ctattggcgg atgtcttatc aataacaaag 9000

acggaagtgt tctcggtcgt ggtcacaaca tgagatttca aaagggatcc gccacactac 9060

atggtgagat ctccactttg gaaaactgtg ggagattaga gggcaaagtg tacaaagata 9120

ccactttgta tacgacgctg tctccatgcg acatgtgtac aggtgccatc atcatgtatg 9180

gtattccacg ctgtgttgtc ggtgagaacg ttaatttcaa aagtaagggc gagaaatatt 9240

tacaaactag aggtcacgag gttgttgttg ttgacgatga gaggtgtaaa aagatcatga 9300

aacaatttat cgatgaaaga cctcaggatt ggtttgaaga tattggtgag taggcggccg 9360

cagataaaat aaaagatttt atttagtctc cagaaaaagg ggggaatgaa agaccccacc 9420

tgtaggtttg gcaagctagc ttaagtaacg ccattttgca aggcatggaa aaatacataa 9480

ctgagaatag agaagttcag atcaaggtca ggaacagatg gaacagctga atatgggcca 9540

aacaggatat ctgtggtaag cagttcctgc cccggctcag ggccaagaac agatggaaca 9600

gctgaatatg ggccaaacag gatatctgtg gtaagcagtt cctgccccgg ctcagggcca 9660

agaacagatg gtccccagat gcggtccagc cctcagcagt ttctagagaa ccatcagatg 9720

tttccagggt gccccaagga cctgaaatga ccctgtgcct tatttgaact aaccaatcag 9780

ttcgcttctc gcttctgttc gcgcgcttct gctccccgag ctcaataaaa gagcccacaa 9840

cccctcactc ggggcgccag tcctccgatt gactgagtcg cccgggtacc cgtgtatcca 9900

ataaaccctc ttgcagttgc atccgacttg tggtctcgct gttccttggg agggtctcct 9960

ctgagtgatt gactacccgt cagcgggggt ctttcattac atgtgagcaa aaggccagca 10020

aaaggccagg aaccgtaaaa aggccgcgtt gctggcgttt ttccataggc tccgcccccc 10080

tgacgagcat cacaaaaatc gacgctcaag tcagaggtgg cgaaacccga caggactata 10140

aagataccag gcgtttcccc ctggaagctc cctcgtgcgc tctcctgttc cgaccctgcc 10200

gcttaccgga tacctgtccg cctttctccc ttcgggaagc gtggcgcttt ctcaatgctc 10260

acgctgtagg tatctcagtt cggtgtaggt cgttcgctcc aagctgggct gtgtgcacga 10320

accccccgtt cagcccgacc gctgcgcctt atccggtaac tatcgtcttg agtccaaccc 10380

ggtaagacac gacttatcgc cactggcagc agccactggt aacaggatta gcagagcgag 10440

gtatgtaggc ggtgctacag agttcttgaa gtggtggcct aactacggct acactagaag 10500

gacagtattt ggtatctgcg ctctgctgaa gccagttacc ttcggaaaaa gagttggtag 10560

ctcttgatcc ggcaaacaaa ccaccgctgg tagcggtggt ttttttgttt gcaagcagca 10620

gattacgcgc agaaaaaaag gatctcaaga agatcctttg atcttttcta cggggtctga 10680

cgctcagtgg aacgaaaact cacgttaagg gattttggtc atgagattat caaaaaggat 10740

cttcacctag atccttttaa attaaaaatg aagttttaaa tcaatctaaa gtatatatga 10800

gtaaacttgg tctgacagtt accaatgctt aatcagtgag gcacctatct cagcgatctg 10860

tctatttcgt tcatccatag ttgcctgact ccccgtcgtg tagataacta cgatacggga 10920

gggcttacca tctggcccca gtgctgcaat gataccgcga gacccacgct caccggctcc 10980

agatttatca gcaataaacc agccagccgg aagggccgag cgcagaagtg gtcctgcaac 11040

tttatccgcc tccatccagt ctattaattg ttgccgggaa gctagagtaa gtagttcgcc 11100

agttaatagt ttgcgcaacg ttgttgccat tgctgcaggc atcgtggtgt cacgctcgtc 11160

gtttggtatg gcttcattca gctccggttc ccaacgatca aggcgagtta catgatcccc 11220

catgttgtgc aaaaaagcgg ttagctcctt cggtcctccg atcgttgtca gaagtaagtt 11280

ggccgcagtg ttatcactca tggttatggc agcactgcat aattctctta ctgtcatgcc 11340

atccgtaaga tgcttttctg tgactggtga gtactcaacc aagtcattct gagaatagtg 11400

tatgcggcga ccgagttgct cttgcccggc gtcaacacgg gataataccg cgccacatag 11460

cagaacttta aaagtgctca tcattggaaa acgttcttcg gggcgaaaac tctcaaggat 11520

cttaccgctg ttgagatcca gttcgatgta acccactcgt gcacccaact gatcttcagc 11580

atcttttact ttcaccagcg tttctgggtg agcaaaaaca ggaaggcaaa atgccgcaaa 11640

aaagggaata agggcgacac ggaaatgttg aatactcata ctcttccttt ttcaatatta 11700

ttgaagcatt tatcagggtt attgtctcat gagcggatac atatttgaat gtatttagaa 11760

aaataaacaa ataggggttc cgcgcacatt tccccgaaaa gtgccacctg acgtctaaga 11820

aaccattatt atcatgacat taacctataa aaataggcgt atcacgaggc cctttcgtct 11880

tcaagaattc at 11892

<210> 21
<211> 12007
<212> DNA
<213> Artificial Sequence

<220>
<223> RCR Vector - pACE-CD

<400> 21
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cggcgccagt cctccgattg 600

actgagtcgc ccgggtaccc gtgtatccaa taaaccctct tgcagttgca tccgacttgt 660

ggtctcgctg ttccttggga gggtctcctc tgagtgattg actacccgtc agcgggggtc 720

tttcatttgg gggctcgtcc gggatcggga gacccctgcc cagggaccac cgacccacca 780

ccgggaggta agctggccag caacttatct gtgtctgtcc gattgtctag tgtctatgac 840

tgattttatg cgcctgcgtc ggtactagtt agctaactag ctctgtatct ggcggacccg 900

tggtggaact gacgagttcg gaacacccgg ccgcaaccct gggagacgtc ccagggactt 960

cgggggccgt ttttgtggcc cgacctgagt ccaaaaatcc cgatcgtttt ggactctttg 1020

gtgcaccccc cttagaggag ggatatgtgg ttctggtagg agacgagaac ctaaaacagt 1080

tcccgcctcc gtctgaattt ttgctttcgg tttgggaccg aagccgcgcc gcgcgtcttg 1140

tctgctgcag catcgttctg tgttgtctct gtctgactgt gtttctgtat ttgtctgaga 1200

atatgggcca gactgttacc actcccttaa gtttgacctt aggtcactgg aaagatgtcg 1260

agcggatcgc tcacaaccag tcggtagatg tcaagaagag acgttgggtt accttctgct 1320

ctgcagaatg gccaaccttt aacgtcggat ggccgcgaga cggcaccttt aaccgagacc 1380

tcatcaccca ggttaagatc aaggtctttt cacctggccc gcatggacac ccagaccagg 1440

tcccctacat cgtgacctgg gaagccttgg cttttgaccc ccctccctgg gtcaagccct 1500

ttgtacaccc taagcctccg cctcctcttc ctccatccgc cccgtctctc ccccttgaac 1560

ctcctcgttc gaccccgcct cgatcctccc tttatccagc cctcactcct tctctaggcg 1620

ccaaacctaa acctcaagtt ctttctgaca gtggggggcc gctcatcgac ctacttacag 1680

aagacccccc gccttatagg gacccaagac cacccccttc cgacagggac ggaaatggtg 1740

gagaagcgac ccctgcggga gaggcaccgg acccctcccc aatggcatct cgcctacgtg 1800

ggagacggga gccccctgtg gccgactcca ctacctcgca ggcattcccc ctccgcgcag 1860

gaggaaacgg acagcttcaa tactggccgt tctcctcttc tgacctttac aactggaaaa 1920

ataataaccc ttctttttct gaagatccag gtaaactgac agctctgatc gagtctgttc 1980

tcatcaccca tcagcccacc tgggacgact gtcagcagct gttggggact ctgctgaccg 2040

gagaagaaaa acaacgggtg ctcttagagg ctagaaaggc ggtgcggggc gatgatgggc 2100

gccccactca actgcccaat gaagtcgatg ccgcttttcc cctcgagcgc ccagactggg 2160

attacaccac ccaggcaggt aggaaccacc tagtccacta tcgccagttg ctcctagcgg 2220

gtctccaaaa cgcgggcaga agccccacca atttggccaa ggtaaaagga ataacacaag 2280

ggcccaatga gtctccctcg gccttcctag agagacttaa ggaagcctat cgcaggtaca 2340

ctccttatga ccctgaggac ccagggcaag aaactaatgt gtctatgtct ttcatttggc 2400

agtctgcccc agacattggg agaaagttag agaggttaga agatttaaaa aacaagacgc 2460

ttggagattt ggttagagag gcagaaaaga tctttaataa acgagaaacc ccggaagaaa 2520

gagaggaacg tatcaggaga gaaacagagg aaaaagaaga acgccgtagg acagaggatg 2580

agcagaaaga gaaagaaaga gatcgtagga gacatagaga gatgagcaag ctattggcca 2640

ctgtcgttag tggacagaaa caggatagac agggaggaga acgaaggagg tcccaactcg 2700

atcgcgacca gtgtgcctac tgcaaagaaa aggggcactg ggctaaagat tgtcccaaga 2760

aaccacgagg acctcgggga ccaagacccc agacctccct cctgacccta gatgactagg 2820

gaggtcaggg tcaggagccc ccccctgaac ccaggataac cctcaaagtc ggggggcaac 2880

ccgtcacctt cctggtagat actggggccc aacactccgt gctgacccaa aatcctggac 2940

ccctaagtga taagtctgcc tgggtccaag gggctactgg aggaaagcgg tatcgctgga 3000

ccacggatcg caaagtacat ctagctaccg gtaaggtcac ccactctttc ctccatgtac 3060

cagactgtcc ctatcctctg ttaggaagag atttgctgac taaactaaaa gcccaaatcc 3120

actttgaggg atcaggagcc caggttatgg gaccaatggg gcagcccctg caagtgttga 3180

ccctaaatat agaagatgag catcggctac atgagacctc aaaagagcca gatgtttctc 3240

tagggtccac atggctgtct gattttcctc aggcctgggc ggaaaccggg ggcatgggac 3300

tggcagttcg ccaagctcct ctgatcatac ctctgaaagc aacctctacc cccgtgtcca 3360

taaaacaata ccccatgtca caagaagcca gactggggat caagccccac atacagagac 3420

tgttggacca gggaatactg gtaccctgcc agtccccctg gaacacgccc ctgctacccg 3480

ttaagaaacc agggactaat gattataggc ctgtccagga tctgagagaa gtcaacaagc 3540

gggtggaaga catccacccc accgtgccca acccttacaa cctcttgagc gggctcccac 3600

cgtcccacca gtggtacact gtgcttgatt taaaggatgc ctttttctgc ctgagactcc 3660

accccaccag tcagcctctc ttcgcctttg agtggagaga tccagagatg ggaatctcag 3720

gacaattgac ctggaccaga ctcccacagg gtttcaaaaa cagtcccacc ctgtttgatg 3780

aggcactgca cagagaccta gcagacttcc ggatccagca cccagacttg atcctgctac 3840

agtacgtgga tgacttactg ctggccgcca cttctgagct agactgccaa caaggtactc 3900

gggccctgtt acaaacccta gggaacctcg ggtatcgggc ctcggccaag aaagcccaaa 3960

tttgccagaa acaggtcaag tatctggggt atcttctaaa agagggtcag agatggctga 4020

ctgaggccag aaaagagact gtgatggggc agcctactcc gaagacccct cgacaactaa 4080

gggagttcct agggacggca ggcttctgtc gcctctggat ccctgggttt gcagaaatgg 4140

cagccccctt gtaccctctc accaaaacgg ggactctgtt taattggggc ccagaccaac 4200

aaaaggccta tcaagaaatc aagcaagctc ttctaactgc cccagccctg gggttgccag 4260

atttgactaa gccctttgaa ctctttgtcg acgagaagca gggctacgcc aaaggtgtcc 4320

taacgcaaaa actgggacct tggcgtcggc cggtggccta cctgtccaaa aagctagacc 4380

cagtagcagc tgggtggccc ccttgcctac ggatggtagc agccattgcc gtactgacaa 4440

aggatgcagg caagctaacc atgggacagc cactagtcat tctggccccc catgcagtag 4500

aggcactagt caaacaaccc cccgaccgct ggctttccaa cgcccggatg actcactatc 4560

aggccttgct tttggacacg gaccgggtcc agttcggacc ggtggtagcc ctgaacccgg 4620

ctacgctgct cccactgcct gaggaagggc tgcaacacaa ctgccttgat atcctggccg 4680

aagcccacgg aacccgaccc gacctaacgg accagccgct cccagacgcc gaccacacct 4740

ggtacacgga tggaagcagt ctcttacaag agggacagcg taaggcggga gctgcggtga 4800

ccaccgagac cgaggtaatc tgggctaaag ccctgccagc cgggacatcc gctcagcggg 4860

ctgaactgat agcactcacc caggccctaa agatggcaga aggtaagaag ctaaatgttt 4920

atactgatag ccgttatgct tttgctactg cccatatcca tggagaaata tacagaaggc 4980

gtgggttgct cacatcagaa ggcaaagaga tcaaaaataa agacgagatc ttggccctac 5040

taaaagccct ctttctgccc aaaagactta gcataatcca ttgtccagga catcaaaagg 5100

gacacagcgc cgaggctaga ggcaaccgga tggctgacca agcggcccga aaggcagcca 5160

tcacagagac tccagacacc tctaccctcc tcatagaaaa ttcatcaccc tacacctcag 5220

aacattttca ttacacagtg actgatataa aggacctaac caagttgggg gccatttatg 5280

ataaaacaaa gaagtattgg gtctaccaag gaaaacctgt gatgcctgac cagtttactt 5340

ttgaattatt agactttctt catcagctga ctcacctcag cttctcaaaa atgaaggctc 5400

tcctagagag aagccacagt ccctactaca tgctgaaccg ggatcgaaca ctcaaaaata 5460

tcactgagac ctgcaaagct tgtgcacaag tcaacgccag caagtctgcc gttaaacagg 5520

gaactagggt ccgcgggcat cggcccggca ctcattggga gatcgatttc accgagataa 5580

agcccggatt gtatggctat aaatatcttc tagtttttat agataccttt tctggctgga 5640

tagaagcctt cccaaccaag aaagaaaccg ccaaggtcgt aaccaagaag ctactagagg 5700

agatcttccc caggttcggc atgcctcagg tattgggaac tgacaatggg cctgccttcg 5760

tctccaaggt gagtcagaca gtggccgatc tgttggggat tgattggaaa ttacattgtg 5820

catacagacc ccaaagctca ggccaggtag aaagaatgaa tagaaccatc aaggagactt 5880

taactaaatt aacgcttgca actggctcta gagactgggt gctcctactc cccttagccc 5940

tgtaccgagc ccgcaacacg ccgggccccc atggcctcac cccatatgag atcttatatg 6000

gggcaccccc gccccttgta aacttccctg accctgacat gacaagagtt actaacagcc 6060

cctctctcca agctcactta caggctctct acttagtcca gcacgaagtc tggagacctc 6120

tggcggcagc ctaccaagaa caactggacc gaccggtggt acctcaccct taccgagtcg 6180

gcgacacagt gtgggtccgc cgacaccaga ctaagaacct agaacctcgc tggaaaggac 6240

cttacacagt cctgctgacc acccccaccg ccctcaaagt agacggcatc gcagcttgga 6300

tacacgccgc ccacgtgaag gctgccgacc ccgggggtgg accatcctct agactgacat 6360

ggcgcgttca acgctctcaa aaccccctca agataagatt aacccgtgga agcccttaat 6420

agtcatggga gtcctgttag gagtagggat ggcagagagc ccccatcagg tctttaatgt 6480

aacctggaga gtcaccaacc tgatgactgg gcgtaccgcc aatgccacct ccctcctggg 6540

aactgtacaa gatgccttcc caaaattata ttttgatcta tgtgatctgg tcggagagga 6600

gtgggaccct tcagaccagg aaccgtatgt cgggtatggc tgcaagtacc ccgcagggag 6660

acagcggacc cggacttttg acttttacgt gtgccctggg cataccgtaa agtcggggtg 6720

tgggggacca ggagagggct actgtggtaa atgggggtgt gaaaccaccg gacaggctta 6780

ctggaagccc acatcatcgt gggacctaat ctcccttaag cgcggtaaca cccccctgga 6840

cacgggatgc tctaaagttg cctgtggccc ctgctacgac ctctccaaag tatccaattc 6900

cttccaaggg gctactcgag ggggcagatg caaccctcta gtcctagaat tcactgatgc 6960

aggaaaaaag gctaactggg acgggcccaa atcgtgggga ctgagactgt accggacagg 7020

aacagatcct attaccatgt tctccctgac ccggcaggtc cttaatgtgg gaccccgagt 7080

ccccataggg cccaacccag tattacccga ccaaagactc ccttcctcac caatagagat 7140

tgtaccggct ccacagccac ctagccccct caataccagt tacccccctt ccactaccag 7200

tacaccctca acctccccta caagtccaag tgtcccacag ccacccccag gaactggaga 7260

tagactacta gctctagtca aaggagccta tcaggcgctt aacctcacca atcccgacaa 7320

gacccaagaa tgttggctgt gcttagtgtc gggacctcct tattacgaag gagtagcggt 7380

cgtgggcact tataccaatc attccaccgc tccggccaac tgtacggcca cttcccaaca 7440

taagcttacc ctatctgaag tgacaggaca gggcctatgc atgggggcag tacctaaaac 7500

tcaccaggcc ttatgtaaca ccacccaaag cgccggctca ggatcctact accttgcagc 7560

acccgccgga acaatgtggg cttgcagcac tggattgact ccctgcttgt ccaccacggt 7620

gctcaatcta accacagatt attgtgtatt agttgaactc tggcccagag taatttacca 7680

ctcccccgat tatatgtatg gtcagcttga acagcgtacc aaatataaaa gagagccagt 7740

atcattgacc ctggcccttc tactaggagg attaaccatg ggagggattg cagctggaat 7800

agggacgggg accactgcct taattaaaac ccagcagttt gagcagcttc atgccgctat 7860

ccagacagac ctcaacgaag tcgaaaagtc aattaccaac ctagaaaagt cactgacctc 7920

gttgtctgaa gtagtcctac agaaccgcag aggcctagat ttgctattcc taaaggaggg 7980

aggtctctgc gcagccctaa aagaagaatg ttgtttttat gcagaccaca cggggctagt 8040

gagagacagc atggccaaat taagagaaag gcttaatcag agacaaaaac tatttgagac 8100

aggccaagga tggttcgaag ggctgtttaa tagatccccc tggtttacca ccttaatctc 8160

caccatcatg ggacctctaa tagtactctt actgatctta ctctttggac cttgcattct 8220

caatcgatta gtccaatttg ttaaagacag gatatcagtg gtccaggctc tagttttgac 8280

tcaacaatat caccagctga agcctataga gtacgagcca tgacgtacgt tactggccga 8340

agccgcttgg aataaggccg gtgtgcgttt gtctatatgt tattttccac catattgccg 8400

tcttttggca atgtgagggc ccggaaacct ggccctgtct tcttgacgag cattcctagg 8460

ggtctttccc ctctcgccaa aggaatgcaa ggtctgttga atgtcgtgaa ggaagcagtt 8520

cctctggaag cttcttgaag acaaacaacg tctgtagcga ccctttgcag gcagcggaac 8580

cccccacctg gcgacaggtg cctctgcggc caaaagccac gtgtataaga tacacctgca 8640

aaggcggcac aaccccagtg ccacgttgtg agttggatag ttgtggaaag agtcaaatgg 8700

ctctcctcaa gcgtattcaa caaggggctg aaggatgccc agaaggtacc ccattgtatg 8760

ggatctgatc tggggcctcg gtgcacatgc tttacatgtg tttagtcgag gttaaaaaaa 8820

cgtctaggcc ccccgaacca cggggacgtg gttttccttt gaaaaacacg ataataccat 8880

ggtgacaggg ggaatggcaa gcaagtggga tcagaagggt atggacattg cctatgagga 8940

ggcggcctta ggttacaaag agggtggtgt tcctattggc ggatgtctta tcaataacaa 9000

agacggaagt gttctcggtc gtggtcacaa catgagattt caaaagggat ccgccacact 9060

acatggtgag atctccactt tggaaaactg tgggagatta gagggcaaag tgtacaaaga 9120

taccactttg tatacgacgc tgtctccatg cgacatgtgt acaggtgcca tcatcatgta 9180

tggtattcca cgctgtgttg tcggtgagaa cgttaatttc aaaagtaagg gcgagaaata 9240

tttacaaact agaggtcacg aggttgttgt tgttgacgat gagaggtgta aaaagatcat 9300

gaaacaattt atcgatgaaa gacctcagga ttggtttgaa gatattggtg agtaggcggc 9360

cgcgccatag ataaaataaa agattttatt tagtctccag aaaaaggggg gaatgaaaga 9420

ccccacctgt aggtttggca agctagctta agtaacgcca ttttgcaagg catggaaaaa 9480

tacataactg agaatagaga agttcagatc aaggtcagga acagatggaa cagctgaata 9540

tgggccaaac aggatatctg tggtaagcag ttcctgcccc ggctcagggc caagaacaga 9600

tggaacagct gaatatgggc caaacaggat atctgtggta agcagttcct gccccggctc 9660

agggccaaga acagatggtc cccagatgcg gtccagccct cagcagtttc tagagaacca 9720

tcagatgttt ccagggtgcc ccaaggacct gaaatgaccc tgtgccttgt ttaaactaac 9780

caatcagttc gcttctcgct tctgttcgcg cgcttctgct ccccgagctc aataaaagag 9840

cccacaaccc ctcactcggg gcgccagtcc tccgattgac tgagtcgccc gggtacccgt 9900

gtatccaata aaccctcttg cagttgcatc cgacttgtgg tctcgctgtt ccttgggagg 9960

gtctcctctg agtgattgac tacccgtcag cgggggtctt tcatttgggg gctcgtccgg 10020

gatcgggaga cccctgccca gggaccaccg acccaccacc gggaggtaag ctggctgcct 10080

cgcgcgtttc ggtgatgacg gtgaaaacct ctgacatgtg agcaaaaggc cagcaaaagg 10140

ccaggaaccg taaaaaggcc gcgttgctgg cgtttttcca taggctccgc ccccctgacg 10200

agcatcacaa aaatcgacgc tcaagtcaga ggtggcgaaa cccgacagga ctataaagat 10260

accaggcgtt tccccctgga agctccctcg tgcgctctcc tgttccgacc ctgccgctta 10320

ccggatacct gtccgccttt ctcccttcgg gaagcgtggc gctttctcaa tgctcacgct 10380

gtaggtatct cagttcggtg taggtcgttc gctccaagct gggctgtgtg cacgaacccc 10440

ccgttcagcc cgaccgctgc gccttatccg gtaactatcg tcttgagtcc aacccggtaa 10500

gacacgactt atcgccactg gcagcagcca ctggtaacag gattagcaga gcgaggtatg 10560

taggcggtgc tacagagttc ttgaagtggt ggcctaacta cggctacact agaaggacag 10620

tatttggtat ctgcgctctg ctgaagccag ttaccttcgg aaaaagagtt ggtagctctt 10680

gatccggcaa acaaaccacc gctggtagcg gtggtttttt tgtttgcaag cagcagatta 10740

cgcgcagaaa aaaaggatct caagaagatc ctttgatctt ttctacgggg tctgacgctc 10800

agtggaacga aaactcacgt taagggattt tggtcatgag attatcaaaa aggatcttca 10860

cctagatcct tttaaattaa aaatgaagtt ttaaatcaat ctaaagtata tatgagtaaa 10920

cttggtctga cagttaccaa tgcttaatca gtgaggcacc tatctcagcg atctgtctat 10980

ttcgttcatc catagttgcc tgactccccg tcgtgtagat aactacgata cgggagggct 11040

taccatctgg ccccagtgct gcaatgatac cgcgagaccc acgctcaccg gctccagatt 11100

tatcagcaat aaaccagcca gccggaaggg ccgagcgcag aagtggtcct gcaactttat 11160

ccgcctccat ccagtctatt aattgttgcc gggaagctag agtaagtagt tcgccagtta 11220

atagtttgcg caacgttgtt gccattgctg caggcatcgt ggtgtcacgc tcgtcgtttg 11280

gtatggcttc attcagctcc ggttcccaac gatcaaggcg agttacatga tcccccatgt 11340

tgtgcaaaaa agcggttagc tccttcggtc ctccgatcgt tgtcagaagt aagttggccg 11400

cagtgttatc actcatggtt atggcagcac tgcataattc tcttactgtc atgccatccg 11460

taagatgctt ttctgtgact ggtgagtact caaccaagtc attctgagaa tagtgtatgc 11520

ggcgaccgag ttgctcttgc ccggcgtcaa cacgggataa taccgcgcca catagcagaa 11580

ctttaaaagt gctcatcatt ggaaaacgtt cttcggggcg aaaactctca aggatcttac 11640

cgctgttgag atccagttcg atgtaaccca ctcgtgcacc caactgatct tcagcatctt 11700

ttactttcac cagcgtttct gggtgagcaa aaacaggaag gcaaaatgcc gcaaaaaagg 11760

gaataagggc gacacggaaa tgttgaatac tcatactctt cctttttcaa tattattgaa 11820

gcatttatca gggttattgt ctcatgagcg gatacatatt tgaatgtatt tagaaaaata 11880

aacaaatagg ggttccgcgc acatttcccc gaaaagtgcc acctgacgtc taagaaacca 11940

ttattatcat gacattaacc tataaaaata ggcgtatcac gaggcccttt cgtcttcaag 12000

aattcat 12007

<210> 22
<211> 11893
<212> DNA
<213> Artificial Sequence

<220>
<223> RCR Vector - pAC3-yCD2

<400> 22
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cggcgccagt cctccgattg 600

actgagtcgc ccgggtaccc gtgtatccaa taaaccctct tgcagttgca tccgacttgt 660

ggtctcgctg ttccttggga gggtctcctc tgagtgattg actacccgtc agcgggggtc 720

tttcatttgg gggctcgtcc gggatcggga gacccctgcc cagggaccac cgacccacca 780

ccgggaggta agctggccag caacttatct gtgtctgtcc gattgtctag tgtctatgac 840

tgattttatg cgcctgcgtc ggtactagtt agctaactag ctctgtatct ggcggacccg 900

tggtggaact gacgagttcg gaacacccgg ccgcaaccct gggagacgtc ccagggactt 960

cgggggccgt ttttgtggcc cgacctgagt ccaaaaatcc cgatcgtttt ggactctttg 1020

gtgcaccccc cttagaggag ggatatgtgg ttctggtagg agacgagaac ctaaaacagt 1080

tcccgcctcc gtctgaattt ttgctttcgg tttgggaccg aagccgcgcc gcgcgtcttg 1140

tctgctgcag catcgttctg tgttgtctct gtctgactgt gtttctgtat ttgtctgaaa 1200

atatgggcca gactgttacc actcccttaa gtttgacctt aggtcactgg aaagatgtcg 1260
agcggatcgc tcacaaccag tcggtagatg tcaagaagag acgttgggtt accttctgct 1320
ctgcagaatg gccaaccttt aacgtcggat ggccgcgaga cggcaccttt aaccgagacc 1380
tcatcaccca ggttaagatc aaggtctttt cacctggccc gcatggacac ccagaccagg 1440
tcccctacat cgtgacctgg gaagccttgg cttttgaccc ccctccctgg gtcaagccct 1500
ttgtacaccc taagcctccg cctcctcttc ctccatccgc cccgtctctc ccccttgaac 1560
ctcctcgttc gaccccgcct cgatcctccc tttatccagc cctcactcct tctctaggcg 1620
ccaaacctaa acctcaagtt ctttctgaca gtgggggccc gctcatcgac ctacttacag 1680
aagacccccc gccttatagg gacccaagac cacccccttc cgacagggac ggaaatggtg 1740
gagaagcgac ccctgcggga gaggcaccgg acccctcccc aatggcatct cgcctacgtg 1800
ggagacggga gccccctgtg gccgactcca ctacctcgca ggcattcccc ctccgcgcag 1860
gaggaaacgg acagcttcaa tactggccgt tctcctcttc tgacctttac aactggaaaa 1920
ataataaccc ttctttttct gaagatccag gtaaactgac agctctgatc gagtctgtcc 1980
tcatcaccca tcagcccacc tgggacgact gtcagcagct gttggggact ctgctgaccg 2040
gagaagaaaa acaacgggtg ctcttagagg ctagaaaggc ggtgcggggc gatgatgggc 2100
gccccactca actgcccaat gaagtcgatg ccgcttttcc cctcgagcgc ccagactggg 2160
attacaccac ccaggcaggt aggaaccacc tagtccacta tcgccagttg ctcctagcgg 2220
gtctccaaaa cgcgggcaga agccccacca atttggccaa ggtaaaagga ataacacaag 2280
ggcccaatga gtctccctcg gccttcctag agagacttaa ggaagcctat cgcaggtaca 2340
ctccttatga ccctgaggac ccagggcaag aaactaatgt gtctatgtct ttcatttggc 2400
agtctgcccc agacattggg agaaagttag agaggttaga agatttaaaa aacaagacgc 2460
ttggagattt ggttagagag gcagaaaaga tctttaataa acgagaaacc ccggaagaaa 2520
gagaggaacg tatcaggaga gaaacagagg aaaaagaaga acgccgtagg acagaggatg 2580
agcagaaaga gaaagaaaga gatcgtagga gacatagaga gatgagcaag ctattggcca 2640
ctgtcgttag tggacagaaa caggatagac agggaggaga acgaaggagg tcccaactcg 2700
atcgcgacca gtgtgcctac tgcaaagaaa aggggcactg ggctaaagat tgtcccaaga 2760
aaccacgagg acctcgggga ccaagacccc agacctccct cctgacccta gatgactagg 2820
gaggtcaggg tcaggagccc ccccctgaac ccaggataac cctcaaagtc ggggggcaac 2880
ccgtcacctt cctggtagat actggggccc aacactccgt gctgacccaa aatcctggac 2940
ccctaagtga taagtctgcc tgggtccaag gggctactgg aggaaagcgg tatcgctgga 3000
ccacggatcg caaagtacat ctagctaccg gtaaggtcac ccactctttc ctccatgtac 3060
cagactgtcc ctatcctctg ttaggaagag atttgctgac taaactaaaa gcccaaatcc 3120

actttgaggg atcaggagcc caggttatgg gaccaatggg gcagcccctg caagtgttga 3180

ccctaaatat agaagatgag tatcggctac atgagacctc aaaagagcca gatgtttctc 3240

tagggtccac atggctgtct gattttcctc aggcctgggc ggaaaccggg ggcatgggac 3300

tggcagttcg ccaagctcct ctgatcatac ctctgaaagc aacctctacc cccgtgtcca 3360

taaaacaata ccccatgtca caagaagcca gactggggat caagccccac atacagagac 3420

tgttggacca gggaatactg gtaccctgcc agtccccctg gaacacgccc ctgctacccg 3480

ttaagaaacc agggactaat gattataggc ctgtccagga tctgagagaa gtcaacaagc 3540

gggtggaaga catccacccc accgtgccca acccttacaa cctcttgagc gggctcccac 3600

cgtcccacca gtggtacact gtgcttgatt taaaggatgc ctttttctgc ctgagactcc 3660

accccaccag tcagcctctc ttcgcctttg agtggagaga tccagagatg ggaatctcag 3720

gacaattgac ctggaccaga ctcccacagg gtttcaaaaa cagtcccacc ctgtttgatg 3780

aggcactgca cagagaccta gcagacttcc ggatccagca cccagacttg atcctgctac 3840

agtacgtgga tgacttactg ctggccgcca cttctgagct agactgccaa caaggtactc 3900

gggccctgtt acaaacccta gggaacctcg ggtatcgggc ctcggccaag aaagcccaaa 3960

tttgccagaa acaggtcaag tatctggggt atcttctaaa agagggtcag agatggctga 4020

ctgaggccag aaaagagact gtgatggggc agcctactcc gaagacccct cgacaactaa 4080

gggagttcct agggacggca ggcttctgtc gcctctggat ccctgggttt gcagaaatgg 4140

cagccccctt gtaccctctc accaaaacgg ggactctgtt taattggggc ccagaccaac 4200

aaaaggccta tcaagaaatc aagcaagctc ttctaactgc cccagccctg gggttgccag 4260

atttgactaa gccctttgaa ctctttgtcg acgagaagca gggctacgcc aaaggtgtcc 4320

taacgcaaaa actgggacct tggcgtcggc cggtggccta cctgtccaaa aagctagacc 4380

cagtagcagc tgggtggccc ccttgcctac ggatggtagc agccattgcc gtactgacaa 4440

aggatgcagg caagctaacc atgggacagc cactagtcat tctggccccc catgcagtag 4500

aggcactagt caaacaaccc cccgaccgct ggctttccaa cgcccggatg actcactatc 4560

aggccttgct tttggacacg gaccgggtcc agttcggacc ggtggtagcc ctgaacccgg 4620

ctacgctgct cccactgcct gaggaagggc tgcaacacaa ctgccttgat atcctggccg 4680

aagcccacgg aacccgaccc gacctaacgg accagccgct cccagacgcc gaccacacct 4740

ggtacacgga tggaagcagt ctcttacaag agggacagcg taaggcggga gctgcggtga 4800

ccaccgagac cgaggtaatc tgggctaaag ccctgccagc cgggacatcc gctcagcggg 4860

ctgaactgat agcactcacc caggccctaa agatggcaga aggtaagaag ctaaatgttt 4920

atactgatag ccgttatgct tttgctactg cccatatcca tggagaaata tacagaaggc 4980

gtgggttgct cacatcagaa ggcaaagaga tcaaaaataa agacgagatc ttggccctac 5040
taaaagccct ctttctgccc aaaagactta gcataatcca ttgtccagga catcaaaagg 5100
gacacagcgc cgaggctaga ggcaaccgga tggctgacca agcggcccga aaggcagcca 5160
tcacagagac tccagacacc tctaccctcc tcatagaaaa ttcatcaccc tacacctcag 5220
aacattttca ttacacagtg actgatataa aggacctaac caagttgggg gccatttatg 5280
ataaaacaaa gaagtattgg gtctaccaag gaaaacctgt gatgcctgac cagtttactt 5340
ttgaattatt agactttctt catcagctga ctcacctcag cttctcaaaa atgaaggctc 5400
tcctagagag aagccacagt ccctactaca tgctgaaccg ggatcgaaca ctcaaaaata 5460
tcactgagac ctgcaaagct tgtgcacaag tcaacgccag caagtctgcc gttaaacagg 5520
gaactagggt ccgcgggcat cggcccggca ctcattggga gatcgatttc accgagataa 5580
agcccggatt gtatggctat aaatatcttc tagtttttat agataccttt tctggctgga 5640
tagaagcctt cccaaccaag aaagaaaccg ccaaggtcgt aaccaagaag ctactagagg 5700
agatcttccc caggttcggc atgcctcagg tattgggaac tgacaatggg cctgccttcg 5760
tctccaaggt gagtcagaca gtggccgatc tgttggggat tgattggaaa ttacattgtg 5820
catacagacc ccaaagctca ggccaggtag aaagaatgaa tagaaccatc aaggagactt 5880
taactaaatt aacgcttgca actggctcta gagactgggt gctcctactc cccttagccc 5940
tgtaccgagc ccgcaacacg ccgggccccc atggcctcac cccatatgag atcttatatg 6000
gggcaccccc gccccttgta aacttccctg accctgacat gacaagagtt actaacagcc 6060
cctctctcca agctcactta caggctctct acttagtcca gcacgaagtc tggagacctc 6120
tggcggcagc ctaccaagaa caactggacc gaccggtggt acctcaccct taccgagtcg 6180
gcgacacagt gtgggtccgc cgacaccaga ctaagaacct agaacctcgc tggaaaggac 6240
cttacacagt cctgctgacc acccccaccg ccctcaaagt agacggcatc gcagcttgga 6300
tacacgccgc ccacgtgaag gctgccgacc ccgggggtgg accatcctct agactgacat 6360
ggcgcgttca acgctctcaa aaccccctca agataagatt aacccgtgga agcccttaat 6420
agtcatggga gtcctgttag gagtagggat ggcagagagc ccccatcagg tctttaatgt 6480
aacctggaga gtcaccaacc tgatgactgg gcgtaccgcc aatgccacct ccctcctggg 6540
aactgtacaa gatgccttcc caaaattata ttttgatcta tgtgatctgg tcggagagga 6600
gtgggaccct tcagaccagg aaccgtatgt cgggtatggc tgcaagtacc ccgcagggag 6660
acagcggacc cggacttttg acttttacgt gtgccctggg cataccgtaa agtcggggtg 6720
tgggggacca ggagagggct actgtggtaa atgggggtgt gaaaccaccg gacaggctta 6780
ctggaagccc acatcatcgt gggacctaat ctcccttaag cgcggtaaca ccccctggga 6840
cacgggatgc tctaaagttg cctgtggccc ctgctacgac ctctccaaag tatccaattc 6900

cttccaaggg gctactcgag ggggcagatg caaccctcta gtcctagaat tcactgatgc 6960

aggaaaaaag gctaactggg acgggcccaa atcgtgggga ctgagactgt accggacagg 7020

aacagatcct attaccatgt tctccctgac ccggcaggtc cttaatgtgg gaccccgagt 7080

ccccataggg cccaacccag tattacccga ccaaagactc ccttcctcac caatagagat 7140

tgtaccggct ccacagccac ctagccccct caataccagt tacccccctt ccactaccag 7200

tacaccctca acctccccta caagtccaag tgtcccacag ccacccccag gaactggaga 7260

tagactacta gctctagtca aaggagccta tcaggcgctt aacctcacca atcccgacaa 7320

gacccaagaa tgttggctgt gcttagtgtc gggacctcct tattacgaag gagtagcggt 7380

cgtgggcact tataccaatc attccaccgc tccggccaac tgtacggcca cttcccaaca 7440

taagcttacc ctatctgaag tgacaggaca gggcctatgc atgggggcag tacctaaaac 7500

tcaccaggcc ttatgtaaca ccacccaaag cgccggctca ggatcctact accttgcagc 7560

acccgccgga acaatgtggg cttgcagcac tggattgact ccctgcttgt ccaccacggt 7620

gctcaatcta accacagatt attgtgtatt agttgaactc tggcccagag taatttacca 7680

ctcccccgat tatatgtatg gtcagcttga acagcgtacc aaatataaaa gagagccagt 7740

atcattgacc ctggcccttc tactaggagg attaaccatg ggagggattg cagctggaat 7800

agggacgggg accactgcct taattaaaac ccagcagttt gagcagcttc atgccgctat 7860

ccagacagac ctcaacgaag tcgaaaagtc aattaccaac ctagaaaagt cactgacctc 7920

gttgtctgaa gtagtcctac agaaccgcag aggcctagat ttgctattcc taaaggaggg 7980

aggtctctgc gcagccctaa aagaagaatg ttgtttttat gcagaccaca cggggctagt 8040

gagagacagc atggccaaat taagagaaag gcttaatcag agacaaaaac tatttgagac 8100

aggccaagga tggttcgaag ggctgtttaa tagatccccc tggtttacca ccttaatctc 8160

caccatcatg ggacctctaa tagtactctt actgatctta ctctttggac cttgcattct 8220

caatcgattg gtccaatttg ttaaagacag gatctcagtg gtccaggctc tggttttgac 8280

tcagcaatat caccagctaa aacccataga gtacgagcca tgaacgcgtt actggccgaa 8340

gccgcttgga ataaggccgg tgtgcgtttg tctatatgtt attttccacc atattgccgt 8400

cttttggcaa tgtgagggcc cggaaacctg gccctgtctt cttgacgagc attcctaggg 8460

gtctttcccc tctcgccaaa ggaatgcaag gtctgttgaa tgtcgtgaag gaagcagttc 8520

ctctggaagc ttcttgaaga caaacaacgt ctgtagcgac cctttgcagg cagcggaacc 8580

ccccacctgg cgacaggtgc ctctgcggcc aaaagccacg tgtataagat acacctgcaa 8640

aggcggcaca accccagtgc cacgttgtga gttggatagt tgtggaaaga gtcaaatggc 8700

tctcctcaag cgtattcaac aaggggctga aggatgccca gaaggtaccc cattgtatgg 8760

gatctgatct ggggcctcgg tgcacatgct ttacatgtgt ttagtcgagg ttaaaaaaac 8820

gtctaggccc cccgaaccac ggggacgtgg ttttcctttg aaaaacacga ttataaatgg 8880

tgaccggcgg catggcctcc aagtgggatc aaaagggcat ggatatcgct tacgaggagg 8940

ccctgctggg ctacaaggag ggcggcgtgc ctatcggcgg ctgtctgatc aacaacaagg 9000

acggcagtgt gctgggcagg ggccacaaca tgaggttcca gaagggctcc gccaccctgc 9060

acggcgagat ctccaccctg gagaactgtg gcaggctgga gggcaaggtg tacaaggaca 9120

ccaccctgta caccaccctg tccccttgtg acatgtgtac cggcgctatc atcatgtacg 9180

gcatccctag gtgtgtgatc ggcgagaacg tgaacttcaa gtccaagggc gagaagtacc 9240

tgcaaaccag gggccacgag gtggtggttg ttgacgatga gaggtgtaag aagctgatga 9300

agcagttcat cgacgagagg cctcaggact ggttcgagga tatcggcgag taagcggccg 9360

cagataaaat aaaagatttt atttagtctc cagaaaaagg ggggaatgaa agaccccacc 9420

tgtaggtttg gcaagctagc ttaagtaacg ccattttgca aggcatggaa aaatacataa 9480

ctgagaatag agaagttcag atcaaggtca ggaacagatg gaacagctga atatgggcca 9540

aacaggatat ctgtggtaag cagttcctgc cccggctcag ggccaagaac agatggaaca 9600

gctgaatatg ggccaaacag gatatctgtg gtaagcagtt cctgccccgg ctcagggcca 9660

agaacagatg gtccccagat gcggtccagc cctcagcagt ttctagagaa ccatcagatg 9720

tttccagggt gccccaagga cctgaaatga ccctgtgcct tatttgaact aaccaatcag 9780

ttcgcttctc gcttctgttc gcgcgcttct gctccccgag ctcaataaaa gagcccacaa 9840

cccctcactc ggggcgccag tcctccgatt gactgagtcg cccgggtacc cgtgtatcca 9900

ataaaccctc ttgcagttgc atccgacttg tggtctcgct gttccttggg agggtctcct 9960

ctgagtgatt gactacccgt cagcgggggt ctttcattac atgtgagcaa aaggccagca 10020

aaaggccagg aaccgtaaaa aggccgcgtt gctggcgttt ttccataggc tccgcccccc 10080

tgacgagcat cacaaaaatc gacgctcaag tcagaggtgg cgaaacccga caggactata 10140

aagataccag gcgtttcccc ctggaagctc cctcgtgcgc tctcctgttc cgaccctgcc 10200

gcttaccgga tacctgtccg cctttctccc ttcgggaagc gtggcgcttt ctcatagctc 10260

acgctgtagg tatctcagtt cggtgtaggt cgttcgctcc aagctgggct gtgtgcacga 10320

accccccgtt cagcccgacc gctgcgcctt atccggtaac tatcgtcttg agtccaaccc 10380

ggtaagacac gacttatcgc cactggcagc agccactggt aacaggatta gcagagcgag 10440

gtatgtaggc ggtgctacag agttcttgaa gtggtggcct aactacggct acactagaag 10500

gacagtattt ggtatctgcg ctctgctgaa gccagttacc ttcggaaaaa gagttggtag 10560

ctcttgatcc ggcaaacaaa ccaccgctgg tagcggtggt ttttttgttt gcaagcagca 10620

gattacgcgc agaaaaaaag gatctcaaga agatcctttg atcttttcta cggggtctga 10680

cgctcagtgg aacgaaaact cacgttaagg gattttggtc atgagattat caaaaaggat 10740

cttcacctag atccttttaa attaaaaatg aagttttaaa tcaatctaaa gtatatatga 10800

gtaaacttgg tctgacagtt accaatgctt aatcagtgag gcacctatct cagcgatctg 10860

tctatttcgt tcatccatag ttgcctgact ccccgtcgtg tagataacta cgatacggga 10920

gggcttacca tctggcccca gtgctgcaat gataccgcga gacccacgct caccggctcc 10980

agatttatca gcaataaacc agccagccgg aagggccgag cgcagaagtg gtcctgcaac 11040

tttatccgcc tccatccagt ctattaattg ttgccgggaa gctagagtaa gtagttcgcc 11100

agttaatagt ttgcgcaacg ttgttgccat tgctgcaggc atcgtggtgt cacgctcgtc 11160

gtttggtatg gcttcattca gctccggttc ccaacgatca aggcgagtta catgatcccc 11220

catgttgtgc aaaaaagcgg ttagctcctt cggtcctccg atcgttgtca gaagtaagtt 11280

ggccgcagtg ttatcactca tggttatggc agcactgcat aattctctta ctgtcatgcc 11340

atccgtaaga tgcttttctg tgactggtga gtactcaacc aagtcattct gagaatagtg 11400

tatgcggcga ccgagttgct cttgcccggc gtcaacacgg gataataccg cgccacatag 11460

cagaacttta aaagtgctca tcattggaaa acgttcttcg gggcgaaaac tctcaaggat 11520

cttaccgctg ttgagatcca gttcgatgta acccactcgt gcacccaact gatcttcagc 11580

atcttttact ttcaccagcg tttctgggtg agcaaaaaca ggaaggcaaa atgccgcaaa 11640

aaagggaata agggcgacac ggaaatgttg aatactcata ctcttccttt ttcaatatta 11700

ttgaagcatt tatcagggtt attgtctcat gagcggatac atatttgaat gtatttagaa 11760

aaataaacaa ataggggttc cgcgcacatt tccccgaaaa gtgccacctg acgtctaaga 11820

aaccattatt atcatgacat taacctataa aaataggcgt atcacgaggc cctttcgtct 11880

tcaagaattc cat 11893

<210> 23
<211> 4473
<212> DNA
<213> Homo sapiens

<220>
<221> CDS
<222> (175)..(3942)

<400> 23
aaggggaggt aaccctggcc cctttggtcg gggccccggg cagccgcgcg ccccttccca 60
cggggccctt tactgcgccg cgcgcccggc ccccacccct cgcagcaccc cgcgccccgc 120
gccctcccag ccgggtccag ccggagccat ggggccggag ccgcagtgag cacc atg 177
gag ctg gcg gcc ttg tgc cgc tgg ggg ctc ctc ctc gcc ctc ttg ccc     225
ccc gga gcc gcg agc acc caa gtg tgc acc ggc aca gac atg aag ctg     273
cgg ctc cct gcc agt ccc gag acc cac ctg gac atg ctc cgc cac ctc     321
tac cag ggc tgc cag gtg gtg cag gga aac ctg gaa ctc acc tac ctg     369
ccc acc aat gcc agc ctg tcc ttc ctg cag gat atc cag gag gtg cag     417
ggc tac gtg ctc atc gct cac aac caa gtg agg cag gtc cca ctg cag     465
agg ctg cgg att gtg cga ggc acc cag ctc ttt gag gac aac tat gcc     513
ctg gcc gtg cta gac aat gga gac ccg ctg aac aat acc acc cct gtc     561
aca ggg gcc tcc cca gga ggc ctg cgg gag ctg cag ctt cga agc ctc     609
aca gag atc ttg aaa gga ggg gtc ttg atc cag cgg aac ccc cag ctc     657
tgc tac cag gac acg att ttg tgg aag gac atc ttc cac aag aac aac     705
cag ctg gct ctc aca ctg ata gac acc aac cgc tct cgg gcc tgc cac     753
ccc tgt tct ccg atg tgt aag ggc tcc cgc tgc tgg gga gag agt tct     801
gag gat tgt cag agc ctg acg cgc act gtc tgt gcc ggt ggc tgt gcc     849
cgc tgc aag ggg cca ctg ccc act gac tgc tgc cat gag cag tgt gct     897
gcc ggc tgc acg ggc ccc aag cac tct gac tgc ctg gcc tgc ctc cac     945
ttc aac cac agt ggc atc tgt gag ctg cac tgc cca gcc ctg gtc acc 993
tac aac aca gac acg ttt gag tcc atg ccc aat ccc gag ggc cgg tat 1041
aca ttc ggc gcc agc tgt gtg act gcc tgt ccc tac aac tac ctt tct 1089
acg gac gtg gga tcc tgc acc ctc gtc tgc ccc ctg cac aac caa gag 1137
gtg aca gca gag gat gga aca cag cgg tgt gag aag tgc agc aag ccc 1185
tgt gcc cga gtg tgc tat ggt ctg ggc atg gag cac ttg cga gag gtg 1233
agg gca gtt acc agt gcc aat atc cag gag ttt gct ggc tgc aag aag 1281
atc ttt ggg agc ctg gca ttt ctg ccg gag agc ttt gat ggg gac cca 1329
gcc tcc aac act gcc ccg ctc cag cca gag cag ctc caa gtg ttt gag 1377
act ctg gaa gag atc aca ggt tac cta tac atc tca gca tgg ccg gac 1425
agc ctg cct gac ctc agc gtc ttc cag aac ctg caa gta atc cgg gga 1473
cga att ctg cac aat ggc gcc tac tcg ctg acc ctg caa ggg ctg ggc 1521
atc agc tgg ctg ggg ctg cgc tca ctg agg gaa ctg ggc agt gga ctg 1569
gcc ctc atc cac cat aac acc cac ctc tgc ttc gtg cac acg gtg ccc 1617
tgg gac cag ctc ttt cgg aac ccg cac caa gct ctg ctc cac act gcc 1665
aac cgg cca gag gac gag tgt gtg ggc gag ggc ctg gcc tgc cac cag 1713
ctg tgc gcc cga ggg cac tgc tgg ggt cca ggg ccc acc cag tgt gtc        1761
aac tgc agc cag ttc ctt cgg ggc cag gag tgc gtg gag gaa tgc cga        1809
gta ctg cag ggg ctc ccc agg gag tat gtg aat gcc agg cac tgt ttg        1857
ccg tgc cac cct gag tgt cag ccc cag aat ggc tca gtg acc tgt ttt        1905
gga ccg gag gct gac cag tgt gtg gcc tgt gcc cac tat aag gac cct        1953
ccc ttc tgc gtg gcc cgc tgc ccc agc ggt gtg aaa cct gac ctc tcc        2001
tac atg ccc atc tgg aag ttt cca gat gag gag ggc gca tgc cag cct        2049
tgc ccc atc aac tgc acc cac tcc tgt gtg gac ctg gat gac aag ggc        2097
tgc ccc gcc gag cag aga gcc agc cct ctg acg tcc atc atc tct gcg        2145
gtg gtt ggc att ctg ctg gtc gtg gtc ttg ggg gtg gtc ttt ggg atc        2193
ctc atc aag cga cgg cag cag aag atc cgg aag tac acg atg cgg aga        2241
ctg ctg cag gaa acg gag ctg gtg gag ccg ctg aca cct agc gga gcg        2289
atg ccc aac cag gcg cag atg cgg atc ctg aaa gag acg gag ctg agg        2337
aag gtg aag gtg ctt gga tct ggc gct ttt ggc aca gtc tac aag ggc        2385
atc tgg atc cct gat ggg gag aat gtg aaa att cca gtg gcc atc aaa        2433
gtg ttg agg gaa aac aca tcc ccc aaa gcc aac aaa gaa atc tta gac        2481
gaa gca tac gtg atg gct ggt gtg ggc tcc cca tat gtc tcc cgc ctt      2529
ctg ggc atc tgc ctg aca tcc acg gtg cag ctg gtg aca cag ctt atg      2577
ccc tat ggc tgc ctc tta gac cat gtc cgg gaa aac cgc gga cgc ctg      2625
ggc tcc cag gac ctg ctg aac tgg tgt atg cag att gcc aag ggg atg      2673
agc tac ctg gag gat gtg cgg ctc gta cac agg gac ttg gcc gct cgg      2721
aac gtg ctg gtc aag agt ccc aac cat gtc aaa att aca gac ttc ggg      2769
ctg gct cgg ctg ctg gac att gac gag aca gag tac cat gca gat ggg      2817
ggc aag gtg ccc atc aag tgg atg gcg ctg gag tcc att ctc cgc cgg      2865
cgg ttc acc cac cag agt gat gtg tgg agt tat ggt gtg act gtg tgg      2913
gag ctg atg act ttt ggg gcc aaa cct tac gat ggg atc cca gcc cgg      2961
gag atc cct gac ctg ctg gaa aag ggg gag cgg ctg ccc cag ccc ccc      3009
atc tgc acc att gat gtc tac atg atc atg gtc aaa tgt tgg atg att      3057
gac tct gaa tgt cgg cca aga ttc cgg gag ttg gtg tct gaa ttc tcc      3105
cgc atg gcc agg gac ccc cag cgc ttt gtg gtc atc cag aat gag gac      3153
ttg ggc cca gcc agt ccc ttg  gac agc acc ttc tac  cgc tca ctg ctg      3201
gag gac gat gac atg ggg gac ctg gtg gat gct gag gag tat ctg 3246
gta ccc cag cag ggc ttc ttc tgt cca gac cct gcc ccg ggc gct 3291
ggg ggc atg gtc cac cac agg cac cgc agc tca tct acc agg agt 3336
ggc ggt ggg gac ctg aca cta ggg ctg gag ccc tct gaa gag gag 3381
gcc ccc agg tct cca ctg gca ccc tcc gaa ggg gct ggc tcc gat 3426
gta ttt gat ggt gac ctg gga atg ggg gca gcc aag ggg ctg caa 3471
agc ctc ccc aca cat gac ccc agc cct cta cag cgg tac agt gag 3516
gac ccc aca gta ccc ctg ccc tct gag act gat ggc tac gtt gcc 3561
ccc ctg acc tgc agc ccc cag cct gaa tat gtg aac cag cca gat 3606
gtt cgg ccc cag ccc cct tcg ccc cga gag ggc cct ctg cct gct 3651
gcc cga cct gct ggt gcc act ctg gaa agg ccc aag act ctc tcc 3696
cca ggg aag aat ggg gtc gtc aaa gac gtt ttt gcc ttt ggg ggt 3741
gcc gtg gag aac ccc gag tac ttg aca ccc cag gga gga gct gcc 3786
cct cag ccc cac cct cct cct gcc ttc agc cca gcc ttc gac aac 3831
ctc tat tac tgg gac cag gac cca cca gag cgg ggg gct cca ccc 3876
agc acc ttc aaa ggg aca cct acg gca gag aac cca gag tac ctg 3921
ggt ctg gac gtg cca gtg tga accagaaggc caagtccgca gaagccctga 3972
tgtgtcctca gggagcaggg aaggcctgac ttctgctggc atcaagaggt gggagggccc 4032
tccgaccact tccaggggaa cctgccatgc caggaacctg tcctaaggaa ccttccttcc 4092
tgcttgagtt cccagatggc tggaaggggt ccagcctcgt tggaagagga acagcactgg 4152
ggagtctttg tggattctga ggccctgccc aatgagactc tagggtccag tggatgccac 4212
agcccagctt ggccctttcc ttccagatcc tgggtactga aagccttagg gaagctggcc 4272
tgagagggga agcggcccta agggagtgtc taagaacaaa agcgacccat tcagagactg 4332
tccctgaaac ctagtactgc cccccatgag gaaggaacag caatggtgtc agtatccagg 4392
ctttgtacag agtgcttttc tgtttagttt ttactttttt tgttttgttt ttttaaagat 4452
gaaataaaga cccaggggga g 4473

<210> 24
<211> 1255
<212> PRT
<213> Homo sapiens

<400> 24

<210> 25
<211> 1212
<212> DNA
<213> Homo sapiens

<220>
<221> CDS
<222> (1)..(1212)

<400> 25
atg aca gcc atc atc aaa gag atc gtt agc aga aac aaa agg aga tat 48
caa gag gat gga ttc gac tta gac ttg acc tat att tat cca aac att 96
att gct atg gga ttt cct gca gaa aga ctt gaa ggc gta tac agg aac 144
aat att gat gat gta gta agg ttt ttg gat tca aag cat aaa aac cat 192
tac aag ata tac aat ctt tgt gct gaa aga cat tat gac acc gcc aaa     240
ttt aat tgc aga gtt gca caa tat cct ttt gaa gac cat aac cca cca     288
cag cta gaa ctt atc aaa ccc ttt tgt gaa gat ctt gac caa tgg cta     336
agt gaa gat gac aat cat gtt gca gca att cac tgt aaa gct gga aag     384
gga cga act ggt gta atg ata tgt gca tat tta tta cat cgg ggc aaa     432
ttt tta aag gca caa gag gcc cta gat ttc tat ggg gaa gta agg acc     480
aga gac aaa aag gga gta act att ccc agt cag agg cgc tat gtg tat     528
tat tat agc tac ctg tta aag aat cat ctg gat tat aga cca gtg gca     576
ctg ttg ttt cac aag atg atg ttt gaa act att cca atg ttc agt ggc     624
gga act tgc aat cct cag ttt gtg gtc tgc cag cta aag gtg aag ata     672
tat tcc tcc aat tca gga ccc aca cga cgg gaa gac aag ttc atg tac     720
ttt gag ttc cct cag ccg tta cct gtg tgt ggt gat atc aaa gta gag     768
ttc ttc cac aaa cag aac aag atg cta aaa aag gac aaa atg ttt cac     816
ttt tgg gta aat aca ttc ttc ata cca gga cca gag gaa acc tca gaa     864
aaa gta gaa aat gga agt cta tgt gat caa gaa atc gat agc att tgc     912
agt ata gag cgt gca gat aat gac aag gaa tat cta gta ctt act tta     960
aca aaa aat gat ctt gac aaa gca aat aaa gac aaa gcc aac cga tac          1008
ttt tct cca aat ttt aag gtg aag ctg tac ttc aca aaa aca gta gag          1056
gag ccg tca aat cca gag gct agc agt tca act tct gta aca cca gat          1104
gtt agt gac aat gaa cct gat cat tat aga tat tct gac acc act gac          1152
tct gat cca gag aat gaa cct ttt gat gaa gat cag cat aca caa att          1200
aca aaa gtc tga                                                          1212

<210>  26
<211>  403
<212>  PRT
<213>  Homo sapiens

<400>  26

<210>  27
<211>  597
<212>  DNA
<213>  Homo sapiens

<220>
<221>  CDS
<222>  (1)..(597)

<400>  27
atg tca aac gtg cga gtg tct aac ggg agc cct agc ctg gag cgg atg      48
Met Ser Asn Val Arg Val Ser Asn Gly Ser Pro Ser Leu Glu Arg Met
1               5                   10                  15

gac gcc agg cag gcg gag cac ccc aag ccc tcg gcc tgc agg aac ctc      96
Asp Ala Arg Gln Ala Glu His Pro Lys Pro Ser Ala Cys Arg Asn Leu
            20                  25                  30

ttc ggc ccg gtg gac cac gaa gag tta acc cgg gac ttg gag aag cac      144
Phe Gly Pro Val Asp His Glu Glu Leu Thr Arg Asp Leu Glu Lys His
        35                  40                  45

tgc aga gac atg gaa gag gcg agc cag cgc aag tgg aat ttc gat ttt      192
Cys Arg Asp Met Glu Glu Ala Ser Gln Arg Lys Trp Asn Phe Asp Phe
    50                  55                  60

cag aat cac aaa ccc cta gag ggc aag tac gag tgg caa gag gtg gag      240
Gln Asn His Lys Pro Leu Glu Gly Lys Tyr Glu Trp Gln Glu Val Glu
65                  70                  75                  80

aag ggc agc ttg ccc gag ttc tac tac aga ccc ccg cgg ccc ccc aaa      288
Lys Gly Ser Leu Pro Glu Phe Tyr Tyr Arg Pro Pro Arg Pro Pro Lys
                85                  90                  95

ggt gcc tgc aag gtg ccg gcg cag gag agc cag gat gtc agc ggg agc      336
Gly Ala Cys Lys Val Pro Ala Gln Glu Ser Gln Asp Val Ser Gly Ser
            100                 105                 110

cgc ccg gcg gcg cct tta att ggg gct ccg gct aac tct gag gac acg      384
Arg Pro Ala Ala Pro Leu Ile Gly Ala Pro Ala Asn Ser Glu Asp Thr
        115                 120                 125

cat ttg gtg gac cca aag act gat ccg tcg gac agc cag acg ggg tta      432
His Leu Val Asp Pro Lys Thr Asp Pro Ser Asp Ser Gln Thr Gly Leu
    130                 135                 140

gcg gag caa tgc gca gga ata agg aag cga cct gca acc gac gat tct      480
Ala Glu Gln Cys Ala Gly Ile Arg Lys Arg Pro Ala Thr Asp Asp Ser
145                 150                 155                 160

tct act caa aac aaa aga gcc aac aga aca gaa gaa aat gtt tca gac      528

Ser Thr Gln Asn Lys Arg Ala Asn Arg Thr Glu Glu Asn Val Ser Asp
                165                 170                 175

ggt tcc cca aat gcc ggt tct gtg gag cag acg ccc aag aag cct ggc       576
Gly Ser Pro Asn Ala Gly Ser Val Glu Gln Thr Pro Lys Lys Pro Gly
            180                 185                 190

ctc aga aga cgt caa acg taa                                           597
Leu Arg Arg Arg Gln Thr
        195

<210>  28
<211>  198
<212>  PRT
<213>  Homo sapiens

<400>  28

Met Ser Asn Val Arg Val Ser Asn Gly Ser Pro Ser Leu Glu Arg Met
1               5                   10                  15

Asp Ala Arg Gln Ala Glu His Pro Lys Pro Ser Ala Cys Arg Asn Leu
            20                  25                  30

Phe Gly Pro Val Asp His Glu Glu Leu Thr Arg Asp Leu Glu Lys His
        35                  40                  45

Cys Arg Asp Met Glu Glu Ala Ser Gln Arg Lys Trp Asn Phe Asp Phe
    50                  55                  60

Gln Asn His Lys Pro Leu Glu Gly Lys Tyr Glu Trp Gln Glu Val Glu
65                  70                  75                  80

Lys Gly Ser Leu Pro Glu Phe Tyr Tyr Arg Pro Pro Arg Pro Pro Lys
                85                  90                  95

Gly Ala Cys Lys Val Pro Ala Gln Glu Ser Gln Asp Val Ser Gly Ser
            100                 105                 110

Arg Pro Ala Ala Pro Leu Ile Gly Ala Pro Ala Asn Ser Glu Asp Thr
        115                 120                 125

His Leu Val Asp Pro Lys Thr Asp Pro Ser Asp Ser Gln Thr Gly Leu
    130                 135                 140

Ala Glu Gln Cys Ala Gly Ile Arg Lys Arg Pro Ala Thr Asp Asp Ser
145                 150                 155                 160

Ser Thr Gln Asn Lys Arg Ala Asn Arg Thr Glu Glu Asn Val Ser Asp
                165                 170                 175

Gly Ser Pro Asn Ala Gly Ser Val Glu Gln Thr Pro Lys Lys Pro Gly
            180                 185                 190

Leu Arg Arg Arg Gln Thr
        195

<210>  29
<211>  894
<212>  DNA
<213>  Homo sapiens

<220>
<221>  CDS
<222>  (1)..(894)

<400>  29
atg aca aca ccc aga aat tca gta aat ggg act ttc ccg gca gag cca        48
Met Thr Thr Pro Arg Asn Ser Val Asn Gly Thr Phe Pro Ala Glu Pro
1               5                   10                  15

atg aaa ggc cct att gct atg caa tct ggt cca aaa cca ctc ttc agg        96
Met Lys Gly Pro Ile Ala Met Gln Ser Gly Pro Lys Pro Leu Phe Arg
            20                  25                  30

agg atg tct tca ctg gtg ggc ccc acg caa agc ttc ttc atg agg gaa       144
Arg Met Ser Ser Leu Val Gly Pro Thr Gln Ser Phe Phe Met Arg Glu
        35                  40                  45

tct aag act ttg ggg gct gtc cag att atg aat ggg ctc ttc cac att       192
Ser Lys Thr Leu Gly Ala Val Gln Ile Met Asn Gly Leu Phe His Ile
    50                  55                  60

gcc ctg ggg ggt ctt ctg atg atc cca gca ggg atc tat gca ccc atc       240
Ala Leu Gly Gly Leu Leu Met Ile Pro Ala Gly Ile Tyr Ala Pro Ile
65                  70                  75                  80

tgt gtg act gtg tgg tac cct ctc tgg gga ggc att atg tat att att       288
Cys Val Thr Val Trp Tyr Pro Leu Trp Gly Gly Ile Met Tyr Ile Ile
                85                  90                  95

tcc gga tca ctc ttg gca gca acg gag aaa aac tct agg aag tgt ttg       336
Ser Gly Ser Leu Leu Ala Ala Thr Glu Lys Asn Ser Arg Lys Cys Leu
            100                 105                 110

gtc aaa gga aaa atg ata atg aat tca ttg agc ctc ttt gct gcc att       384
Val Lys Gly Lys Met Ile Met Asn Ser Leu Ser Leu Phe Ala Ala Ile
        115                 120                 125

tct gga atg att ctt tca atc atg gac ata ctt aat att aaa att tcc       432
Ser Gly Met Ile Leu Ser Ile Met Asp Ile Leu Asn Ile Lys Ile Ser
    130                 135                 140

cat ttt tta aaa atg gag agt ctg aat ttt att aga gct cac aca cca       480
His Phe Leu Lys Met Glu Ser Leu Asn Phe Ile Arg Ala His Thr Pro
145                 150                 155                 160

tat att aac ata tac aac tgt gaa cca gct aat ccc tct gag aaa aac       528
Tyr Ile Asn Ile Tyr Asn Cys Glu Pro Ala Asn Pro Ser Glu Lys Asn
                165                 170                 175

tcc cca tct acc caa tac tgt tac agc ata caa tct ctg ttc ttg ggc 576
Ser Pro Ser Thr Gln Tyr Cys Tyr Ser Ile Gln Ser Leu Phe Leu Gly
            180                 185                 190

att ttg tca gtg atg ctg atc ttt gcc ttc ttc cag gaa ctt gta ata 624
Ile Leu Ser Val Met Leu Ile Phe Ala Phe Phe Gln Glu Leu Val Ile
        195                 200                 205

gct ggc atc gtt gag aat gaa tgg aaa aga acg tgc tcc aga ccc aaa 672
Ala Gly Ile Val Glu Asn Glu Trp Lys Arg Thr Cys Ser Arg Pro Lys
    210                 215                 220

tct aac ata gtt ctc ctg tca gca gaa gaa aaa aaa gaa cag act att 720
Ser Asn Ile Val Leu Leu Ser Ala Glu Glu Lys Lys Glu Gln Thr Ile
225                 230                 235                 240

gaa ata aaa gaa gaa gtg gtt ggg cta act gaa aca tct tcc caa cca 768
Glu Ile Lys Glu Glu Val Val Gly Leu Thr Glu Thr Ser Ser Gln Pro
                245                 250                 255

aag aat gaa gaa gac att gaa att att cca atc caa gaa gag gaa gaa 816
Lys Asn Glu Glu Asp Ile Glu Ile Ile Pro Ile Gln Glu Glu Glu Glu
            260                 265                 270

gaa gaa aca gag acg aac ttt cca gaa cct ccc caa gat cag gaa tcc 864
Glu Glu Thr Glu Thr Asn Phe Pro Glu Pro Pro Gln Asp Gln Glu Ser
        275                 280                 285

tca cca ata gaa aat gac agc tct cct taa 894
Ser Pro Ile Glu Asn Asp Ser Ser Pro
    290                 295

<210> 30
<211> 297
<212> PRT
<213> Homo sapiens

<400> 30

Met Thr Thr Pro Arg Asn Ser Val Asn Gly Thr Phe Pro Ala Glu Pro
1               5                   10                  15

Met Lys Gly Pro Ile Ala Met Gln Ser Gly Pro Lys Pro Leu Phe Arg
            20                  25                  30

Arg Met Ser Ser Leu Val Gly Pro Thr Gln Ser Phe Phe Met Arg Glu
        35                  40                  45

Ser Lys Thr Leu Gly Ala Val Gln Ile Met Asn Gly Leu Phe His Ile
    50                  55                  60

Ala Leu Gly Gly Leu Leu Met Ile Pro Ala Gly Ile Tyr Ala Pro Ile
65                  70                  75                  80

Cys Val Thr Val Trp Tyr Pro Leu Trp Gly Gly Ile Met Tyr Ile Ile
                85                  90                  95

Ser Gly Ser Leu Leu Ala Ala Thr Glu Lys Asn Ser Arg Lys Cys Leu
            100                 105                 110

Val Lys Gly Lys Met Ile Met Asn Ser Leu Ser Leu Phe Ala Ala Ile
        115                 120                 125

Ser Gly Met Ile Leu Ser Ile Met Asp Ile Leu Asn Ile Lys Ile Ser
    130                 135                 140

His Phe Leu Lys Met Glu Ser Leu Asn Phe Ile Arg Ala His Thr Pro
145                 150                 155                 160

Tyr Ile Asn Ile Tyr Asn Cys Glu Pro Ala Asn Pro Ser Glu Lys Asn
                165                 170                 175

Ser Pro Ser Thr Gln Tyr Cys Tyr Ser Ile Gln Ser Leu Phe Leu Gly
            180                 185                 190

Ile Leu Ser Val Met Leu Ile Phe Ala Phe Phe Gln Glu Leu Val Ile
        195                 200                 205

Ala Gly Ile Val Glu Asn Glu Trp Lys Arg Thr Cys Ser Arg Pro Lys
    210                 215                 220

Ser Asn Ile Val Leu Leu Ser Ala Glu Glu Lys Lys Glu Gln Thr Ile
225                 230                 235                 240

Glu Ile Lys Glu Glu Val Val Gly Leu Thr Glu Thr Ser Ser Gln Pro
                245                 250                 255

Lys Asn Glu Glu Asp Ile Glu Ile Ile Pro Ile Gln Glu Glu Glu Glu
            260                 265                 270

Glu Glu Thr Glu Thr Asn Phe Pro Glu Pro Pro Gln Asp Gln Glu Ser
        275                 280                 285

Ser Pro Ile Glu Asn Asp Ser Ser Pro
    290                 295

## Description

### CROSS REFERENCE TO RELATED APPLICATIONS

This application claims priority to U.S. Provisional Application Serial No. 61/100,666, filed September 26, 2008, U.S. Provisional Application Serial No. 61/120,618, filed December 8, 2008 and U.S. Provisional Application Serial No. 61/186,823, filed June 13, 2009 the disclosures of which are incorporated herein by reference.

### TECHNICAL FIELD

This disclosure relates to replication competent retroviral vectors for treating cell proliferative. The disclosure further relates to the use of such replication competent retroviral fectors for delivery and expression of heterologous nucleic acids.

### BACKGROUND

Effective methods of delivering genes and heterologous nucleic acids to cells and subjects has been a goal researchers for scientific development and for possible treatments of diseases and disorders.

### SUMMARY

The disclosure provides a recombinant replication competent retrovirus (RCR) comprising: a retroviral GAG protein; a retroviral POL protein; a retroviral envelope; a retroviral polynucleotide comprising Long-Terminal Repeat (LTR) sequences at the 3' end of the retroviral polynucleotide sequence, a promoter sequence at the 5' end of the retroviral polynucleotide, said promoter being suitable for expression in a mammalian cell, a gag nucleic acid domain, a pol nucleic acid domain and an env nucleic acid domain; a cassette comprising an internal ribosome entry site (IRES) operably linked to a heterologous polynucleotide, wherein the cassette is positioned 5' to the 3' LTR and 3' to the env nucleic acid domain encoding the retroviral envelope; and cis-acting sequences necessary for reverse transcription, packaging and integration in a target cell,wherein the RCR maintains higher replication competency after 6 passages compared to a pACE vector (SEQ ID NO:21). In one embodiment, the the retroviral polynucleotide sequence is derived from murine leukemia virus (MLV), Moloney murine leukemia virus (MoMLV),Feline leukemia virus (FeLV) or Gibbon ape leukemia virus (GALV). In another embodiment, the MLV is an amphotropic MLV. In yet another embodiment, the retrovirus is an oncoretrovirus or gamma retrovirus. In yet another embodiment, the target cell is a cell having a cell proliferative disorder. The cell proliferative disorder can be selected from the group consisting of, but is not limited to, lung cancer, colon-rectum cancer, breast cancer, prostate cancer, urinary tract cancer, uterine cancer, brain cancer, head and neck cancer, pancreatic cancer, melanoma, stomach cancer and ovarian cancer, rheumatoid arthritis and other autoimmune diseases. In one embodiment, the promoter comprises a CMV promoter having a sequence as set forth in SEQ ID NO:19, 20 or 22 from nucleotide 1 to about nucleotide 582 and may include modification to one or more nucleic acid bases and which is capable of directing and initiating transcription. In yet a further embodiment, the promoter comprises a sequence as set forth in SEQ ID NO:19, 20 or 22 from nucleotide 1 to about nucleotide 582. In a further embodiment, the promoter comprises a CMV-R-U5 domain polynucleotide. In one embodiment, the CMV-R-U5 domain comprise the immediately early promoter from human cytomegalovirus linked to an MLV R-U5 region. In yet another embodiment, the CMV-R-U5 domain polynucleotide comprises a sequence as set forth in SEQ ID NO:19, 20 or 22 from about nucleotide 1 to about nucleotide 1202 or sequences that are at least 95% identical to a sequence as set forth in SEQ ID NO:19, 20 or 22, wherein the polynucleotide promotes transcription of a nucleic acid molecule operably linked thereto. In another embodiment, the gag and pol of the polynucleotide are derived from an oncoretrovirus or gamma retrovirus. The gag nucleic acid domain can comprise a sequence from about nucleotide number 1203 to about nucleotide 2819 of SEQ ID NO: 19 or 22 or a sequence having at least 95%, 98%, 99% or 99.8% identity thereto. The pol domain can comprise a sequence from about nucleotide number 2820 to about nucleotide 6358 of SEQ ID NO:19 or 22 or a sequence having at least 95%, 98%, 99% or 99.9% identity thereto. In one embodiment, the env domain encodes an amphotropic env protein. The env domain can comprise a sequence from about nucleotide number 6359 to about nucleotide 8323 of SEQ ID NO:19 or 22 or a sequence having at least 95%, 98%, 99% or 99.8% identity thereto. The IRES domain of the vector can be any IRES, however, in one embodiment the IRES is derived from an encephalomyocarditis virus. In a further embodiment, the IRES comprises a sequence from about nucleotide number 8327 to about nucleotide 8876 of SEQ ID NO:19 or 22 or a sequence having at least 95%, 98%, or 99% identity thereto.

The vector can comprise any number of different heterologous polynucleotides. For example, the heterologous polynucleotide can comprise a cytokine, an siRNA, miRNA or RNAi molecules, a targeting sequence, a binding domain, a cytotoxic gene, a single chain antibody or any combination thereof. When the heterologous polynucleotide is for a non-translated RNA such as siRNA, miRNA or RNAi then no IRES is necessary, but may be included for another translated gene RNA, and any kind of retrovirua can be used. In yet a further embodiment, the heterologous polynucleotide comprises a polynucleotide having a sequence as set forth in SEQ ID NO:3, 5, 11, 13, 15 or 17. In a further embodiment, the heterologous sequence encodes a polypeptide comprising a sequence as set forth in SEQ ID NO:4. The heterologous nucleic acid is human codon optimized and encodes a polypeptide as set forth in SEQ ID NO:4. In a further embodiment, the heterologous nucleic acid comprises a sequence as set forth in SEQ ID NO: 19 or 22 from about nucleotide number 8877 to about 9353. In one embodiment, the 3' LTR is derived from an oncoretrovirus or gamma-retrovirus. In a further embodiment, the 3' LTR comprises a U3-R-U5 domain. In yet a further embodiment, the 3' LTR comprises a sequence as set forth in SEQ ID NO:19 from about nucleotide 9405 to about 9998 or a sequence that is at least 95%, 98% or 99.5% identical thereto.

The disclosure provides a polynucleotide comprising a sequence as set forth in SEQ ID NO:19, 20 or 22.

The disclosure provides an isolated polynucleotide comprising from 5' to 3': a CMV-R-U5 fusion of the immediate early promoter from human cytomegalovirus to an MLV R-U5 region; a PBS, primer binding site for reverse transcriptase; a 5' splice site; ψ packaging signal; a gag coding sequence for MLV group specific antigen; a pol coding sequence for MLV polymerase polyprotein; a 3' splice site; a 4070A env coding sequence for envelope protein of MLV strain 4070A; an internal ribosome entry site (IRES) from encephalomyocarditis virus; a modified cytosine deaminase coding sequence; a polypurine tract; and a U3-R-U5 MLV long terminal repeat.

The disclosure provides a method of treating a subject with a cell proliferative disorder comprising contacting the subject with a polynucleotide encoding a polypeptide of the disclosure having cytosine deaminase activity under conditions such that the polynucleotide is expressed, and contacting the subject with 5-fluorocytosine.

The disclosure also provides a method of treating a cell proliferative disorder in a subject comprising contacting the subject with a retrovirus of the disclosure, wherein the heterologous nucleic acid sequence encodes a therapeutic protein that inhibits proliferation of a neoplastic cell. In one embodiment, the retrovirus comprises a polynucleotide encoding a polypeptide having a sequence as set forth in SEQ ID NO:4, 12, 14, 16, or 18.

The details of one or more embodiments of the disclosure are set forth in the accompanying drawings and the description below. Other features, objects, and advantages will be apparent from the description and drawings, and from the claims.

### BRIEF DESCRIPTION OF DRAWINGS

**Figure 1A-C** shows an alignment of the Wild-type yeast cytosine deaminase (SEQ ID NO:2) and a cytosine deaminase of the disclosure (SEQ ID NO:4) and other sequences of the disclosure.
**Figure 2** shows a graph of cell killing data showing that modified vectors are more effective compared to the original wildtype CD. The graph also shows that the new modified backbone (T5.0007) is more effective at killing than the old backbone (pACE-CD).Also shown is a table cataloguing the various vector constructs and their names
**Figure 3A-D** shows (a) a schematic of a recombinant retroviral vector of the disclosure; (b) a plasmid map of a polynucleotide of the disclosure; (c and d) a sequence of a polynucleotide of the disclosure (SEQ ID NO:19).
**Figure 4** shows that higher levels of yCD2 protein are observed compared to wild type yCD protein in infected U-87 cells.

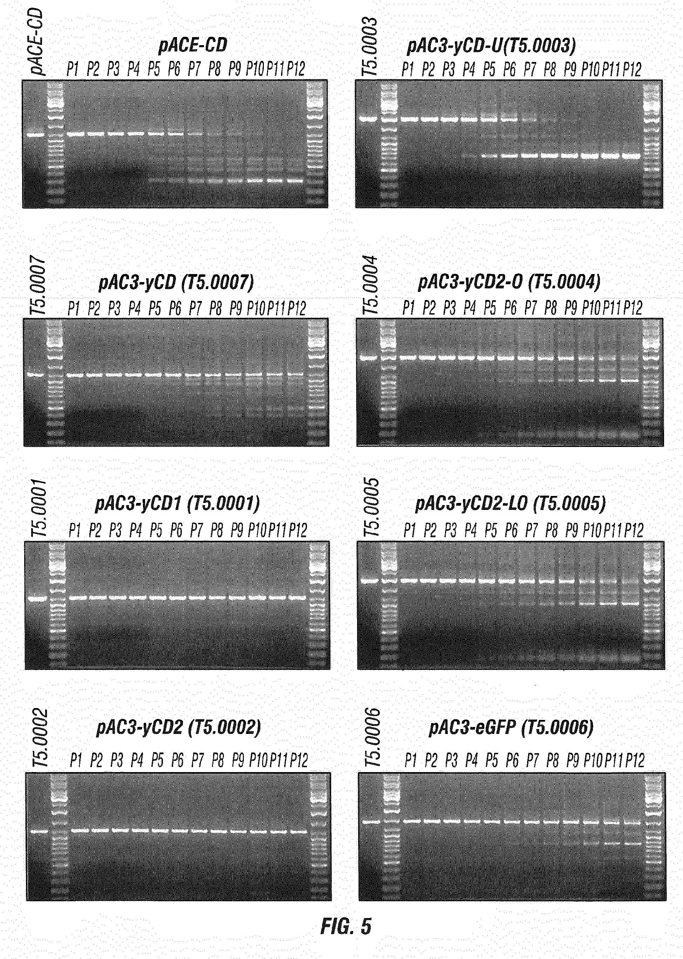

**Figure 5** shows that a vector of the disclosure is genetically stable after 12 cycles of viral passages as assessed using PCR amplification. The figure also demonstrates that the vectors of the disclosure are more stable after longer passages compared to the vector pACE-CD (Kasahara *et al*.). In particular pAC3-CD is more stable than pACE-CD, demonstrating that the changed backbone has made the vector more stable. In addition pACE-yCD1 (T5.0001) and -yCD2 (T5-0002) are very much more stable than pAC-yCD, demonstrating that small and silent changes to the coding sequence of the transgene can have a very large effect on stability, leading to superior properties. T5.0003 is the least stable and T5.0004 and T5.0005 seem about equivalent to pACE-CD, which has shown efficacy in mouse tumors (W. Wang et al. Hum Gene Ther 14:117 2003, Tai et al. Mol Ther 12:842 2005).
**Figure 6** shows (A) cell killing assays; and (B) cytosine deaminase specific activity of cells in fected with different vectors. (A) shows that cytosine deaminase and vector of the disclosure kill infected cells at least as well and perhaps better than the original pACE-CD when U87 infected cells are exposed to increasing levels of 5-FC. (B) shows tha the specific CD activity of the disclosure (T5.0007, T5.0001 and T5.0002) are all increased compared to pACE-CD (T5.0000), and is in the order T5.0000<T5.0007<T5.0001<T5.0002.
**Figure 7** shows U-87 tumors treated with CD vector of the disclosure *in vivo* and explanted from mice treated with 4 cycles of 5-FC are still sensitive to the drug.
**Figure 8** shows dosing information and therapeutic effect in a Kaplan-Meyer survival analysis in a mouse model of brain cancer.
**Figure 9** shows dosing information and therapeutic effect in a Kaplan-Meyer survival analysis in a syngeneic mouse model.
**Figure 10A-D** shows schemes for the generation of various embodiments of the disclosure comprising polypeptide with CD, OPRT and UPRT activity.
Figure 11: A. is a schematic vector map of the MLV retroviral vector pAC3 backbone containing polynucleotide sequences of human primary precursor miR-128-1, human primary precursor miR-128-2 and human precursor miR-128 linked to a human H1 promoter, designated pAC3-miR128-1, pAC3-miR-128-2, and pAC3-H1-shRNAmiR128, respectively. B. is a schematic vector map of the MLV retroviral vector pAC3-yCD2 backbone containing polynucleotide sequences of a human precursor miR-128 linked to a human H1 promoter, designated pAC3-yCD2-H1-shRNAmiR128.

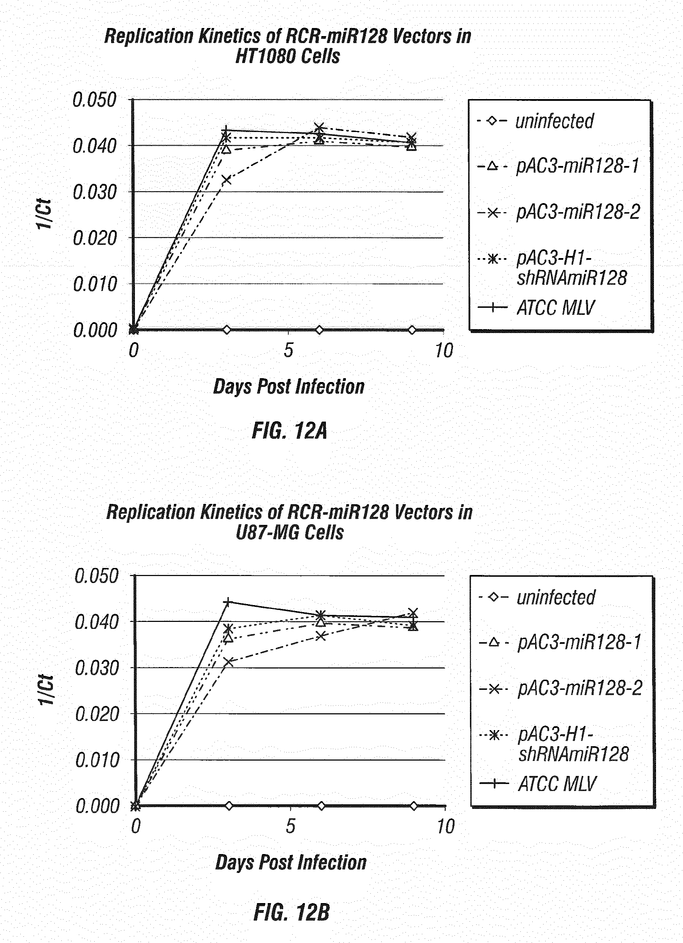

Figure 12: A. shows a comparison of replication kinetics of miR-128 containing vectors (pAC3-miR-128-1, pAC3-miR-128-2, and , pAC3-H1-shRNAmiR128) in human fibrosarcoma cells HT1080 analyzed by qPCR. The graph is generated by plotting of inversed C(t) values obtained from qPCR vs. various time points during viral replication. B. shows a comparison of replication kinetics of miR-128 containing vectors (pAC3-miR-128-1, pAC3-miR-128-2, and, pAC3-H1-shRNAmiR128) in human glioma cells U87-MG analyzed by qPCR. The graph is generated by plotting of inversed C(t) values obtained from qPCR vs. various time points during viral replication.
Figure 13 shows a relative quantification of mature miR-128 expression from cells transduced with miR-128 containing vectors.
Figure 14 shows a relative quantification of Bmi-1 gene expression from cells transduced with miR-128 containing vectors.
Figure 15 is a schematic vector map of the MLV retroviral vector pAC3-emd containing a single copy of 142-3pT target sequence, designated pAC3-emd-142-3pT and 4 tandem repeats of 142-3pT, designated pAC3-emd-142-3pT4X.
Figure 16 is a schematic vector map of the MLV retroviral vector pAC3-yCD2 containing a single copy of 142-3pT target sequence, designated pAC3-yCD2-142-3pT and 4 tandem repeats of 142-3pT, designated pAC3-yCD2-142-3pT4X.

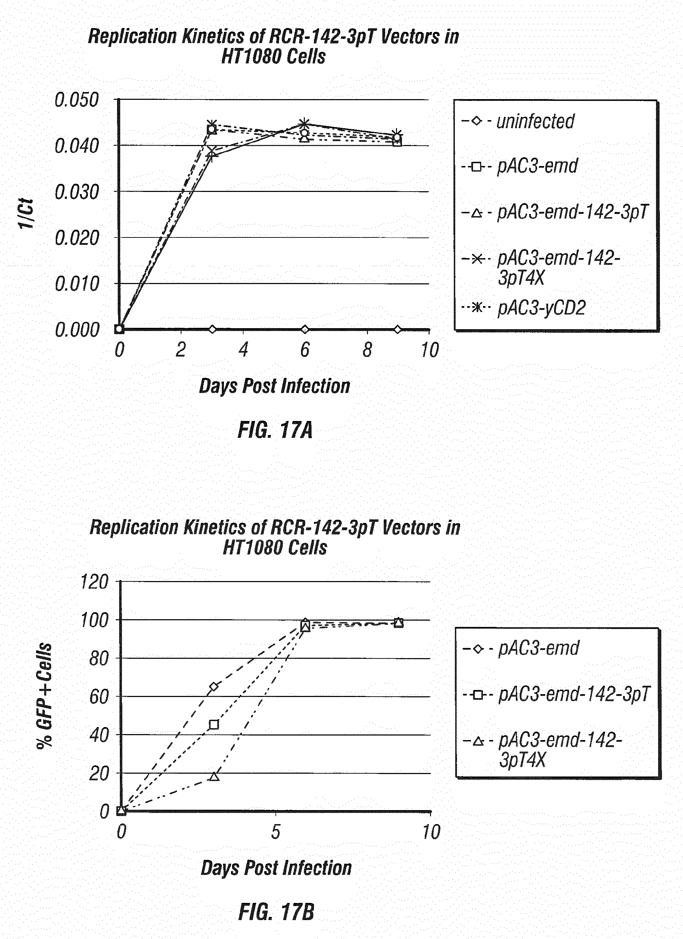

Figure 17: A. shows a comparison of replication kinetics of 142-3pT containing vectors (pAC3-emd-142-3pT pAC3-emd-142-3pT4X, pAC3-yCD2-142-3pT and pAC3-yCD2-142-3pT4X) and their parental vectors (pAC3-emd and pAC3-yCD2) in human fibrosarcoma cells HT1080 analyzed qPCR. The graph is generated by plotting by inversed C(t) values obtained from qPCR vs. various time points during viral replication. B. shows a comparison of replication kinetics of GFP containing vectors (pAC3-emd, pAC3-emd-142-3pT and pAC3-emd-142-3pT4X) in human fibrosarcoma cells HT1080 analyzed by flow cytometric analysis of GFP expression at various time points during vector spread.

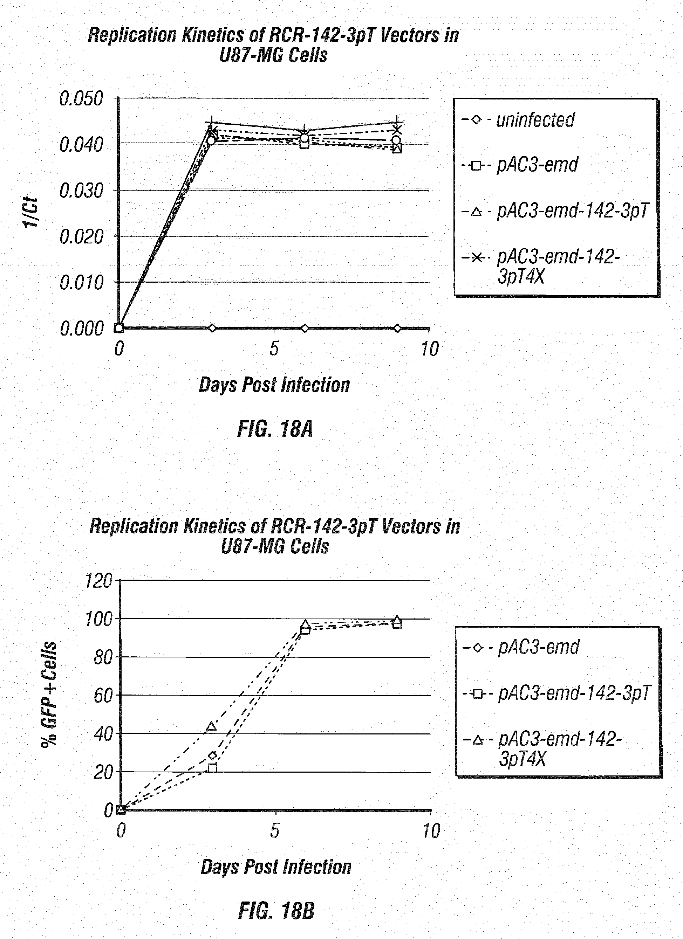

Figure 18: A. shows a comparison of replication kinetics of 142-3pT containing vectors (pAC3-emd-142-3pT pAC3-emd-142-3pT4X, pAC3-yCD2-142-3pT and pAC3-yCD2-142-3pT4X) and their parental vectors (pAC3-emd and pAC3-yCD2) in human glioma cells U87-MG analyzed qPCR. The graph is generated by plotting by inversed C(t) values obtained from qPCR vs. various time points during viral replication. B. shows a comparison of replication kinetics of GFP containing vectors (pAC3-emd, pAC3-emd-142-3pT and pAC3-emd-142-3pT4X) in human glioma cells U87-MG analyzed by flow cytometric analysis of GFP expression at various time points during vector spread.
Figure 19 shows the replication kinetics of GFP containing vector (pAC3-emd) in mouse and human hematopoietic cells analyzed by flow cytometric analysis of GFP expression at various time points during vector spread.

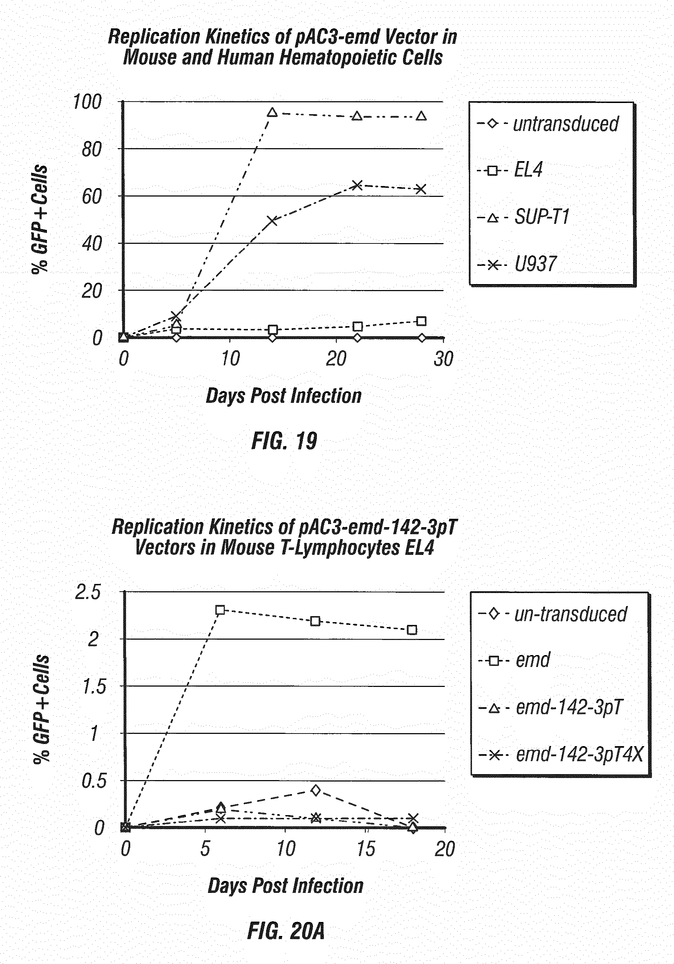

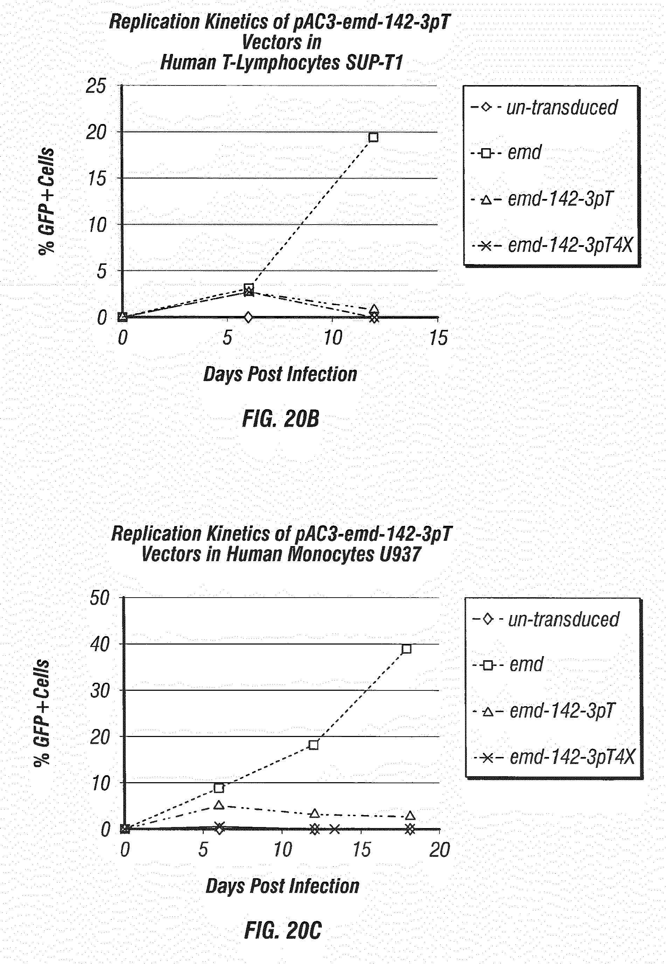

Figure 20: A. shows a comparison of replication kinetics of GFP containing vectors (pAC3-emd, pAC3-emd-142-3pT and pAC3-emd-142-3pT4X) in mouse T-lymphocytes EL4 analyzed by flow cytometric analysis of GFP expression at various time points during vector spread. B. shows a comparison of replication kinetics of GFP containing vectors (pAC3-emd, pAC3-emd-142-3pT and pAC3-emd-142-3pT4X) in human T-lymphocytes SUP-T1 analyzed by flow cytometric analysis of GFP expression at various time points during vector spread. C. shows a comparison of replication kinetics of GFP containing vectors (pAC3-emd, pAC3-emd-142-3pT and pAC3-emd-142-3pT4X) in human monocytes U937 analyzed by flow cytometric analysis of GFP expression at various time points during vector spread.
Figures 21A and 21B are still frames from the MRI images obtained from the patient dog during intratumoral CED infusion of Toca 511 and gadolinium. Note the large tumor on the left side of the image compressing both sides of the brain and shifting midline structures to the right. The white areas are the gadolinium-Toca 511 infusion. Figure 21B shows the placement of the two catheters into the tumor.
Figure 22 is a schematic vector map of the MLV retroviral vectors encoding the human IFN-gamma (hIFNg) and mouse IFN-gamma (mIFNg), respectively, in pAC3 backbone.
Figure 23 shows the expression of mIFN-gamma at the RNA level from human fibrosarcoma cell line HT1080 infected with pAC3-mIFNg vector. Expression is detected by RT-PCR.
Figure 24 shows the expression of hIFN-gamma protein secreted from human fibrosarcoma cell line HT1080 infected with pAC3-hIFNg vector.
Figure 25 shows the expression of mIFN-gamma protein secreted from human fibrosarcoma cell line HT1080 infected with pAC3-mIFNg vector.
Figure 26 shows flow cytometry analysis of GFP expression in U87 cells after intratumor or intravenous delivery of AC3-GFP vector in a nude mouse model. Cells are measured by flow cytometry for percent GFP positive. Cells isolated from naive nude mouse brains, U87 cells from tissue culture, or U87 cells transduced at an multiplicity of infection of 1 with AC3-GFP(V) *in vitro* serve as controls. From example 27(iv injection of GFP vector).
Figure 27 shows a histogram analysis was also done on groups 1,3, and 5 from example 27 (iv injection of GFP vector) to measure the distribution of GFP signal in isolated U87 cells. GFP expression is from U87 tumor cells isolated from mouse brains after 14 days after vector treatment.
Figure 28 is a schematic vector map of the MLV retroviral vectors encoding the human IL-2 in the pAC3 backbone.

Like reference symbols in the various drawings indicate like elements.

### DETAILED DESCRIPTION

As used herein and in the appended claims, the singular forms "a," "and," and "the" include plural referents unless the context clearly dictates otherwise. Thus, for example, reference to "a cell" includes a plurality of such cells and reference to "the agent" includes reference to one or more agents known to those skilled in the art, and so forth.

Also, the use of "or" means "and/or" unless stated otherwise. Similarly, "comprise," "comprises," "comprising" "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of" or "consisting of."

Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein.

General texts which describe molecular biological techniques useful herein, including the use of vectors, promoters and many other relevant topics, include Berger and Kimmel, Guide to Molecular Cloning Techniques, Methods in Enzymology Volume 152, (Academic Press, Inc., San Diego, Calif.) ("Berger"); Sambrook et al., Molecular Cloning--A Laboratory Manual, 2d ed., Vol. 1-3, Cold Spring Harbor Laboratory, Cold Spring Harbor, N.Y., 1989 ("Sambrook") and Current Protocols in Molecular Biology, F. M. Ausubel et al., eds., Current Protocols, a joint venture between Greene Publishing Associates, Inc. and John Wiley & Sons, Inc., (supplemented through 1999) ("Ausubel"). Examples of protocols sufficient to direct persons of skill through *in vitro* amplification methods, including the polymerase chain reaction (PCR), the ligase chain reaction (LCR), Qβ-replicase amplification and other RNA polymerase mediated techniques (*e.g*., NASBA), *e.g*., for the production of the homologous nucleic acids of the disclosure are found in Berger, Sambrook, and Ausubel, as well as in Mullis et al. (1987) U.S. Pat. No. 4,683,202; Innis et al., eds. (1990) PCR Protocols: A Guide to Methods and Applications (Academic Press Inc. San Diego, Calif.) ("Innis"); Arnheim & Levinson (Oct. 1, 1990) C&EN 36-47; The Journal Of NIH Research (1991) 3: 81-94; Kwoh et al. (1989) Proc. Natl. Acad. Sci. USA 86: 1173; Guatelli et al. (1990) Proc. Nat'l. Acad. Sci. USA 87: 1874; Lomell et al. (1989) J. Clin. Chem 35: 1826; Landegren et al. (1988) Science 241: 1077-1080; Van Brunt (1990) Biotechnology 8: 291-294; Wu and Wallace (1989) Gene 4:560; Barringer et al. (1990) Gene 89:117; and Sooknanan and Malek (1995) Biotechnology 13: 563-564. Improved methods for cloning *in vitro* amplified nucleic acids are described in Wallace et al., U.S. Pat. No. 5,426,039. Improved methods for amplifying large nucleic acids by PCR are summarized in Cheng et al. (1994) Nature 369: 684-685 and the references cited therein, in which PCR amplicons of up to 40 kb are generated. One of skill will appreciate that essentially any RNA can be converted into a double stranded DNA suitable for restriction digestion, PCR expansion and sequencing using reverse transcriptase and a polymerase. See, *e.g.,* Ausubel, Sambrook and Berger, all supra.

The publications discussed throughout the text are provided solely for their disclosure prior to the filing date of the present application. Nothing herein is to be construed as an admission that the inventors are not entitled to antedate such disclosure by virtue of prior disclosure.

The disclosure provides methods and compositions useful for gene or protein delivery to a cell or subject. Such methods and compositions can be used to treat various diseases and disorders in a subject including cance and other cell proliferative diseases and disorders. The disclosure provides replication comptent retroviral vectors for gene delivery.

The terms "vector", "vector construct" and "expression vector" mean the vehicle by which a DNA or RNA sequence (*e.g*. a foreign gene) can be introduced into a host cell, so as to transform the host and promote expression (*e.g*. transcription and translation) of the introduced sequence. Vectors typically comprise the DNA of a transmissible agent, into which foreign DNA encoding a protein is inserted by restriction enzyme technology. A common type of vector is a "plasmid", which generally is a self-contained molecule of double-stranded DNA that can readily accept additional (foreign) DNA and which can readily introduced into a suitable host cell. A large number of vectors, including plasmid and fungal vectors, have been described for replication and/or expression in a variety of eukaryotic and prokaryotic hosts. Non-limiting examples include pKK plasmids (Clonetech), pUC plasmids, pET plasmids (Novagen, Inc., Madison, Wis.), pRSET or pREP plasmids (Invitrogen, San Diego, Calif.), or pMAL plasmids (New England Biolabs, Beverly, Mass.), and many appropriate host cells, using methods disclosed or cited herein or otherwise known to those skilled in the relevant art. Recombinant cloning vectors will often include one or more replication systems for cloning or expression, one or more markers for selection in the host, *e.g*., antibiotic resistance, and one or more expression cassettes.

The terms "express" and "expression" mean allowing or causing the information in a gene or DNA sequence to become manifest, for example producing a protein by activating the cellular functions involved in transcription and translation of a corresponding gene or DNA sequence. A DNA sequence is expressed in or by a cell to form an "expression product" such as a protein. The expression product itself, e.g. the resulting protein, may also be said to be "expressed" by the cell. A polynucleotide or polypeptide is expressed recombinantly, for example, when it is expressed or produced in a foreign host cell under the control of a foreign or native promoter, or in a native host cell under the control of a foreign promoter.

The disclosure provides replication competent viral vectors the contain a heterologous polynucleotide encoding, for example, a cytosine deaminase or mutant thereof, an miRNA or siRNA, a cytokine, an antibody binding domain etc., that can be delivered to a cell or subject. The viral vector can be an adenoviral vector, a measles vector, a herpes vector, a retroviral vector (including a lentiviral vector), a rhabdoviral vector such as a Vesicular Stomatitis viral vector, a reovirus vector, a Seneca Valley Virus vector, a poxvirus vector (including animal pox or vaccinia derived vectors), a parvovirus vector (including an AAV vector), an alphavirus vector or other viral vector known to one skilled in the art (see also, *e.g.,* Concepts in Genetic Medicine, ed. Boro Dropulic and Barrie Carter, Wiley, 2008, Hoboken, NJ.; The Development of Human Gene Therapy, ed. Theodore Friedmann, Cold Springs Harbor Laboratory Press, Cold springs Harbor, New York, 1999; Gene and Cell Therapy, ed. Nancy Smyth Templeton, Marcel Dekker Inc., New York, New York, 2000 and Gene Therapy: Therapeutic Mechanism and Strategies, ed. Nancy Smyth Templetone and Danilo D Lasic, Marcel Dekker, Inc., New York, New York, 2000; the disclosures of which are incorporated herein by reference).

In one embodiment, the viral vector can be a replication competent retroviral vector capable of infecting only replicating mammalian cells. In one embodiment, a replication competent retroviral vector comprises an internal ribosomal entry site (IRES) 5' to the heterologous polynucleotide encoding, e.g., a cytosine deaminase, miRNA, siRNA, cytokine, receptor, antibody or the like. When the heterologous polynucleotide encodes a non-translated RNA such as siRNA, miRNA or RNAi then no IRES is necessary, but may be included for another translated gene, and any kind of retrovirus (see below) can be used.In one embodiment, the polynucleotide is 3' to a ENV polynucleotide of a retroviral vector

In other embodiments, host cells transfected with a replication competent retroviral vector of the disclosure are provided. Host cells include eucaryotic cells such as yeast cells, insect cells, or animal cells. Host cells also include prokaryotic cells such as bacterial cells.

Also provided are engineered host cells that are transduced (transformed or transfected) with a vector provided herein (*e.g*., a replication competent retroviral vector). The engineered host cells can be cultured in conventional nutrient media modified as appropriate for activating promoters, selecting transformants, or amplifying a coding polynucleotide. Culture conditions, such as temperature, pH and the like, are those previously used with the host cell selected for expression, and will be apparent to those skilled in the art and in the references cited herein, including, *e.g*., Sambrook, Ausubel and Berger, as well as *e.g*., Freshney (1994) Culture of Animal Cells: A Manual of Basic Technique, 3rd ed. (Wiley-Liss, New York) and the references cited therein.

Examples of appropriate expression hosts include: bacterial cells, such as *E. coli, B. subtilis, Streptomyces,* and *Salmonella typhimurium;* fungal cells, such as *Saccharomyces cerevisiae, Pichia pastoris,* and *Neurospora crassa;* insect cells such as Drosophila and *Spodoptera frugiperda;* mammalian cells such as CHO, COS, BHK, HEK 293 br Bowes melanoma; or plant cells or explants, etc. Typically human cells or cell lines will be used, however, it may be desirable to clone vectors and polynucleotides of the disclosure into non-human host cells for purposes of sequencing, amplification and cloning.

The disclosure also provides replication competent retroviral vectors having increased stability relative to prior retroviral vectors. Such increased stability during infection and replication is important for the treatment of cell proliferative disorders. The combination of transduction efficiency, transgene stability and target selectivity is provided by the replication competent retrovirus. The compositions and methods provide insert stability and maintain transcription activity of the transgene and the translational viability of the encoded polypeptide.

The disclosure provides modified retroviral vectors. The modified retroviral vectors can be derived from members of the retroviridae family. The Retroviridae family consists of three groups: the spumaviruses-(or foamy viruses) such as the human foamy virus (HFV); the lentiviruses, as well as visna virus of sheep; and the oncoviruses (although not all viruses within this group are oncogenic). The term "lentivirus" is used in its conventional sense to describe a genus of viruses containing reverse transcriptase. The lentiviruses include the "immunodeficiency viruses" which include human immunodeficiency virus (HIV) type 1 and type 2 (HIV-1 and HIV-2) and simian immunodeficiency virus (SIV). The oncoviruses have historically been further subdivided into groups A, B, C and D on the basis of particle morphology, as seen under the electron microscope during viral maturation. A-type particles represent the immature particles of the B- and D-type viruses seen in the cytoplasm of infected cells. These particles are not infectious. B-type particles bud as mature virion from the plasma membrane by the enveloping of intracytoplasmic A-type particles. At the membrane they possess a toroidal core of 75 nm, from which long glycoprotein spikes project. After budding, B-type particles contain an eccentrically located, electron-dense core. The prototype B-type virus is mouse mammary tumor virus (MMTV). No intracytoplasmic particles can be observed in cells infected by C-type viruses. Instead, mature particles bud directly from the cell surface via a crescent 'C'-shaped condensation which then closes on itself and is enclosed by the plasma membrane. Envelope glycoprotein spikes may be visible, along with a uniformly electron-dense core. Budding may occur from the surface plasma membrane or directly into intracellular vacuoles. The C-type viruses are the most commonly studied and include many of the avian and murine leukemia viruses (MLV). Bovine leukemia virus (BLV), and the human T-cell leukemia viruses types I and II (HTLV-I/II) are similarly classified as C-type particles because of the morphology of their budding from the cell surface. However, they also have a regular hexagonal morphology and more complex genome structures than the prototypic C-type viruses such as the murine leukemia viruses (MLV). D-type particles resemble B-type particles in that they show as ring-like structures in the infected cell cytoplasm, which bud from the cell surface, but the virion incorporate short surface glycoprotein spikes. The electron-dense cores are also eccentrically located within the particles. Mason Pfizer monkey virus (MPMV) is the prototype D-type virus.

Retroviruses have been classified in various ways but the nomenclature has been standardized in the last decade (see ICTVdB - The Universal Virus Database, v 4 on the World Wide Web (www) at ncbi.nlm.nih.gov/ICTVdb/ICTVdB/ and the text book "Retroviruses" Eds Coffin, Hughs and Varmus, Cold Spring Harbor Press 1997; the disclosures of which are incorporated herein by reference). In one embodiment, the replication competent retroviral vector can comprise an Orthoretrovirus or more typically a gamma retrovirus vector.

Retroviruses are defined by the way in which they replicate their genetic material. During replication the RNA is converted into DNA. Following infection of the cell a double-stranded molecule of DNA is generated from the two molecules of RNA which are carried in the viral particle by the molecular process known as reverse transcription. The DNA form becomes covalently integrated in the host cell genome as a provirus, from which viral RNAs are expressed with the aid of cellular and/or viral factors. The expressed viral RNAs are packaged into particles and released as infectious virion.

The retrovirus particle is composed of two identical RNA molecules. Each wild-type genome has a positive sense, single-stranded RNA molecule, which is capped at the 5' end and polyadenylated at the 3' tail. The diploid virus particle contains the two RNA strands complexed with gag proteins, viral enzymes (pol gene products) and host tRNA molecules within a 'core' structure of gag proteins. Surrounding and protecting this capsid is a lipid bilayer, derived from host cell membranes and containing viral envelope (env) proteins. The env proteins bind to a cellular receptor for the virus and the particle typically enters the host cell via receptor-mediated endocytosis and/or membrane fusion.

After the outer envelope is shed, the viral RNA is copied into DNA by reverse transcription. This is catalyzed by the reverse transcriptase enzyme encoded by the pol region and uses the host cell tRNA packaged into the virion as a primer for DNA synthesis. In this way the RNA genome is converted into the more complex DNA genome.

The double-stranded linear DNA produced by reverse transcription may, or may not, have to be circularized in the nucleus. The provirus now has two identical repeats at either end, known as the long terminal repeats (LTR). The termini of the two LTR sequences produces the site recognized by a pol product--the integrase protein--which catalyzes integration, such that the provirus is always joined to host DNA two base pairs (bp) from the ends of the LTRs. A duplication of cellular sequences is seen at the ends of both LTRs, reminiscent of the integration pattern of transposable genetic elements. Integration is thought to occur essentially at random within the target cell genome. However, by modifying the long-terminal repeats it is possible to control the integration of a retroviral genome.

Transcription, RNA splicing and translation of the integrated viral DNA is mediated by host cell proteins. Variously spliced transcripts are generated. In the case of the human retroviruses HIV-1/2 and HTLV-I/II viral proteins are also used to regulate gene expression. The interplay between cellular and viral factors is a factor in the control of virus latency and the temporal sequence in which viral genes are expressed.

Retroviruses can be transmitted horizontally and vertically. Efficient infectious transmission of retroviruses requires the expression on the target cell of receptors which specifically recognize the viral envelope proteins, although viruses may use receptor-independent, nonspecific routes of entry at low efficiency. In addition, the target cell type must be able to support all stages of the replication cycle after virus has bound and penetrated. Vertical transmission occurs when the viral genome becomes integrated in the germ line of the host. The provirus will then be passed from generation to generation as though it were a cellular gene. Hence endogenous proviruses become established which frequently lie latent, but which can become activated when the host is exposed to appropriate agents.

As mentioned above, the integrated DNA intermediate is referred to as a provirus. Prior gene therapy or gene delivery systems use methods and retroviruses that require transcription of the provirus and assembly into infectious virus while in the presence of an appropriate helper virus or in a cell line containing appropriate sequences enabling encapsidation without coincident production of a contaminating helper virus. As described below, a helper virus is not required for the production of the recombinant retrovirus of the disclosure, since the sequences for encapsidation are provided in the genome thus providing a replication competent retroviral vector for gene delivery or therapy.

Existing replication competent retroviral vectors also tend to be lost from an infected cell or host cell during horizontal or vertical transmission and during replication. This may be due in-part from the presence of extra nucleotide sequences that include repeats or which reduce the efficiency of a polymerase.

The retroviral genome and the proviral DNA of the disclosure have at least three genes: the *gag,* the *pol,* and the *env,* these genes may be flanked by one or two long terminal (LTR) repeat, or in the provirus are flanked by two long terminal repeat (LTR) and sequences containing cis-acting sequences such as psi. The gag gene encodes the internal structural (matrix, capsid, and nucleocapsid) proteins; the pol gene encodes the RNA-directed DNA polymerase (reverse transcriptase), protease and integrase; and the env gene encodes viral envelope glycoproteins. The 5' and/or 3' LTRs serve to promote transcription and polyadenylation of the virion RNAs. The LTR contains all other cis-acting sequences necessary for viral replication. Lentiviruses have additional genes including vif, vpr, tat, rev, vpu, nef, and vpx (in HIV-1, HIV-2 and/or SIV).

Adjacent to the 5' LTR are sequences necessary for reverse transcription of the genome (the tRNA primer binding site) and for efficient encapsidation of viral RNA into particles (the Psi site). If the sequences necessary for encapsidation (or packaging of retroviral RNA into infectious virion) are missing from the viral genome, the result is a cis defect which prevents encapsidation of genomic viral RNA. This type of modified vector is what has typically been used in prior gene delivery systems (*i.e*., systems lacking elements which are required for encapsidation of the virion).

In a first embodiment, the disclosure provides a recombinant retrovirus capable of infecting a non-dividing cell, a dividing cell, or a cell having a cell proliferative disorder. The recombinant replication competent retrovirus of the disclosure comprises a polynucleotide sequence encoding a viral GAG, a viral POL, a viral ENV, a heterologous polynucleotide preceded by an internal ribosome entry site (IRES) enpasulated within a virion.

The phrase "non-dividing" cell refers to a cell that does not go through mitosis. Non-dividing cells may be blocked at any point in the cell cycle, (*e.g*., G₀/G₁, G_{1/S}, G_{2/M}), as long as the cell is not actively dividing. For *ex vivo* infection, a dividing cell can be treated to block cell division by standard techniques used by those of skill in the art, including, irradiation, aphidocolin treatment, serum starvation, and contact inhibition. However, it should be understood that *ex vivo* infection is often performed without blocking the cells since many cells are already arrested (*e.g*., stem cells). For example, a recombinant lentivirus vector is capable of infecting any non-dividing cell, regardless of the mechanism used to block cell division or the point in the cell cycle at which the cell is blocked. Examples of pre-existing non-dividing cells in the body include neuronal, muscle, liver, skin, heart, lung, and bone marrow cells, and their derivatives. For dividing cells onco-retroviral vectors can be used.

By "dividing" cell is meant a cell that undergoes active mitosis, or meiosis. Such dividing cells include stem cells, skin cells (*e.g*., fibroblasts and keratinocytes), gametes, and other dividing cells known in the art. Of particular interest and encompassed by the term dividing cell are cells having cell proliferative disorders, such as neoplastic cells. The term "cell proliferative disorder" refers to a condition characterized by an abnormal number of cells. The condition can include both hypertrophic (the continual multiplication of cells resulting in an overgrowth of a cell population within a tissue) and hypotrophic (a lack or deficiency of cells within a tissue) cell growth or an excessive influx or migration of cells into an area of a body. The cell populations are not necessarily transformed, tumorigenic or malignant cells, but can include normal cells as well. Cell proliferative disorders include disorders associated with an overgrowth of connective tissues, such as various fibrotic conditions, including scleroderma, arthritis and liver cirrhosis. Cell proliferative disorders include neoplastic disorders such as head and neck carcinomas. Head and neck carcinomas would include, for example, carcinoma of the mouth, esophagus, throat, larynx, thyroid gland, tongue, lips, salivary glands, nose, paranasal sinuses, nasopharynx, superior nasal vault and sinus tumors, esthesioneuroblastoma, squamous call cancer, malignant melanoma, sinonasal undifferentiated carcinoma (SNUC), brain (including glioblastomas) or blood neoplasia. Also included are carcinoma's of the regional lymph nodes including cervical lymph nodes, prelaryngeal lymph nodes, pulmonary juxtaesophageal lymph nodes and submandibular lymph nodes (Harrison's Principles of Internal Medicine (eds., Isselbacher, et al., McGraw-Hill, Inc., 13th Edition, pp1850-1853, 1994). Other cancer types, include, but are not limited to, lung cancer, colon-rectum cancer, breast cancer, prostate cancer, urinary tract cancer, uterine cancer lymphoma, oral cancer, pancreatic cancer, leukemia, melanoma, stomach cancer, skin cancer and ovarian cancer. The cell prolifereative disease also includes rheumatoid arthritis (O'Dell NEJM 350:2591 2004)and other auto-immune disorders (Mackay et al NEJM 345:340 2001) that are often characterized by inappropriate proliferation of cells of the immune system.

The heterologous nucleic acid sequence is operably linked to an IRES. As used herein, the term "heterologous" nucleic acid sequence or transgene refers to (i) a sequence that does not normally exist in a wild-type retrovirus, (ii) a sequence that originates from a foreign species, or (iii) if from the same species, it may be substantially modified from its original form. Alternatively, an unchanged nucleic acid sequence that is not normally expressed in a cell is a heterologous nucleic acid sequence.

Depending upon the intended use of the retroviral vector of the disclosure any number of heterologous polynucleotide or nucleic acid sequences may be inserted into the retroviral vector. For example, for *in vitro* studies commonly used marker genes or reporter genes may be used, including, antibiotic resistance and fluorescent molecules (*e.g*., GFP). Additional polynucleotide sequences encoding any desired polypeptide sequence may also be inserted into the vector of the disclosure. Where *in vivo* delivery of a heterologous nucleic acid sequence is sought both therapeutic and non-therapeutic sequences may be used. For example, the heterologous sequence can encode a therapeutic molecule including antisense molecules (miRNA, siRNA) or ribozymes directed to a particular gene associated with a cell proliferative disorder or other gene-associated disease or disorder, the heterologous sequence can be a suicide gene (*e.g*., HSV-tk or PNP or cytosine deaminase; either modified or unmodified), a growth factor or a therapeutic protein (*e.g*., Factor IX, IL2, and the like). Other therapeutic proteins applicable to the disclosure are easily identified in the art.

In one embodiment, the heterologous polynucleotide within the vector comprises a cytosine deaminase that has been optimized for expression in a human cell. In a further embodiment, the cytosine deaminase comprises a sequence that has been human codon optimized and comprises mutations that increase the cytosine deaminase's stability (*e.g*., reduced degradation or increased thermo-stability) compared to a wild-type cytosine deaminase. In yet another embodiment, the heterologous polynucleotide encodes a fusion construct comprising a cytosine deaminase (either human codon optimized or non-optimized, either mutated or non-mutated) operably linked to a polynucleotide encoding a polypeptide having UPRT or OPRT activity. In another embodiment, the heterologous polynucleotide comprises a CD polynucleotide of the disclosure (*e.g*., SEQ ID NO:3, 5, 11, 13, 15, or 17).

In another embodiment, replication competent retroviral vector can comprise a heterologus polynucleotide encoding a polypeptide comprising a cytosine deaminase (as described herein) and may further comprise a polynucleotide comprising a miRNA or siRNA molecule either as part of the primary transcript from the viral promoter or linked to a promoter, which can be cell-type or tissue specific.

MicroRNAs (miRNA) are small, non-coding RNAs. They are located within introns of coding or non-coding gene, exons of non-coding genes or in inter-genic regions. miRNA genes are transcribed by RNA polymerase II that generate precursor polynucleotides called primary precursor miRNA (pri-miRNA). The pri-miRNA in the nucleus is processed by the ribonuclease Drosha to produce the miRNA precursor (pre-miRNA) that forms a short hairpin structure. Subsequently, pre-miRNA is transported to the cytoplasma via Exportin 5 and further processed by another ribonuclease called Dicer to generate an active, mature miRNA.

A mature miRNA is approximately 21 nucleotides in length. It exerts in function by binding to the 3' untranslated region of mRNA of targeted genes and suppressing protein expression either by repression of protein translation or degradation of mRNA. miRNA are involved in biological processes including development, cell proliferation, differentiation and cancer progression. Studies of miRNA profiling indicate that some miRNA expressions are tissue specific or enriched in certain tissues. For example, , miR-142-3p, miR-181 and miR-223 expressions have demonstrated to be enriched in hematopoietic tissues in human and mouse (Baskerville et al., 2005 RNA 11, 241-247; Chen et al., 2004 Science 303, 83-86).

Some miRNAs have been observed to be up-regulated (oncogenic miRNA) or down-regulated (repressor)in several tumors (Spizzo et al., 2009 Cell 137, 586e1). For example, miR-21 is overexpressed in glioblastoma, breast, lung, prostate, colon, stomach, esophageal, and cervical cancer, uterine leiomyosarcoma, DLBCL, head and neck cancer. In contrast, members of let-7 have reported to be down-regulated in glioblastoma, lung, breast, gastric, ovary, prostate and colon cancers. Re-establishment of homeostasis of miRNA expression in cancer is an imperative mechanism to inhibit or reverse cancer progression.

As a consequence of the vital functions modulated by miRNAs in cancers, focus in developing potential therapeutic approaches has been directed toward antisense-mediated inhibition (antigomers) of oncogenic miRNAs. However, miRNA replacement might represent an equally efficacious strategy. In this approach, the most therapeutically useful miRNAs are the ones expressed at low levels in tumors but at high level, and therefore tolerated, in normal tissues.

miRNAs that are down-regulated in cancers could be useful as anticancer agents. Examples include mir-128-1, let-7, miR-26, miR-124, and miR-137 (Esquela-Kerscher et al., 2008 Cell Cycle 7, 759-764; Kumar et al., 2008 Proc Natl Acad Sci USA 105, 3903-3908; Kota et al., 2009 Cell 137, 1005-1017; Silber et al., 2008 BMC Medicine 6:14 1-17). miR-128 expression has reported to be enriched in the central nervous sytem and has been observed to be down-regulated in glioblastomas (Sempere et al., 2004 Genome Biology 5:R13.5-11; Godlewski et al., 2008 Cancer Res 68: (22) 9125-9130). miR-128 is encoded by two distinct genes, miR-128-1 and miR-128-2. Both are processed into identical mature sequence. Bmi-1 and E2F3a have been reported to be the direct targets of miR-128 (Godlewski et al., 2008 Cancer Res 68: (22) 9125-9130; Zhang et al., 2009 J. Mol Med 87:43-51). In addition, Bmi-1 expression has been observed to be up-regulated in a variety of human cancers, including gliomas, mantle cell lymphomas, non-small cell lung cancer B-cell non-Hodgkin's lymphoma, breast, colorectal and prostate cancer. Furthermore, Bmi-1 has been demonstrated to be required for the self-renewal of stem cells from diverse tissues, including neuronal stem cells as well as "stem-like" cell population in gliomas.

Although there have been a number of in vitro demonstrations of the possibilities of miRNA mediated inhibition of cellular function, it has been difficult to deliver these as oligonucleotides or in viral vectors as efficiently as necessary to have in vivo effects (e.g. Li et al. Cell Cycle 5:2103-2109 2006), as has been true for other molecules. Non-replicative vectors do not appear to be efficient enough in any case to achieve delivery of a therapeutic gene into a significant portion of tumors. However it is also not simple to see how to use replicative vectors to deliver miRNA types of agents. In particular it is not clear how to incorporate extra RNA sequences into the RNA genome of replication competent retroviruses and maintain the replication efficiency and keep the addition stably incorporated into the genome.

Replication-defective retroviral and lentiviral vectors have been used to stably express pri-mi RNA by a polymerase II promoter such as CMV or LTR and demonstrated production of mature miRNA. However, these vectors do not have to go through the entire lifecycle of the retrovirus or lentivirus multiple times as is required for replicating vectors. The genome has to be able to accommodate many more events than simple entry, integration and transcription. The concerns associated with the use of a RNA-based virus to express miRNA include: (1) the integrity of the viral RNA genome at post transcriptional step during RNA processing; (2) the stability of the inserted cassette during replication; and (3) proper processing of pri-miRNA as part of the viral RNA transcribed from the LTR promoter producing mature miRNA.

Thus, incorporation of type III RNA polymerase III promoters such as the U6 and the H1 promoter in non-replicative retroviral and lentiviral vectors has been used widely to express functional small interference RNA (siRNA) producing a short hairpin structured RNA (Bromberg-White et al., 2004 J Virol 78:9, 4914-4916; Sliva et al., 2006 Virology 351, 218-225; Haga et al., 2006, Transplant Proc 38(10):3184-8). The loop sequence is cleaved by Dicer producing the mature siRNAs that are 21-22 nucleotides in length. shRNA can be stably expressed in cells to down-regulate target gene expression. However the incorporation of such cassettes into the recombinant replication competent retroviral vector, the expression and the processing by Dicer to produce mature miRNA remain problematic.
In one embodiment, the present invention provides a recombinant replication competent retroviral vector that contains a heterologous polynucleotide sequence of a primary precursor miRNA.

In a further embodiment the primary precursor miRNA is of human origin. In another embodiment the primary precursor RNA sequence is downstream of the env gene.

In another embodiment, the present invention provides a recombinant replication competent retroviral vector that contains a heterologous polynucleotide sequence of the human primary precursor miR-128-2 (SEQ ID NO:32) downstream of the env gene. miRNAs that are down-regulated in cancers can be incorporated into the vector for therapeutic gene delivery. For example, let-7, miR-26, miR-124, and miR-137 (Esquela-Kerscher et al., 2008 Cell Cycle 7, 759-764; Kumar et al., 2008 Proc Natl Acad Sci USA 105, 3903-3908; Kota et al., 2009 Cell 137, 1005-1017; Silber et al., 2008 BMC Medicine 6:14 1-17).

In yet another embodiment, the present invention provides a recombinant replication competent retroviral vector that contains a heterologous polynucleotide sequence of the short hairpin structured human pre-miR-128 linked to a human H1 promoter (SEQ ID NO: 33 and SEQ ID NO:34) downstream of the env gene. miRNAs that are down-regulated in cancers can be incorporated into the vector for therapeutic gene delivery. For example, let-7, miR-26, miR-124, and miR-137 (Esquela-Kerscher et al., 2008 Cell Cycle 7, 759-764; Kumar et al., 2008 Proc Natl Acad Sci USA 105, 3903-3908; Kota et al., 2009 Cell 137, 1005-1017; Silber et al., 2008 BMC Medicine 6:14 1-17).

Antisense nucleic acids are DNA or RNA molecules that are complementary to at least a portion of a specific mRNA molecule (Weintraub, Scientific American, 262:40, 1990). In the cell, the antisense nucleic acids hybridize to the corresponding mRNA, forming a double-stranded molecule. The antisense nucleic acids interfere with the translation of the mRNA, since the cell will not translate a mRNA that is double-stranded. Antisense oligomers of about 15 nucleotides are preferred, since they are easily synthesized and are less likely to cause problems than larger molecules when introduced into the target cell. The use of antisense methods to inhibit the *in vitro* translation of genes is well known in the art (Marcus-Sakura, Anal. Biochem., 172:289, 1988).

The antisense nucleic acid can be used to block expression of a mutant protein or a dominantly active gene product, such as amyloid precursor protein that accumulates in Alzheimer's disease. Such methods are also useful for the treatment of Huntington's disease, hereditary Parkinsonism, and other diseases. Of particular interest are the blocking of genes associated with cell-proliferative disorders. Antisense nucleic acids are also useful for the inhibition of expression of proteins associated with toxicity.

Use of an oligonucleotide to stall transcription is known as the triplex strategy since the oligomer winds around double-helical DNA, forming a three-strand helix. Therefore, these triplex compounds can be designed to recognize a unique site on a chosen gene (Maher, et al., Antisense Res. and Dev., 1(3):227, 1991; Helene, C., Anticancer Drug Design, 6(6):569, 1991).

Ribozymes are RNA molecules possessing the ability to specifically cleave other single-stranded RNA in a manner analogous to DNA restriction endonucleases. Through the modification of nucleotide sequences which encode these RNAs, it is possible to engineer molecules that recognize specific nucleotide sequences in an RNA molecule and cleave it (Cech, J. Amer. Med. Assn., 260:3030, 1988). A major advantage of this approach is that, because they are sequence-specific, only mRNAs with particular sequences are inactivated.

As used herein, the term "RNA interference" (RNAi) refers to the process of sequence-specific post-transcriptional gene silencing mediated by short interfering nucleic acids (siRNAs or microRNAs (miRNA)). The term "agent capable of mediating RNA interference" refers to siRNAs as well as DNA and RNA vectors that encode siRNAs when transcribed within a cell. The term siRNA or miRNA is meant to encompass any nucleic acid molecule that is capable of mediating sequence specific RNA interference, for example short interfering RNA (siRNA), double-stranded RNA (dsRNA), micro-RNA (miRNA), short hairpin RNA (shRNA), short interfering oligonucleotide, short interfering nucleic acid, short interfering modified oligonucleotide, chemically-modified siRNA, post-transcriptional gene silencing RNA (ptgsRNA), and others.

Suitable range for designing stem lengths of a hairpin duplex, includes stem lengths of 20-30 nucleotides, 30-50 nucleotides, 50-100 nucleotides, 100-150 nucleotides, 150-200 nucleotides, 200-300 nucleotides, 300-400 nucleotides, 400-500 nucleotides, 500-600 nucleotides, and 600-700 nucleotides. Suitable range for designing loop lengths of a hairpin duplex, includes loop lengths of 4-25 nucleotides, 25-50 nucleotides, or longer if the stem length of the hair duplex is substantial. In certain context, hairpin structures with duplexed regions that are longer than 21 nucleotides may promote effective siRNA-directed silencing, regardless of the loop sequence and length.

The replicating retroviral vectors of the disclosure can be used to treat disease by expressing engineered siRNA or miRNA (Dennis, Nature, 418: 122 2002) that switches off or lowers expression of key genes that govern the proliferation or survival of diseased cells including tumor cells. Such targets include genes like Rad 51 a central enzyme in DNA repair, and without which cell growth is drastically restricted. Other targets include many of the signaling pathway molecules that control cell growth (Marquez & McCaffrey Hum Gene Ther. 19:27 2008). The siRNA or miRNA may be combined with expression of a cytotoxic gene from the same or different retroviral vector of the disclosure. An example of a suitable cytotoxic gene comprise a cytosine deaminase or modified cytosine deaminase of the disclosure.

In use, the retroviral vector(s) will replicate through the tumor or other target tissue and before growth inhibition occurs the virus first integrates into the host genome and continues to make virus after growth of that cell is inhibited. Methods for selecting functional miRNA or siRNA sequences are known in the art. Key feature in general in designing effective siRNA or miRNA sequences is usually avoiding "off-target" effects. However for the use of replicating vectors that are highly specific to tumor cells such as those of the disclosure, these side effects are not very important, as the cells are expected to eventually die. A retroviral vector of this disclosure can be made using cells from other species for which the corresponding protein is not significantly targeted. Such cells include dog cell lines or chicken cell line. Alternatively the virus is made by transient transfection on human 293 derived cells or other cell line that allows efficient transient transfection. For this use the virus does not need to utilize an IRES, and the siRNA or miRNA sequence can simply be inserted at a convenient site on the viral genome. This site includes the region downstream of the envelope and upstream of the 3'LTR of the replicating retrovirus. Alternatively polIII transcription units can be inserted in the viral genome with the appropriate siRNA or miRNA's, typically downstream of the 3' envelope gene. Several different siRNA or miRNA sequences can be inserted to ensure efficient down regulation of the target gene or down regulation of more than one gene. Suitable sequences and targets can be obtained from sources known to those skilled in the art. For example:
- The MIT/ICBP siRNA Database http:(//)web.mit.edu/sirna/ - "The MIT [Massachusetts Institute of Technology]/ICBP [Integrative Cancer Biology Program] siRNA Database is a university-wide effort to catalog these experimentally validated reagents and make that information available to other researchers, both within and outside the MIT community. (Massachusetts Institute of Technology).
- RNAi Central - http:(//)katahdin.cshl.org:9331/RNAi_web/ scripts/main2.pl RNAi resources, including siRNA and shRNA design tools. (Hannon Lab, Cold Spring Harbor Laboratory)
- The RNAi Web - http:(//)www.rnaiweb.com/ General resource.
- siDIRECT - http:(//)genomics.jp/sidirect/ Online target-specific siRNA design program for mammalian RNA interference. (University of Tokyo, Japan).
- siRNA Database - A comprehensive siRNA database that contains siRNA targets against all known mRNA sequences throughout a variety of organisms. (Part of the Protein Lounge systems biology Web site)
- siRNA Database and Resources for RNA Interference Studies http:(//)www.rnainterference.org/
- siRNA Selector - http:(//)bioinfo.wistar.upenn.edu/siRNA /siRNA.htm. A set of rules was used for evaluating siRNA functionality based on thermodynamics parameters (Khvorova et *al.,* 2003, Schwarz *et al.,* 2003) and sequence-related determinants developed by Dharmacon (Reynolds *et al.,* 2004). Specificity is determined using BLAST against UniGene databases. (Wistar Institute)
- siRNA Target Finder http:(//)www(.)ambion.com/techlib/misc/ siRNA_finder.html (Ambion).

The replicating retroviruses of the disclosure can also express targets for naturally occurring siRNA's that are restricted in expression to particular cell types so that replication of the vector is significantly inhibited in those cell types. The generation of murine leukemia virus-based recombinant replication competent retroviral vector allows high level of transduction and thus high efficiency of gene delivery *in vivo.* One major concern of using replication competent retroviral vector has been the uncontrolled spread of virus as reported previously (Donahue et al., J. Exp Med. 1992, 176:1124-1135; Calmes et al., Blood 2005, 106: 2530-2533; Seggewiss et al., Blood 2006, 107: 3865-3867). Because of the nature of the virus, the viral spread may be achieved initially within lymphatic cells and subsequently spread to peripheral tissues. For anti-tumor purposes some normal cells in the body that are naturally replicating at some level are hematopoietic cells, cells of the lining of the gut, and some endothelial cells. These are then potential sites where virus that is in the circulation could productively infect. In general this would be undesirable. Any stray infection of cells such as these can be inhibited by including a target for naturally occurring miRNA's or for a combination of miRNA's in these cell types. Some feasibility of using miRNA targets to suppress immune responses has already been shown. (Brown et al. Nat Biotechnol. 2007 25:1457-67). These targets are small RNA sequences with a homologous match to the miRNA sequences that are naturally occurring. These sequences can be inserted in any convenient site in the vectors of the current invention without, in general significant deleterious consequence for vector viability, other than in a cell of the type desired. Vectors can be made and used as described herein.

In one embodiment, the present invention provides a recombinant replication competent retroviral vector that contains a single copy of the miR-142-3p target sequence (142-3pT, SEQ ID NO:35) downstream of the transgene, such as yCD2 or GFP, linked to the IRES. In addition to miR181 and miR-223, the target sequence of other tissue or cell-enriched miRNA can be incorporated into the vector to restrict viral spread in specific tissue or cell type manner. For example, miR-133 and miR206 expressions are highly enriched in muscle cells (Kelly et al., 2008 Nature Medicine 14:11 1278-1283.

In another embodiment, the present invention provides a recombinant replication competent retroviral vector that contains 4 copies of the 142-3pT (SEQ ID NO: 36) downstream of the transgene, such as yCD2 or GFP, linked to the IRES. In addition to miR181 and miR-223, the target sequence of other tissue or cell-enriched miRNA can be incorporated into the vector to restrict viral spread in specific tissue or cell type manner. For example, miR-133 and miR206 expressions are highly enriched in muscle cells. The present invention provides flexibility of single, multiple or combination of target sequence of miRNA and thereby provides restriction of uncontrolled viral spread in a tissue- and/or cell-specific fashion *in vitro* and *in vivo* (e. g. hematopoietic and/or muscle cells), (Kelly et al., 2008 Nature Medicine 14:11 1278-1283).

The miRNA target can be inserted 3' to the transgene but before the 3'LTR or upstream of the IRES but after the 3' end of the envelope. In general the target would not be inserted into protein coding sequences.

In yet further embodiments, the heterologous polynucleotide may comprise a cytokine such as an interleukin, interferon gamma or the like. Cytokines that may expressed from a retroviral vector of the disclosure include, but are not limited to, IL-1alpha, IL-1beta, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, and IL-21, anti-CD40, CD40L, IFN-gamma and TNF-alpha, soluble forms of TNF-alpha, lymphotoxin-alpha (LT-alpha, also known as TNF-beta), LT-beta (found in complex heterotrimer LT-alpha2-beta), OPGL, FasL, CD27L, CD30L, CD40L, 4-1BBL, DcR3, OX40L, TNF-gamma (International Publication No. WO 96/14328), AIM-I (International Publication No. WO 97/33899), endokine-alpha (International Publication No. WO 98/07880), OPG, and neutrokine-alpha (International Publication No. WO 98/18921, OX40, and nerve growth factor (NGF), and soluble forms of Fas, CD30, CD27, CD40 and 4-IBB, TR2 (International Publication No. WO 96/34095), DR3 (International Publication No. WO 97/33904), DR4 (International Publication No. WO 98/32856), TR5 (International Publication No. WO 98/30693), TRANK, TR9 (International Publication No. WO 98/56892), TR10 (International Publication No. WO 98/54202), 312C2 (International Publication No. WO 98/06842), and TR12, and soluble forms CD154, CD70, and CD153. Angiogenic proteins may be useful in some embodiments, particularly for protein production from cell lines. Such angiogenic factors include, but are not limited to, Glioma Derived Growth Factor (GDGF), Platelet Derived Growth Factor-A (PDGF-A), Platelet Derived Growth Factor-B (PDGF-B), Placental Growth Factor (PIGF), Placental Growth Factor-2 (PIGF-2), Vascular Endothelial Growth Factor (VEGF), Vascular Endothelial Growth Factor-A (VEGF-A), Vascular Endothelial Growth Factor-2 (VEGF-2), Vascular Endothelial Growth Factor B (VEGF-3), Vascular Endothelial Growth Factor B-1 86 (VEGF-B186), Vascular Endothelial Growth Factor-D (VEGF-D), Vascular Endothelial Growth Factor-D (VEGF-D), and Vascular Endothelial Growth Factor-E (VEGF-E). Fibroblast Growth Factors may be delivered by a vector of the disclosure and include, but are not limited to, FGF-1, FGF-2, FGF-3, FGF-4, FGF-5, FGF-6, FGF-7, FGF-8, FGF-9, FGF-10, FGF-11, FGF-12, FGF-13, FGF-14, and FGF-15. Hematopoietic growth factors may be delivered using vectors of the disclosure, such growth factors include, but are not limited to, granulocyte macrophage colony stimulating factor (GM-CSF) (sargramostim), granulocyte colony stimulating factor (G-CSF) (filgrastim), macrophage colony stimulating factor (M-CSF, CSF-1) erythropoietin (epoetin alfa), stem cell factor (SCF, c-kit ligand, steel factor), megakaryocyte colony stimulating factor, PIXY321 (a GMCSF/IL-3) fusion protein and the like.

Generally, the recombinant virus of the disclosure is capable of transferring a nucleic acid sequence into a target cell.

The term "regulatory nucleic acid sequence" refers collectively to promoter sequences, polyadenylation signals, transcription termination sequences, upstream regulatory domains, origins of replication, enhancers and the like, which collectively provide for the replication, transcription and translation of a coding sequence in a recipient cell. Not all of these control sequences need always be present so long as the selected coding sequence is capable of being replicated, transcribed and translated in an appropriate host cell. One skilled in the art can readily identify regulatory nucleic acid sequence from public databases and materials. Furthermore, one skilled in the art can identify a regulatory sequence that is applicable for the intended use, for example, *in vivo, ex vivo,* or *in vitro.*

An internal ribosome entry sites ("IRES") refers to a segment of nucleic acid that promotes the entry or retention of a ribosome during translation of a coding sequence usually 3' to the IRES. In some embodiments the IRES may comprise a splice acceptor/donor site, however, preferred IRESs lack a splice acceptor/donor site. Normally, the entry of ribosomes into messenger RNA takes place via the cap located at the 5' end of all eukaryotic mRNAs. However, there are exceptions to this universal rule. The absence of a cap in some viral mRNAs suggests the existence of alternative structures permitting the entry of ribosomes at an internal site of these RNAs. To date, a number of these structures, designated IRES on account of their function, have been identified in the 5' noncoding region of uncapped viral mRNAs, such as that, in particular, of picornaviruses such as the poliomyelitis virus (Pelletier et al., 1988, Mol. Cell. Biol., 8, 1103-1112) and the EMCV virus (encephalo-myocarditis virus (Jang et al., J. Virol., 1988, 62, 2636-2643). The disclosure provides the use of an IRES in the context of a replication-competent retroviral vector.

The term "promoter region" is used herein in its ordinary sense to refer to a nucleotide region comprising a DNA regulatory sequence, wherein the regulatory sequence is derived from a gene which is capable of binding RNA polymerase and initiating transcription of a downstream (3'-direction) coding sequence. The regulatory sequence may be homologous or heterologous to the desired gene sequence. For example, a wide range of promoters may be utilized, including viral or mammalian promoter as described above.

The heterologous nucleic acid sequence is typically under control of either the viral LTR promoter-enhancer signals or an internal promoter, and retained signals within the retroviral LTR can still bring about efficient integration of the vector into the host cell genome. Accordingly, the recombinant retroviral vectors of the disclosure, the desired sequences, genes and/or gene fragments can be inserted at several sites and under different regulatory sequences. For example, a site for insertion can be the viral enhancer/promoter proximal site (*i.e*., 5' LTR-driven gene locus). Alternatively, the desired sequences can be inserted into a regulatory sequence distal site (*e.g*., the IRES sequence 3' to the env gene) or where two or more heterologous sequences are present one heterologous sequence may be under the control of a first regulatory region and a second heterologous sequence under the control of a second regulatory region. Other distal sites include viral promoter sequences, where the expression of the desired sequence or sequences is through splicing of the promoter proximal cistron, an internal heterologous promoter as SV40 or CMV, or an internal ribosome entry site (IRES) can be used.

In one embodiment, the retroviral genome of the disclosure contains an IRES comprising a cloning site downstream of the IRES for insertion of a desired/heterologous polynucleotide. In one embodiment, the IRES is located 3' to the env gene in the retroviral vector, but 5' to the desired heterologous polynucleotide. Accordingly, a heterologous polynucleotide encoding a desired polypeptide may be operably linked to the IRES.

In another embodiment, a targeting polynucleotide sequence is included as part of the recombinant retroviral vector of the disclosure. The targeting polynucleotide sequence is a targeting ligand (*e.g*., peptide hormones such as heregulin, a single-chain antibodies, a receptor or a ligand for a receptor), a tissue-specific or cell-type specific regulatory element (*e.g*., a tissue-specific or cell-type specific promoter or enhancer), or a combination of a targeting ligand and a tissue-specific/cell-type specific regulatory element. Preferably, the targeting ligand is operably linked to the env protein of the retrovirus, creating a chimeric retroviral env protein. The viral GAG, viral POL and viral ENV proteins can be derived from any suitable retrovirus (*e.g*., MLV or lentivirus-derived). In another embodiment, the viral ENV protein is non-retrovirus-derived (*e.g*., CMV or VSV).

In one embodiment, the recombinant retrovirus of the disclosure is genetically modified in such a way that the virus is targeted to a particular cell type (*e.g*., smooth muscle cells, hepatic cells, renal cells, fibroblasts, keratinocytes, mesenchymal stem cells, bone marrow cells, chondrocyte, epithelial cells, intestinal cells, mammary cells, neoplastic cells, glioma cells, neuronal cells and others known in the art) such that the recombinant genome of the retroviral vector is delivered to a target non-dividing, a target dividing cell, or a target cell having a cell proliferative disorder.

In one embodiment, the retroviral vector is targeted to the cell by binding to cells having a molecule on the external surface of the cell. This method of targeting the retrovirus utilizes expression of a targeting ligand on the coat of the retrovirus to assist in targeting the virus to cells or tissues that have a receptor or binding molecule which interacts with the targeting ligand on the surface of the retrovirus. After infection of a cell by the virus, the virus injects its nucleic acid into the cell and the retrovirus genetic material can integrate into the host cell genome.

In another embodiment, targeting uses cell- or tissue-specific regulatory elements to promote expression and transcription of the viral genome in a targeted cell which actively utilizes the regulatory elements, as described more fully below. The transferred retrovirus genetic material is then transcribed and translated into proteins within the host cell. The targeting regulatory element is typically linked to the 5' and/or 3' LTR, creating a chimeric LTR.

By inserting a heterologous polynucleotide of interest into the viral vector of the disclosure, along with another gene which encodes, for example, the ligand for a receptor on a specific target cell, the vector is now target specific. Viral vectors can be made target specific by attaching, for example, a sugar, a glycolipid, or a protein. Targeting can be accomplished by using an antibody to target the viral vector. Those of skill in the art will know of, or can readily ascertain, specific polynucleotide sequences which can be inserted into the viral genome or proteins which can be attached to a viral envelope to allow target specific delivery of the viral vector containing the nucleic acid sequence of interest.

Thus, the disclosure includes in one embodiment, a chimeric env protein comprising a retroviral ENV protein operably linked to a targeting polypeptide. The targeting polypeptide can be a cell specific receptor molecule, a ligand for a cell specific receptor, an antibody or antibody fragment to a cell specific antigenic epitope or any other ligand easily identified in the art which is capable of binding or interacting with a target cell. Examples of targeting polypeptides or molecules include bivalent antibodies using biotin-streptavidin as linkers (Etienne-Julan et al., J. Of General Virol., 73, 3251-3255 (1992); Roux et al., Proc. Natl. Acad. Sci USA 86, 9079-9083 (1989)), recombinant virus containing in its envelope a sequence encoding a single-chain antibody variable region against a hapten (Russell et al., Nucleic Acids Research, 21, 1081-1085 (1993)), cloning of peptide hormone ligands into the retrovirus envelope (Kasahara et al., Science, 266, 1373-1376 (1994)), chimeric EPO/env constructs (Kasahara et al., 1994), single-chain antibody against the low density lipoprotein (LDL) receptor in the ecotropic MLV envelope, resulting in specific infection of HeLa cells expressing LDL receptor (Somia et al., Proc. Natl. Acad. Sci USA, 92, 7570-7574 (1995)), similarly the host range of ALV can be altered by incorporation of an integrin ligand, enabling the virus to now cross species to specifically infect rat glioblastoma cells (Valsesia-Wittmann et al., J. Virol. 68, 4609-4619 (1994)), and Dornberg and co-workers (Chu and Dornburg, J. Virol 69, 2659-2663 (1995)) have reported tissue-specific targeting of spleen necrosis virus (SNV), an avian retrovirus, using envelopes containing single-chain antibodies directed against tumor markers.

The disclosure provides a method of producing a recombinant retrovirus capable of infecting a target cell comprising transfecting a suitable host cell with the following: a vector comprising a polynucleotide sequence encoding a viral gag, a viral pol and a viral env, and a heterologous polynucleotide, operably linked to a regulatory nucleic acid sequence, and recovering the recombinant virus.

The retrovirus and methods of the disclosure provide a replication competent retrovirus that does not require helper virus or additional nucleic acid sequence or proteins in order to propagate and produce virion. For example, the nucleic acid sequences of the retrovirus of the disclosure encode, for example, a group specific antigen and reverse transcriptase, (and integrase and protease-enzymes necessary for maturation and reverse transcription), respectively, as discussed above. The viral gag and pol can be derived from a lentivirus, such as HIV or an oncovirus or gammaretrovirus such as MoMLV. In addition, the nucleic acid genome of the retrovirus of the disclosure includes a sequence encoding a viral envelope (ENV) protein. The env gene can be derived from any retroviruses. The env may be an amphotropic envelope protein which allows transduction of cells of human and other species, or may be an ecotropic envelope protein, which is able to transduce only mouse and rat cells. Further, it may be desirable to target the recombinant virus by linkage of the envelope protein with an antibody or a particular ligand for targeting to a receptor of a particular cell-type. As mentioned above, retroviral vectors can be made target specific by inserting, for example, a glycolipid, or a protein. Targeting is often accomplished by using an antibody to target the retroviral vector to an antigen on a particular cell-type (*e.g*., a cell type found in a certain tissue, or a cancer cell type). Those of skill in the art will know of, or can readily ascertain without undue experimentation, specific methods to achieve delivery of a retroviral vector to a specific target. In one embodiment, the env gene is derived from a non-retrovirus (*e.g*., CMV or VSV). Examples of retroviral-derived env genes include, but are not limited to: Moloney murine leukemia virus (MoMuLV), Harvey murine sarcoma virus (HaMuSV), murine mammary tumor virus (MuMTV), gibbon ape leukemia virus (GaLV), human immunodeficiency virus (HIV) and Rous Sarcoma Virus (RSV). Other env genes such as Vesicular stomatitis virus (VSV) (Protein G), cytomegalovirus envelope (CMV), or influenza virus hemagglutinin (HA) can also be used.

In one embodiment, the retroviral genome is derived from an onco-retrovirus, and more particularly a mammalian onco-retrovirus. By "derived" is meant that the parent polynucleotide sequence is an wild-type oncovirus which has been modified by insertion or removal of naturally occurring sequences (*e.g*., insertion of an IRES, insertion of a heterologous polynucleotide encoding a polypeptide or inhibitory nucleic acid of interest, swapping of a more effective promoter from a different retrovirus or virus in place of the wild-type promoter and the like).

Unlike recombinant retroviruses produced by standard methods in the art that are defective and require assistance in order to produce infectious vector particles, the disclosure provides a retrovirus that is replication-competent.

In another embodiment, the disclosure provides retroviral vectors that are targeted using regulatory sequences. Cell- or tissue-specific regulatory sequences (*e.g*., promoters) can be utilized to target expression of gene sequences in specific cell populations. Suitable mammalian and viral promoters for the disclosure are described elsewhere herein. Accordingly, in one embodiment, the disclosure provides a retrovirus having tissue-specific promoter elements at the 5' end of the retroviral genome. Typically, the tissue-specific regulatory elements/sequences are in the U3 region of the LTR of the retroviral genome, including for example cell- or tissue-specific promoters and enhancers to neoplastic cells (*e.g*., tumor cell-specific enhancers and promoters), and inducible promoters (*e.g*., tetracycline).

Transcription control sequences of the disclosure can also include naturally occurring transcription control sequences naturally associated with a gene encoding a superantigen, a cytokine or a chemokine.

In some circumstances, it may be desirable to regulate expression. For example, different viral promoters with varying strengths of activity may be utilized depending on the level of expression desired. In mammalian cells, the CMV immediate early promoter if often used to provide strong transcriptional activation. Modified versions of the CMV promoter that are less potent have also been used when reduced levels of expression of the transgene are desired. When expression of a transgene in hematopoietic cells is desired, retroviral promoters such as the LTRs from MLV or MMTV can be used. Other viral promoters that can be used include SV40, RSV LTR, HIV-1 and HIV-2 LTR, adenovirus promoters such as from the E1A, E2A, or MLP region, AAV LTR, cauliflower mosaic virus, HSV-TK, and avian sarcoma virus.

Similarly tissue specific or selective promoters may be used to effect transcription in specific tissues or cells so as to reduce potential toxicity or undesirable effects to non-targeted tissues. For example, promoters such as the PSA, probasin, prostatic acid phosphatase or prostate-specific glandular kallikrein (hK2) may be used to target gene expression in the prostate. The Whey accessory protein (WAP) may be used for breast tissue expression (Andres et al., PNAS 84:1299-1303, 1987). Other promoters/regulatory domains that can be used are set forth in Table 1.

"Tissue-specific regulatory elements" are regulatory elements (*e.g*., promoters) that are capable of driving transcription of a gene in one tissue while remaining largely "silent" in other tissue types. It will be understood, however, that tissue-specific promoters may have a detectable amount of "background" or "base" activity in those tissues where they are silent. The degree to which a promoter is selectively activated in a target tissue can be expressed as a selectivity ratio (activity in a target tissue/activity in a control tissue). In this regard, a tissue specific promoter useful in the practice of the disclosure typically has a selectivity ratio of greater than about 5. Preferably, the selectivity ratio is greater than about 15.

In certain indications, it may be desirable to activate transcription at specific times after administration of the recombinant replication competent retrovirus of the disclosure (RRCR). This may be done with promoters that are hormone or cytokine regulatable. For example in therapeutic applications where the indication is a gonadal tissue where specific steroids are produced or routed to, use of androgen or estrogen regulated promoters may be advantageous. Such promoters that are hormone regulatable include MMTV, MT-1, ecdysone and RuBisco. Other hormone regulated promoters such as those responsive to thyroid, pituitary and adrenal hormones may be used. Cytokine and inflammatory protein responsive promoters that could be used include K and T Kininogen (Kageyama *et al.,* 1987), c-fos, TNF-alpha, C-reactive protein (Arcone *et al.,* 1988), haptoglobin (Oliviero *et al.,* 1987), serum amyloid A2, C/EBP alpha, IL-1, IL-6 (Poli and Cortese, 1989), Complement C3 (Wilson *et al.,* 1990), IL-8, alpha-1 acid glycoprotein (Prowse and Baumann, 1988), alpha-1 antitypsin, lipoprotein lipase (Zechner *et al.,* 1988), angiotensinogen (Ron *et al*., 1990), fibrinogen, c-jun (inducible by phorbol esters, TNF-alpha, UV radiation, retinoic acid, and hydrogen peroxide), collagenase (induced by phorbol esters and retinoic acid), metallothionein (heavy metal and glucocorticoid inducible), Stromelysin (inducible by phorbol ester, interleukin-1 and EGF), alpha-2 macroglobulin and alpha-1 antichymotrypsin. Tumor specific promoters such as osteocalcin, hypoxia-responsive element (HRE), MAGE-4, CEA, alpha-fetoprotein, GRP78/BiP and tyrosinase may also be used to regulate gene expression in tumor cells.

In addition, this list of promoters should not be construed to be exhaustive or limiting, those of skill in the art will know of other promoters that may be used in conjunction with the promoters and methods disclosed herein.

**TABLE 1 TISSUE SPECIFIC PROMOTERS**

| Tissue | Promoter |
|---|---|
| Pancreas | Insulin Elastin Amylase pdr-1 pdx-1 glucokinase |
| Liver | Albumin PEPCK HBV enhancer α fetoprotein apolipoprotein C α-1 antitrypsin vitellogenin, NF-AB Transthyretin |
| Skeletal muscle | Myosin H chain Muscle creatine kinase Dystrophin Calpain p94 Skeletal alpha-actin fast troponin 1 |
| Skin Lung | Keratin K6 Keratin K1 CFTR Human cytokeratin 18 (K18) Pulmonary surfactant proteins A, B and C CC-10 P1 |
| Smooth muscle Endothelium | sm22 α SM-alpha-actin Endothelin-1 E-selectin von Willebrand factor TIE (Korhonen et al., 1995) KDR/flk-1 Melanocytes Tyrosinase |
| Adipose tissue | Lipoprotein lipase (Zechner *et al.,* 1988) Adipsin (Spiegelman *et al.,* 1989) acetyl-CoA carboxylase (Pape and Kim, 1989) glycerophosphate dehydrogenase (Dani *et al.,* 1989) adipocyte P2 (Hunt *et al.,* 1986) |
| Breast | Whey Acidic Protien (WAP)(Andres et al. PNAS 84:1299-1303 1987 |
| Blood | β-globin |

It will be further understood that certain promoters, while not restricted in activity to a single tissue type, may nevertheless show selectivity in that they may be active in one group of tissues, and less active or silent in another group. Such promoters are also termed "tissue specific", and are contemplated for use with the disclosure. For example, promoters that are active in a variety of central nervous system (CNS) neurons may be therapeutically useful in protecting against damage due to stroke, which may effect any of a number of different regions of the brain. Accordingly, the tissue-specific regulatory elements used in the disclosure, have applicability to regulation of the heterologous proteins as well as a applicability as a targeting polynucleotide sequence in the present retroviral vectors.

In yet another embodiment, the disclosure provides plasmids comprising a recombinant retroviral derived construct. The plasmid can be directly introduced into a target cell or a cell culture such as NIH 3T3 or other tissue culture cells. The resulting cells release the retroviral vector into the culture medium.

The disclosure provides a polynucleotide construct comprising from 5' to 3': a promoter or regulatory region useful for initiating transcription; a psi packaging signal; a *gag* encoding nucleic acid sequence, a *pol* encoding nucleic acid sequence; an env encoding nucleic acid sequence; an internal ribosome entry site nucleic acid sequence; a heterologous polynucleotide encoding a marker, therapeutic or diagnostic polypeptide; and a LTR nucleic acid sequence. As described elsewhere herein and as follows the various segment of the polynucleotide construct of the disclosure (*e.g*., a recombinant replication competent retoviral polynucleotide) are engineered depending in part upon the desired host cell, expression timing or amount, and the heterologous polynucleotide. A replication competent retroviral construct of the disclosure can be divided up into a number of domains that may be individually modified by those of skill in the art.

For example, the promoter can comprise a CMV promoter having a sequence as set forth in SEQ ID NO:19, 20 or 22 from nucleotide 1 to about nucleotide 582 and may include modification to one or more (*e.g*., 2-5, 5-10, 10-20, 20-30, 30-50, 50-100 or more nucleic acid bases) so long as the modified promoter is capable of directing and initiating transcription. In one embodiment, the promoter or regulatory region comprises a CMV-R-U5 domain polynucleotide. The CMV-R-U5 domain comprise the immediately early promoter from human cytomegalovirus to the MLV R-U5 region. In one embodiment, the CMV-R-U5 domain polynucleotide comprises a sequence as set forth in SEQ ID NO:19, 20 or 22 from about nucleotide 1 to about nucleotide 1202 or sequences that are at least 95% identical to a sequence as set forth in SEQ ID NO:19, 20, or 22 wherein the polynucleotide promotes transcription of a nucleic acid molecule operably linked thereto. The gag domain of the polynucleotide may be derived from any number of retroviruses, but will typically be derived from an oncoretrovirus and more particularly from a mammalian oncoretrovirus. In one embodiment the gag domain comprises a sequence from about nucleotide number 1203 to about nucleotide 2819 or a sequence having at least 95%, 98%, 99% or 99.8% (rounded to the nearest 10^{th}) identity thereto. The pol domain of the polynucleotide may be derived from any number of retroviruses, but will typically be derived from an oncoretrovirus and more particularly from a mammalian oncoretrovirus. In one embodiment the pol domain comprises a sequence from about nucleotide number 2820 to about nucleotide 6358 or a sequence having at least 95%, 98%, 99% or 99.9% (roundest to the nearest 10^{th}) identity thereto. The env domain of the polynucleotide may be derived from any number of retroviruses, but will typically be derived from an oncoretrovirus or gamma-retrovirus and more particularly from a mammalian oncoretrovirus or gamma-retrovirus. In some embodiments the env coding domain comprises an amphotropic env domain. In one embodiment the env domain comprises a sequence from about nucleotide number 6359 to about nucleotide 8323 or a sequence having at least 95%, 98%, 99% or 99.8% (roundest to the nearest 10^{th}) identity thereto. The IRES domain of the polynucleotide may be obtained from any number of internal ribosome entry sites. In one embodiment, IRES is derived from an encephalomyocarditis virus. In one embodiment the IRES domain comprises a sequence from about nucleotide number 8327 to about nucleotide 8876 or a sequence having at least 95%, 98%, or 99% (roundest to the nearest 10^{th}) identity thereto so long as the domain allows for entry of a ribosome. The heterologous domain can comprise a cytosine deaminase of the disclosure. In one embodiment, the CD polynucleotide comprises a human codon optimized sequence. In yet another embodiment, the CD polynucleotide encodes a mutant polypeptide having cytosine deaminase, wherein the mutations confer increased thermal stabilization that increase the melting temperature (Tm) by 10°C allowing sustained kinetic activity over a broader temperature range and increased accumulated levels of protein. In one embodiment, the cytosine deaminase comprises a sequence as set forth in SEQ ID NO:19 or 22 from about nucleotide number 8877 to about 9353. The heterologous domain may be followed by a polypurine rich domain. The 3' LTR can be derived from any number of retroviruses, typically an oncoretrovirus and preferably a mammalian oncoretrovirus. In one embodiment, the 3' LTR comprises a U3-R-U5 domain. In yet another embodiment the LTR comprises a sequence as set forth in SEQ ID NO:19 or 22 from about nucleotide 9405 to about 9998 or a sequence that is at least 95%, 98% or 99.5% (rounded to the nearest 10^{th}) identical thereto.

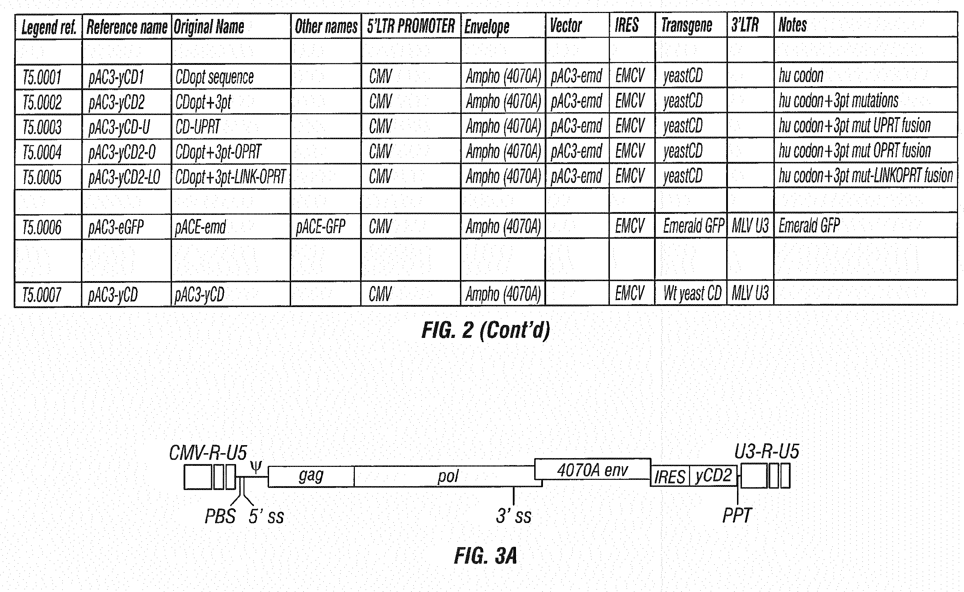

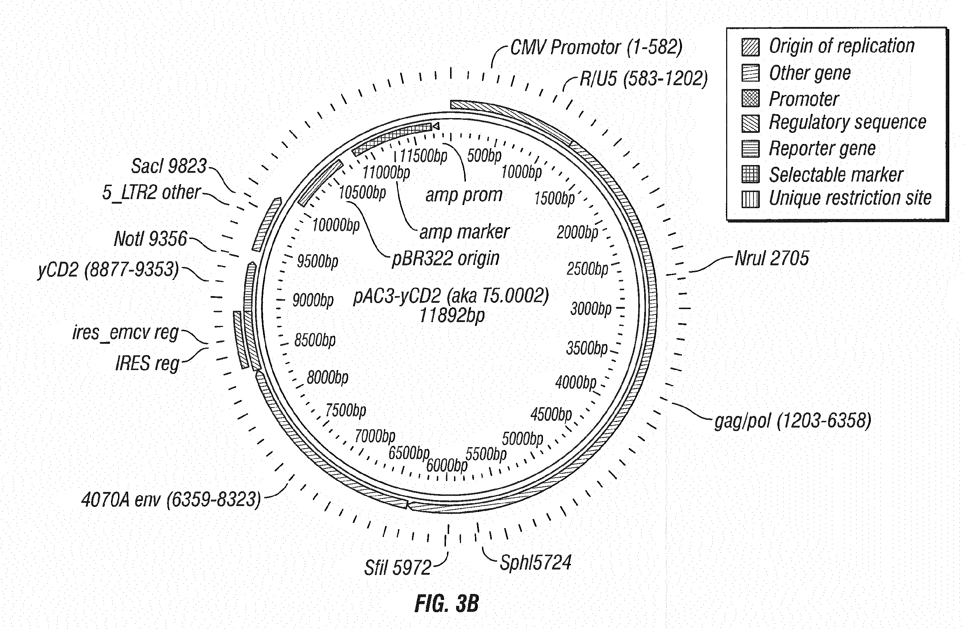

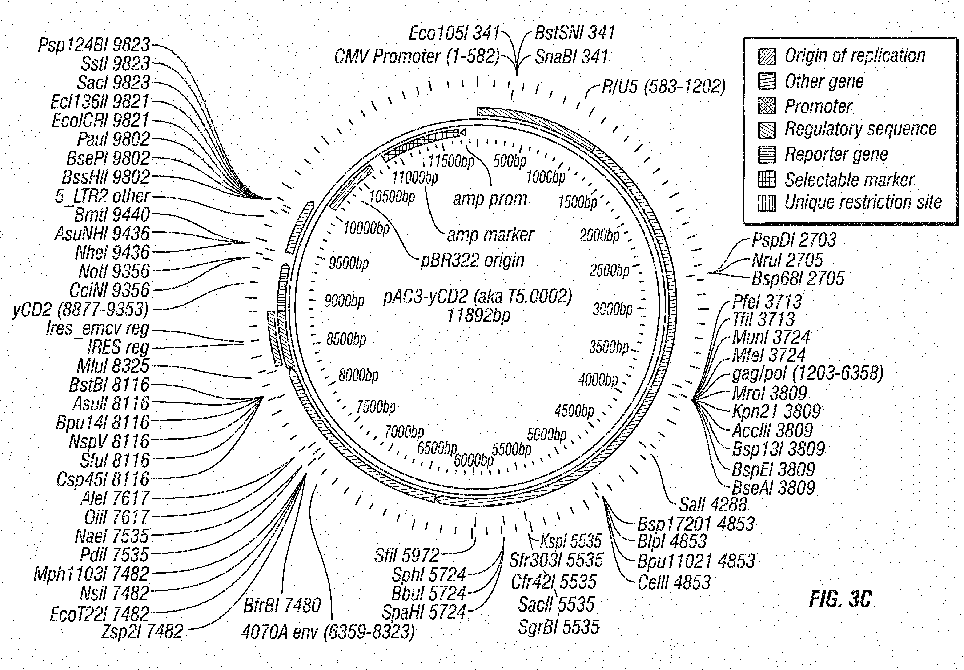

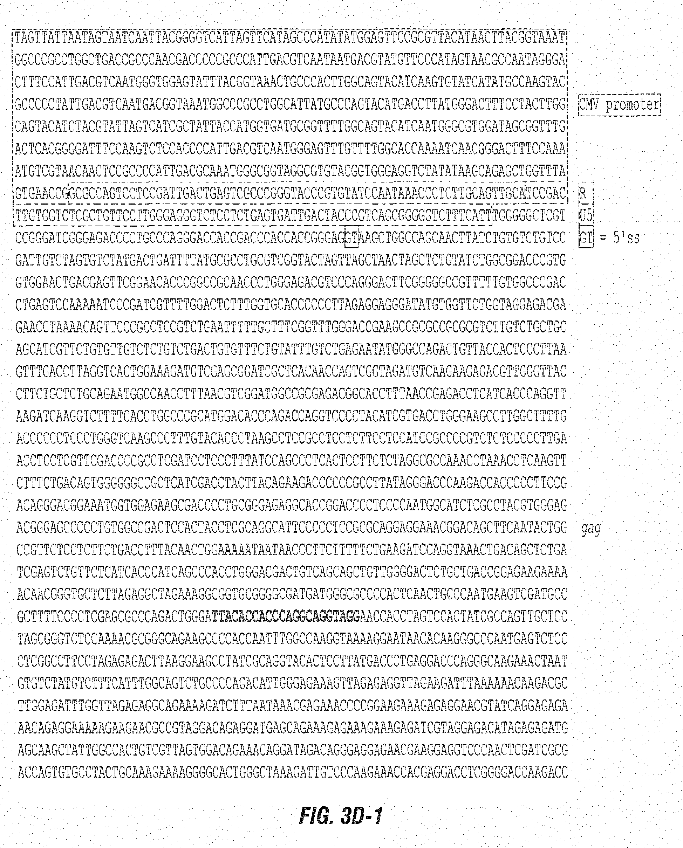

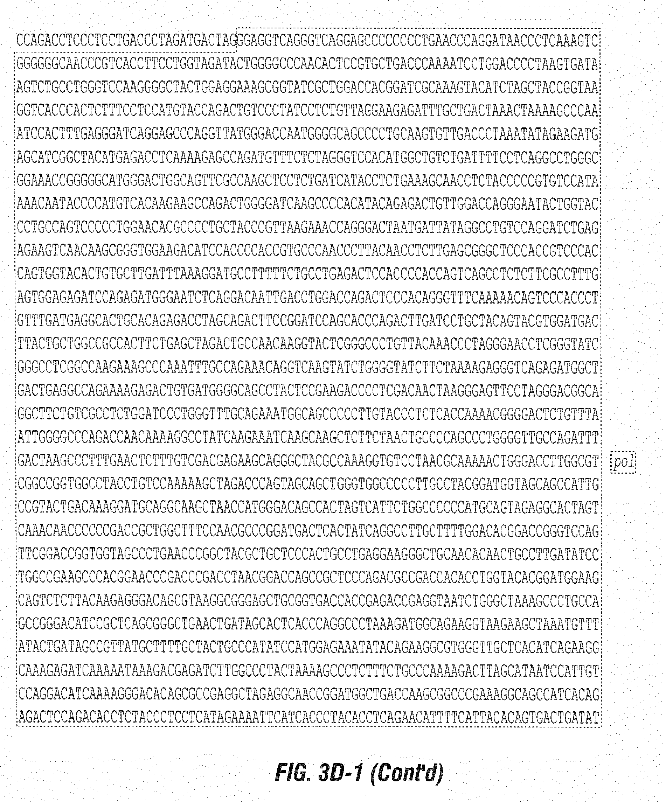

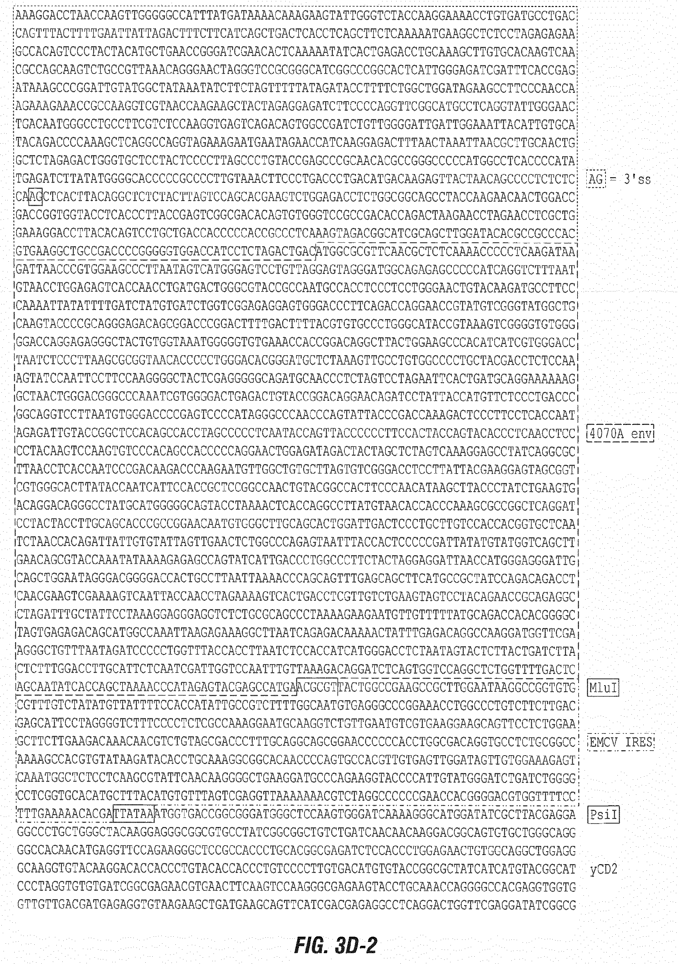

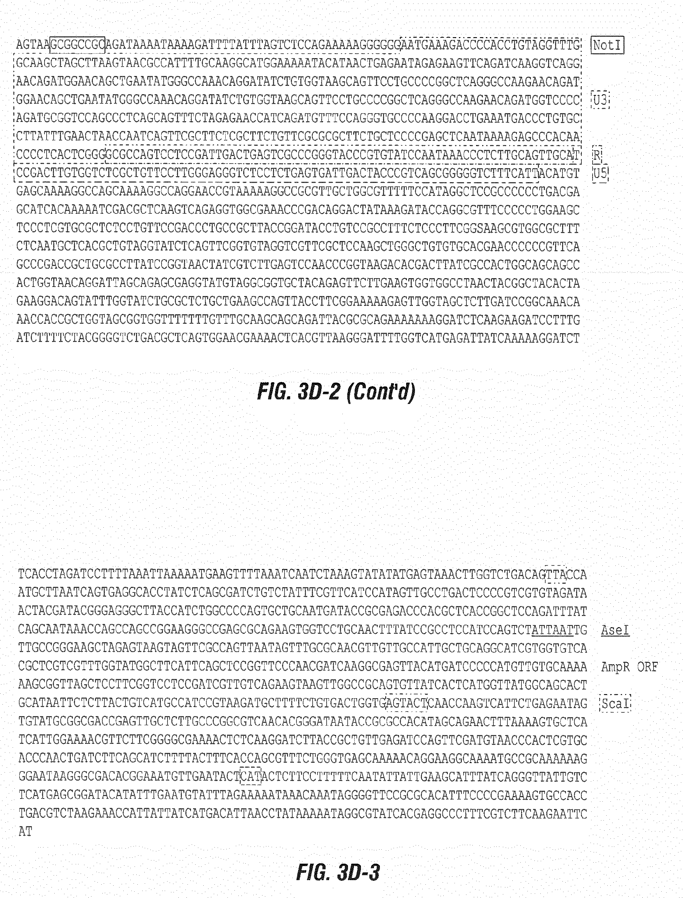

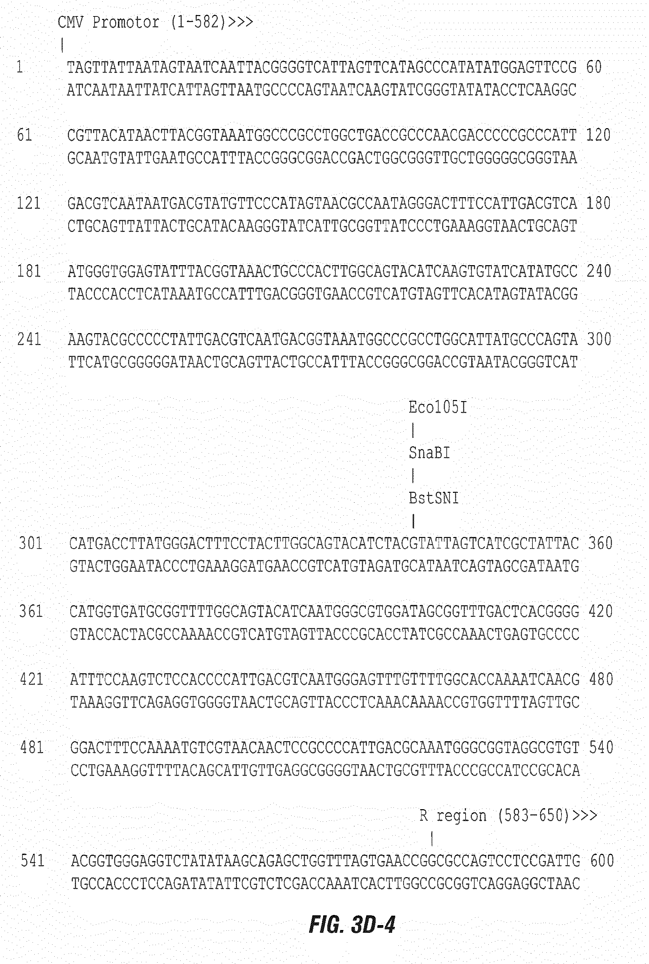

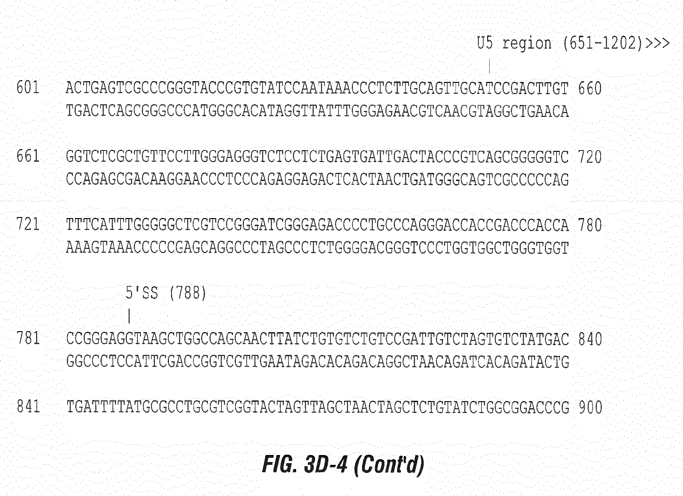

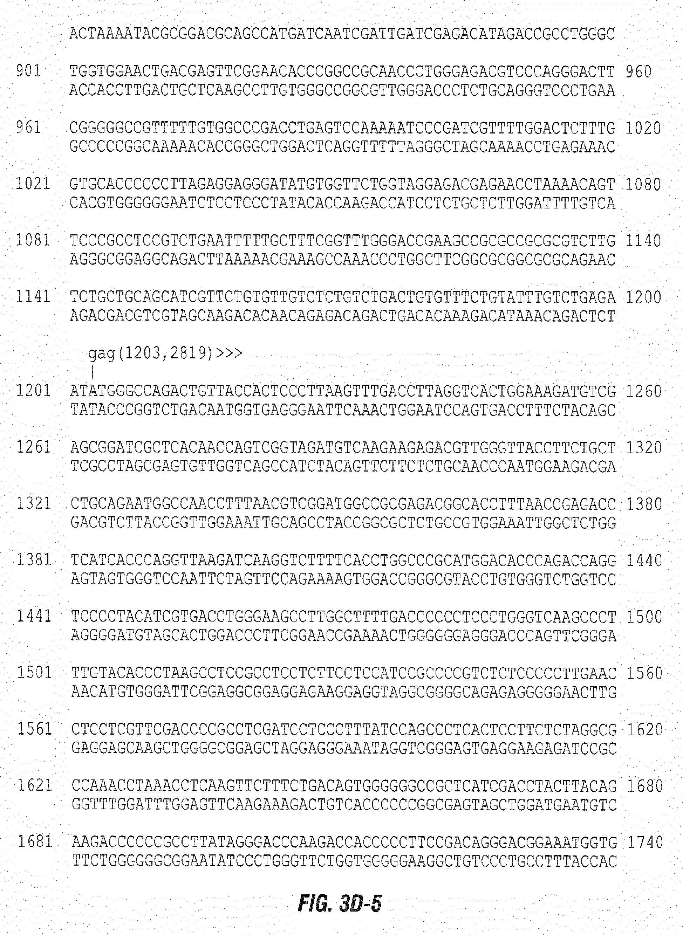

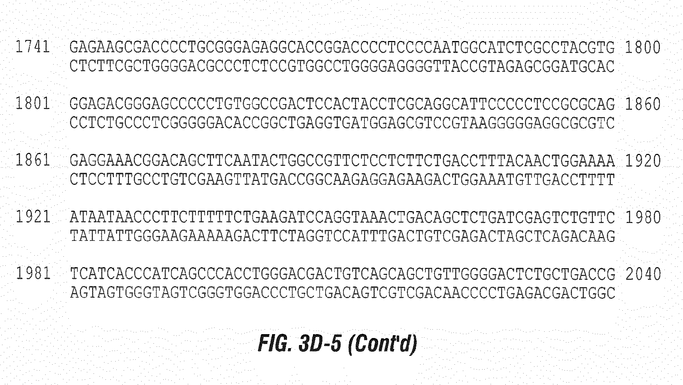

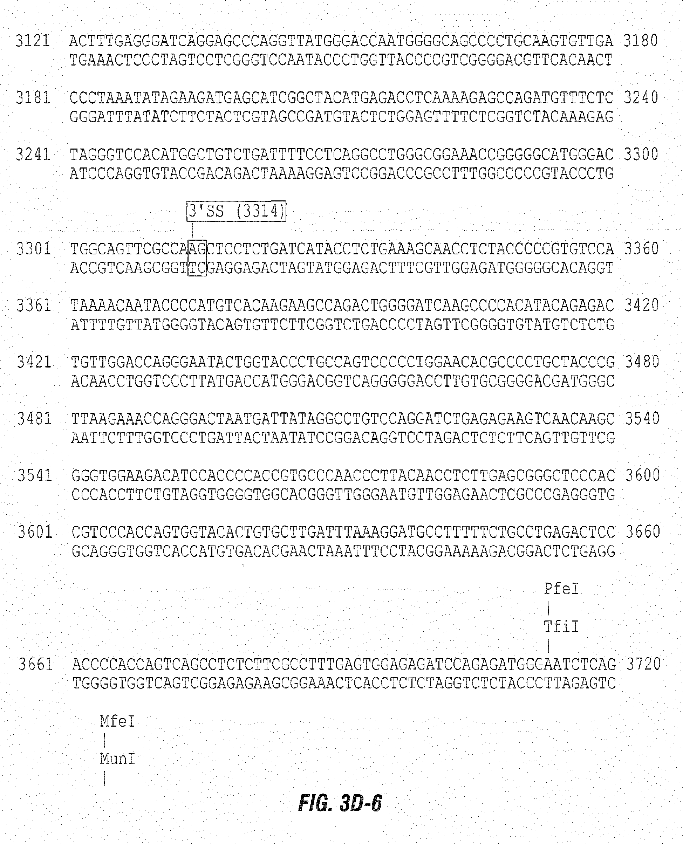

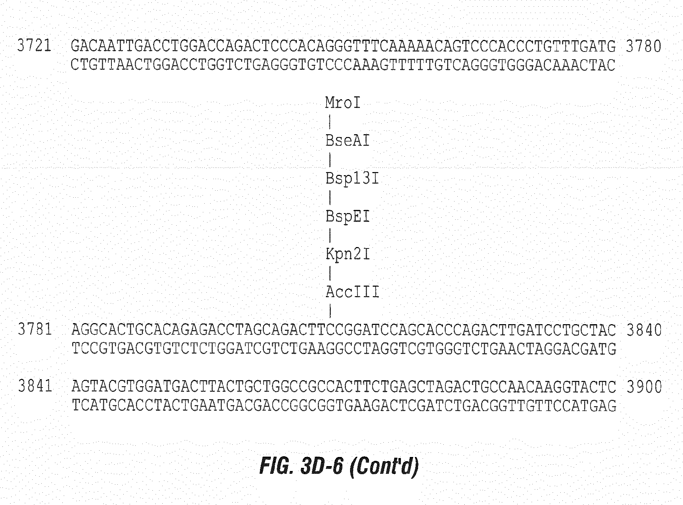

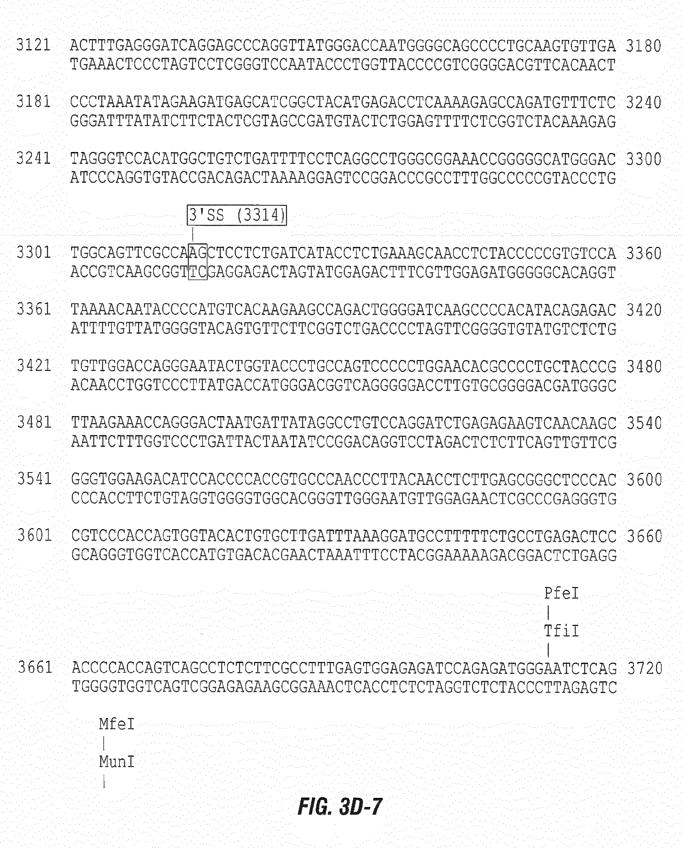

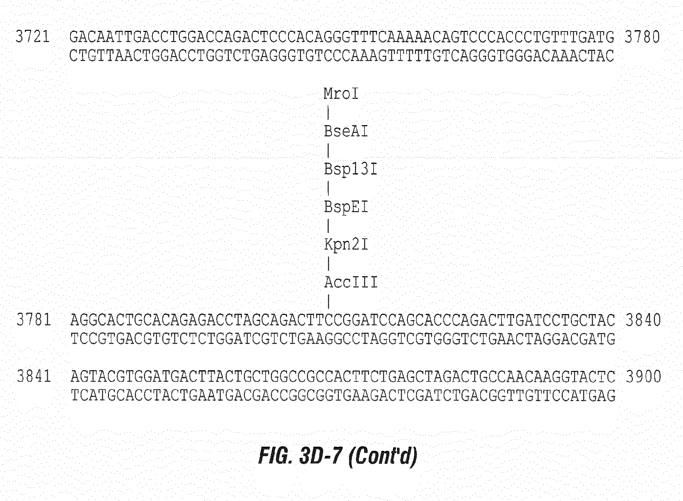

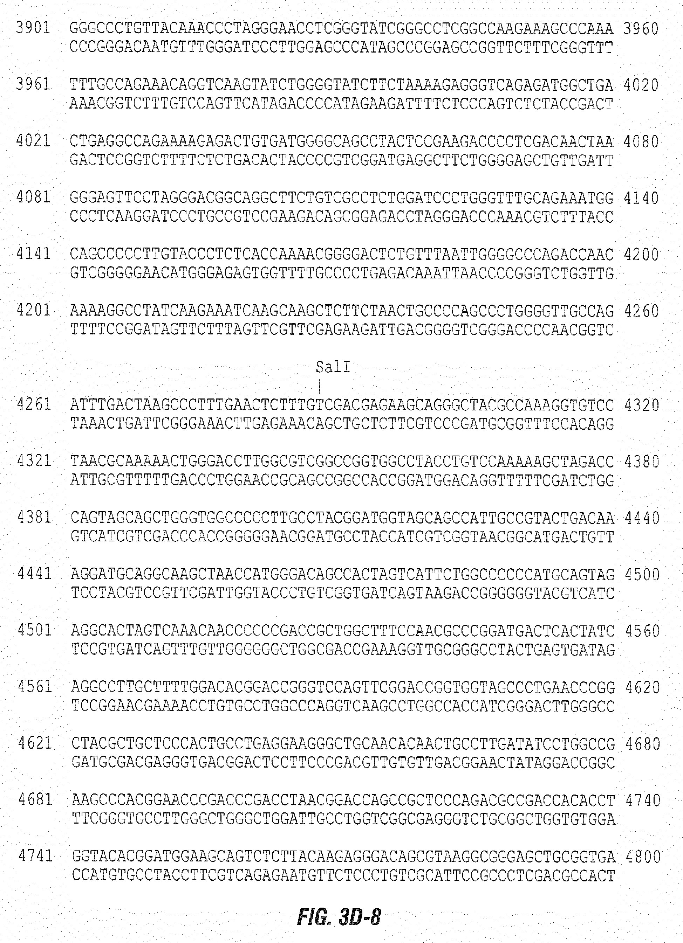

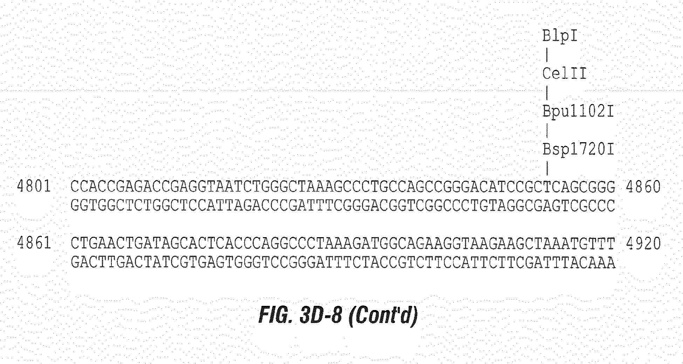

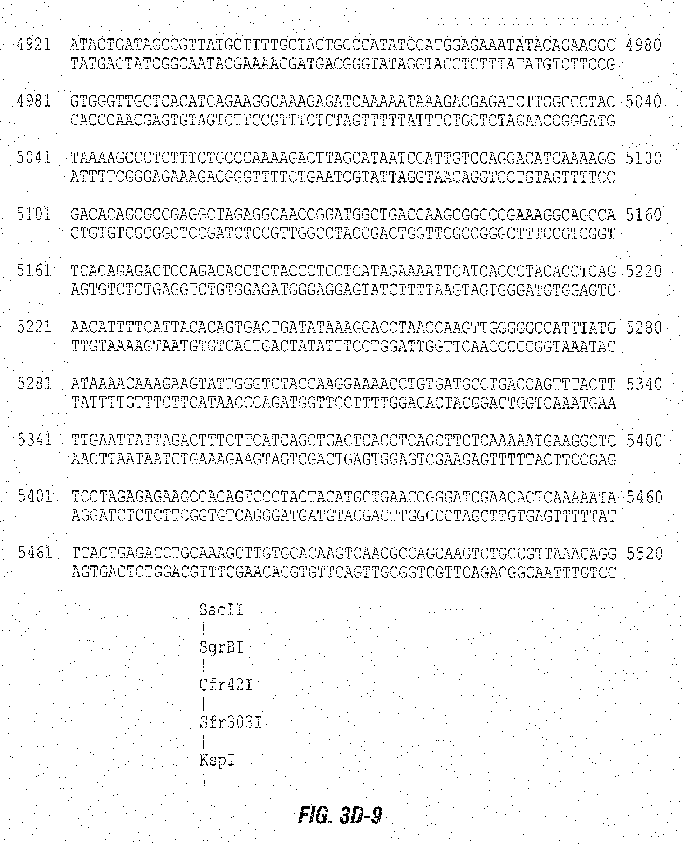

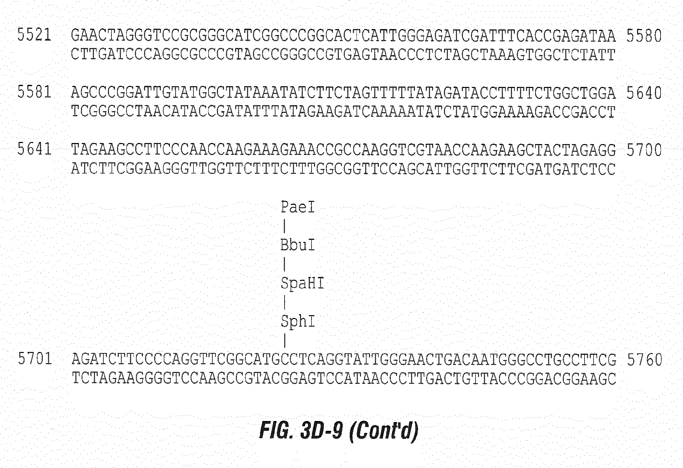

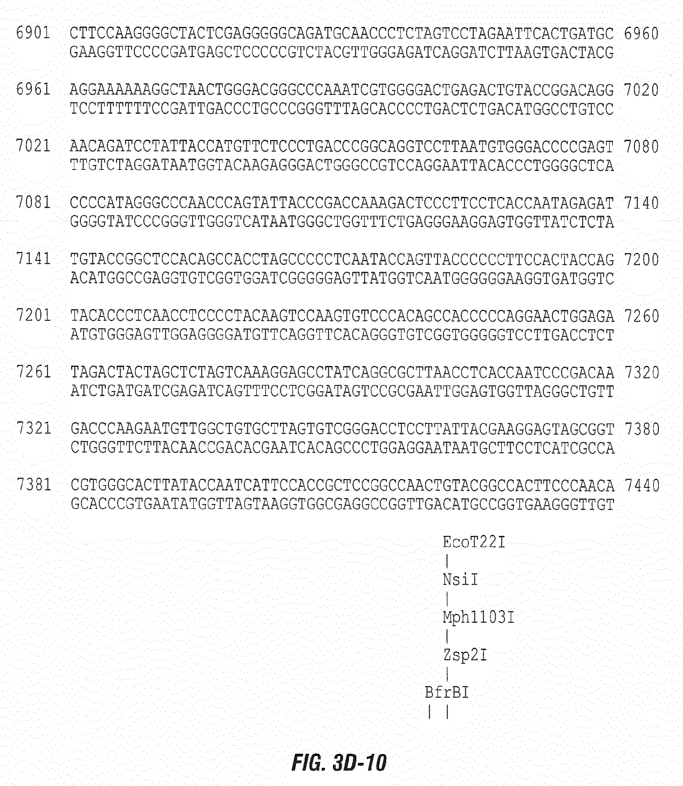

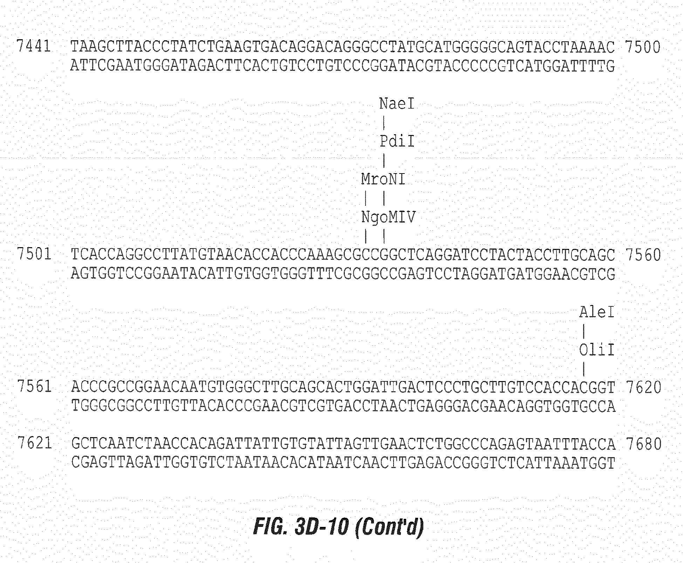

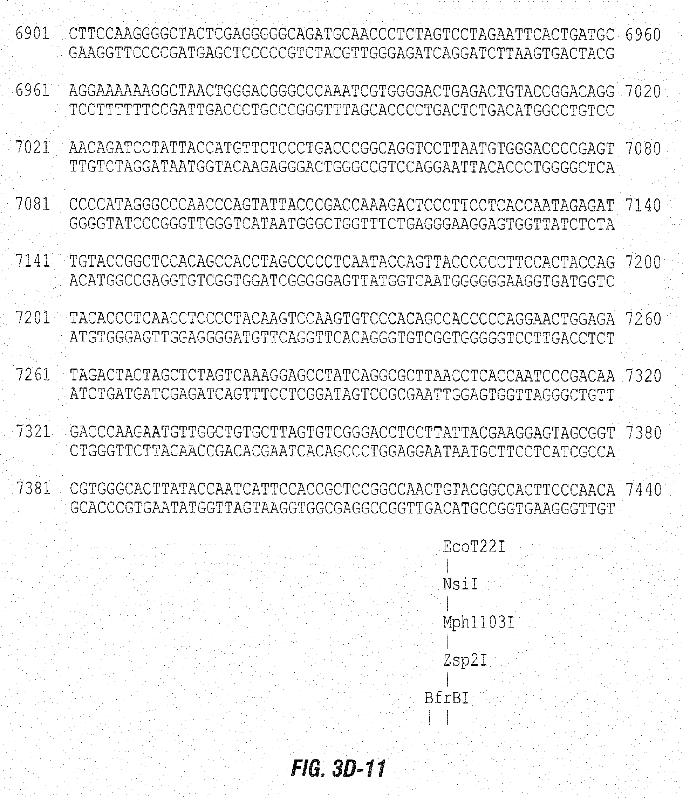

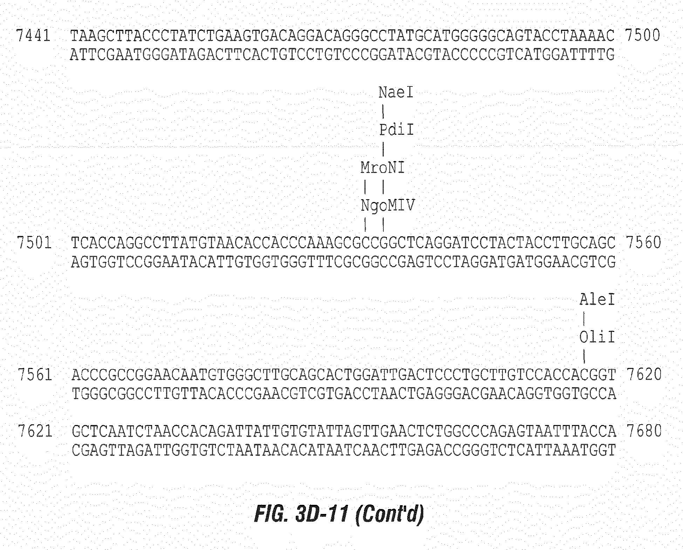

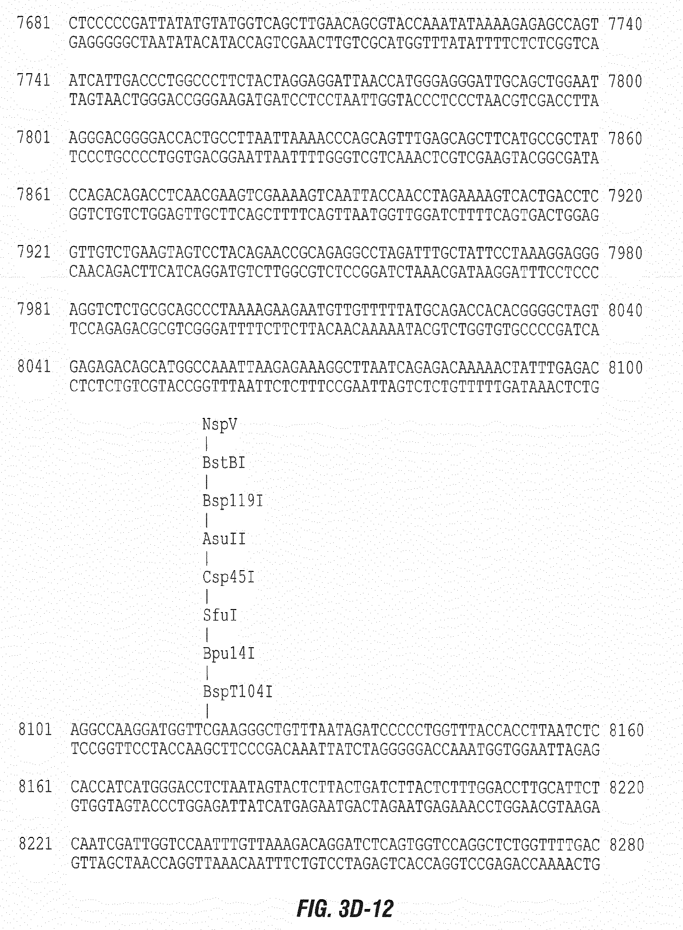

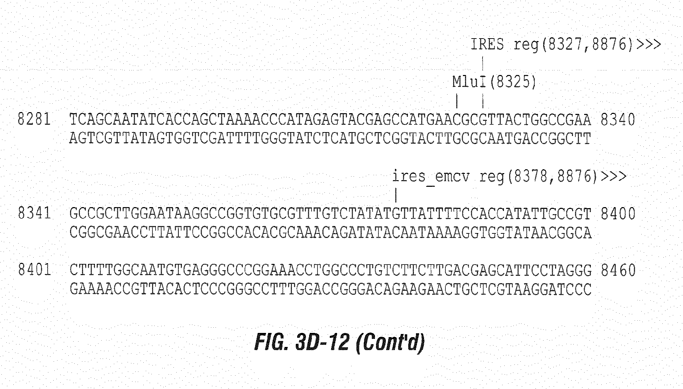

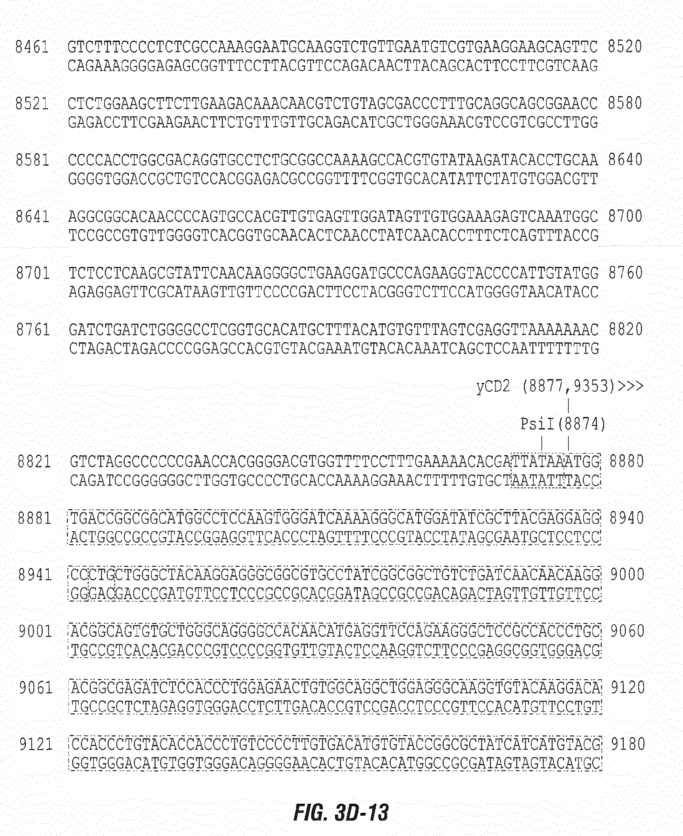

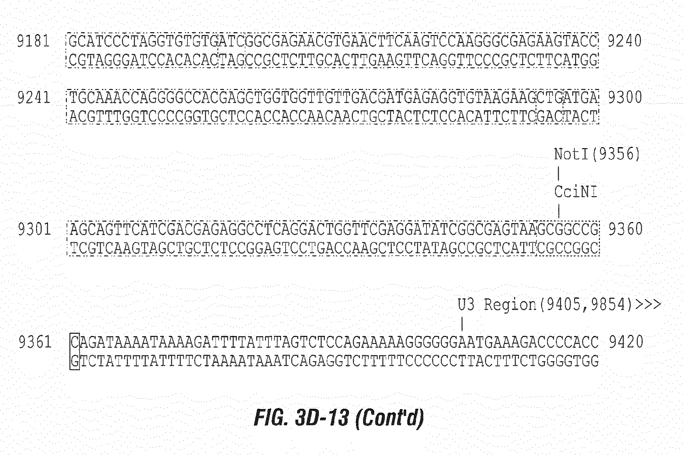

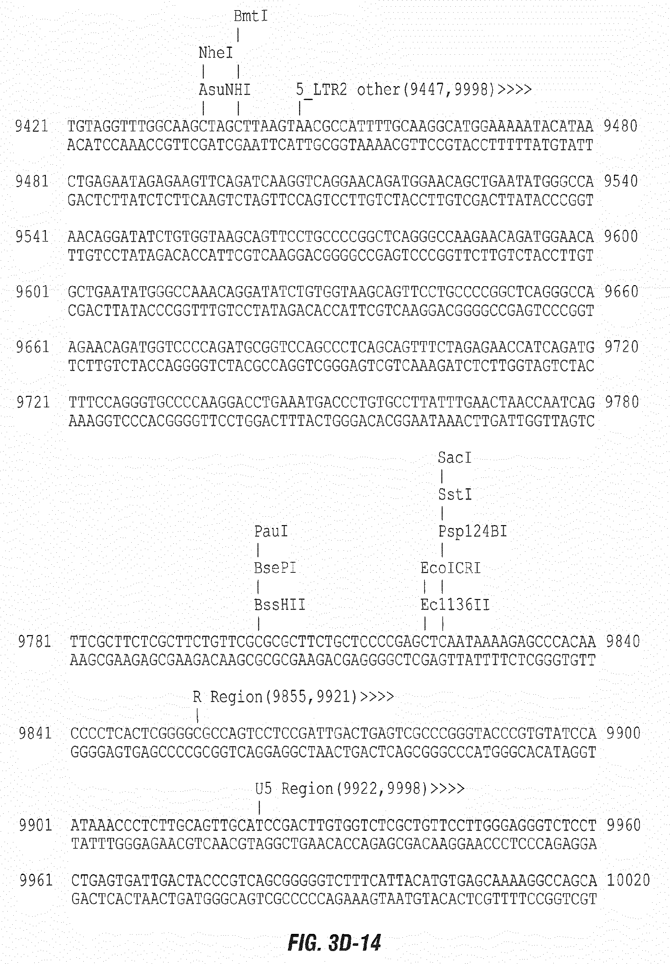

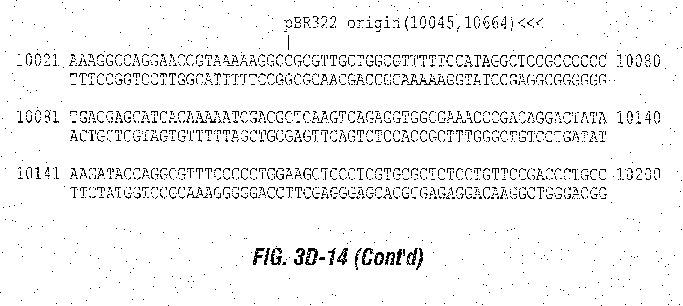

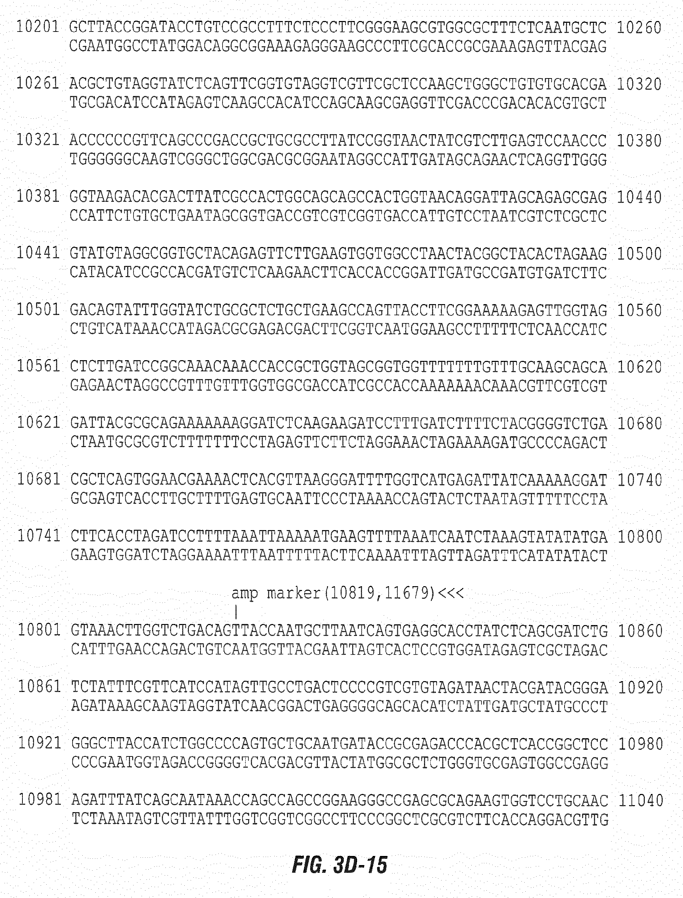

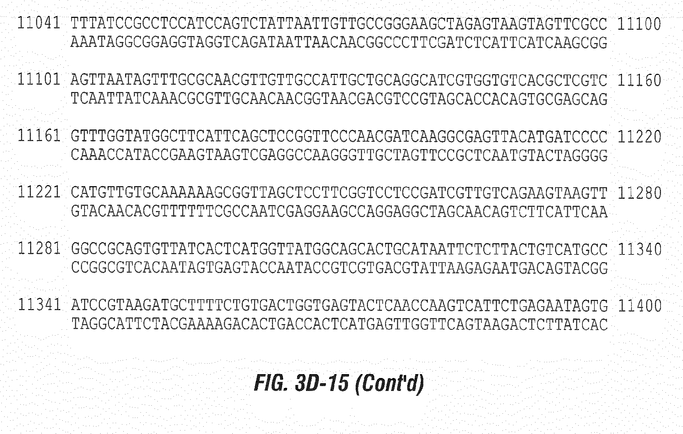

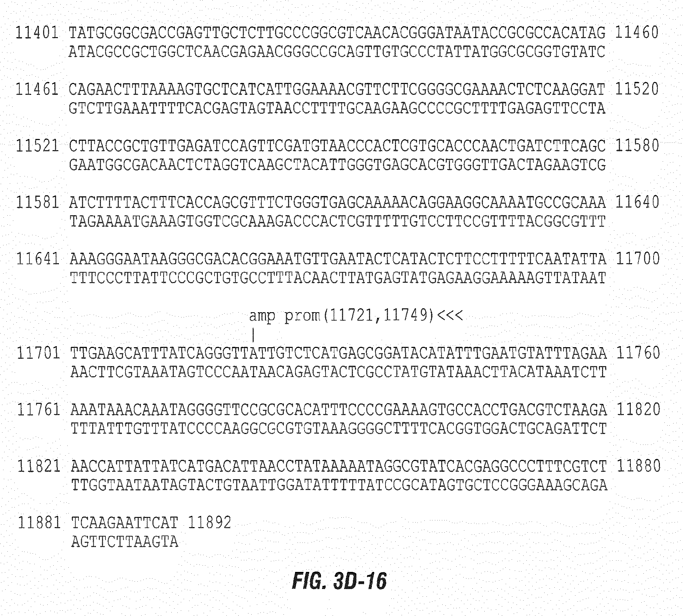

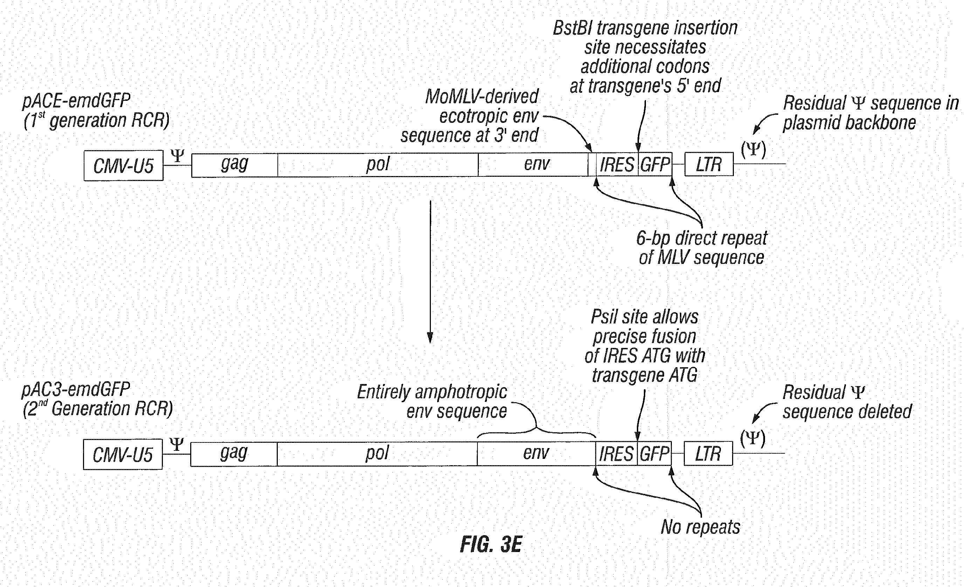

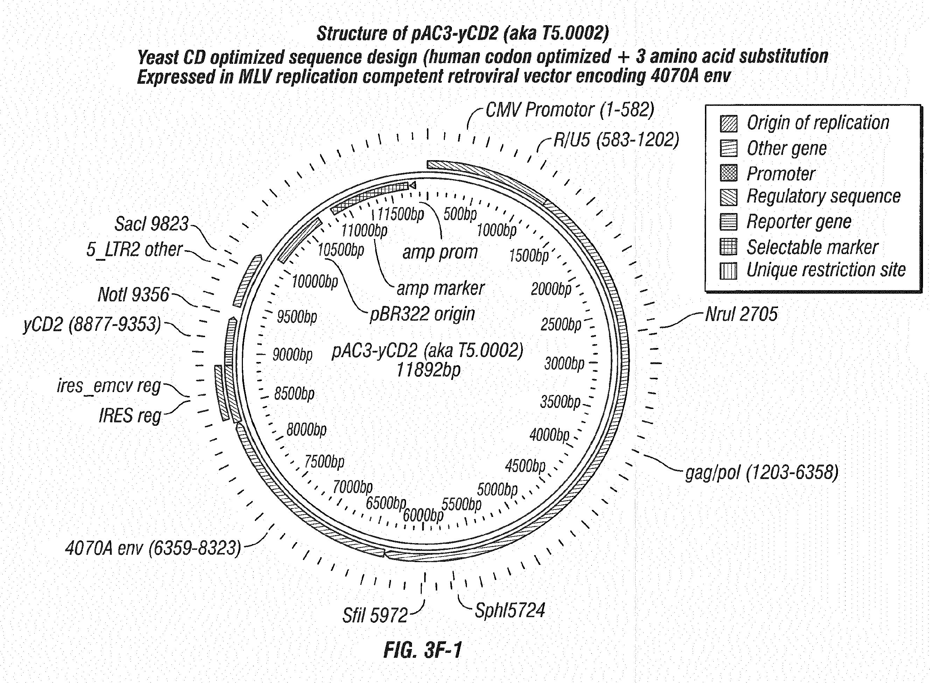

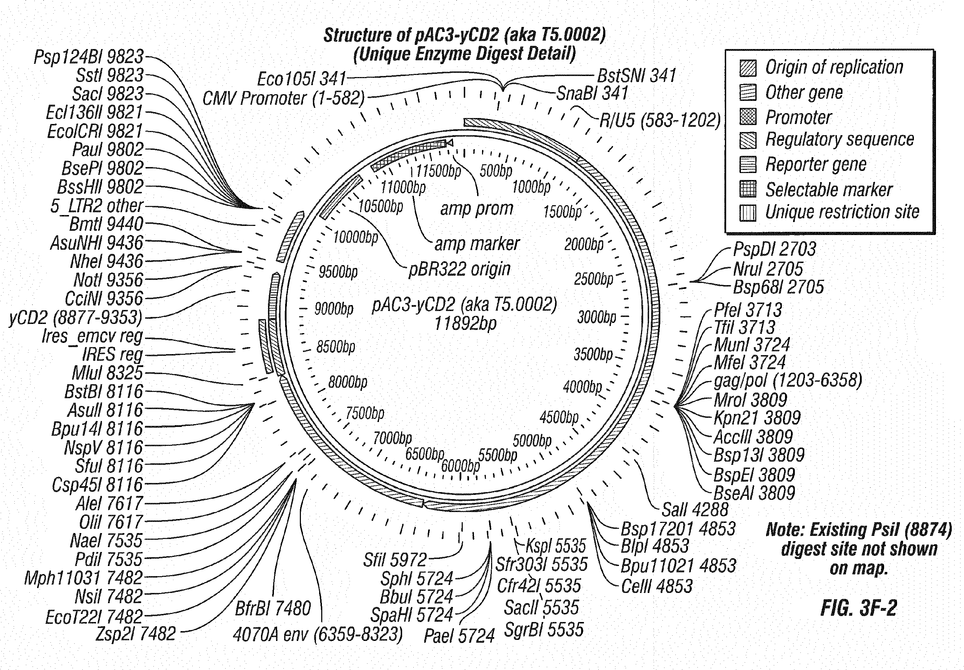

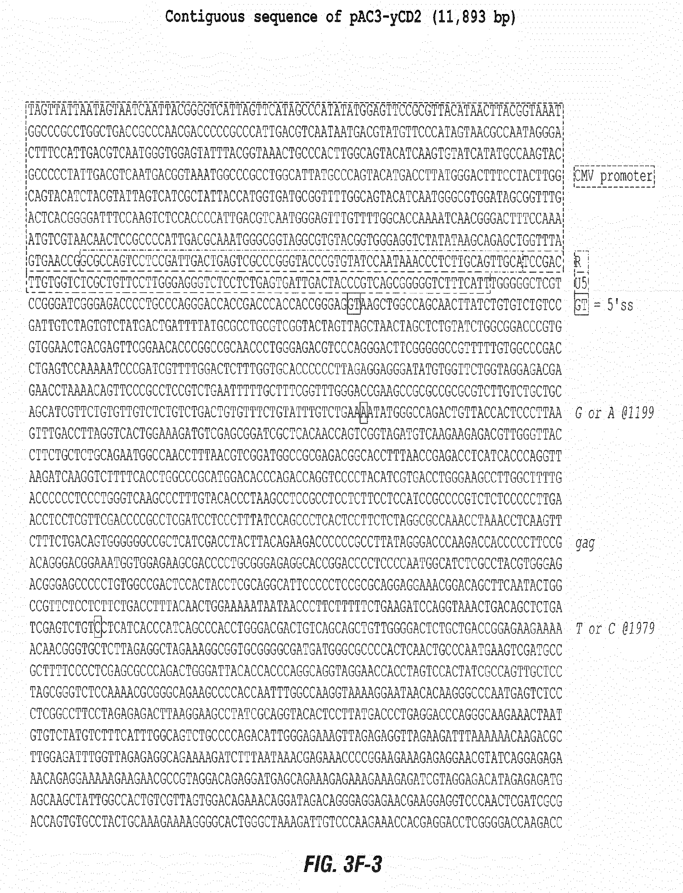

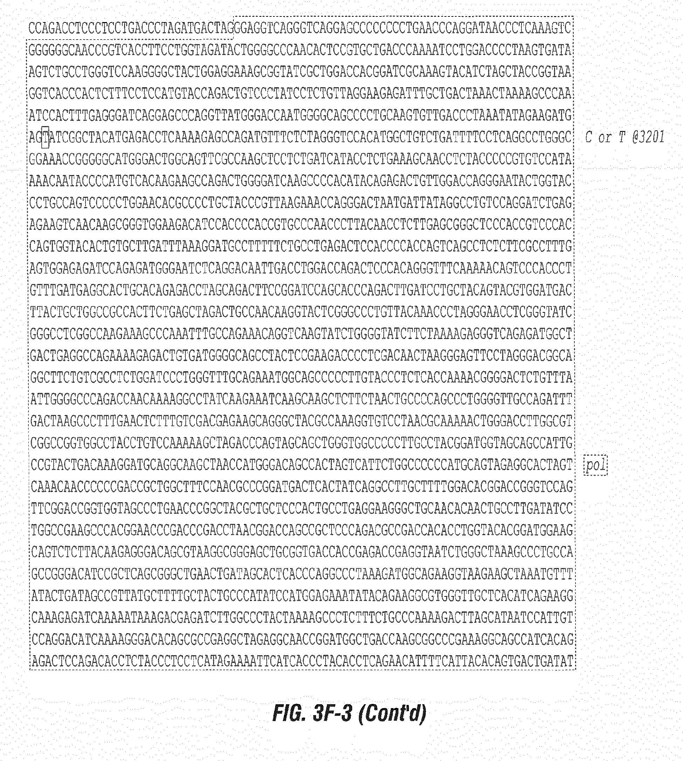

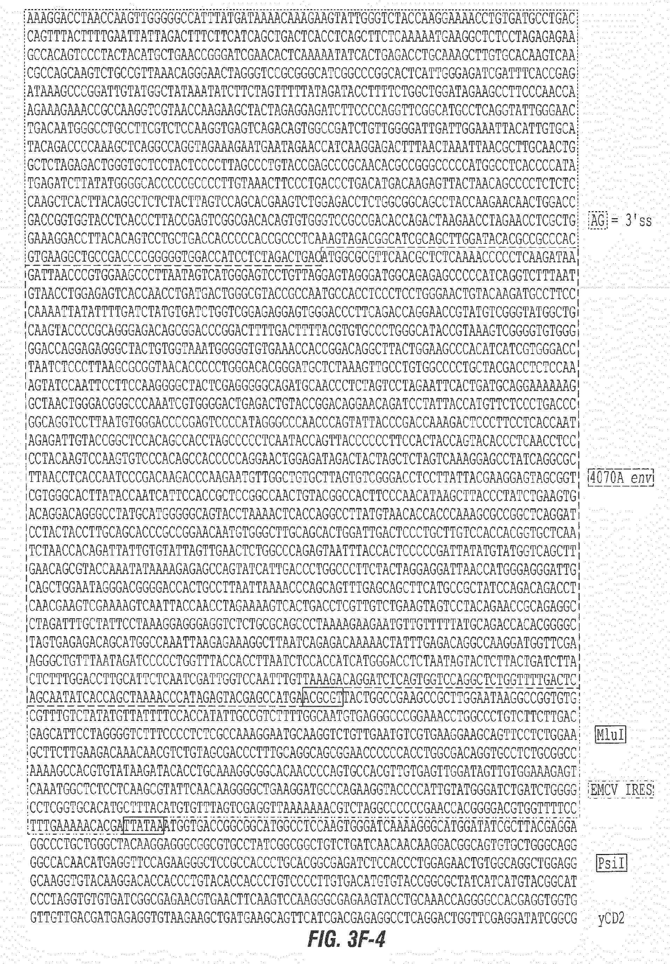

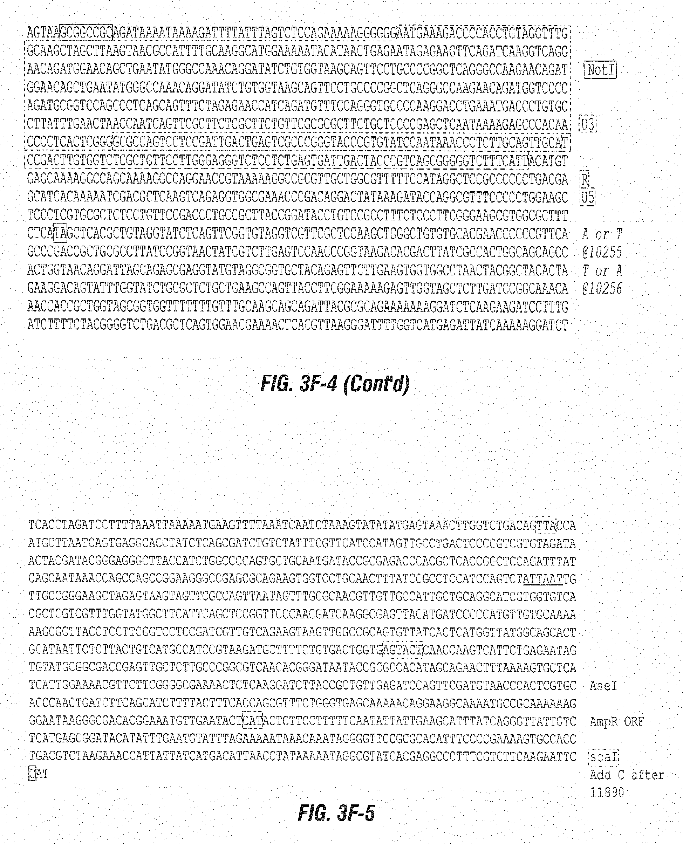

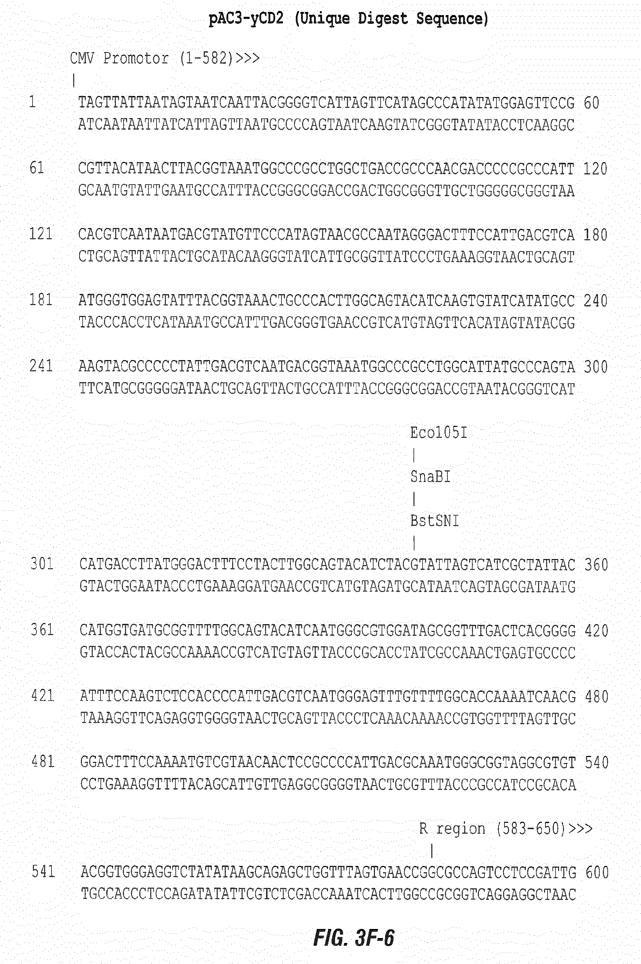

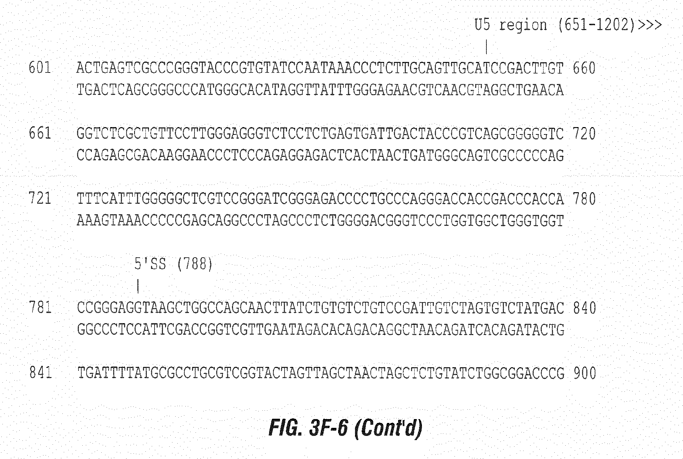

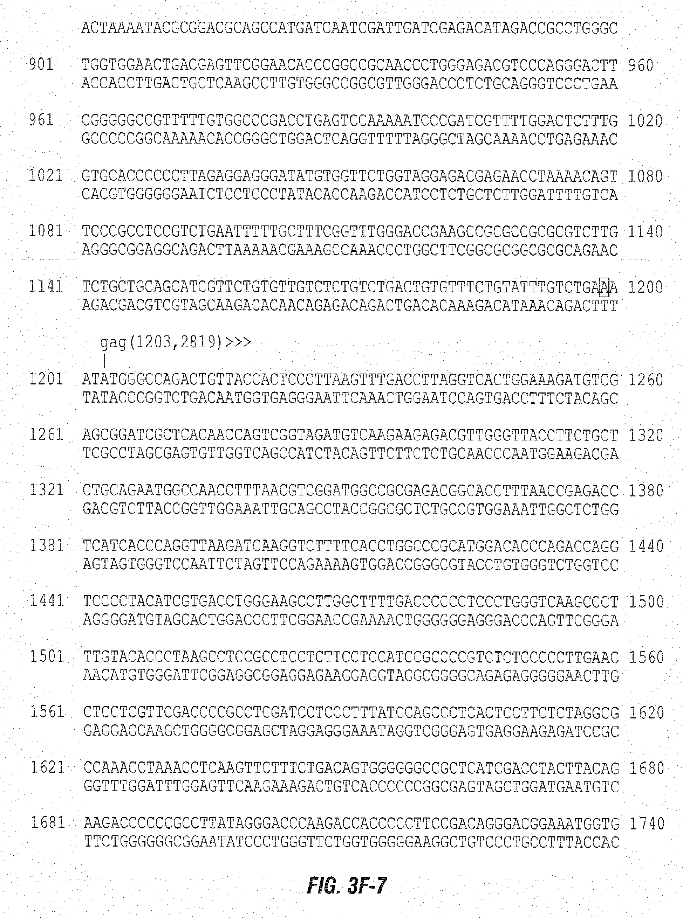

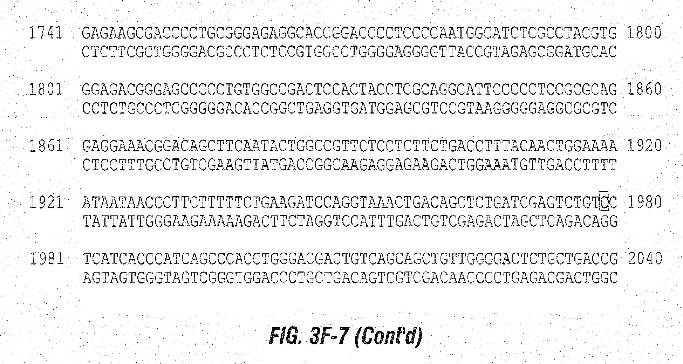

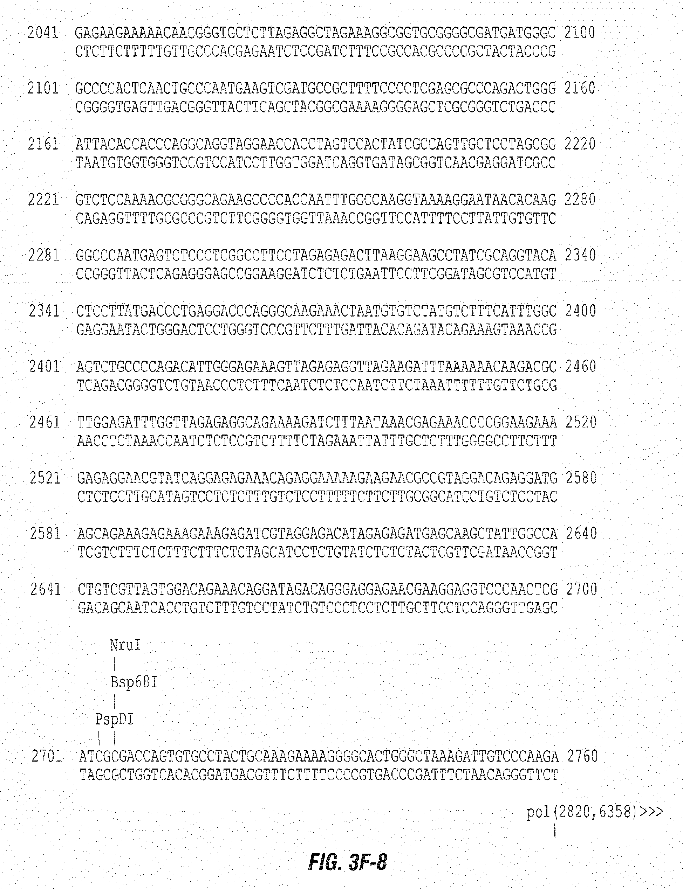

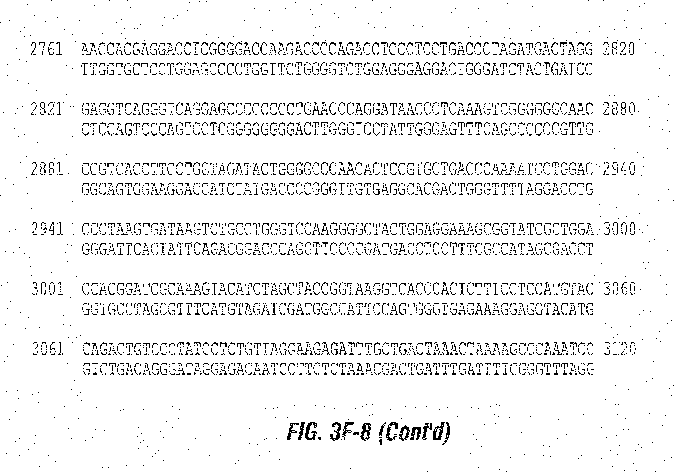

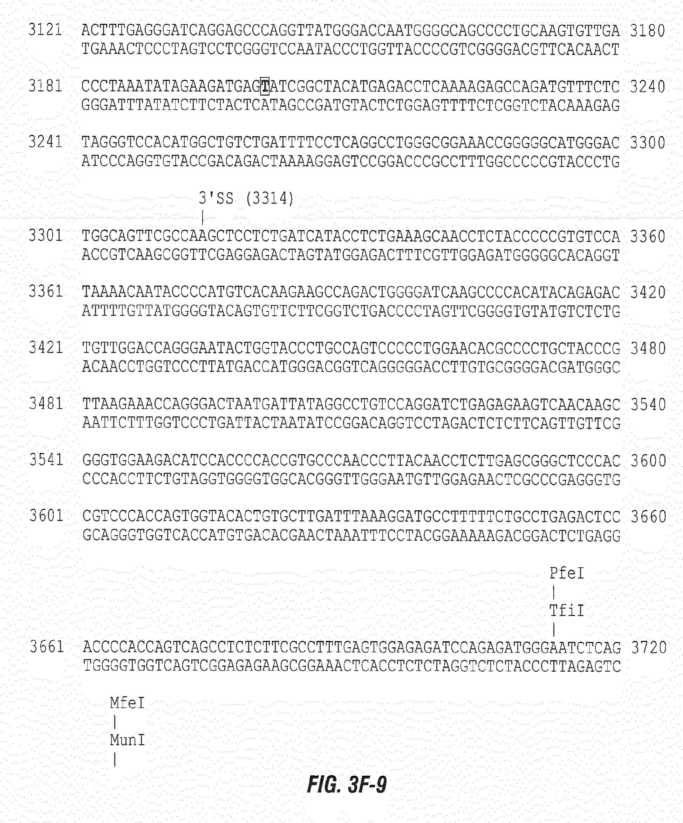

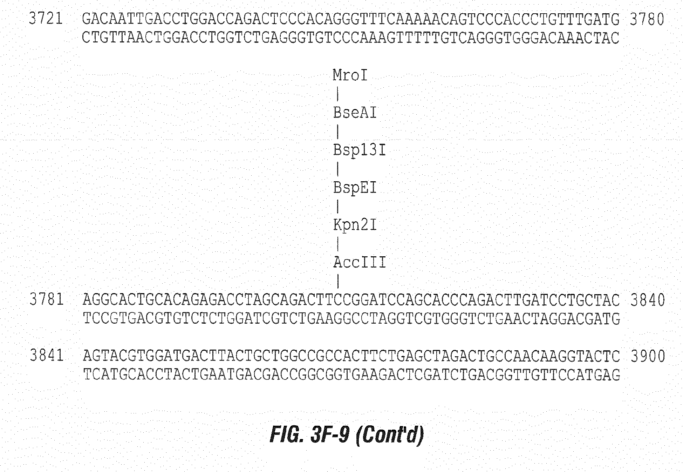

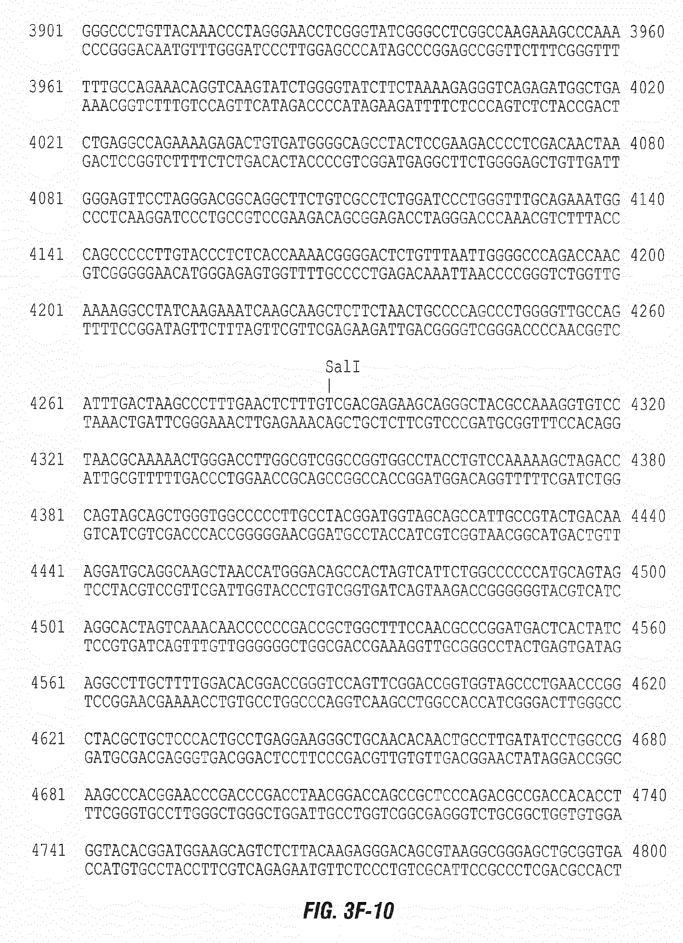

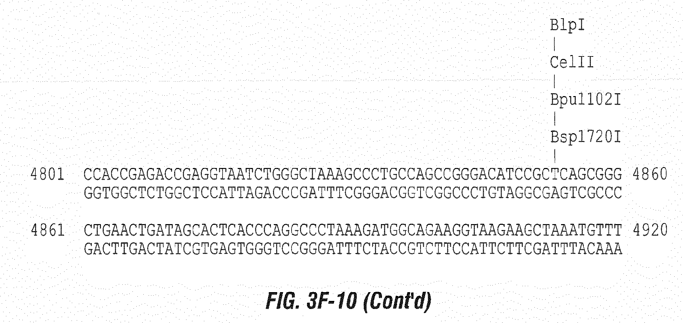

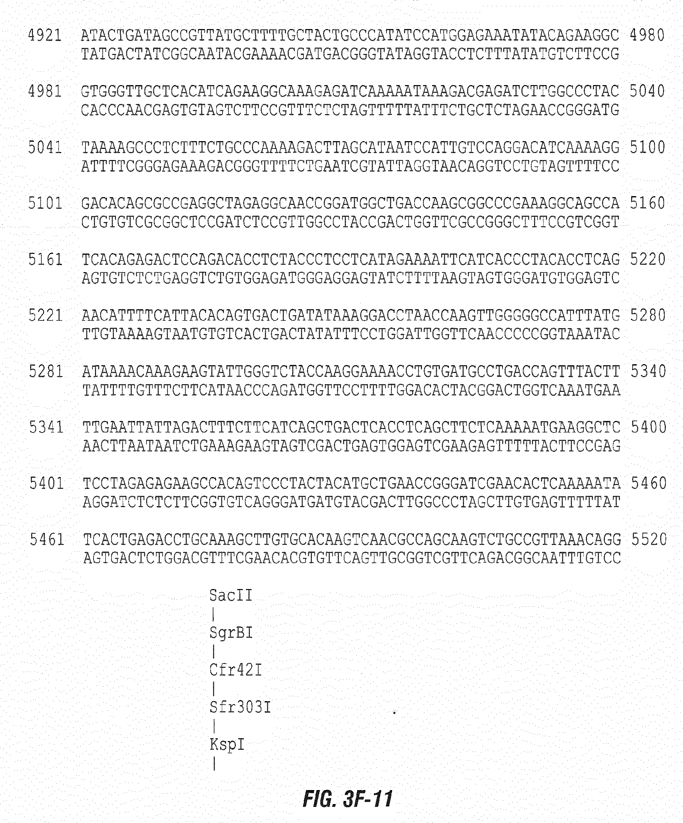

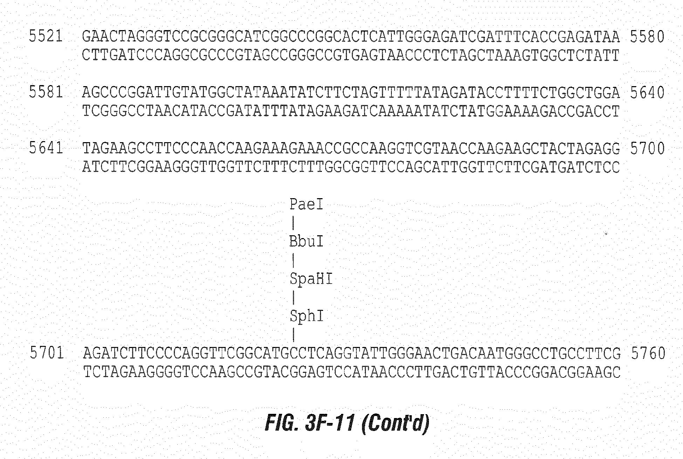

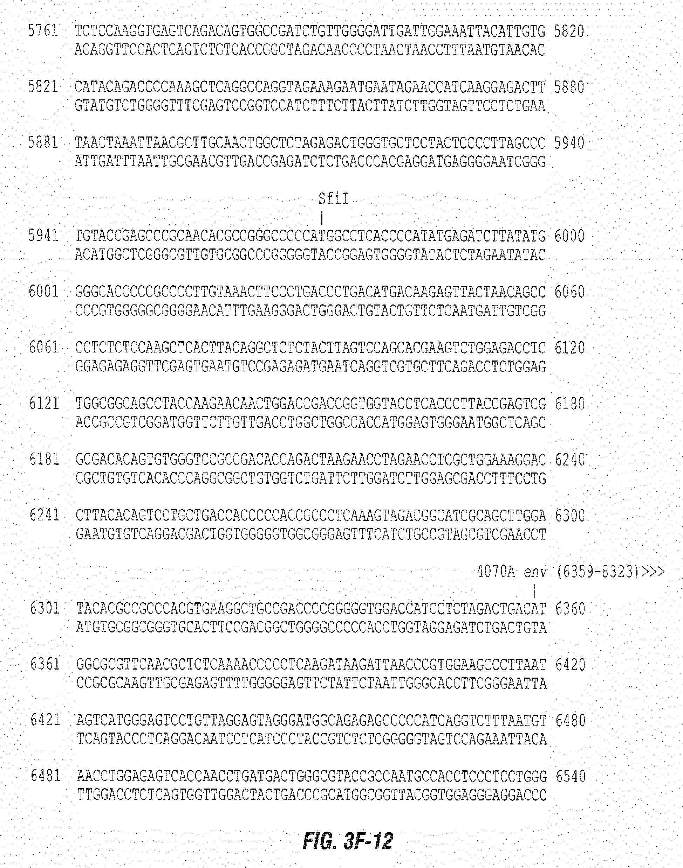

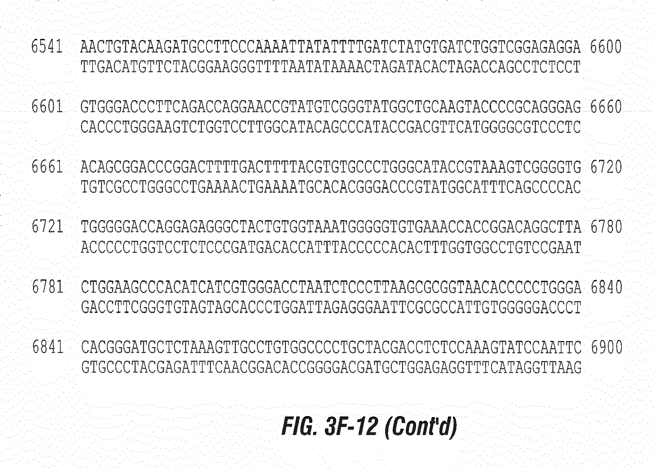

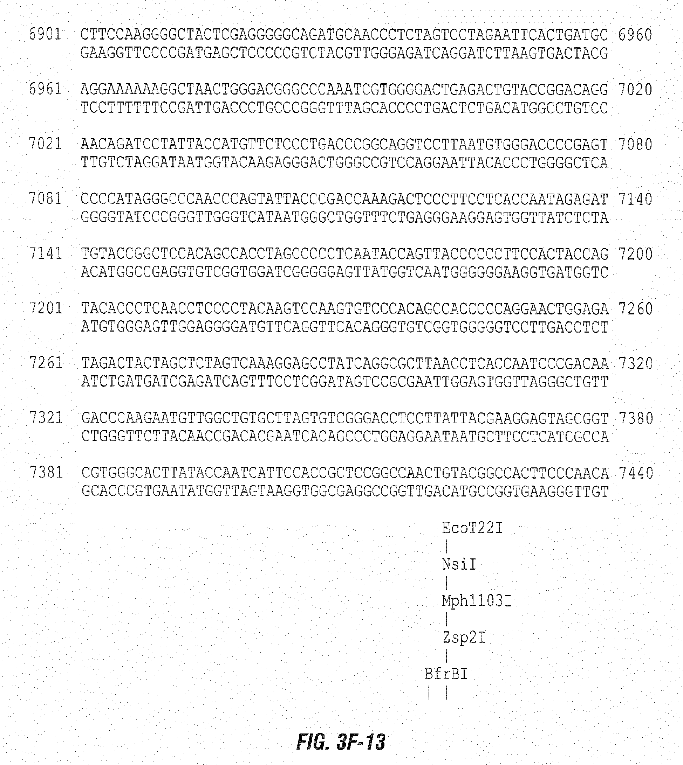

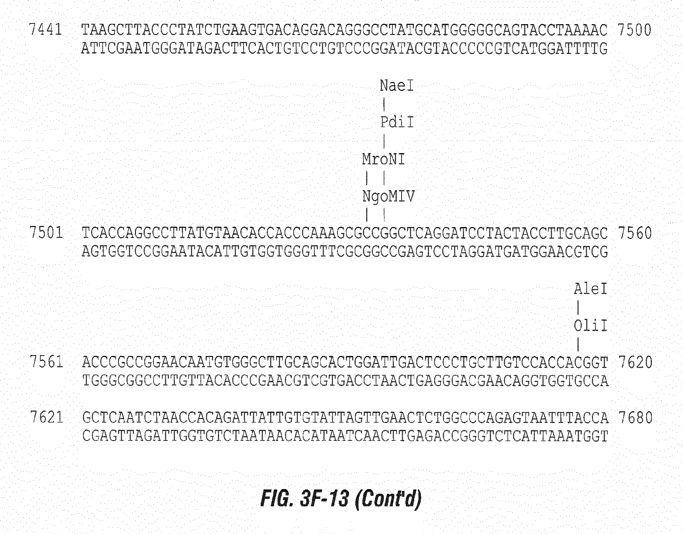

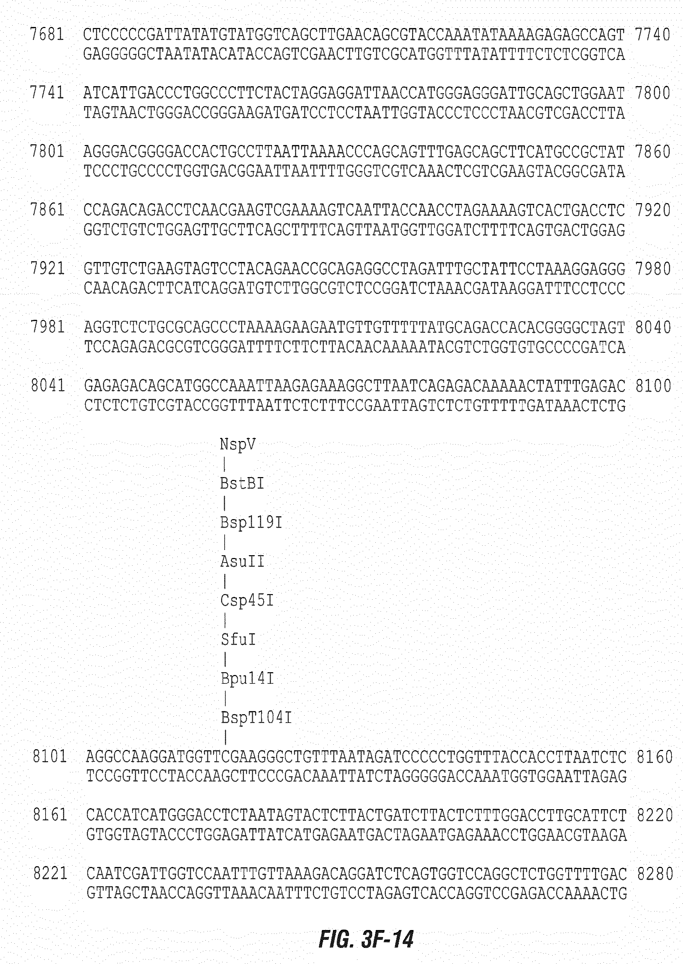

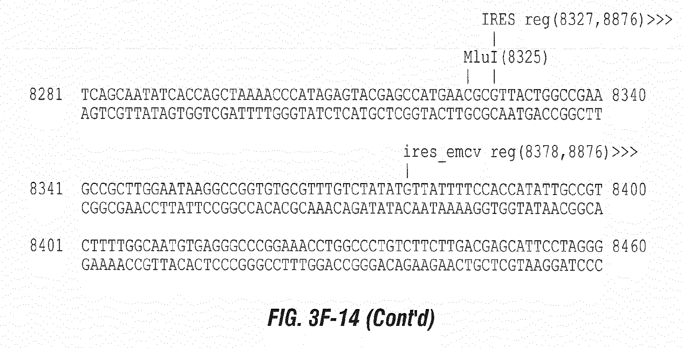

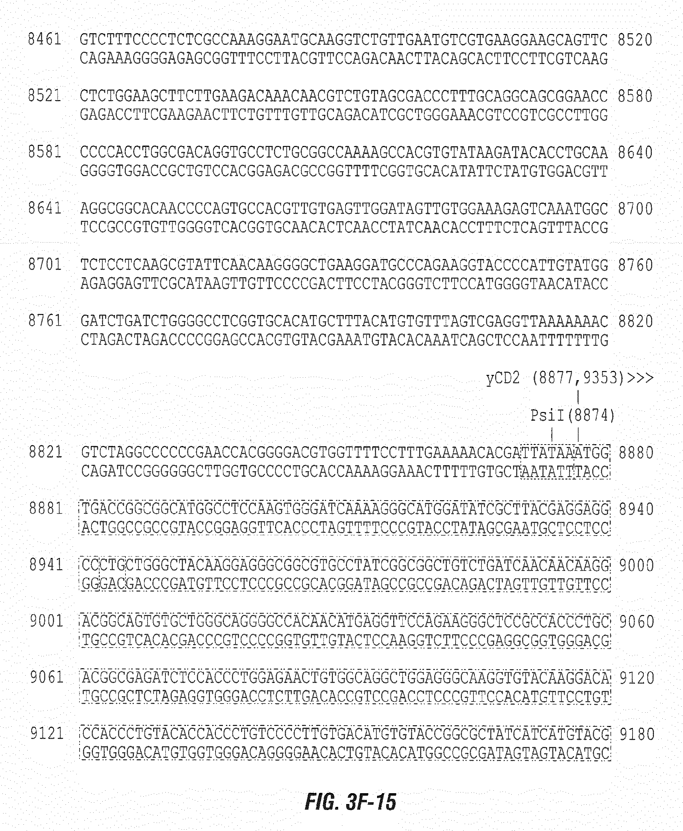

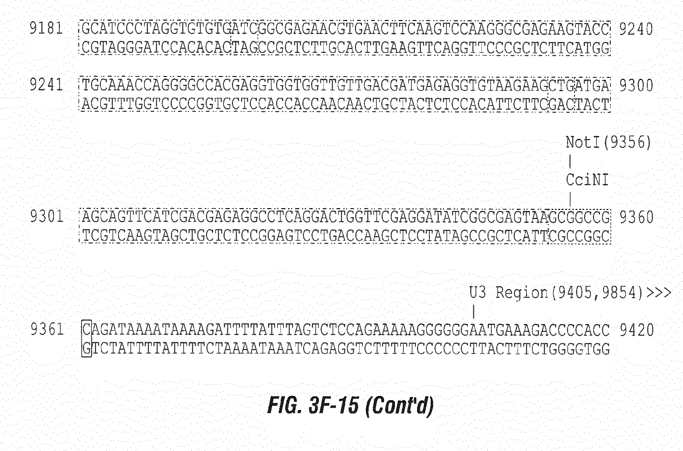

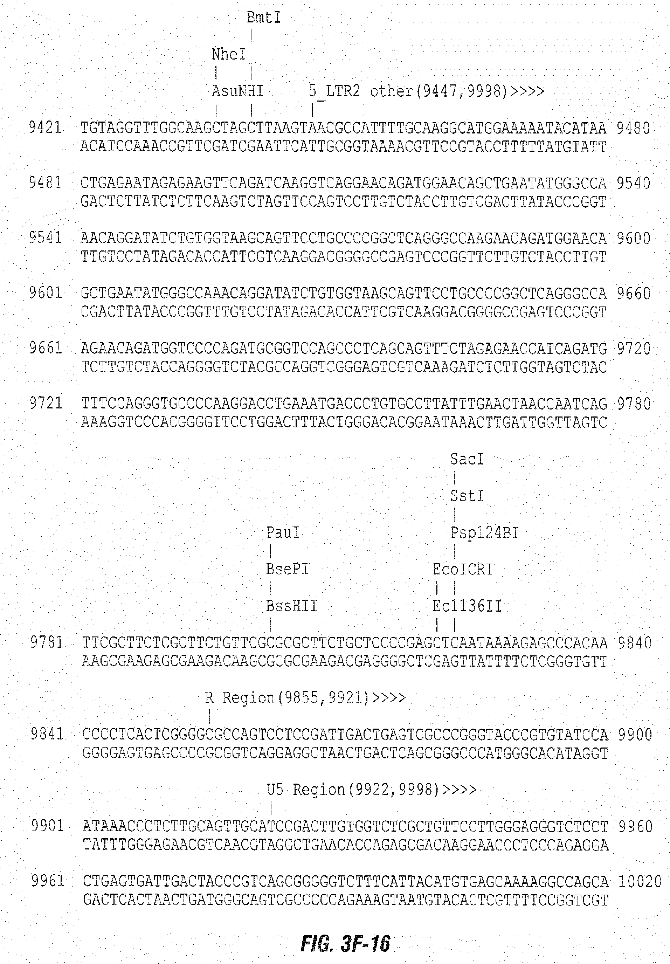

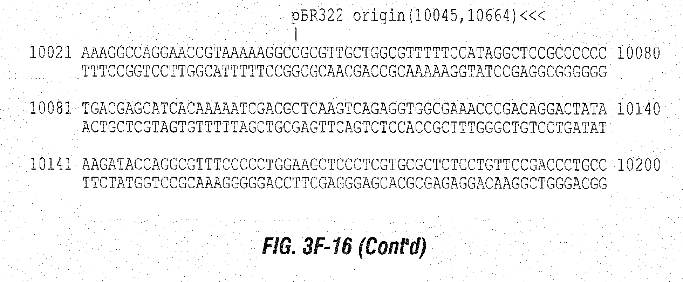

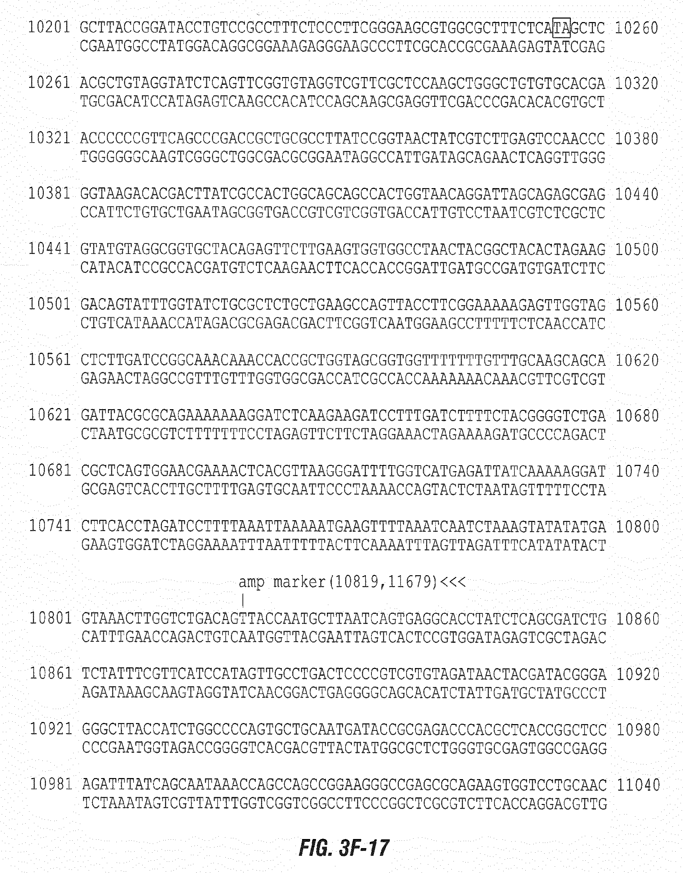

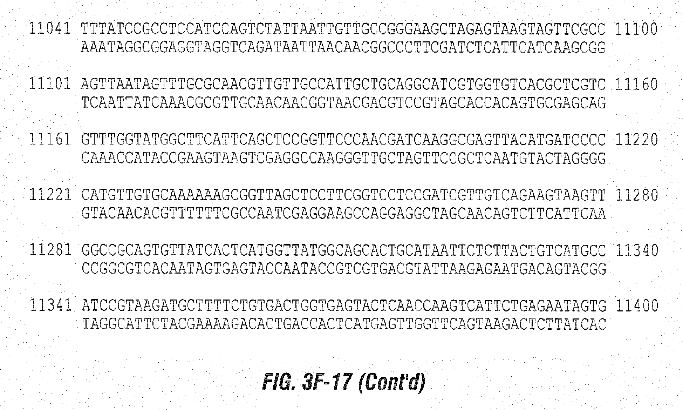

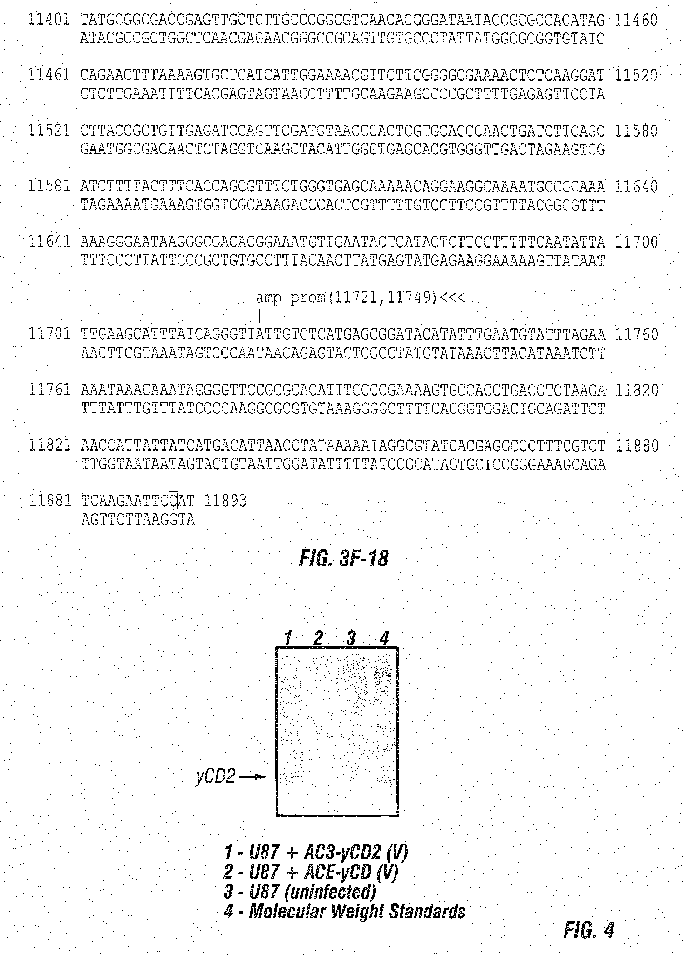

The disclosure also provides a recombinant retroviral vector comprising from 5' to 3' a CMV-R-U5, fusion of the immediate early promoter from human cytomegalovirus to the MLV R-U5 region; a PBS, primer binding site for reverse transcriptase; a 5' splice site; a ψ packaging signal; a gag, ORF for MLV group specific antigen; a pol, ORF for MLV polymerase polyprotein; a 3' splice site; a 4070A env, ORF for envelope protein of MLV strain 4070A; an IRES, internal ribosome entry site of encephalomyocarditis virus; a modified cytosine deaminase (thermostablized and codon optimized); a PPT, polypurine tract; and a U3-R-U5, MLV long terminal repeat. This structure is further depicted in Figure 3.

The disclosure also provides a retroviral vector comprising a sequence as set forth in SEQ ID NO:19, 20 or 22.

The retroviral vectors can be used to treat a wide range of disease and disorders including a number of cell proliferative diseases and disorders (see, *e.g*., U.S. Pat. Nos. 4,405,712 and 4,650,764; Friedmann, 1989, Science, 244:1275-1281; Mulligan, 1993, Science, 260:926-932, R. Crystal, 1995, Science 270:404-410, each of which are incorporated herein by reference in their entirety, see also, The Development of Human Gene Therapy, Theodore Friedmann, Ed., Cold Spring Harbor Laboratory Press, Cold Spring Harbor, N.Y., 1999. ISBN 0-87969-528-5, which is incorporated herein by reference in its entirety).

The disclosure also provides gene therapy for the treatment of cell proliferative disorders. Such therapy would achieve its therapeutic effect by introduction of an appropriate therapeutic polynucleotide (*e.g*., antisense, ribozymes, suicide genes, siRNA), into cells of subject having the proliferative disorder. Delivery of polynucleotide constructs can be achieved using the recombinant retroviral vector of the disclosure, particularly if it is based on MLV, which will is capable of infecting dividing cells.

In addition, the therapeutic methods (*e.g*., the gene therapy or gene delivery methods) as described herein can be performed *in vivo* or *ex vivo.* It may be preferable to remove the majority of a tumor prior to gene therapy, for example surgically or by radiation. In some aspects, the retroviral therapy may be preceded or followed by surgery, chemotherapy or radiation therapy.

Thus, the disclosure provides a recombinant retrovirus capable of infecting a non-dividing cell, a dividing cell or a neoplastic cell, therein the recombinant retrovirus comprises a viral GAG; a viral POL; a viral ENV; a heterologous nucleic acid operably linked to an IRES; and cis-acting nucleic acid sequences necessary for packaging, reverse transcription and integration. The recombinant retrovirus can be a lentivirus, such as HIV, or can be an oncovirus. As described above for the method of producing a recombinant retrovirus, the recombinant retrovirus of the disclosure may further include at least one of VPR, VIF, NEF, VPX, TAT, REV, and VPU protein. While not wanting to be bound by a particular theory, it is believed that one or more of these genes/protein products are important for increasing the viral titer of the recombinant retrovirus produced (*e.g*., NEF) or may be necessary for infection and packaging of virion.

The disclosure also provides a method of nucleic acid transfer to a target cell to provide expression of a particular nucleic acid (*e.g*., a heterologous sequence). Therefore, in another embodiment, the disclosure provides a method for introduction and expression of a heterologous nucleic acid in a target cell comprising infecting the target cell with the recombinant virus of the disclosure and expressing the heterologous nucleic acid in the target cell. As mentioned above, the target cell can be any cell type including dividing, non-dividing, neoplastic, immortalized, modified and other cell types recognized by those of skill in the art, so long as they are capable of infection by a retrovirus.

It may be desirable to modulate the expression of a gene in a cell by the introduction of a nucleic acid sequence (*e.g*., the heterologous nucleic acid sequence) by the method of the disclosure, wherein the nucleic acid sequence give rise, for example, to an antisense or ribozyme molecule. The term "modulate" envisions the suppression of expression of a gene when it is overexpressed, or augmentation of expression when it is under-expressed. Where a cell proliferative disorder is associated with the expression of a gene, nucleic acid sequences that interfere with the gene's expression at the translational level can be used. This approach utilizes, for example, antisense nucleic acid, ribozymes, or triplex agents to block transcription or translation of a specific mRNA, either by masking that mRNA with an antisense nucleic acid or triplex agent, or by cleaving it with a ribozyme.

It may be desirable to transfer a nucleic acid encoding a biological response modifier (*e.g*., a cytokine) into a cell or subject. Included in this category are immunopotentiating agents including nucleic acids encoding a number of the cytokines classified as "interleukins". These include, for example, interleukins 1 through 15, as well as other response modifiers and factors described elsewhere herein. Also included in this category, although not necessarily working according to the same mechanisms, are interferons, and in particular gamma interferon, tumor necrosis factor (TNF) and granulocyte-macrophage-colony stimulating factor (GM-CSF). Other polypeptides include, for example, angiogenic factors and anti-angiogenic factors. It may be desirable to deliver such nucleic acids to bone marrow cells or macrophages to treat enzymatic deficiencies or immune defects. Nucleic acids encoding growth factors, toxic peptides, ligands, receptors, or other physiologically important proteins can also be introduced into specific target cells.

The disclosure can be used for delivery of heterologous polynucleotides that promote drug specific targeting and effects. For example, HER2 (see, *e.g*., SEQ ID NO:23 and 24), a member of the EGF receptor family, is the target for binding of the drug trastuzumab (Herceptin™, Genentech). Trastuzumab is a mediator of antibody-dependent cellular cytotoxicity (ADCC). Activity is preferentially targeted to HER2-expressing cells with 2+ and 3+ levels of overexpression by immunohistochemistry rather than 1+ and non-expressing cells (Herceptin prescribing information, Crommelin 2002). Enhancement of expression of HER2 by introduction of vector expressing HER2 or truncated HER2 (expressing only the extracellular and transmembrane domains) in HER2 low tumors may facilitate optimal triggering of ADCC and overcome the rapidly developing resistance to HER2 that is observed in clinical use.

The substitution of yCD2 (comprising SEQ ID NO:19 from about 8877 to 9353) for the intracellular domain of HER2 allows for cell surface expression of HER2 and cytosolic localization of yCD2. The HER2 extracellular domain (ECD) and transmembrane domain (TM) (approximately 2026 bp from about position 175 to 2200 of SEQ ID NO:23) can be amplified by PCR (Yamamoto et al., Nature 319:230-234, 1986; Chen et al., Canc. Res., 58:1965-1971, 1998) or chemically synthesized (BioBasic Inc., Markham, Ontario, Canada) and inserted between the IRES and yCD2 gene in the vector pAC3-yCD2 SEQ ID NO: 19 (e.g., between about nucleotide 8876 and 8877 of SEQ ID NO:19). Alternatively, the yCD gene can be excised and replaced with a polynucleotide encoding a HER2 polypeptide or fragment thereof. A further truncated HER2 with only the Herceptin binding domain IV of the ECD and TM domains (approximately 290 bp from position 1910 to 2200) can be amplified or chemically synthesized and used as above (Landgraf 2007; Garrett et al., J. of Immunol., 178:7120-7131, 2007). A further modification of this truncated form with the native signal peptide (approximately 69 bp from position 175-237) fused to domain IV and the TM can be chemically synthesized and used as above. The resulting viruses can be used to treat a cell proliferative disorder in a subject in combination with trastuzumab or trastuzumab and 5-FC.

Alternatively, HER2 and the modifications described above can be expressed in a separate vector containing a different ENV gene or other appropriate surface protein. This vector can be replication competent (Logg et al. J.Mol Biol. 369:1214 2007) or non replicative "first generation" retroviral vector that encodes the envelope and the gene of interest (Emi et al. J.Virol 65:1202 1991). In the latter case the pre-exisitng viral infection will provide complementary gag and pol to allow infective spread of the "non-replicative" vector from any previously infected cell. Alternate ENV and glycoproteins include xenotropic and polytropic ENV and glycoproteins capable of infecting human cells, for example ENV sequences from the NZB strain of MLV and glycoproteins from MCF, VSV, GALV and other viruses (Palu 2000, Baum et al., Mol. Therapy, 13(6):1050-1063, 2006). For example, a polynucleotide can comprise a sequence wherein the GAG and POL and yCD2 genes of SEQ ID NO: 19 are deleted, the ENV corresponds to a xenotropic ENV domain of NZB MLV or VSV-g, and the IRES or a promoter such as RSV is operatively linked directly to HER2, HER2 ECDTM, HER2 ECDIVTM, or HER2 SECDIVTM.

Mixed infection of cells by VSVG pseudotyped virus and amphotropic retrovirus results in the production of progeny virions bearing the genome of one virus encapsidated by the envelope proteins of the other. The same is true for other envelopes that pseudotype retroviral particles. For example, infection by retroviruses derived as above results in production of progeny virions capable of encoding yCD2 and HER2 (or variant) in infected cells. The resulting viruses can be used to treat a cell proliferative disorder in a subject in combination with trastuzumab or trastuzumab and 5-FC.

Recently, a gamma retrovirus, XMRV, has been associated with prostate cancer in humans with the virus showing a strong preference for replication in prostate tissue (R.Schlaberg et al. PNAS106: 16351-16356 2009). The virus appears very similar too xenotropic MLV. In one embodiment of the current invention non-replicative retroviral vectors are provided which carry both a therapeutic gene (cytosine deaminase, thymidine kinase, other prodrug activating genes, interferons , IL-2, IL-12, other cytokines, p53 other anti-oncogenes, anti cancer miRNA or the like) and an envelope gene that is capable of being complemented by the XMRV gag and gagpol functions such as an amphotropic envelope, a GALV envelope, a VSVg protein envelope, or other envelopes known to those skilled in the art. The non-replicative vector polynucleotide is delivered to the prostate cancer in a patient or animal as a DNA or RNA molecule using one of: non-viral or physical delivery systems; a heterologous viral delivery system such as an adenoviral vector, or as a manufactured retroviral particle. Once delivered the non-replicative vector will be spread by complementation by XMRV and infection of neighboring cells will take place until the boundary of XMRV infection is reached, when the XMRV complementation will not be available. The therapeutic gene can then have its effect (e.g. after a prodrug is administered) in the XMRV infected area only. The same rescue effect can be achieved using a replicative retrovirlavector of the current invention. This strategy (complementary non-replicative vector with a therapeutic gene) can be used with any retroviral disease (HIV infection, HTLV1 infection, other cancer associated retroviruses), or with any viral or viral associated disease (HPV infection and HPV E6 & E7 expression in cervical cancers, EBV associated lymphomas or carcinomas etc.).

Another aspect of the development of resistance to trastuzumab relates to the interference with intracellular signaling required for the activity of trastuzumab. Resistant cells show loss of PTEN and lower expression of p27kip1 [Fujita, Brit J. Cancer, 94:247, 2006; Lu et al., Journal of the National Cancer Institute, 93(24): 1852-1857, 2001; Kute et al., Cytometry Part A 57A:86-93, 2004). For example, a polynucleotide encoding PTEN (SEQ ID NO:25) can be recombinantly generated or chemically synthesized (BioBasic Inc., Markham, Canada) and operably inserted directly after the yCD2 polynucleotide in the vector pAC3-yCD2 SEQ ID NO: 19 or 22, or with a linker sequence as previously described, or as a replacement for yCD2. In a further example, the PTEN encoding polynucleotide can be synthesized as above and inserted between the IRES and yCD2 sequences or with a linker as previously described.

Alternatively, PTEN can be expressed in a separate vector containing a different ENV gene or other appropriate surface protein. This vector can be replication competent (Logg et al. J.Mol Biol. 369:1214 2007) or non replicative "first generation" retroviral vector that encodes the envelope and the gene of interest (Emi et al., J.Virol 65:1202 1991). In the latter case the pre-exisitng viral infection will provide complementary gag and pol to allow infective spread of the "non-replicative" vector from any previously infected cell. Alternate ENV and glycoproteins include xenotropic and polytropic ENV and glycoproteins capable of infecting human cells, for example ENV sequences from the NZB strain of MLV and glycoproteins from MCF, VSV, GALV and other viruses (Palu, Rev Med Virol. 2000, Baum, Mol. Ther. 13(6):1050-1063, 2006). For example, a polynucleotide can comprise a sequence wherein the GAG and POL and yCD2 genes of SEQ ID NO: 19 are deleted, the ENV corresponds to a xenotropic ENV domain of NZB MLV or VSV-g, and the IRES or a promoter such as RSV is operatively linked directly to PTEN.

Mixed infection of cells by VSVG pseudotyped virus and amphotropic retrovirus results in the production of progeny virions bearing the genome of one virus encapsidated by the envelope proteins of the other [Emi 1991]. The same is true for other envelopes that pseudotype retroviral particles. For example, infection by retroviruses derived as above results in production of progeny virions capable of encoding yCD2 and PTEN (or variant) or PTEN alone in infected cells. The resulting viruses can be used to treat a cell proliferative disorder in a subject in combination with trastuzumab or trastuzumab and 5-FC.

Similarly, a polynucleotide encoding p27kip1 (SEQ ID NO:27 and 28) can be chemically synthesized (BioBasic Inc., Markham, Canada) and operably inserted directly after the yCD2 gene in the vector pAC3-yCD2 SEQ ID NO: 19 or with a linker sequence. In a further example, the p27kip1 encoding polynucleotide can be synthesized as above and inserted between the IRES and yCD2 sequences or with a linker as previously described or in place of the yCD2 gene.

Alternatively, p27kip1 can be expressed in a separate vector containing a different ENV gene or other appropriate surface protein. This vector can be replication competent (CR.Logg et al. J.Mol Biol. 369:1214 2007) or non replicative "first generation" retroviral vector that encodes the envelope and the gene of interest (Emi et al. J.Virol 65:1202 1991). In the latter case the pre-exisitng viral infection will provide complementary gag and pol to allow infective spread of the "non-replicative" vector from any previously infected cell. Alternate ENV and glycoproteins include xenotropic and polytropic ENV and glycoproteins capable of infecting human cells, for example ENV sequences from the NZB strain of MLV and glycoproteins from MCF, VSV, GALV and other viruses (Palu 2000, Baum 2006, *supra*). For example, a polynucleotide can comprise a sequence wherein the GAG and POL and yCD2 genes of SEQ ID NO: 19 are deleted, the ENV corresponds to a xenotropic ENV domain of NZB MLV or VSV-g, and the IRES or a promoter such as RSV is operatively linked directly to p27kip1.

Mixed infection of cells by VSVG pseudotyped virus and amphotropic retrovirus results in the production of progeny virions bearing the genome of one virus encapsidated by the envelope proteins of the other [Emi 1991]. The same is true for other envelopes that pseudotype retroviral particles. For example, infection by retroviruses derived as above from both SEQ ID NO: 19 and 22 results in production of progeny virions capable of encoding yCD2 and p27kip1 (or variant) in infected cells. The resulting viruses can be used to treat a cell proliferative disorder in a subject in combination with trastuzumab or trastuzumab and 5-FC.

In another example, CD20 is the target for binding of the drug rituximab (Rituxan™, Genentech). Rituximab is a mediator of complement-dependent cytotoxicity (CDC) and ADCC. Cells with higher mean fluorescence intensity by flow cytometry show enhanced sensitivity to rituximab (van Meerten et al., Clin Cancer Res 2006; 12(13):4027-4035, 2006). Enhancement of expression of CD20 by introduction of vector expressing CD20 in CD20 low B cells may facilitate optimal triggering of ADCC.

For example, a polynucleotide encoding CD20 (SEQ ID NO:29 and 30) can be chemically synthesized (BioBasic Inc., Markham, Canada) and operably inserted directly after the yCD2 gene in the vector pAC3-yCD2(-2) SEQ ID NO: 19 or 22 with a linker sequence as previously described, or as a replacement for the yCD2 gene. In a further example, the CD20 encoding polynucleotide can be synthesized as above and inserted between the IRES and yCD2 sequences or with a linker as previously described. As a further alternative the CD20 sequence can be inserted into the pAC3-yCD2 vector after excision of the CD gene by Psi1 and Not1 digestion.

In still a further example, a polynucleotide encoding CD20 (SEQ ID NO:29 and 30) can be chemically synthesized (BioBasic Inc., Markham, Canada)and inserted into a vector containing a non amphotropic ENV gene or other appropriate surface protein (Tedder et al., PNAS, 85:208-212, 1988). Alternate ENV and glycoproteins include xenotropic and polytropic ENV and glycoproteins capable of infecting human cells, for example ENV sequences from the NZB strain of MLV and glycoproteins from MCF, VSV, GALV and other viruses [Palu 2000, Baum 2006]. For example, a polynucleotide can comprise a sequence wherein the GAG and POL and yCD2 genes of SEQ ID NO: 19 are deleted, the ENV corresponds to a xenotropic ENV domain of NZB MLV or VSV-g, and the IRES or a promoter such as RSV is operatively linked directly to CD20.

Mixed infection of cells by VSVG pseudotyped virus and amphotropic retrovirus results in the production of progeny virions bearing the genome of one virus encapsidated by the envelope proteins of the other [Emi 1991]. The same is true for other envelopes that pseudotype retroviral particles. For example, infection by retroviruses derived as above from both SEQ ID NO: 19 or 22 results in production of progeny virions capable of encoding yCD2 and CD20 in infected cells. The resulting viruses can be used to treat a cell proliferative disorder in a subject in combination with Rituxan and/or 5-FC. Similarly, infection of a tumor with a vector encoding only the CD20 marker can make the tumor treatable by the use of Rituxan.

Levels of the enzymes and cofactors involved in pyrimidine anabolism can be limiting. OPRT, thymidine kinase (TK), Uridine monophosphate kinase, and pyrimidine nucleoside phosphorylase expression is low in 5-FU resistant cancer cells compared to sensitive lines (Wang et al., Cancer Res., 64:8167-8176, 2004). Large population analyses show correlation of enzyme levels with disease outcome (Fukui et al., Int'1. J. OF Mol. Med., 22:709-716, 2008). Coexpression of CD and other pyrimidine anabolism enzymes (PAE) can be exploited to increase the activity and therefore therapeutic index of fluoropyrimidine drugs.

To further increase the genetic stability (see, e.g., Fig. 5) of yCD2/PAE containing vectors, the enzyme encoding gene can be chemically synthesized with random mutations throughout the sequence. These mutations can be essentially random or can consist of only mutations at the wobble position for each amino acids. The library of mutated sequences is inserted downstream or in place of the yCD2 gene as was previously described for SEQ ID NO: 11 and 13 to create a library of plasmids, that can then be used to generate a library of infectious particles by transient transfection of 293T cells or equivalent. Sensitive cells can be infected with retrovirus encoding the fusion polypeptide and subjected to selection with appropriate chemicals.

DNA shuffling or "molecular breeding" allows genetic information to be shuffled, leading to recombinants with desired properties. Different proteins and enzymes have been improved using DNA shuffling (Stemmer 1994 Proc Natl Acad Sci USA 91(22): 10747-51; Stemmer 1994 Nature 370 (6488):389-91). Genetic recombination is a major force driving the evolution of many viruses. In retrovirus, recombination between two co-packaged retroviral genomes may occur at rates as high as 40% per replication cycle. High rates of recombination at each replication cycle enables genetic information to be shuffled rapidly, leading to recombinants with new pattern of mutations and phenotypes within a short period of time. For example, molecular breeding of retrovirus containing a library of recombinant ecotropic envelope sequences from six murine leukemia virus resulted in a viral clone with a new tropism. Using the same method, several viral clones were selected with improved stability and processing yields (Soong et al., 2000 Nat Genet 25(4):436-9; Powell et al., 2000 Nat Biotechnol 18(12):1279-82).

Polynucleotide sequence incorporated into the present invention are sometimes unstable resulting in deletion of the polynucleotide sequence from the viral genome over time. The basis for this is not well understood, but it is clearly sequence dependent Molecular breeding using the present invention to select for recombinant viral clones that have acquired optimal recombinations within the heterologous polynucleotide sequence is employed to select for viral clones that have greater vector stability.

For example, the HSV-TK coding sequence (SEQ ID NO:37)is not as stable as desired in some situations Molecular breeding of recombinant retroviral vectors encompass a pool of degenerated coding sequence of HSV-TK is performed to select recombinant vectors that have great vector stability. Randomly mutagenized Herpes Thymidine Kinase (TK) is chemically synthesized (Bio Basic Inc, Markham, Canada). The synthetic sequence is inserted 3' of the yCD2 sequence in SEQ ID NO:19, or by itself in the pAC3-yCD2 vector back bones after excision of the CD2 gene. The retroviral vector mixture is packaged as previously described. Mouse fibroblast LMTK- cells or humans 143Tk- are infected with vector and selected for TK activity in HAT media (Hiller et al., Mol. Cell Biol. 8(8):3298-3302, 1988). Serial passage of supernatants of resistant cells to fresh LMTK-/143Tk- cells again selected in HAT media results in selection of stable vectors expressing TK. TK+ resistant cells can be isolated and TK sequences rescued by standard PCR based techniques for mutation analysis (Cowell et al., CDNA Library Protocols, Published by Humana Press, 1996). In this manner, sequences are selected for both expression of functional protein and genomic stability of retroviral vector construct. Similar strategies can be employed for UPRT (SEQ ID NO: 11, 13), OPRT (SEQ ID NO: 15, 17) (Olah et al., Cancer Res. 40:2869-2875, 1980; and Suttle, Somatic Cell & Mol. Genet., 15(5):435-443, 1989) and other genes of interest. In adition the serial passage strategy can be used for non selectable genes and the genomic DNA after serial passage screened for full length inserts by PCR across the IRES-insert gene (see Fig 5). The full length inserts can be purified and cloned out back inot the viral vector then retested. Several cycles of this procedure can be performed to select the most stable gene. This strategy can also be used for passage in animals with or without tumors, and even in patient tissue.

Alternatively, OPRT, UPRT, TK or other PAE can be expressed in a separate vector containing a different ENV gene or other appropriate surface glycoprotein. This vector can be replication competent (Logg et al. J.Mol Biol. 369:1214 2007) or non replicative "first generation" retroviral vector that encodes the envelope and the gene of interest (Emi et al. J.Virol 65:1202 1991). In the latter case the pre-exisitng viral infection will provide complementary gag and pol to allow infective spread of the "non-replicative" vector from any previously infected cell. Alternate ENV and glycoproteins include xenotropic and polytropic ENV and glycoproteins capable of infecting human cells, for example ENV sequences from the NZB strain of MLV and glycoproteins from MCF, VSV, GALV and other viruses [Palu 2000, Baum 2006, *supra*]. For example, a polynucleotide can comprise a sequence wherein the GAG and POL genes are deleted, the ENV corresponds to a xenotropic ENV domain from NZB MLV or VSV-g, and the IRES or a promoter such as RSV is operatively linked directly to OPRT, UPRT, TK, or other PAE gene.

Mixed infection of cells by VSV-g pseudotyped virus and amphotropic retrovirus results in the production of progeny virions bearing the genome of one virus encapsidated by the envelope proteins of the other (Emi et al., J. Virol. 65:1202, 1991). The same is true for other envelopes that pseudotype retroviral particles. For example, infection by retroviruses derived as above from both SEQ ID NO: 19 and 22 results in production of progeny virions capable of encoding yCD2 and OPRT in infected cells. The resulting viruses can be used to treat a cell proliferative disorder in a subject in combination with 5-FC.

The recombinant retrovirus of the disclosure can be used for the treatment of a neuronal disorder for example, may optionally contain an exogenous gene, for example, a gene which encodes a receptor or a gene which encodes a ligand. Such receptors include receptors which respond to dopamine, GABA, adrenaline, noradrenaline, serotonin, glutamate, acetylcholine and other neuropeptides, as described above. Examples of ligands which may provide a therapeutic effect in a neuronal disorder include dopamine, adrenaline, noradrenaline, acetylcholine, gamma-aminobutyric acid and serotonin. The diffusion and uptake of a required ligand after secretion by an infected donor cell would be beneficial in a disorder where the subject's neural cell is defective in the production of such a gene product. A cell genetically modified to secrete a neurotrophic factor, such as nerve growth factor, (NGF), might be used to prevent degeneration of cholinergic neurons that might otherwise die without treatment.

Alternatively, cells be grafted into a subject with a disorder of the basal ganglia, such as Parkinson's disease, can be modified to contain an exogenous gene encoding L-DOPA, the precursor to dopamine. Parkinson's disease is characterized by a loss of dopamine neurons in the substantia-nigra of the midbrain, which have the basal ganglia as their major target organ.

Other neuronal disorders that can be treated similarly by the method of the disclosure include Alzheimer's disease, Huntington's disease, neuronal damage due to stroke, and damage in the spinal cord. Alzheimer's disease is characterized by degeneration of the cholinergic neurons of the basal forebrain. The neurotransmitter for these neurons is acetylcholine, which is necessary for their survival. Engraftment of cholinergic cells infected with a recombinant retrovirus of the disclosure containing an exogenous gene for a factor which would promote survival of these neurons can be accomplished by the method of the disclosure, as described. Following a stroke, there is selective loss of cells in the CA1 of the hippocampus as well as cortical cell loss which may underlie cognitive function and memory loss in these patients. Once identified, molecules responsible for CA1 cell death can be inhibited by the methods of this disclosure. For example, antisense sequences, or a gene encoding an antagonist can be transferred to a neuronal cell and implanted into the hippocampal region of the brain.

For diseases due to deficiency of a protein product, gene transfer could introduce a normal gene into the affected tissues for replacement therapy, as well as to create animal models for the disease using antisense mutations. For example, it may be desirable to insert a Factor IX encoding nucleic acid into a retrovirus for infection of a muscle or liver cell.

The disclosure also provides gene therapy for the treatment of cell proliferative or immunologic disorders. Such therapy would achieve its therapeutic effect by introduction of an antisense or dominant negative encoding polynucleotide into cells having the proliferative disorder, wherein the polynucleotide binds to and prevents translation or expression of a gene associated with a cell-proliferative disorder. Delivery of heterologous nucleic acids useful in treating or modulating a cell proliferative disorder (*e.g*., antisense polynucleotides) can be achieved using a recombinant retroviral vector of the disclosure. In another embodiment, a cell proliferative disorder is treated by introducing a CD polynucleotide of the disclosure, expressing the polynucleotide to produce a polypeptide comprising cytosine deaminase activity and contacting the cell with 5-fluorocytosine in an amount and for a period of time to produce a cytotoxic amount of 5-FU.

A number of chemotherapeutic agents are currently on the market having varying degrees of success from full remission to temporary remission and prolonged life with expected recurrence. Some of the cancer therapeutic agents on the market target the vascular angiogenic properties of tumor. The composition target the angiogenesis of tumors seeking to reduces blood supply and nutrients to the tumor or cancer and thereby reduce the tumor and prolong a subject's life. VEGF is an angiogenic factor known to play a role in tumor growth. Thus, antagonists of VEGF have been developed as anti-cancer agents.

Human VEGF mediates neoangiogenesis in normal and malignant vasculature; it is overexpressed in most malignancies and high levels have correlated with a greater risk of metastases and poor prognosis in many. When VEGF interacts with its receptor in *in vitro* models of angiogenesis, endothelial cell proliferation and new blood vessel formation occur. In animal models, VEGF has been demonstrated to induce vascular endothelial-cell proliferation/migration, sustain survival of newly-formed blood vessels, and enhance vascular permeability.

A VEGF antagonist agent is one that targets or negatively regulates the VEGF signaling pathway. Examples include VEGF inhibitors (*e.g*., agents that directly inhibit VEGF (*e.g*., VEGF-A, -B, -C, or -D), such as by binding VEGF (*e.g*., anti-VEGF antibodies such as bevacizumab (AVASTIN®) or ranibizumab (LUCENTIS®), or other inhibitors such as pegaptanib, NEOVASTAT®, AE-941, VEGF Trap, and PI-88)), modulators of VEGF expression (*e.g*., INGN-241, oral tetrathiomolybdate, 2-methoxyestradiol, 2-methoxyestradiol nanocrystal dispersion, bevasiranib sodium, PTC-299, Veglin), inhibitors of a VEGF receptor (*e.g*., KDR or VEGF receptor III (Flt4), for example anti-KDR antibodies, VEGFR2 antibodies such as CDP-791, IMC-1121B, VEGFR2 blockers such as CT-322), modulators of VEGFR expression (*e.g*., VEGFR1 expression modulator Sirna-027) or inhibitors of VEGF receptor downstream signaling. In some aspects described herein, the VEGF antagonist agent is bevacizumab, pegaptanib, ranibizumab, sorafenib, sunitinib, AE-941, VEGF Trap, pazopanib, vandetanib, vatalanib, cediranib, fenretinide, squalamine, INGN-241, oral tetrathiomolybdate, tetrathiomolybdate, Panzem NCD, 2-methoxyestradiol, AEE-788, AG-013958, bevasiranib sodium, AMG-706, axitinib, BIBF-1120, CDP-791, CP-547632, PI-88, SU-14813, SU-6668, XL-647, XL-999, IMC-1121B, ABT-869, BAY-57-9352, BAY-73-4506, BMS-582664, CEP-7055, CHIR-265, CT-322, CX-3542, E-7080, ENMD-1198, OSI-930, PTC-299, Sirna-027, TKI-258, Veglin, XL-184, or ZK-304709.

Bevacizumab (AVASTATIN®) (rhuMAb-VEGF) (Anti-VEGF monoclonal antibody) is a recombinant human/murine chimeric monoclonal antibody directed against vascular endothelial growth factor (VEGF)). It is prepared by engineering VEGF-binding residues of a murine anti-VEGF monoclonal antibody into framework regions of human immunoglobulin-1 (IgG1) (Prod Info Avastin, 2004). Only 7% of the amino acid sequence is derived from the murine antibody, with 93% from IgG1. Bevacizumab binds and neutralizes all human VEGF forms via recognition of binding sites for the two human VEGF receptor types (flt-1 and flk-1). In animal models, the antibody has been shown to stabilize established tumors or suppress tumor growth by inhibiting angiogenesis induced by VEGF.

The pharmacokinetics of bevacizumab are linear after doses of 0.3 mg/kg or greater (Anon, 2002). Following 90-minute intravenous infusions of 0.3, 1, 3, and 10 mg/kg in advanced cancer patients (n=25), peak serum concentrations of bevacizumab ranged from 5 to 9 mcg/mL, 21 to 39 mcg/mL, 52 to 92 mcg/mL, and 186 to 294 mcg/mL, respectively; slight accumulation was observed with repeat doses (weekly), but this was not significant and pharmacokinetics remained linear. Steady-state levels of bevacizumab were obtained in 100 days after 1 to 20 mg/kg weekly, every 2 weeks, or every 3 week.

The recommended dose of bevacizumab is 5 milligrams/kilogram infused intravenously over 30 minutes every 2 weeks until disease progression diminishes. Bevacizumab should follow chemotherapy. Efficacy of single-agent bevacizumab has not been established. Bevacizumab (which may be coadministered with the gemcitabine and docetaxel, or within a week before or after chemotherapy), is administered intravenously, at about 1 mg/kg to about 15 mg/kg, preferably about 5 mg/kg.

The methods and compositions of the disclosure are useful in combination therapies including therapies with bevacizumab. As described herein a replication competent retrovirus (RCR) of the disclosure comprising a therapeutic (*e.g*., a cytotoxic gene) is useful in treating cell proliferative disorders. An advantage of the RCR of the disclosure includes its ability to infect replicating cells cancer cells. Where the transgene of the vector comprises a cytotoxic gene (*e.g*., a gene that encodes a polypeptide that converts a non-cytotoxic agent to a cytotoxic agent) provides the ability to kill cancer cells.

The disclosure provides methods for treating cell proliferative disorders such as cancer and neoplasms comprising administering an RCR vector of the disclosure followed by treatment with a chemotherapeutic agent or anti-cancer agent. In one aspect, the RCR vector is administered to a subject for a period of time prior to administration of the chemotherapeutic or anti-cancer agent that allows the RCR to infect and replicate. The subject is then treated with a chemotherapeutic agent or anti-cancer agent for a period of time and dosage to reduce proliferation or kill the cancer cells. In one aspect, if the treatment with the chemotherapeutic or anti-cancer agent reduces, but does not kill the cancer/tumor (*e.g*., partial remission or temporary remission), the subject may then be treated with a non-toxic therapeutic agent (*e.g*., 5-FC) that is converted to a toxic therapeutic agent in cells expression a cytotoxic gene (*e.g*., cytosine deaminase) from the RCR. Using such methods the RCXR vectors of the disclosure are spread during a replication process of the tumor cells, such cells can then be killed by treatment with an anti-cancer or chemotherapeutic agent and further killing can occur using the RCR treatment process described herein.

In yet another embodiment of the disclosure, the heterologous gene can comprise a coding sequence for a target antigen (*e.g*., a cancer antigen). In this embodiment, cells comprising a cell proliferative disorder are infected with an RCR comprising a heterologous polynucleotide encoding the target antigen to provide expression of the target antigen (*e.g*., overexpression of a cancer antigen). An anticancer agent comprising a targeting cognate moiety that specifically interacts with the target antigen is then administered to the subject. The targeting cognate moiety can be operably linked to a cytotoxic agent or can itself be an anticancer agent. Thus, a cancer cell infected by the RCR comprising the targeting antigen coding sequences increases the expression of target on the cancer cell resulting in increased efficiency/efficacy of cytotoxic targeting.

Blocking of interactions between cells of the immune sytem have been shown to have significant immunological effects, either activating or suppressing (Waldmann Annu Rev Med. 57:65 2006). For example, blockade of the interaction of CTLA-4 (CD 152) and B7.1 (CD80) which modulates the activation of T cells has been shown to cause immune stimulation, presumably by blocking this suppressive interaction (Peggs et al. Curr. Opin. Immunol. 18:206, 2006). This blockade can potentially be achieved either by antibodies against CTLA-4 or by soluble B7.1. Systemic administration of these types of molecules can have undesirable global effects which can at a minimum lead to deleterious side - effects or even death in the case of one C28 agonist (Suntharalingam et al. NEJM 355 1018 2006). Pfizer has been developing one such anti-CTLA-4 blockading antibody (CP-675,206) as an anticancer reagent but has recently stopped development because of significant side effects. Local delivery of blockading molecules that are released into the local environment, from the tumor after infection with a replication competent vector encoding such molecules that are released into the extracellular space, provides the immune modulation locally and avoid these serious side effects. The blockading molecules are antibodies, single chain antibodies, soluble versions of the natural ligand or other peptides that bind such receptors.

In yet another embodiment, an RCR of the disclosure can comprise a coding sequence comprising a binding domain (*e.g*., an antibody, antibody fragment, antibody domain or receptor ligand) that specifically interacts with a cognate antigen or ligand. The RCR comprising the coding sequence for the binding domain can then be used to infect cells in a subject comprising a cell proliferative disorder such as a cancer cell or neoplastic cell. The infected cell will then express the binding domain or antibody. An antigen or cognate operably linked to a cytotoxic agent or which is cytotoxic itself can then be administered to a subject. The cytotoxic cognate will then selectively kill infected cells expressing the binding domain. Alternatively the binding domain itself can be an anti-cancer agent.

As used herein, the term "antibody" refers to a protein that includes at least one immunoglobulin variable domain or immunoglobulin variable domain sequence. For example, an antibody can include a heavy (H) chain variable region (abbreviated herein as VH), and a light (L) chain variable region (abbreviated herein as VL). In another example, an antibody includes two heavy (H) chain variable regions and two light (L) chain variable regions. The term "antibody" encompasses antigen-binding fragments of antibodies (*e.g*., single chain antibodies, Fab fragments, F(ab').sub.2, a Fd fragment, a Fv fragments, and dAb fragments) as well as complete antibodies.

The VH and VL regions can be further subdivided into regions of hypervariability, termed "complementarity determining regions" (CDR), interspersed with regions that are more conserved, termed "framework regions" (FR). The extent of the framework region and CDRs has been precisely defined (see, Kabat, E. A., et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917). Kabat definitions are used herein. Each VH and VL is typically composed of three CDRs and four FRs, arranged from amino-terminus to carboxy-terminus in the following order: FR1, CDR1, FR2, CDR2, FR3, CDR3, FR4.

An "immunoglobulin domain" refers to a domain from the variable or constant domain of immunoglobulin molecules. Immunoglobulin domains typically contain two .beta.-sheets formed of about seven .beta.-strands, and a conserved disulphide bond (see, e*.g*., A. F. Williams and A. N. Barclay 1988 Ann. Rev Immunol. 6:381-405). The canonical structures of hypervariable loops of an immunoglobulin variable can be inferred from its sequence, as described in Chothia et al. (1992) J. Mol. Biol. 227:799-817; Tomlinson et al. (1992) J. Mol. Biol. 227:776-798); and Tomlinson et al. (1995) EMBO J. 14(18):4628-38.

As used herein, an "immunoglobulin variable domain sequence" refers to an amino acid sequence which can form the structure of an immunoglobulin variable domain. For example, the sequence may include all or part of the amino acid sequence of a naturally-occurring variable domain. For example, the sequence may omit one, two or more N- or C-terminal amino acids, internal amino acids, may include one or more insertions or additional terminal amino acids, or may include other alterations. In one embodiment, a polypeptide that includes immunoglobulin variable domain sequence can associate with another immunoglobulin variable domain sequence to form a target binding structure (or "antigen binding site"), *e.g*., a structure that interacts with Tie1, *e.g*., binds to or inhibits Tie1.

The VH or VL chain of the antibody can further include all or part of a heavy or light chain constant region, to thereby form a heavy or light immunoglobulin chain, respectively. In one embodiment, the antibody is a tetramer of two heavy immunoglobulin chains and two light immunoglobulin chains, wherein the heavy and light immunoglobulin chains are inter-connected by, *e.g*., disulfide bonds. The heavy chain constant region includes three domains, CH1, CH2 and CH3. The light chain constant region includes a CL domain. The variable region of the heavy and light chains contains a binding domain that interacts with an antigen. The constant regions of the antibodies typically mediate the binding of the antibody to host tissues or factors, including various cells of the immune system (*e.g*., effector cells) and the first component (Clq) of the classical complement system. The term "antibody" includes intact immunoglobulins of types IgA, IgG, IgE, IgD, IgM (as well as subtypes thereof). The light chains of the immunoglobulin may be of types kappa or lambda. In one embodiment, the antibody is glycosylated. An antibody can be functional for antibody-dependent cytotoxicity and/or complement-mediated cytotoxicity.

The term "monospecific antibody" refers to an antibody that displays a single binding specificity and affinity for a particular target, *e.g*., epitope. This term includes a "monoclonal antibody" which refers to an antibody that is produced as a single molecular species, *e.g*., from a population of homogenous isolated cells. A "monoclonal antibody composition" refers to a preparation of antibodies or fragments thereof of in a composition that includes a single molecular species of antibody. In one embodiment, a monoclonal antibody is produced by a mammalian cell. One or more monoclonal antibody species may be combined.

One or more regions of an antibody can be human or effectively human. For example, one or more of the variable regions can be human or effectively human. For example, one or more of the CDRs can be human, *e.g*., HC CDR1, HC CDR2, HC CDR3, LC CDR1, LC CDR2, and LC CDR3. Each of the light chain CDRs can be human. HC CDR3 can be human. One or more of the framework regions can be human, *e.g*., FR1, FR2, FR3, and FR4 of the HC or LC. In one embodiment, all the framework regions are human, *e.g*., derived from a human somatic cell, *e.g*., a hematopoietic cell that produces immunoglobulins or a non-hematopoietic cell. In one embodiment, the human sequences are germline sequences, *e.g*., encoded by a germline nucleic acid. One or more of the constant regions can be human or effectively human. In another embodiment, at least 70, 75, 80, 85, 90, 92, 95, or 98% of the framework regions (*e.g*., FR1, FR2, and FR3, collectively, or FR1, FR2, FR3, and FR4, collectively) or the entire antibody can be human or effectively human. For example, FR1, FR2, and FR3 collectively can be at least 70, 75, 80, 85, 90, 92, 95, 98, or 99% identical to a human sequence encoded by a human germline V segment of a locus encoding a light or heavy chain sequence.

All or part of an antibody can be encoded by an immunoglobulin gene or a segment thereof. Exemplary human immunoglobulin genes include the kappa, lambda, alpha (IgA1 and IgA2), gamma (IgG1, IgG2, IgG3, IgG4), delta, epsilon and mu constant region genes, as well as the myriad immunoglobulin variable region genes. Full-length immunoglobulin light chains (about 25 Kd or 214 amino acids) are encoded by a variable region gene at the NH2-terminus (about 110 amino acids) and a kappa or lambda constant region gene at the COOH-- terminus. Full-length immunoglobulin heavy chains (about 50 Kd or 446 amino acids), are similarly encoded by a variable region gene (about 116 amino acids) and one of the other aforementioned constant region genes, *e.g*., gamma (encoding about 330 amino acids). A light chain refers to any polypeptide that includes a light chain variable domain. A heavy chain refers to any polypeptide that a heavy chain variable domain.

The term "antigen-binding fragment" of a full-length antibody (or simply "antibody portion," or "fragment"), as used herein, refers to one or more fragments of a full-length antibody that retain the ability to specifically bind to a target of interest. Examples of binding fragments encompassed within the term "antigen-binding fragment" of a full length antibody include (i) a Fab fragment, a monovalent fragment consisting of the VL, VH, CL and CH1 domains; (ii) a F(ab').sub.2 fragment, a bivalent fragment including two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR) that retains functionality. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules known as single chain Fv (scFv). See *e.g*., Bird et al. (1988) Science 242:423-426; and Huston et al. (1988) Proc. Natl. Acad. Sci. USA 85:5879-5883.

Antibody fragments can be obtained using any appropriate technique including conventional techniques known to those with skill in the art. The term "monospecific antibody" refers to an antibody that displays a single binding specificity and affinity for a particular target, *e.g*., epitope. This term includes a "monoclonal antibody" or "monoclonal antibody composition," which as used herein refer to a preparation of antibodies or fragments thereof of single molecular composition. As used herein, "isotype" refers to the antibody class (*e.g*., IgM or IgG1) that is encoded by heavy chain constant region genes.

The disclosure provides a method of treating a subject having a cell proliferative disorder. The subject can be any mammal, and is preferably a human. The subject is contacted with a recombinant replication competent retroviral vector of the disclosure. The contacting can be *in vivo* or *ex vivo.* Methods of administering the retroviral vector of the disclosure are known in the art and include, for example, systemic administration, topical administration, intraperitoneal administration, intra-muscular administration, intracranial, cerebrospinal, as well as administration directly at the site of a tumor or cell-proliferative disorder. Other routes of administration known in the art.

Thus, the disclosure includes various pharmaceutical compositions useful for treating a cell proliferative disorder. The pharmaceutical compositions according to the disclosure are prepared by bringing a retroviral vector containing a heterologous polynucleotide sequence useful in treating or modulating a cell proliferative disorder according to the disclosure into a form suitable for administration to a subject using carriers, excipients and additives or auxiliaries. Frequently used carriers or auxiliaries include magnesium carbonate, titanium dioxide, lactose, mannitol and other sugars, talc, milk protein, gelatin, starch, vitamins, cellulose and its derivatives, animal and vegetable oils, polyethylene glycols and solvents, such as sterile water, alcohols, glycerol and polyhydric alcohols. Intravenous vehicles include fluid and nutrient replenishers. Preservatives include antimicrobial, anti-oxidants, chelating agents and inert gases. Other pharmaceutically acceptable carriers include aqueous solutions, non-toxic excipients, including salts, preservatives, buffers and the like, as described, for instance, in Remington's Pharmaceutical Sciences, 15th ed. Easton: Mack Publishing Co., 1405-1412, 1461-1487 (1975) and The National Formulary XIV., 14th ed. Washington: American Pharmaceutical Association (1975), the contents of which are hereby incorporated by reference. The pH and exact concentration of the various components of the pharmaceutical composition are adjusted according to routine skills in the art. See Goodman and Gilman's The Pharmacological Basis for Therapeutics (7th ed.).

For example, and not by way of limitation, a retroviral vector useful in treating a cell proliferative disorder will include an amphotropic ENV protein, GAG, and POL proteins, a promoter sequence in the U3 region retroviral genome, and all cis-acting sequence necessary for replication, packaging and integration of the retroviral genome into the target cell.

The following Examples are intended to illustrate, but not to limit the disclosure. While such Examples are typical of those that might be used, other procedures known to those skilled in the art may alternatively be utilized.

### EXAMPLES

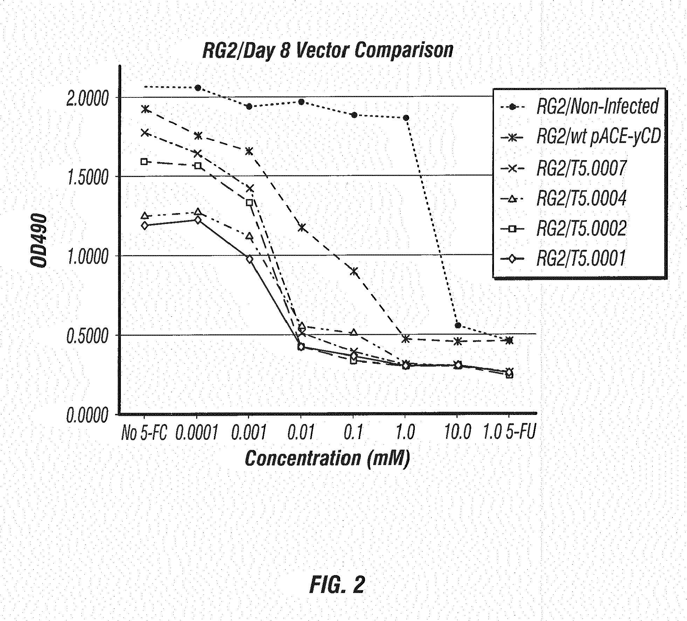

**Example 1. Modification of vector backbone of pACE-GFPemd to pAC3-GFPemd and insertion of cytosine deaminase gene sequences in place of GFP.** The previous back bone of the pACE-GFPemd plasmid (US6899871, Wang et al. Hum Gene Ther 14:117 2003) was modified in 3 ways as shown in Figure 3E. The modifications were made by PCR-mediated, oligonucleotide-directed mutagenesis (Logg et al., J. Mol Biol 369: 1214, 2007; see also "Molecular Biology and Biotechnology" Eds. J M. Walker, R.Rapley, Royal Society of Chemistry, London UK, 2000). The following modifications were made. 1) The nucleic acid sequence at the p15 region at 3' end of the amphotropic env gene was originally derived from the ecotropic envelope - this sequence was replaced by the corresponding sequence from the 4070A amphotropic envelope; the encoded envelope amino acids are identical in the two constructs. 2) The IRES sequence 3' end was modified to allow easier insertion of transgenes of choice with insertion of a PstI1 site and small imperfect repeats at either end of the IRES transgene site were removed. 3) Residual viral sequence downstream of the 3'LTR was removed. The resultant plasmid is pACE-emdGFP (aka pACE-GFP, pACE-eGFP and T5.0006) was used as a basis for the vectors encoding cytosine deaminase and variants. Two methods of inserting the coding sequence cassettes were used initially. The first method resulted in the sequence 5'TTATAAT3', and the second in the sequence 5'TTATAA3' immediately upstream of the ATG start codon. The second method was was simpler, as it involved simple PstI1 and Not1 enzyme cuts in the vector and the synthetic cytosine deaminase genes, followed by religation. Vectors with cytosine deaminase inserts were made both ways with the CDopt (CD1) and the CDopt+3pt (CD2) (see Figure 2) coding sequences and infectious virus preps made by transient transfection of 293T cells as described in Example 3. U87 cells were then infected in culture, at an MOI of 0.1, and the cells grown until 100% infected. Cell extracts of 100% infected cells were assayed for cytosine deaminase activity as described in Example 6 and the specific activity of the enzyme was found to be equivalent for constructs with either upstream sequence, that were otherwise identical. Therefore in the table in Figure 2, pACE-eGFP (T5.0006) and pACE-yCD (T5.0007) have the first upstream sequence, while all other constructs that were further tested have the second. Subsequently vectors with different gene inserts have been routinely constructed with straightforward PStI1 and Not 1 cuts.

See Figure 3A below for a diagram of the vector construct for the initial transfected replication-competent retrovirus. CMV is the human CMV immediate early promoter, U3, R and U5 are the corresponding regions of the viral long terminal repeat (LTR). *Gag, pol* and *env* are the viral protein coding regions. Figure 3B and 3D shows the plasmid structure and a sequence of the disclosure.

The vector of the disclosure provides a number of differences compared to the vector of Tai et al., Mol. Ther. 12:842, 2005. The Tai *et al.* vector has been altered to eliminate about 70bp of MLV sequence downstream from the 3'LTR. The DNA sequence downstream of the ClaI site in the envelope was changed to an amphotropic envelope sequence. This change does not change the amino acid sequence of the envelope. In addition, small repeats on either side of the IRES-CD cassette have been eliminated to avoid instability due to homologous recombination. These changes also unexpectedly provided increased stability of the vector during replication and passaging in host cells (Figure 5).

It is recognized that after reverse transcription and the first integration event into treated cells, the DNA provirus and any subsequent progeny retrovirus has a conventional LTR structure from MLV on either end. This configuration has been shown to be stable after multiple cycles of infection (See Figure 5 below).

### Example 2 Genetic enhancements to the wild type yeast cytosine deaminase gene.

Two sets of changes have been made: (1) three positional mutations which change three amino acids (A23L, I140L and V108I) to increase thermal stability of the yeast cytosine deaminase protein and (2) additional gene sequence modifications to enhance human codon usage sequences to improve protein translation efficiency in human cells without further changes to the amino acid sequence.

Sequence design for CD included CDoptimized, CD-UPRT (+/- linker) and CD-OPRTase (+/- linker). The final cytosine deaminase coding sequence can comprise at the 5' end a PSI1 site (full length) and 3' end Not1 site plus poly A tail for PSI1/Not1 cassette based strategy. Sequences cassettes were ordered from, and provided by, a commercial vendor (BioBasic Inc., Markham , Ontario, Canada).

The following sequence comprising a yeast cytosine deaminase was used for cloning, optimizing and mutation (the boxed nucleic acids comprise the restriction sites -Psi1 and Not1 - used in subsequent methods for cloning:

AACACGATTATAAATGGTGACAGGGGGAATGGCAAGCAAGTGGGATCAGAAGGGTATGGACATTGCC
TATGAGGAGGCGGCCTTAGGTTACAAAGAGGGTGGTGTTCCTATTGGCGGATGTCTTATCAATAACA
AAGACGGAAGTGTTCTCGGTCGTGGTCACAACATGAGATTTCAAAAGGATCCGCCACACTACATGG
TGAGATCTCCACTTTGGAAAACTGTGGGAGATTAGAGGGCAAAGTGTACAAAGATACCACTTTGTAT
ACGACGCTGTCTCCATGCGACATGTGTACAGGTGCCATCATCATGTATGGTATTCCACGCTGTGTTG
TCGGTGAGAACGTTAATTTCAAAAGTAAGGGCGAGAAATATTTACAAACTAGAGGTCACGAGGTTGT
TGTTGTTGACGATGAGAGGTGTAAAAAGATCATGAAACAATTTATCGATGAAAGACCTCAGGATTGG
TTTGAAGATATTGGTGAGTAGGCGGCCGCGCCATAGATAAAATAAAAGATTTTATTTAGTCTCCAGA
AAAAGGGGGG (SEQ ID NO:32)

The following Table summarizes the genes and resulting plasmid vectors that were made and their names.

**Table: Vector constructs and names**

| **Tocagen Code** | **Reference name** | **Original Name** | **5'LTR Prom** | **Envelope** | **Vector** | **IRES** | **Trans-gene** | **3'LTR** |
|---|---|---|---|---|---|---|---|---|
| | | | | | | | | |
| T5.0000 | pACE-yCD | pACE-CD (Tai et al. 2005) | CMV | Ampho (4070A) | pACE | EMCV | Wt yeast CD | MLV U3 |
| T5.0001 | pAC3-yCD1 | CDopt sequence | CMV | Ampho (4070A) | pAC3 | EMCV | modified CD | MLV U3 |
| T5.0002 | pAC3-yCD2 | CDopt+3pt | CMV | Ampho (4070A) | pAC3 | EMCV | Modif-ied CD | MLV U3 |
| T5.0003 | pAC3-yCD2-U | Cdopt+3pt-UPRT | CMV | Ampho (4070A) | pAC3 | EMCV | CD2-UPRT | MLV U3 |
| T5.0004 | pAC3-yCD2-O | CDopt+3pt-OPRT | CMV | Ampho (4070A) | pAC3 | EMCV | CD2-OPRT | MLV U3 |
| T5.0005 | pAC3-yCD2-LO | CDopt+3pt-LINK-OPRT | CMV | Ampho (4070A) | pAC3 | EMCV | CD2-L-OPRT | MLV U3 |
| T5.0006 | pAC3-eGFP | pAC3-emd, pAC3GFP | CMV | Ampho (4070A) | pAC3 | EMCV | Emera Id GFP | MLV U3 |
| T5.0007 | pAC3-yCD | pAC3-yCD | CMV | Ampho (4070A) | pAC3 | EMCV | Wt yeast CD | MLV U3 |

The replication competent retroviral vector described by Kasahara *et al.* pACE-CD (U.S. Patent No. 6,899,871, the disclosure of which is incorporated herein) was used as a basis for additional modifications. A vector (pAC3-yCD)was modified to express a modified yeast cytosine deaminase gene as described herein and was used in the contructs. See Figure 3A below for a diagram of the vector construct for the initial transfected replication-competent retrovirus. CMV is the human CMV immediate early promoter, U3, R and U5 are the corresponding regions of the viral long terminal repeat (LTR). *Gag, pol* and *env* are the viral protein coding regions. Fig3B and 3D shows the plasmid structure and a sequence of the disclosure.
After the genes were synthesized at a contractor (Bio Basic Inc., Markham, Ontario, Canada)they were inserted into the Psi1 -Not1 site of the pAC3 vector backbone (Figure 3). The plasmid backbone was normally generated by cutting the plasmid pAC3-eGFP with Psi1 and Not1 and purifying the large (about 11kb) fragment encoding the plasmid and retroviral backbone)

A. Humanized codon optimized CD gene (CDopt,aka CD1, T5.0001) A comparison of a human codon optimized cytosine deaminase of Conrad et al. and PCT WO 99/60008 indicates 91 total codons optimized in both, 36 codons identical, 47 codons had third base pair changes (all encode same amino acid) and 9 codons were different (however they encoded same amino acid). Of the 9 codons that differed:
AGC (Ser) to TCC (Ser)
CGT (Arg) to AGG (Arg)
CCA (Pro) to CCT (Pro)

All have equivalent GC content and encode the same amino acid. The native yeast gene sequence above was separately codon optimized to give the following CD gene (CD1) and was called T5.0001 when when inserted into the plasmid vector pAC3 which encodes the replication competent retrovirus (RCR) with IRES.

TTATAAATGGTGACCGGCGGCATGGCCTCCAAGTGGGATCAAAAGGGCATGGATATCGCTT
ACGAGGAGGCCGCCCTGGGCTACAAGGAGGGCGGCGTGCCTATCGGCGGCTGTCTGATCAA
CAACAAGGACGGCAGTGTGCTGGGCAGGGGCCACAACATGAGGTTCCAGAAGGGCTCCGCC

ACCCTGCACGGCGAGATCTCCACCCTGGAGAACTGTGGCAGGCTGGAGGGCAAGGTGTACA
AGGACACCACCCTGTACACCACCCTGTCCCCTTGTGACATGTGTACCGGCGCTATCATCAT
GTACGGCATCCCTAGGTGTGTGGTGGGCGAGAACGTGAACTTCAAGTCCAAGGGCGAGAAG
TACCTGCAAACCAGGGGCCACGAGGTGGTGGTTGTTGACGATGAGAGGTGTAAGAAGATCA
TGAAGCAGTTCATCGACGAGAGGCCTCAGGACTGGTTCGAGGATATCGGCGAGTGATAAGC
GGCCGCAGATAAAATAAAAGATTTTATTTAGTCTCCAGAAAAAGGGGGG.

B. Heat stabilized CD gene Additional modifications were made to enhance the stability of the cytosine deaminase. Genetic enhancements to the wild type yeast cytosine deaminase gene were made to include three positional mutations which change three amino acids (A23L, I140L and V108I) to increase thermal stability of the yeast cytosine deaminase protein.

The following primer pairs were used in the generation of the gene for the cytosine deaminase polypeptide of the disclosure:
Site directed mutagenesis primers: Primers sense: 5'-tcgaggatatcggcgagtgaaacccgttattctttttggc-3' (SEQ ID NO:33)
Primers antisense: 5'-gccaaaaagaataacgggtttcactcgccgatatcctcga-3' (SEQ ID NO:34)

### Primers sense:

5'tcggcgagtgatccggcggcggcgcctccggcggcggcgcctccggcggcggcgcctccggcggc ggcgccaacccgttatt-3' (SEQ ID NO:35)

### Primers antisense:

5'-aataacgggttggcgccgccgccggaggcgccgccgccggaggcgccgccgccggag gcgccgccgccggatcactcgccga-3' (SEQ ID NO:36)

To increase the stability of the native yeast CD protein, three amino acid substitutions were engineered into the protein. These substitutions were alone or in combination with human codon optimization.

The three amino acid substitutions are : A23L, V108I, I140L. A sequence encoding these substitutions is shown below.

ATGGTGACAGGGGGAATGGCAAGCAAGTGGGATCAGAAGGGTATGGACATTGCCTATGAGGAGGCGT
TATTAGGTTACAAAGAGGGTGGTGTTCCTATTGGCGGATGTCTTATCAATAACAAAGACGGAAGTGT
TCTCGGTCGTGGTCACAACATGAGATTTCAAAAGGGATCCGCCACACTACATGGTGAGATCTCCACT
TTGGAAAACTGTGGGAGATTAGAGGGCAAAGTGTACAAAGATACCACTTTGTATACGACGCTGTCTC
CATGCGACATGTGTACAGGTGCCATCATCATGTATGGTATTCCACGCTGTGTCATCGGTGAGAACGT
TAATTTCAAAAGTAAGGGCGAGAAATATTTACAAACTAGAGGTCACGAGGTTGTTGTTGTTGACGAT

GAGAGGTGTAAAAAGTTAATGAAACAATTTATCGATGAAAGACCTCAGGATTGGTTTGAAGATATTG
GTGAGTAGGCGGCCGCGCCATAGATAAAATAAAAGATTTTATTTAGTCTCCAGAAAAAGGGGGG(SE
Q ID NO:3)

The encoded polypeptide comprises the following sequence (substituted amino acids in underlined):

1 MVTGGMASKWDQKGMDIAYEEALLGYKEGGVPIGGCLINNKDGSVLGRGHNMRFQKGSAT
61 LHGEISTLENCGRLEGKVYKDTTLYTTLSPCDMCTGAIIMYGIPRCVIGENVNFKSKGEK
121 YLQTRGHEVVVVDDERCKKLMKQFIDERPQDWFEDIGE- (SEQ ID NO:4)

Final construct design that integrates 3 amino acid substitutions A23L/V108I/I140L utilizing preferred codons and uses preferred human codon usage for entire sequence (this gene is called CDopt+3pt [aka CD2] (SEQ ID NO:37): 
1 ATGGTGACCGGCGGCATGGCCTCCAAGTGGGATCAAAAGGGCATGGATATCGCTTACGAG
61 GAGGCCCTGCTGGGCTACAAGGAGGGCGGCGTGCCTATCGGCGGCTGTCTGATCAACAAC
121 AAGGACGGCAGTGTGCTGGGCAGGGGCCACAACATGAGGTTCCAGAAGGGCTCCGCCACC
181 CTGCACGGCGAGATCTCCACCCTGGAGAACTGTGGCAGGCTGGAGGGCAAGGTGTACAAG
241 GACACCACCCTGTACACCACCCTGTCCCCTTGTGACATGTGTACCGGCGCTATCATCATG
301 TACGGCATCCCTAGGTGTGTGATCGGCGAGAACGTGAACTTCAAGTCCAAGGGCGAGAAG
361 TACCTGCAAACCAGGGGCCACGAGGTGGTGGTTGTTGACGATGAGAGGTGTAAGAAGCTG
421 ATGAAGCAGTTCATCGACGAGAGGCCTCAGGACTGGTTCGAGGATATCGGCGAGTGATAA
 and T5.0002 when inserted into the plasmid vector pAC3 which encodes the RCR with IRES. Underlined codons denotes preferred codons for amino acid substitutions.

Protein translation sequence alignment indicates preferred codon changes and amino acid substitutions result in desired protein structure:

CDoptimized sequence design (human codon preference + 3 amino acid substitutions)

AACACGATTATAAATGGTGACCGGCGGCATGGCCTCCAAGTGGGATCAAAAGGGCATGGATATCGCT
TACGAGGAGGCCCTGCTGGGCTACAAGGAGGGCGGCGTGCCTATCGGCGGCTGTCTGATCAACAACA
AGGACGGCAGTGTGCTGGGCAGGGGCCACAACATGAGGTTCCAGAAGGGCTCCGCCACCCTGCACGG
CGAGATCTCCACCCTGGAGAACTGTGGCAGGCTGGAGGGCAAGGTGTACAAGGACACCACCCTGTAC
ACCACCCTGTCCCCTTGTGACATGTGTACCGGCGCTATCATCATGTACGGCATCCCTAGGTGTGTGA
TCGGCGAGAACGTGAACTTCAAGTCCAAGGGCGAGAAGTACCTGCAAACCAGGGGCCACGAGGTGGT
GGTTGTTGACGATGAGAGGTGTAAGAAGCTGATGAAGCAGTTCATCGACGAGAGGCCTCAGGACTGG
TTCGAGGATATCGGCGAGTAAGCGGCCGCGCCATAGATAAAATAAAAGATTTTATTTAGTCTCCAGA
AAAAGGGGGG (SEQ ID NO:38)

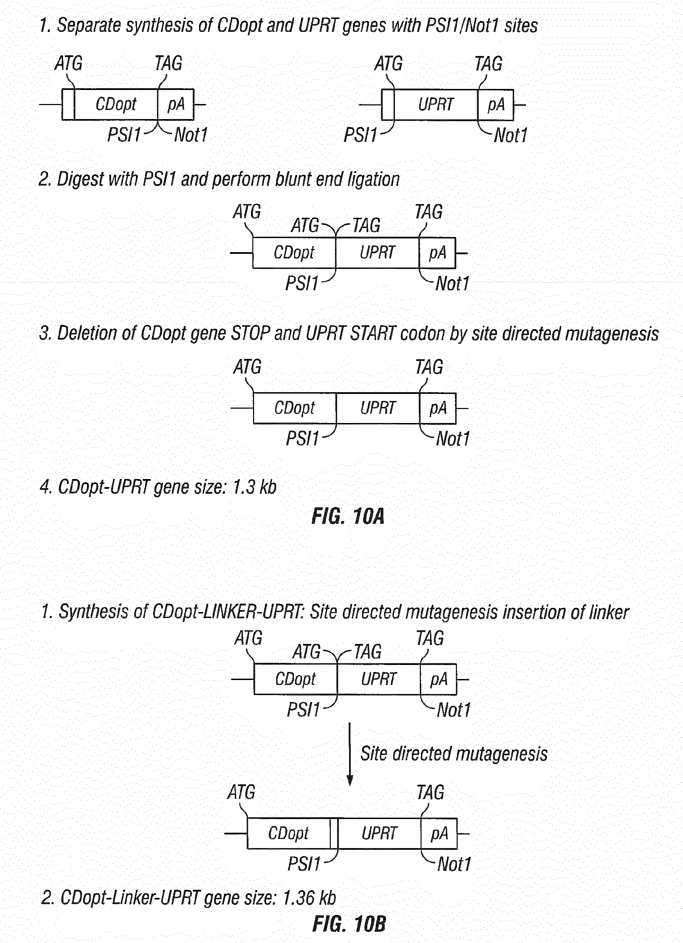

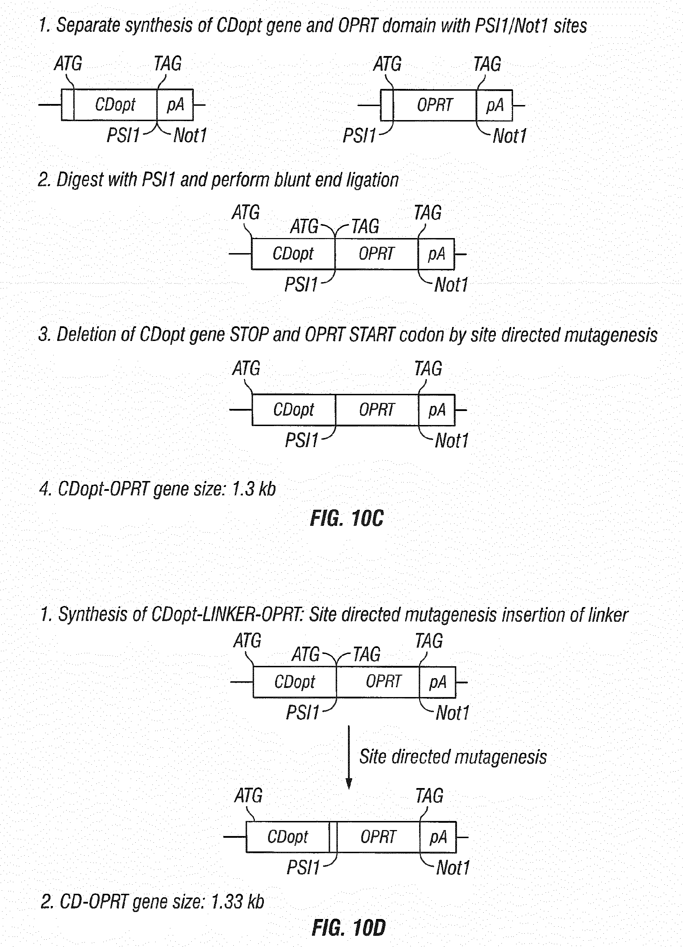

C. Construction of CD-UPRT fusion gene (CDopt+3pt-UPRT, [aka CDopt-UPRT and CD2-UPRT], T5.0003 in the pAC3 plasmid RCR vector) A fusion construct was also developed comprising a CD polypeptide as described above linked to a UPRT polypeptide to generate a CDoptimized-UPRT sequence using Scheme I as set forth in Figure 10A. The following primers were used to delete the stop-start between the CD and UPRT.

### Primer sequences:

| **Primer Name** | **Primer Sequence (5' to 3')** | | | |
|---|---|---|---|---|
| del118-123 | 5'-tcgaggatatcggcgagtgaaacccgttattctttttggc-3' | | | |
| del118-123-antisense | 5'-gccaaaaagaataacgggtttcactcgccgatatcctcga-3' | | | |

| **Primer Name** | **Length (nt.)** | **Tm** | **Duplex Energy at 68°C** | **Energy Cost of Mismatches** |
|---|---|---|---|---|
| del118-123 | 40 | 79.06°C | -44.37 kcal/mole | 21.1% |
| del118-123-antisense | 40 | 79.06°C | -47.95 kcal/mole | 20.3% |

| **Primer Name** | **Primer-Template Duplex** | | | |
|---|---|---|---|---|
| del118-123 | 5'-tcgaggatatcggcgagtga------aacccgttattctttttggc-3' ;;;;;;;;;;;;;;;;;;;;      ;;;;;;;;;;;;;;;;;;;; ccaagctcctatagccgctcactatctacttgggcaataagaaaaaccgaag | | | |
| del118-123-antisense | ggttcgaggatatcggcgagtgatagatgaacccgttattctttttggcttc ;;;;;;;;;;;;;;;;;;;;      ;;;;;;;;;;;;;;;;;;;; 3'-agctcctatagccgctcact------ttgggcaataagaaaaaccg | | | |

The resulting fusion polynucleotide comprises 1296 bp and the sequence set forth immediately below:

AACACGATTATAAATGGTGACCGGCGGCATGGCCTCCAAGTGGGATCAAAAGGGCATGGATATCGCT
TACGAGGAGGCCCTGCTGGGCTACAAGGAGGGCGGCGTGCCTATCGGCGGCTGTCTGATCAACAACA
AGGACGGCAGTGTGCTGGCAGGGGCCACAACATGAGGTTCCAGAAGGGCTCCGCCACCCTGCACGG
CGAGATCTCCACCCTGGAGAACTGTGGCAGGCTGGAGGGCAAGGTGTACAAGGACACCACCCTGTAC
ACCACCCTGTCCCCTTGTGACATGTGTACCGGCGCTATCATCATGTACGGCATCCCTAGGTGTGTGA
TCGGCGAGAACGTGAACTTCAAGTCCAAGGGCGAGAAGTACCTGCAAACCAGGGGCCACGAGGTGGT
GGTTGTTGACGATGAGAGGTGTAAGAAGCTGATGAAGCAGTTCATCGACGAGAGGCCTCAGGACTGG
TTCGAGGATATCGGCGAGAACCCGTTATTCTTTTTGGCTTCTCCATTCTTGTACCTTACATATCTTA
TATATTATCCAAACAAAGGGTCTTTCGTTAGCAAACCTAGAAATCTGCAAAAAATGTCTTCGGAACC
ATTTAAGAACGTCTACTTGCTACCTCAAACAAACCAATTGCTGGGTTTGTACACCATCATCAGAAAT
AAGAATACAACTAGACCTGATTTCATTTTCTACTCCGATAGAATCATCAGATTGTTGGTTGAAGAAG
GTTTGAACCATCTACCTGTGCAAAAGCAAATTGTGGAAACTGACACCAACGAAAACTTCGAAGGTGT
CTCATTCATGGGTAAAATCTGTGGTGTTTCCATTGTCAGAGCTGGTGAATCGATGGAGCAAGGATTA
AGAGACTGTTGTAGGTCTGTGCGTATCGGTAAAATTTTAATTCAAAGGGACGAGGAGACTGCTTTAC
CAAAGTTATTCTACGAAAAATTACCAGAGGATATATCTGAAAGGTATGTCTTCCTATTAGACCCAAT
GCTGGCCACCGGTGGTAGTGCTATCATGGCTACAGAAGTCTTGATTAAGAGAGGTGTTAAGCCAGAG
AGAATTTACTTCTTAAACCTAATCTGTAGTAAGGAAGGGATTGAAAAATACCATGCCGCCTTCCCAG
AGGTCAGAATTGTTACTGGTGCCCTCGACAGAGGTCTAGATGAAAACAAGTATCTAGTTCCAGGGTT
GGGTGACTTTGGTGACAGATACTACTGTGTTTAAGCGGCCGCGCCATAGATAAAATAAAAGATTTTA
TTTAGTCTCCAGAAAAAGGGGGG

D. Construction of CD-linker UPRT fusion gene (CDopt+3pt-LINK-UPRT [aka CDopt-LINKER-UPRT and CD2-L-UPRT] A fusion construct was also developed by cloning a linker (Ser-Gly-Gly-Gly-Gly)₄ domain between and in frame with the CD polyeptpide and the UPRT polyeptpide to generated a CDoptimized-linker-UPRT sequence using Scheme II as depicted in Figure 10B. The following primers were used to insert the linker.

| **Primer Name** | **Primer Sequence (5' to 3')** | | | |
|---|---|---|---|---|
| ins_60nt_after_477 | 5'- ccggcgagtgatccggcggcggcgcctccggcggcggcgcctccggcg gcggcgcctccggcggcggcgccaacccgttatt-3' | | | |
| ins_60nt_after_477-antisense | 5'- aataacgggttggcgccgccgccggaggcgccgccgccggaggcgcc gccgccggaggcgccgccgccggatcactcgccga-3' | | | |

| **Primer Name** | **Length (nt.)** | **Tm** | **Duplex Energy at 68°C** | **Energy Cost of Mismatches** |
|---|---|---|---|---|
| ins_60nt_after_477 | 82 | 79.77°C | -30.19 kcal/mole | 83.3% |
| ins_60nt_after_477-antisense | 82 | 79.77°C | -32.31 kcal/mole | 82.2% |

The resulting contruct has size: 1356 bp and the sequence immediately below:

AACACGATTATAAATGGTGACCGGCGGCATGGCCTCCAAGTGGGATCAAAAGGGCATGGATATCGCT
TACGAGGAGGCCCTGCTGGGCTACAAGGAGGGCGGCGTGCCTATCGGCGGCTGTCTGATCAACAACA
AGGACGGCAGTGTGCTGGGCAGGGGCCACAACATGAGGTTCCAGAAGGGCTCCGCCACCCTGCACGG
CGAGATCTCCACCCTGGAGAACTGTGGCAGGCTGGAGGGCAAGGTGTACAAGGACACCACCCTGTAC
ACCACCCTGTCCCCTTGTGACATGTGTACCGGCGCTATCATCATGTACGGCATCCCTAGGTGTGTGA
TCGGCGAGAACGTGAACTTCAAGTCCAAGGGCGAGAAGTACCTGCAAACCAGGGGCCACGAGGTGGT
GGTTGTTGACGATGAGAGGTGTAAGAAGCTGATGAAGCAGTTCATCGACGAGAGGCCTCAGGACTGG
TTCGAGGATATCGGCGAGTCCGGCGGCGGCGCCTCCGGCGGCGGCGCCTCCGGCGGCGGCGCCTCCG
GCGGCGGCGCCAACCCGTTATTCTTTTTGGCTTCTCCATTCTTGTACCTTACATATCTTATATATTA
TCCAAACAAAGGGTCTTTCGTTAGCAAACCTAGAAATCTGCAAAAAATGTCTTCGGAACCATTTAAG
AACGTCTACTTGCTACCTCAAACAAACCAATTGCTGGGTTTGTACACCATCATCAGAAATAAGAATA
CAACTAGACCTGATTTCATTTTCTACTCCGATAGAATCATCAGATTGTTGGTTGAAGAAGGTTTGAA
CCATCTACCTGTGCAAAAGCAAATTGTGGAAACTGACACCAACGAAAACTTCGAAGGTGTCTCATTC
ATGGGTAAAATCTGTGGTGTTTCCATTGTCAGAGCTGGTGAATCGATGGAGCAAGGATTAAGAGACT
GTTGTAGGTCTGTGCGTATCGGTAAAATTTTAATTCAAAGGGACGAGGAGACTGCTTTACCAAAGTT
ATTCTACGAAAAATTACCAGAGGATATATCTGAAAGGTATGTCTTCCTATTAGACCCAATGCTGGCC
ACCGGTGGTAGTGCTATCATGGCTACAGAAGTCTTGATTAAGAGAGGTGTTAAGCCAGAGAGAATTT
ACTTCTTAAACCTAATCTGTAGTAAGGAAGGGATTGAAAAATACCATGCCGCCTTCCCAGAGGTCAG
AATTGTTACTGGTGCCCTCGACAGAGGTCTAGATGAAAACAAGTATCTAGTTCCAGGGTTGGGTGAC
TTTGGTGACAGATACTACTGTGTTTAA
GCGGCCGCGCCATAGATAAAATAAAAGATTTTATTTAGTCTCCAGAAAAAGGGGGG

E.Construction of CD-OPRT fusion gene (CDopt+3pt-OPRT [aka CDopt-OPRT and CD2-OPRT], T5.0004 when inserted into the pAC3 plasmid RCR vector) A fusion construct was also developed comprising a CD polypeptide as described above linked to an OPRT polypeptide to generated a CDoptimized-OPRTase (CD humanized+3ptmutation + OPRTase functional domain human) using Scheme III as shown in Figurue 10C.

The resulting construct comprises a size of 1269 bp and the sequence immediately below:

AACACGATTATAAATGGTGACCGGCGGCATGGCCTCCAAGTGGGATCAAAAGGGCATGGATATCGCT
TACGAGGAGGCCCTGCTGGGCTACAAGGAGGGCGGCGTGCCTATCGGCGGCTGTCTGATCAACAACA
AGGACGGCAGTGTGCTGGGCAGGGGCCACAACATGAGGTTCCAGAAGGGCTCCGCCACCCTGCACGG
CGAGATCTCCACCCTGGAGAACTGTGGCAGGCTGGAGGGCAAGGTGTACAAGGACACCACCCTGTAC
ACCACCCTGTCCCCTTGTGACATGTGTACCGGCGCTATCATCATGTACGGCATCCCTAGGTGTGTGA
TCGGCGAGAACGTGAACTTCAAGTCCAAGGGCGAGAAGTACCTGCAAACCAGGGGCCACGAGGTGGT
GGTTGTTGACGATGAGAGGTGTAAGAAGCTGATGAAGCAGTTCATCGACGAGAGGCCTCAGGACTGG
TTCGAGGATATCGGCGAGGCGGTCGCTCGTGcagctttggggccattggtgacgggtctgtacgacg
tgcaggctttcaagtttggggacttcgtgctgaagagcgggctttcctcccccatctacatcgatct
gcgggggcatcgtgtctcgaccgcgtcttctgagtcaggttgcagatattttattccaaactgcccaa
aatgcaggcatcagttttgacaccgtgtgtggagtgccttatacagctttgccattggctacagtta
tctgttcaaccaatcaaattccaatgcttattagaaggaaagaaacaaaggattatggaactaagcg
tcttgtagaaggaactattaatccaggagaaacctgtttaatcattgaagatgttgtcaccagtgga
tctagtgttttggaaactgttgaggttcttcagaaggagggcttgaaggtcactgatgccatagtgc
tgttggacagagagcagggaggcaaggacaagttgcaggcgcacgggatccgcctccactcagtgtg
tacattgtccaaaatgctggagattctcgagcagcagaaaaaagttgatgctgagacagttgggaga
gtgaagaggtttattcaggagaatgtctttgtggcagcgaatcataatggttctcccctttctataa
aggaagcacccaaagaactcaGCTTCGGTGCACGTGCAGAGCTGCCCAGGATCCACCCAGTTGCATC
GAAGTAAGCGGCCGCGCCATAGATAAAATAAAAGATTTTATTTAGTCTCCAGAAAAAGGGGGG

F.Construction of CD-linker-OPRT fusion gene (CDopt+3pt-LINK-OPRT, [aka CDopt-LINKER-OPRT and CD2-L-OPRT], T5.0005 in the pAC3 plasmid RCR vector) A fusion construct was also developed by cloning a linker (Ser-Gly-Gly-Gly-Gly)₄) domain between and in frame with the CD polyeptpide and the OPRT polyeptpide to generated a CDoptimized-linker-OPRT sequence using Scheme IV as shown in Figure 10D.

The resulting construct comprises a size of 1329 bp and the sequence immediately below:

AACACGATTATAAATGGTGACCGGCGGCATGGCCTCCAAGTGGGATCAAAAGGGCATGGATATCGCT
TACGAGGAGGCCCTGCTGGGCTACAAGGAGGGCGGCGTGCCTATCGGCGGCTGTCTGATCAACAACA
AGGACGGCAGTGTGCTGGGCAGGGGCCACAACATGAGGTTCCAGAAGGGCTCCGCCACCCTGCACGG
CGAGATCTCCACCCTGGAGAACTGTGGCAGGCTGGAGGGCAAGGTGTACAAGGACACCACCCTGTAC
ACCACCCTGTCCCCTTGTGACATGTGTACCGGCGCTATCATCATGTACGGCATCCCTAGGTGTGTGA
TCGGCGAGAACGTGAACTTCAAGTCCAAGGGCGAGAAGTACCTGCAAACCAGGGGCCACGAGGTGGT
GGTTGTTGACGATGAGAGGTGTAAGAAGCTGATGAAGCAGTTCATCGACGAGAGGCCTCAGGACTGG
TTCGAGGATATCGGCGAGTCCGGCGGCGGCGCCTCCGGCGGCGGCGCCTCCGGCGGCGGCGCCTCCG
GCGGCGGCGCCGCGGTCGCTCGTGcagctttggggccattggtgacgggtctgtacgacgtgcaggc
tttcaagtttggggacttcgtgctgaagagcgggctttcctcccccatctacatcgatctgcggggc
atcgtgtctcgaccgcgtcttctgagtcaggttgcagatattttattccaaactgcccaaaatgcag

gcatcagttttgacaccgtgtgtggagtgccttatacagctttgccattggctacagttatctgttc
aaccaatcaaattccaatgcttattagaaggaaagaacaaaggattatggaactaagcgtcttgta
gaaggaactattaatccaggagaaacctgtttaatcattgaagatgttgtcaccagtggatctagtg
ttttggaaactgttgaggttcttcagaaggagggcttgaaggtcactgatgccatagtgctgttgga
cagagagcagggaggcaaggacaagttgcaggcgcacgggatccgcctccactcagtgtgtacattg
tccaaaatgctggagattctcgagcagcagaaaaagttgatgctgagacagttgggagagtgaaga
ggtttattcaggagaatgtctttgtggcagcgaatcataatggttctcccctttctataaaggaagc
acccaaagaactcaGCTTCGGTGCACGTGCAGAGCTGCCCAGGATCCACCCAGTTGCATCGAAGTAA
GCGGCCGCGCCATAGATAAAATAAAAGATTTTATTTAGTCTCCAGAAAAAGGGGGG

Figure 4 demonstrates that higher levels of the human codon optimized with the three mutations for higher stability are observed compared to wild type yCD protein in infected U-87 cells.

### Example 3 Vector production by transient transfection

Vector can be produced in a number of ways, but the first step is to introduce the DNA vector into cells to allow production of infectious particles, that can then be harvested from the cell supernatant. Once infectious particles have been generated other methods of production can be implemented by those skilled in the art. Vector particles were generated by transient transfection of 293T cells (Pear et al. Proc Natl Acad Sci USA. 90:8392-8396 1993).

The 293T cells were thawed and put into culture, then passaged twice in T-75 flasks containing 15 mL of the DMEM medium that was prepared by mixing DMEM High Glucose medium (Hyclone# 30081, 500 mL) with FBS (Hyclone# SH30070, 50 mL), L-Glutamine (Cellgro# 25-005-CI, 5 mL), NEAA (Hyclone #SH30238, 5 mL), and Penicillin-strep (Cellgro# 30-002-CI, 5 mL). The flasks were incubated at 37°C and 5% CO₂. After the 3^{rd} passage cells were seeded in 6 T-25's, each containing 5 mL of the medium, at a cell density of 1.8 x 10⁶ cells/T-25 (or 7.2 x 10⁴ cells/cm²). One day after seeding the T-25's, the cells were transfected with the T5.0002 plasmid that expressed the viral vector using the Calcium Phosphate Transfection Kit from Promega (Cat# E1200). Eighteen hours following transfection, the media in one set of the flasks (3 flasks each set) were replaced with fresh medium containing 10 mM NaB. The media in the 2^{nd} set of the flasks were not replaced, which served as a control (zero NaB). Eight hours post NaB treatment, the media in all flasks were replaced with the fresh medium containing no NaB. The expression was allowed to continue for both sets of flasks until the next day (22 hours duration). The supernatants from both sets of flasks were harvested and assayed for their titers by qPCR expressed in Transducing Units (TU)/ml (see example 4).

The titer results are shown in the following table.

| Condition | First titer | Second titer (after storing at -80°C for 68 days) |
|---|---|---|
| Without NaB | 1.5 (±0.05) x 10⁶ TU/mL | 1.2 (±0.2) x 10⁶ TU/mL |
| 10 mM NaB | 1.4 (±0.3) x 10⁶ TU/mL | 7.0 (±0.14) x 10⁵ TU/mL |

| | | |
|---|---|---|
| TU= transduction unit | | |

Subsequent vector preparations were produced in this manner, without sodium butyrate. Other vector plasmids (Table 2) have been used in the same way to generate vector preparations with titers between 1E5 TU/ml and 1E7 TU/ml. Such material can be further purified and concentrated, if desired, as described below see also: US 5792643; T. Rodriguez et al. J Gene Med 9:233 2007; US Application 61218063.

In certain embodiments of the disclosure the dosing was calculated by grams of brain weight. In such embodiments, the dosing of a replication competent retroviral vector of the disclosure useful in the methods for treatment can range from 10⁴ to 10⁶ TU per gram brain weight.

### Example 4 Quantitative PCR titering assay

The functional vector concentration, or titer, is determined using a quantitative PCR-based (qPCR) method. In this method, vector is titered by infecting a transducible host cell line (*e.g*. PC-3 human prostatic carcinoma cells, ATCC Cat# CRL-1435) with a standard volume of vector and measuring the resulting amount of provirus present within the host cells after transduction. The cells and vector are incubated under standard culturing condition (37 °C, 5% CO₂) for 24 hr to allow for complete infection prior to the addition of the anti-retroviral AZT to stop vector replication. Next, the cells are harvested from the culture dish and the genomic DNA (gDNA) is purified using an Invitrogen Purelink gDNA purification kit and eluted from the purification column with sterile RNase-/DNase-free water. The A₂₆₀/A₂₈₀ absorbance ratio is measured on a spectrophotometer to determine the concentration and relative purity of the sample. The gDNA concentrations are normalized with additional RNase-/DNase-free water to the lowest concentration of any given set of gDNA preparations such that the input DNA for the qPCR is constant for all samples analyzed. Genomic DNA purity is further assessed by electrophoresis of an aliquot of each sample on an ethidium bromide stained 0.8% agarose gel. If the sample passes an A₂₆₀/A₂₈₀ absorbance range of 1.8-2.0 and shows a single band of gDNA, then the sample is ready for qPCR analysis of provirus copy number of the vector. Using primers that interrogate the LTR region of the provirus (reverse-transcribed vector DNA and vector DNA that is integrated into the host gDNA), qPCR is performed to estimate the total number of transduction events that occurred when the known volume of vector was used to transduce the known number of cells. The number of transduction events per reaction is calculated from a standard curve that utilizes a target-carrying plasmid of known copy-number that is serial diluted from 10⁷ to 10 copies and measured under identical qPCR conditions as the samples. Knowing how many genomic equivalents were used for each qPCR reaction (from the concentration previously determined) and how many transduction events that occurred per reaction, we determine the total number of transduction events that occurred based on the total number of cells that were present at the time of transduction. This value is the titer of the vector after dilution into the medium containing the cells during the initial transduction. To calculate the corrected titer value, the dilution is corrected for by multiplying through by the volume of culture and the volume of titer divided by the volume of titer. These experiments are performed in replicate cultures and analyzed by qPCR using triplicate measurements for each condition to determine an average titer and with its associated standard deviation and coefficient of variance.

### Example 5 Vector Testing

In order to be effective vector constructs and their derived infectious particles need to: 1) make good titer of virus by transient transfection (see examples 3 and 4); 2) be stable upon multiple passages; 3)kill cells efficiently in the presence of 5-FC; and 4) express enzymic activity upon infection of target cells. Example 3 shows that useful titers can be obtained from the vectors.

Genetic Stability of viral vectors. To demonstrate the stability the following experiment was performed. Approximately 10⁶ naive U-87 cells were initially infected with the viral vector at an MOI of 0.01, and grown until fully infected to complete a single cycle of infection. Supernatant is then repassed onto uninfected cells and the cycle repeated. In this experiment,twelve cycles have been completed in duplicate trials (Figure 5 shows one of each of the duplicate trials; the other duplicates gave similar results). Genomic stability of the yCD2 or other trangene sequence is assessed by PCR amplification of the integrated provirus from the infected cells using MLV specific primers flanking the transgene insertion site. The appearance of any bands smaller than full-length amplicon would be an indicator of vector instability. Figure 5 demonstrates that a vector of the disclosure (T5.0007 - comprising the modified vector and CD heterologous polynucleotide) maintains stability for more passages than pACE-CD. Furthermore T5.0003 is somewhat less stable while T5.0004 and T5 appear about as stable as pACE-CD. pACE-CD has been used in mouse tumor studies and shows good anti-tumor effects in mouse models. However a more stable viral genome will be much more potent and long lasting in treatment of animals and humans, especially if multiple cycles of 5-FC treatment are required. Both T5.0001 and T5.0002 are markedy more stable than even T5.0007, showing that silent changes in a protein coding sequence or small changes that result in point mutations can lead to unexpectedly superior properties with more stable vector genomes.

Cell killing experiments. The CellTiter 96 Aqueous One Solution Cell Proliferation Assay (MTS) is a colorimetric method for determining the number of viable cells in proliferation assays. We have utilized this assay to determine cell growth kinetics, as well as to determine the dose response of various cell lines to 5-Fluorocytosine (5-FC) and 5-Fluorouracil (5-FU).

Cells 100% infected with vector were seeded at 1000 cells/well in 96-well plates. They were monitored over an eight day period following treatment with various concentrations of 5-FC (5-FU for controls). An analysis of their cell growth was assessed every two days utilizing Promega's Cell Titer 96 AQueous One Solution reagent (MTS). Briefly, 20 µl of MTS was mixed with 100 µl media (as recommended by the manufacturer) and added to the samples in the 96-well plate. The samples were incubated for 60 minutes in a 37°C/5% CO₂ incubator. Thereafter, absorbance readings were taken on a plate reader at a 490nm wavelength. The plates were then discarded.

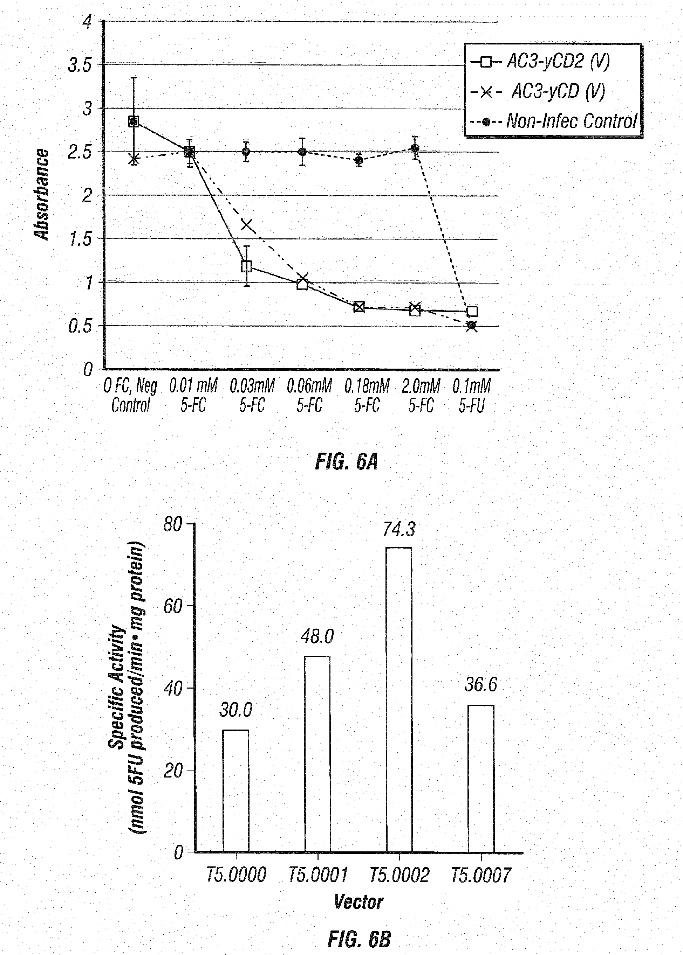

Figure 6A shows the results of an experiment that demonstrates that the cytosine deaminase in cells expressing the yCD2 protein is at least as active as that from cells expressing the wild type yCD protein, by performing 5-FC titrations on U-87 cells infected either with AC3-yCD2 (vector) or AC3-yCD (vector). Briefly, U-87 cells 5 days post infection at a multiplicity of infection of 0.1 (i.e. 100% infected) with either AC3-yCD (wild type CD) vector or AC3-yCD2 (thermostabilized & codon optimized) vector were subject to increasing amounts of 5-FC or 0.1 mM of 5-FU as a positive control for 8 days. On day 8, cell cultures were assessed for viability using an MTS assay (Promega CellTiter 96 AQUEOUS One Solution Proliferation Assay). Data shows comparable killing between the two retroviral vectors at increasing doses of 5-FC treatment.

In similar *in-vitro* cell culture experiments with RG2 cells (ATCC Cat# CRL-2433), the RG2 cell line was transduced with 5 different vectors (pACE-CD, T5.0001, T5.0002, T5.0004, and T5.0007). It was subsequently subject to increasing concentrations of 5-FC (5-FU for controls) for 8 days and monitored as described above. The results are shown in Figure 2. Concentrations of 0.01 mM were sufficient to induce complete killing throughout all vectors tested with the exception of wild type-yeast Cytosine Deaminase (pACE-yCD). It was less sensitive and required 1.0 mM of 5-FC for complete killing.

CD expression assay. U87 cells were transduced at a multiplicity of infection (MOI) of 0.1, cultivated for 5 days to allow viral spread and and cells from day 5 post transduction were harvested. The cells were then collected by centrifugation at 800 x g for 5 min. The supernatant was aspirated away from the cell pellet and washed with 5 mL of phosphate buffered saline (PBS) and again centrifuged at 800 x g for 5 min. The resulting cell pellet was taken up in 1.5 mL of PBS, resuspended by passage through a pipette tip and placed in a freezer at -20C. Cells were lysed by a freeze/thaw method. Previously resuspended cells were allowed to thaw at room temperature, passed through a pipette tip, mixed with protease inhibitor cocktail and again refrozen at -20C. Previous to the enzyme assay, the sample was again thawed at room temperature and passed through a pipette tip. The suspension was then centrifuged at 14,000 rpm in a tabletop centrifuge for 5 min. The supernatant was decanted away from the pellet and placed in a fresh eppendorf tube and placed on ice.

yCD enzyme activity was assessed by using an HPLC assay. The HPLC assay was performed on a Shimadzu LC20AT unit connected in series with a photoarray detector and autoinjector. The solid phase was a Hypersil BDS C₁₈ HPLC column with a 5 um sphere size and 4.0 x 250 mm column dimensions. The mobile phase was 50 mM ammonium phosphate, pH 2.1, containing 0.01% tert-butylammonium perchlorate and 5% methanol; the system was equilibrated at 22 C. All reagents were ACS grade and solvents were HPLC grade. A reaction mix was made consisting of 800 µL with a final concentration of 0.125 mg/mL 5FC (1 mM) in PBS and placed in a 1.5 mL autosampler vial. The reaction was then initiated by adding 200uL of each cell lysate. The reaction / autosampler vials were placed in the auto sampler and 5uL of the reaction mixture was injected. Time points were taken periodically by retrieving a 5 uL aliquot from each reaction vial and analyzing on the HPLC column. The conversion rates of 5FC to 5FU were calculated by comparing the peak areas with known amounts from a previously generated standard curve of 5FU. The rate of 5FC conversion to 5FU was derived by plotting the amount of 5FU (in nmol) generated against its corresponding time interval. Protein concentration for the cell sample was derived and the Specific Activity of the cell lysate samples were calculated by dividing the conversion rate (nmol/min) by the amount of protein used in the assay in mg. Figure 6B shows the specific activity of various vectors after 5 days on transduction at an MOI of 0.1. The data demonstrate that pACE-yCD (T5.0000) < pAC3-yCD1(T5.0001) <pAC3-CD2 (T5.0002)in terms of the specific activity of cytosine deaminase in tissue culture cells.

### Example 6 Vector Purification and concentration

A vector of the disclosure is manufactured by transient transfection on 293T cells (Example 3), followed by harvesting of the cell supernatant, filtration, benzonase treatment, diafiltration/concentration and dialysis. A further chromatography column step may be included, known to those skilled in the art (see for example US5792643; T. Rodriguez et al. J Gene Med 9:233 2007; US Application 61218063). Clinical material is released based on standard testing such as sterility, mycoplasma and endotoxins, plus product specific potency, strength, and identity testing. Titer is determined as Transducing Units (TU) by PCR quantitation of integrated viral vector DNA in target cells (Example 4). The final product is targeted to have a titer of up to 10⁹ TU/ml formulated in isotonic Tris-buffered sucrose solution, as a sterile injectable.

In general, to accurately and precisely determine the strength of vector lots, a quantitative PCR-based titer assay has been developed (described in general tersm in example 4). The details of the assay procedure consist of the following steps:

Transduction. Transductions are performed in a 12-well plate format using the stable human prostate adenocarcinoma derived PC-3 cell line. For each test sample, three dilutions of untitered vector preparation are used to transduce PC-3 cells in triplicate wells. Viral replication is stopped 24 hours post-transduction with azidothymidine (AZT). Cells are maintained for an additional 24 -64 hours prior to harvesting and genomic DNA purification.

Genomic DNA Preparation. Qiagen DNeasy DNA Minikits is used to prepare genomic DNA from transduced harvested cells as per the manufacturer's protocol. DNA concentrations and quality are assessed by direct absorbance measurement using UV/vis spectrophotometry to determine the A260 and A260/A280 ratio.

Real-time Quantitative PCR. The BioRad CFX96 real-time PCR instrument or equivalent is used for performing quantitative PCR. Provector copy numbers present in each test sample are measured by using specific DNA oligonucleotide primers in conjunction with a TaqMan probe designed to amplify the integrated, or pro-retroviral, U3/Psi packaging versus the CMV/Psi plasmid promoter. Vector titer is expressed relative to a copy number standard curve. To generate the vector copy number standard curve, genomic DNA from PC-3 cells is spiked with a unique plasmid containing the pro-retroviral U3/Psi sequence. Vector test sample titers are obtained by calculating the number of transduced genomes in multiple dilutions using multiple reactions per dilution.

For each titer assessment, a non template control (wells containing all components except plasmid or genomic DNA) and a negative control (all components including equivalent genomic DNA from non-transduced PC-3 cells), is performed in triplicate. The titer values are expressed in transduction units per milliliter (TU/mL).

The potency of the vector of the disclosure is dependent on both the replication of the vector and the resultant cytosine deaminase (CD) activity in target cells. Therefore the potency assay measures the increase in CD activity over time as vector infection spreads in a previously uninfected cell line in tissue culture. The assay measures the enzymatic activity of the transferred yCD2 protein in transduced cells during early, middle and late stages of infection by monitoring the conversion of 5-fluorocytosine (5-FC) to 5-fluorouracil (5-FU), using reverse phase HPLC separation with UV detection. The increase of CD activity over the course of the infection is a function of the percent of cells infected over time and indicative of the TOCA 511 vector's ability to replicate. CD activity based on the 5-FC to 5-FU conversion rate is measured for each time point in CD units per mg of protein (the specific activity, SA). The increase in SA is then plotted over time, and reflects both the increase in the percentage of cells transduced as a result of viral replication in the culture, and the resultant transfer of CD activity. Accumulated data from multiple assays and vector lots has been used to determine an appropriate specification for this increase in SA of CD, for product release. The assay has 1, 3 and 5 day timepoints after an initial infection at an MOI of about 0.1 and a non-infected control.

CD activity from late stage infected cells (day 5 time point) was compared between lots to evaluate the use of this activity as an Identity test. The assay includes the following steps:

Transductions. Transductions are performed in multi-well plate format on U87 cells. For each transduction, three suitable dilutions are used and each performed in triplicate. Cells are harvested at 0, 1, 3 and 5 days post transduction.

Set-up of CD Reaction. Cells are lysed and the total protein concentration determined using the BCA protein assay using BSA as a standard. For the yCD2 enzyme assay, an appropriate amount of cell lysate is added to buffer containing 5-FC such that the rate of 5-FU formation remains linear over 1-2 hours at 37°C. The final volume for the reaction mixture is 100 µL. After 2 h, the enzyme reaction is terminated by the addition of trichloroacetic acid, briefly vortexed and prepared for subsequent HPLC analyses. Cell lysates from non-transduced cells are used as a negative control while a similar assay using samples from 100% infected cells is used as a positive control.

HPLC analysis. The terminated reaction mixture is centrifuged at 12,000 rpm for 5 minutes at room temperature in a micro-centrifuge. The supernatant is then decanted away from the pellet and passed through a 0.2µ filter to further remove particulates before injection onto a reverse phase HPLC column previously equilibrated with an aqueous based mobile phase containing phosphate buffer at a pH around 4.0. The chromatograms is followed at 260 nm and 280nm to monitor both substrate consumption and product formation. Concentrations of either substrate or product are determined using the graphing and analysis capabilities of GraphPad by comparing them to previously generated standard curves calculated from known substrate or product concentrations. Amounts of 5-FU generated over 1-2 h are used to determine CD units of activity (1 unit of CD activity is defined as the formation 1 nmol of 5-FU per min) and the Specific Activity is calculated dividing this number by the amount of protein (from the cell lysate) used in the assay.

### Example 7 Construction and use of a vector encoding a single chain antibody to CTLA-4 (CD 152).

Single chain antibodies are derived from known full antibody sequences that have a desired effect. Such sequences are available (*e.g.* WO2006048749, US2006165706, US7034121, Genbank Accession Numbers DJ437648, CS441506, CS441500, CS441494, CS441488, the disclosures of which are incorporated herein by reference). Such conventional antibody gene sequences are converted into single chain antibody (scFv) sequences by commonly used methods known to those skilled in the art (see for example Gilliland et al. "Rapid and reliable cloning of antibody variable regions and generation of recombinant single chain antibody fragments." Tissue Antigens 47, 1-20, 1996). Phage single chain antibodies to CTLA-4 are also available from screening phage-scFv libraries directly, and can be used directly for insertion into the replicating retroviral vectors of the current invention (Pistillo et al. Tissue Antigens 55:229 2000). Regardless of how the sequence is derived scFv are typically about 700-900bp in length and are synthesized by a commercial vendor (BioBasic) with a Psi1 site at the 5' end and compatible Not1 site at the 3' end, as described previously. This sequence is then inserted into the replicating retroviral back bone from pAC3-yCD2 at the Psi1-Not1 sites after removal of the yCD2 sequence. Vector is produced and titered as described, and further purified if necessary as described above. Human and Mouse CTLA4 are very homologous in sequence and the replicating retrovirus of the invention is first tested in a suitable syngeneic immunocompetent mouse models such as the CT26/Balb/c model and s91 mouse melanoma models, well known to those skilled in the art (see for example Hodge et al J.Immunol. 174:5994 2005). Outcome is measured by one or more of: modulation of tumor growth; lack of toxicity; generation of antitumor responses; shrinkage of remote lesions indicating systemic immunity. Doses are in the range of 10³ to 10⁷ TU in mice. In patients the vector is administered by intralesional injection into tumor, or by administration into the circulation that then carries the virus to the tumor. Doses are in the range of 10⁵ to 10¹¹ TU.

### Example 8 Anti-melanoma efficacy studies with anti CD152 single chain antibody expressing vector in a mouse melanoma model. Objective

The objective of this study is to assess the effect of a novel MLV based replication-competent retroviral vector carrying single chain antibody directed against Cytotoxic T-Lymphocyte Antigen 4 (CTLA-4) also referred to as Cluster of differentiation 152 (CD152) sequence (pAC3-αCD152) on melanoma growth, when delivered via intratumoral (IT) injection in DBA/2 mice bearing subcutaneous melanoma (Cloudman S91).

### Mice

Female DBA/2 mice (age ∼8 weeks) are purchased from Jackson Laboratories (Bar Harbor, ME). Mice are acclimated for 7 days after arrival before start of studies.

### Cells

Cloudman S91 cells (ATCC, Manassas VA) are a spontaneously arising melanoma derived from DBA/2 mice. Cells are cultured in Dulbecco's modified Eagles medium with 10% fetal bovine serum, sodium pyruvate, and Glutamax (Hyclone, Logan UT, and Invitrogen, San Diego CA). Cells are resuspended in PBS (Hyclone, Logan UT) for implantation. S91 cells (1E6 in 100 pL) are injected into the right flank of DBA/2 mice.

### Vector

Vectors preparations are made by transient transfection (or from a producer cell line in HT1080 cells see US application) with titers of approximately 7E6TU/ml. For initial studies vector is not further purified or concentrated. For follow on experiments to determine full dose response curves, high titer purified material is prepared with a titer expected around 10E8/ml. Vector is administered IT in a volume of 50-100 µL and IV in 100 µL the total dose/mouse of approximately 7E5 TU/mouse. Vector expressing αCD152 is identified as Toca αCD152.

### Tumor implantation and vector injection

Five groups of female DBA/2 (55 mice, 9-10 weeks of age) are implanted subcutaneously with S91 melanoma cells (Day 0) and then dosed (day 4-7 depending on growth rate of the S91 tumor; approximately 50-100mm³) with vehicle (Groups 1), with control vector [AC3-GFP(V), (Group2), intratumor (IT) Toca αCD152 vector injection (Groups 3), or intravenous Toca αCD152 vector injection (group 4). Group 5 mice have no tumor implanted and are intravenously injected with Toca αCD152 only.

### Data analyses

Tumor growth analysis is carried out to 2000mm³ or to 60 days based on which ever comes first. 10 mice from each group will be plotted for tumor size over time. Statistical significance will be determined using analysis of variance (ANOVA). P values of <0.05 are considered statistically significant in all analyses, which are performed with Prism 5 statistical software (GraphPad Software) or equivalent. In-life observations are also taken to assess any adverse events to αCD152 expression during treatment.

### Results

Delivery of αCD152 by replicating MLV IT shows a statistically significant retardation of growth compared to the controls. Delivery of αCD152 by replicating MLV intravenously shows a statistically significant retardation of growth compared to the controls abrogates melanoma burden from the DBA/2-Cloudman S91 mouse melanoma model.

Further animal studies were performed as follows:

### Example 9 ACE-yCD2 viral vector is therapeutic in an intracranial human xenograft (U87) in nude mice

An intracranial xenograft model using the U87 human glioma cell line was established to test RCR vector spread and biodistribution as well as therapeutic efficacy of RCR-vector mediated cytosine deaminase suicide gene therapy in a nude mouse host.

Following acclimation, mice were randomly assigned to one of 8 Treatment groups (see group description below). Seven groups underwent intracranial administration into the right striatum of 1 x 10⁵ U87 cells administered/mouse on Day 0. Group 8 mice were not implanted with tumor. At Day 5, mice were injected with Formulation Buffer only, or an RCR vector at 9 x 10⁵/5ul, 9 x 10⁴/5ul, or 9 x 10³/5ul. Mice receiving no vector, or vector at 9 x 10⁵/5ul or 9 x 10³/5ul were randomized to receive 5-FC (500 mg/kg/day), administered as a single IP injection, beginning on Day 19, or no 5-FC. Mice receiving vector at mid dose all received 5-FC (ie. No separate control group for this dose). 5-FC administration continued daily for 7 consecutive days followed by 15 days of no treatment. Cycles of drug plus rest were repeated up to 4 cycles. 10 mice from each group except group 8 were randomly assigned to the survival analysis category. The remaining mice were sacrificed according to a predetermined schedule.

### Group Assignments and Dose Levels

| Group | Test article | Volume | Drug TX | N | N per Analysis Category | |
|---|---|---|---|---|---|---|
| | | | | | (A) Surv ival analysi s | (B) Schedul ed Sacrifice |
| 1 | Form buffer | 5 ul | none | 4 | | 4 before first drug cycle |
| 2 | Form buffer | 5 ul | 5FC | 10 | | |
| 3 | T5.0002 | 9e5/5ul | PBS | 10 | | |
| 4 | T5.0002 | 9e5/5ul | 5FC | 25 | 10 | 3 before start of each cycle, 15 total |
| 5 | T5.0002 | 9e4/5ul | 5FC | 10 | | |
| 6 | T5.0002 | 9e3/5ul | 5FC | 25 | 10 | 3 before start of each cycle, 15 total |
| 7 | T5.0002 | 9e3/5ul | PBS | 10 | | |
| 8 NO TUMOR | none | | 5FC | 15 | | 3 before start of each cycle, 15 total |
| Total Number of Animals | | | | **109** | 60 | 49 |

Intravenous dosing was done via injection into the tail vein. Intraperitoneal dosing was done via injection into the abdomen with care taken to avoid the bladder. For intracranial injection mice were anesthetized with isoflurane and positioned in a stereotaxic device with blunt ear bars. The skin was shaved and betadine was used to treat the scalp to prepare the surgical site. The animal was placed on a heating pad and a scalpel used under sterile conditions to make a midline incision through the skin. Retraction of the skin and reflection of the fascia at the incision site will allow for visualization of the skull. A guide cannula with a 3mm projection, fitted with a cap with a 3.5 mm projection, will be inserted through a small burr hole in the skull and attached with dental cement and three small screws to the skull. After hardening of the cement, the skin will be closed with sutures. The projected stereotaxic coordinates are AP= 0.5-1.0 mm, ML=1.8-2.0 mm, DV= 3.0 mm. Exact stereotaxic coordinates for the cohort of animals received will be determined in a pilot experiment (2-3 animals) by injecting dye and determining its location. The animals will be monitored during anesthesia recovery. Analgesics, buprenorphine, will be administered subcutaneously (SC) before the end of the procedure then buprenorphine will be administered approximately every 12 hrs for up to 3 days. Animals will be monitored on a daily basis. Cells or vector were intracranially infused through an injection cannula with a 3.5 mm projection inserted through the guide cannula. The rate was controlled with a syringe pump fitted with a Hamilton syringe and flexible tubing. For cell injection, 1 microliter of cells was delivered at a flow rate of 0.2 microliters per minute (5 minutes total).For vector injection, 5 microliters of vector was delivered at a flow rate 0f 0.33 microliters per minute (15 minutes total).

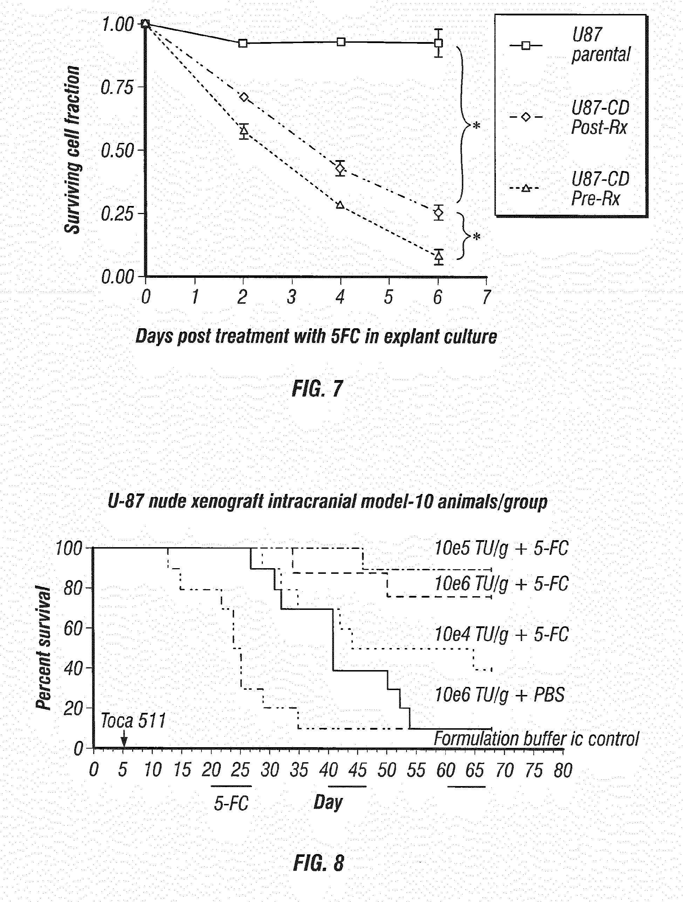

Vector was delivered and calculated as transforming units (TU) per gram of brain weight to the mice. Using such calculation the translation of dose can be calculated for other mammals including humans. Figure 8 shows the effect on vector dose in this mouse model.

### Example 10 AC3-yCD2 (V) is therapeutic in a syngeneic mouse model of brain cancer.

Additional experiments to demonstrate the methods and compositions of the disclosure in a syngeneic animal model were performed.

An intracranial implant model using the CT26 colorectal cancer cell line in syngeneic BALB/c mice was established to test RCR vector spread and biodistribution as well as therapeutic efficacy of RCR-vector mediated cytosine deaminase suicide gene therapy and its immunological impact.

This study included 129 animals, 0 Male, 119 Female and 10 contingency animals (10 Female). Following acclimation, mice were randomly assigned to one of 8 Treatment groups (see group description below). Seven groups underwent intracranial administration into the right striatum of 1 x 10⁴ CT26 cells administered/mouse on Day 0. Group 8 mice were not implanted with tumor. At Day 4, mice were injected with Formulation Buffer only, or vector at 9 x 10⁵/5ul, 9 x 10⁴/5ul, or 9 x 10³/5ul. Mice receiving no vector, or vector at 9 x 10⁵/5ul or 9 x 10³/5ul were randomized to receive 5-FC (500 mg/kg/BID), administered by IP injection, beginning on Day 13, or no 5-FC. Mice receiving vector at mid dose received 5-FC (ie. No separate control group for this dose). 5-FC administration continued daily for 7 consecutive days followed by 10 days of no treatment. Cycles of drug plus rest were repeated up to 4 cycles. 10 mice from each group except group 8 were randomly assigned to the survival analysis category. The remaining mice were sacrificed according to a predetermined schedule.

Naive sentinel mice were co-housed with the scheduled sacrifice animals and taken down at the same timepoints to assess vector transmittal through shedding.

### Group Assignments and Dose Levels

| Group | Test article | Volume | Drug TX | N | N per Analysis Category | | |
|---|---|---|---|---|---|---|---|
| | | | | | (A) Surv ival analysi s | (B) Schedule d Sacrifice | (C) Sentinels |
| 1 | Form buffer | 5 ul | PBS | 4 | | 4 before first drug cycle | |
| 2 | Form buffer | 5 ul | 5FC | 10 | | | |
| 3 | T5.0002 | 9e5/5ul | BS | 10 | | | |
| 4 | T5.0002 | 9e5/5ul | 5FC | 25 | 10 | 3 before start of each cycle, 15 total | 1 before start of each cycle, 5 total |
| 5 | T5.0002 | 9e4/5ul | 5FC | 10 | 10 | | |
| 6 | T5.0002 | 9e3/5ul | 5FC | 25 | 10 | 3 before start of each cycle, 15 total | 1 before start of each cycle, 5 total |
| 7 | T5.0002 | 9e3/5ul | PBS | 10 | | | |
| 8 NO TUMOR | none | | 5FC | 15 | | fore start of each cycle, 15 total | |
| Total Number of Animals | | | | 119 | 60 | 49 | 10 |

Intravenous dosing was done via injection into the tail vein. Intraperitoneal dosing was done via injection into the abdomen with care taken to avoid the bladder. For intracranial administration, mice with a guide cannula with a 3.2 mm projection implanted into the right striatum, and fitted with a cap with a 3.7 mm projection were used. The projected stereotaxic coordinates are AP= 0.5-1.0 mm, ML=1.8-2.0 mm, DV= 3.2 mm (from bregma). Cells or vector were intracranially infused through an injection cannula with a 3.7 mm projection inserted through the guide cannula. The rate was controlled with a syringe pump fitted with a Hamilton syringe and flexible tubing.

For cell injection, 1 microliter of cells wass delivered at a flow rate of 0.2 microliter per minute (5 minutes total). For vector injection, 5 microliter of vector was delivered at a flow rate of 0.33 microliter per minute (15 minutes total).

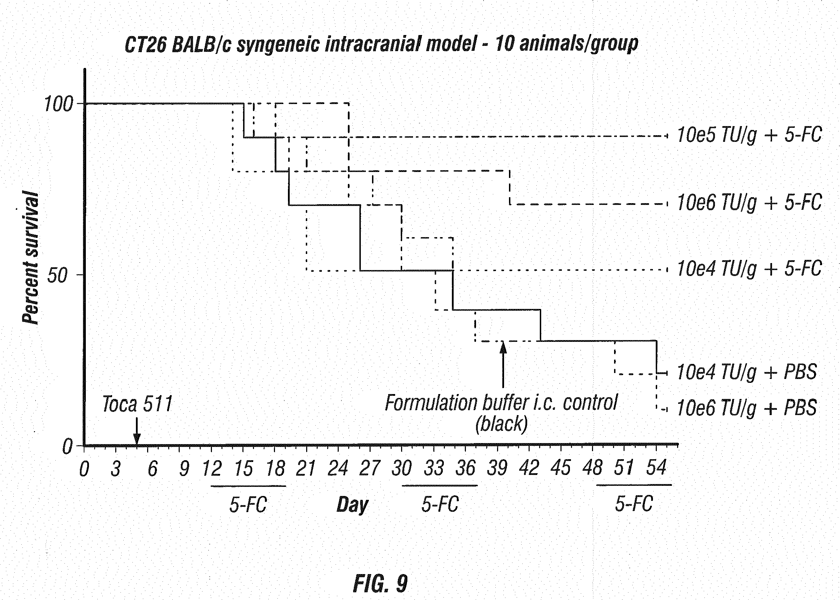

Vector was delivered and calculated as transforming units (TU) per gram of brain weight to the mice. Using such calculation the translation of dose can be calculated for other mammals including humans. Figure 9 shows the effect on vector dose in this mouse model when the vector is delivered intracranially

### Example 11 Construction and testing of RCR vectors expressing miR-128-1 and miR128-2.

### A.Construction of recombinant replication competent retroviral vector containing a heterologous polynucleotide sequence of human pri-miRNA-128-1

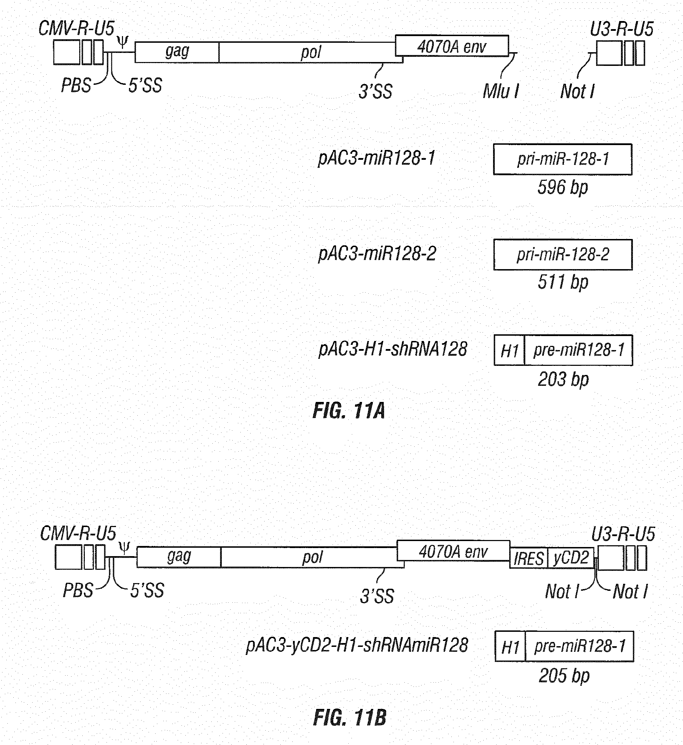

The replication competent retroviral vector, pAC3-miR-128-1 expressing miR-128-1 was derived from the backbone of pAC3-yCD2 described in one of the embodiment. The pAC3 backbone in the pAC3-miR-128-1 vector was isolated by endonuclease digestion of the pAC3-yCD2 plasmid DNA with Mlu I and Not I to remove the IRES-yCD2 polynucleotide sequence. The polynucleotide DNA sequence of pri-miR-128-1 was obtained from the product sheet of the pEP-mir-128-1 expression vector (Cell BioLabs Inc.) (SEQ ID NO: 31). DNA sequence of pri-miR-128-1 was synthesized with a Mlu I restriction enzyme site at the 5' end and a Not I restriction enzyme site at the 3'end of the double-stranded DNA fragment for subsequent insertion at the corresponding site in the Mlu I and Not I digested pAC3-yCD2 plasmid DNA described above. The resulting construct, pAC3-miR-128-1, encodes 3 genes: the gag, the pol, and the env, and the non-coding pri-miR-128-1 sequence (Figure 11).

Vector stock was produced by transient transfection of the vector-encoding plasmid DNA into 293T cells using calcium phosphate method. Eighteen hours post transfection, the culture was replaced with fresh medium. Twenty-four hours post medium replacement, the supernatant containing the vector was collected and filtered through a 0.45 m filter and used immediately or stored in aliquots at -80°C for later use. Twenty micro-liter of the collected vector stocks was used to infect human prostate cancer cells, PC3. Twenty-four hours post infection, AZT was added to the cells to inhibit further viral replication. Forty-eight hours post infection, genomic DNA of infected PC3 cells was extracted for titer assay. The titer of the vector stocks was determined by qPCR with an inclusion of standards of known copy numbers.

### B. Construction of recombinant replication competent retroviral vector containing a heterologous polynucleotide sequence of human pri-miRNA-128-2

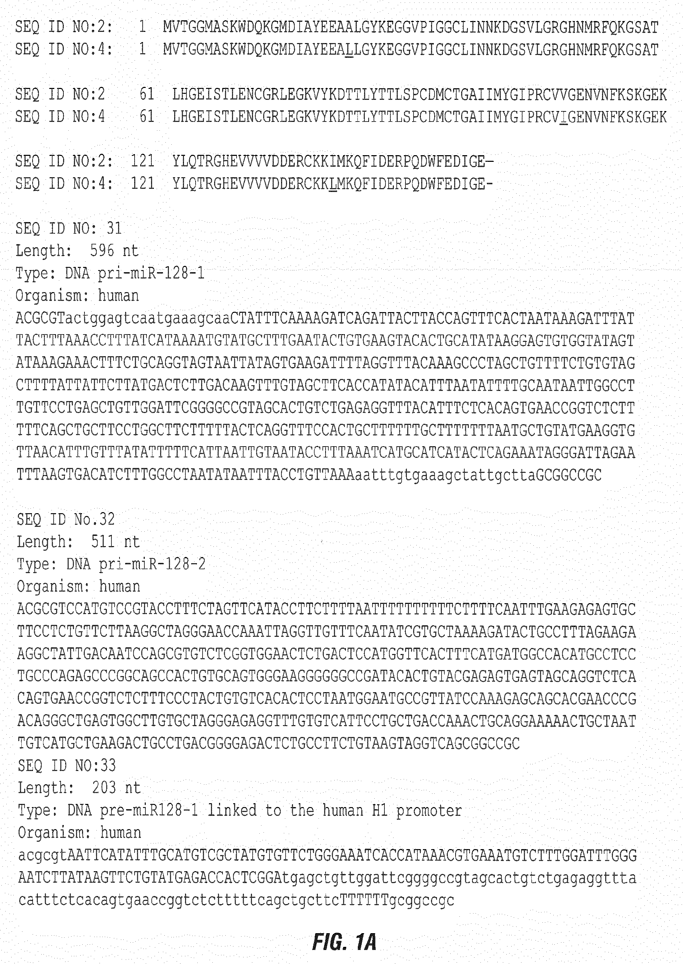

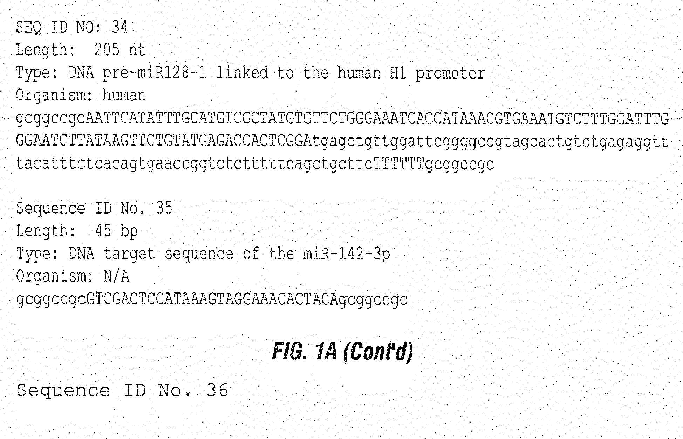

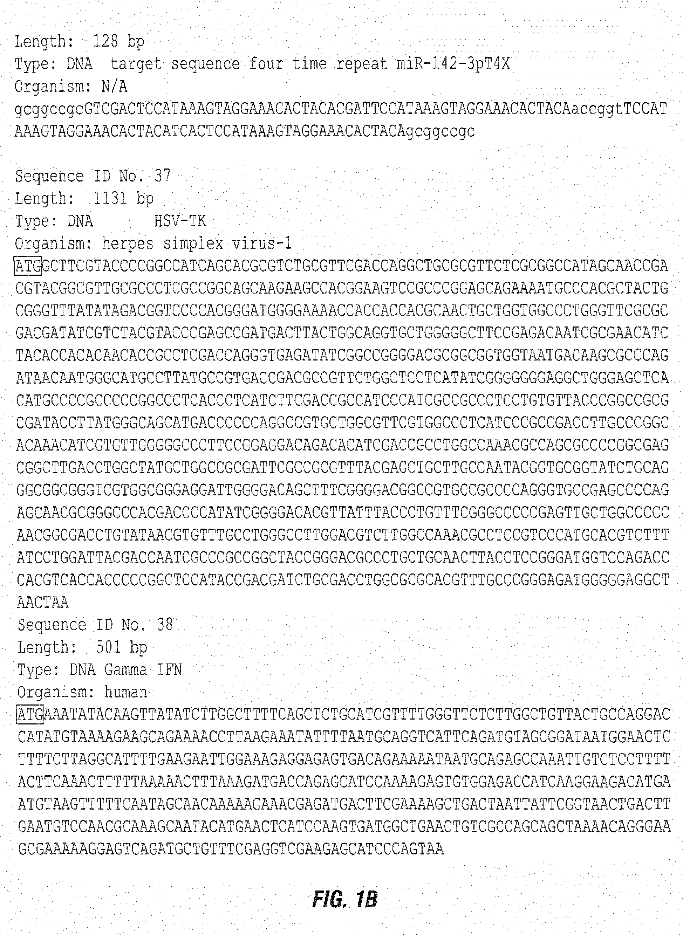

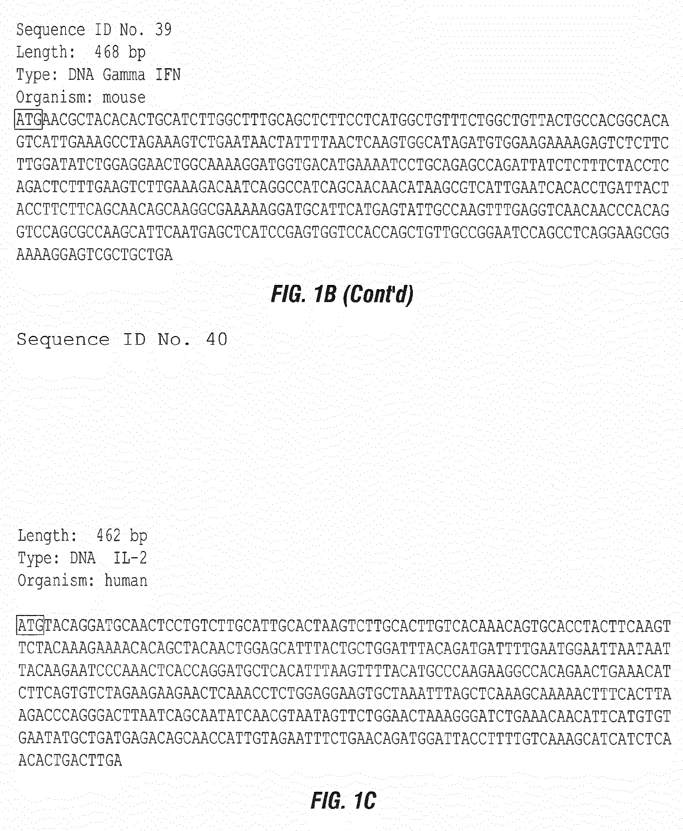

The replication competent retroviral vector, pAC3-miR-128-2 expressing miR-128-2 was derived from the backbone of pAC3-yCD2 described in one of the embodiment. The pAC3 backbone in the pAC3-miR-128-1 vector was isolated by endonuclease digestion of the pAC3-yCD2 plasmid DNA with Mlu I and Not I to remove the IRES-yCD2 polynucleotide sequence. The polynucleotide DNA sequence of pri-miR-128-2 was obtained from the sequence analysis of the expression vector Lenti-miR-128-2 expressing the pri-miR128-2 (System Biosciences) (SEQ ID NO:32). DNA sequence of pri-miR128-2 was synthesized with a Mlu I restriction enzyme site at the 5' end and a Not I restriction enzyme site at the 3'end of the double-stranded DNA fragment for subsequent insertion at the corresponding site in the Mlu I and Not I digested pAC3-yCD2 plasmid DNA described above. The resulting construct, pAC3-miR-128-1, encodes 3 genes: the gag, the pol, and the env, and the non-coding pri-miR-128-2 sequence (Figure 1).

Vector stock was produced by transient transfection of the vector-encoding plasmid DNA into 293T cells using calcium phosphate method. Eighteen hours post transfection, the culture was replaced with fresh medium. Twenty-four hours post medium replacement, the supernatant containing the vector was collected and filtered through a 0.45 m filter and used immediately or stored in aliquots at -80°C for later use. Twenty micro-liter of the collected vector stocks was used to infect human prostate cancer cells, PC3. Twenty-four hours post infection, AZT was added to the cells to inhibit further viral replication. Forty-eight hours post infection, genomic DNA of infected PC3 cells was extracted for titer assay. The titer of the vector stocks was determined by qPCR with an inclusion of standards of known copy numbers.

### C. Construction of recombinant replication competent retroviral vector containing heterologous polynucleotide sequences of human H1 promoter and human pre-miRNA-128-

The replication competent retroviral vector, pAC3-miR-128-2 expressing miR-128-2 was derived from the backbone of pAC3-yCD2 described in one of the embodiment. The pAC3 backbone in the pAC3-miR-128-1 vector was isolated by endonuclease digestion of the pAC3-yCD2 plasmid DNA with Mlu I and Not I to remove the IRES-yCD2 polynucleotide sequence. The polynucleotide DNA sequence of the human H1 promoter was obtained from the product information of pSilencer 3.1 H1 hygro expression vector (Ambion), and the polynucleotide DNA sequence of the short hairpin structured pre-miR-128-1 was obtained from the http://www.mirbase.org/. DNA sequence of pre-miR128-1 linked to the human H1 promoter (SEQ ID NO: 33) was synthesized with a Mlu I restriction enzyme site at the 5' end and a Not I restriction enzyme site at the 3'end of the double-stranded DNA fragment for subsequent insertion at the corresponding site in the Mlu I and Not I digested pAC3-yCD2 plasmid DNA described above. The resulting construct, pAC3-H1-shRNAmiR128, encodes 3 genes: the gag, the pol, and the env, and the non-coding short hairpin structured pre-miR-128-1 sequence (Figure 11).

Vector stock was produced by transient transfection of the vector-encoding plasmid DNA into 293T cells using calcium phosphate method. Eighteen hours post transfection, the culture was replaced with fresh medium. Twenty-four hours post medium replacement, the supernatant containing the vector was collected and filtered through a 0.45 m filter and used immediately or stored in aliquots at -80°C for later use. Twenty micro-liter of the collected vector stocks was used to infect human prostate cancer cells, PC3. Twenty-four hours post infection, AZT was added to the cells to inhibit further viral replication. Forty-eight hours post infection, genomic DNA of infected PC3 cells was extracted for titer assay. The titer of the vector stocks was determined by qPCR with an inclusion of standards of known copy numbers.

### D. Analysis of replication kinetics of recombinant replication competent retroviral vector by qPCR assay

Currently, there are at least two common ways to obtain replication kinetics of recombinant replication competent retroviral vector: (1) analysis of GFP expression with vectors expressing the GFP protein by flow cytometric analysis, and (2) reversed transcriptase assay by measuring the reverse transcriptase activity of the vector stock collected from cultured medium of transduced cells. Titers assessed by DNA analysis of transduced cells provide the most reliable estimate of the functional titers as compared to titers obtained by RNA and transgene expression in which titers were over-estimated and under-estimated, respectively, (Sastry et al., 2002 Gene Therapy 9, 1155-1162). The replication kinetics of viral spread correlates with the percent of a cell population being transduced in which an integrated proviral DNA of the viral genome can be detected by qPCR with primers specific to the viral sequence is being tested in culture. The present assay requires equal amount of genomic DNA in-put from all time points within the same vector tested and among various vectors if a comparison of replication kinetics is being tested. The replication kinetics graph was generated by plotting inversed C(t) values vs. time points. Figure 12A and Figure 12B show comparisons of replication kinetics of various recombinant replication competent retroviral vectors. The assay is sensitive to reveal the small difference in replication kinetics among various vectors.

### E. Testing of replication kinetics of miR-128 containing recombinant replication competent retroviral vectors in cultur

In order to confirm that the incorporation of pri-miR-128-1, pri-miR-128-2 and H1-pre-miR-128-1 sequence, respectively, in the present invention replicates normally, calculated volume of each vector stocks collected from transient transfection mentioned above was used to infect fresh human fibrosarcoma cells, HT1080 and human glioma cells, U87-MG, respectively, at a MOI of 0.1. Transduced cells were passaged at day 3, 6 and 9 post infection. At each time point, a portion of cells were collected for genomic DNA extraction for qPCR. Dilutions of genomic DNA were made to generate aliquots of genomic DNA with same concentration for equal amount of genomic in-put in qPCR. Replication kinetics of each vectors were generated by plotting inversed C(t) values vs. time points. Figure 12A and 12B show that all vectors tested replicated at similar kinetics compared to control MLV virus.

### E. Testing of expression of mature miR-128 from cells transduced with miR-128 containing recombinant replication competent retroviral vector

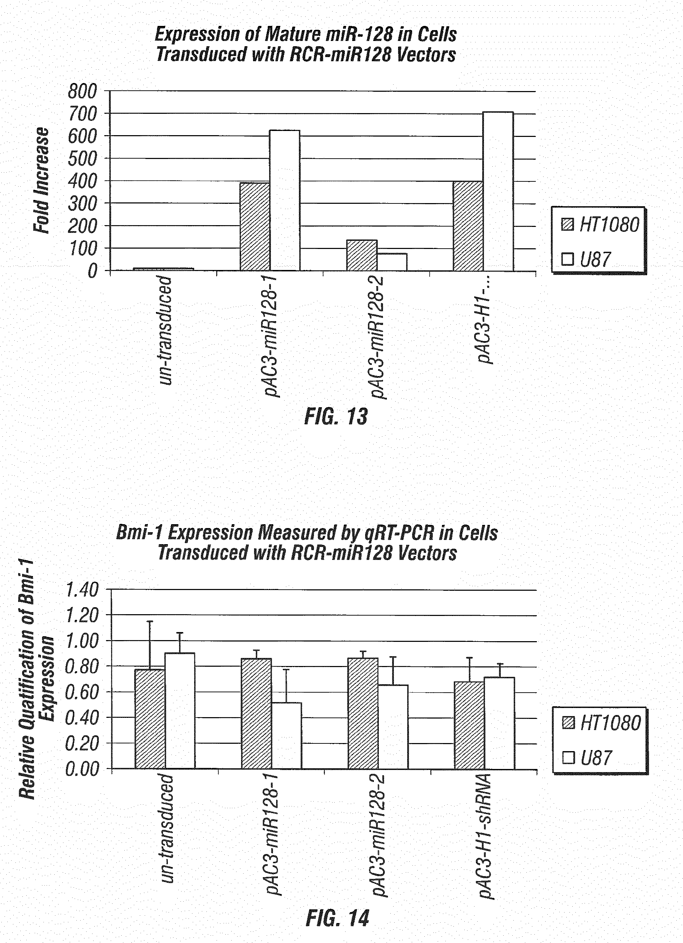

In order to confirm the expression of miR-128 from cells transduced with miR-128 containing recombinant replication competent retroviral vectors, cells from day 9 post infection at which the maximal infectivity has reached (Figure 12A and Figure 12B) were expanded and harvested to extract total RNA for detection of mature miRNA expression. The results from Taqman microRNA assay showed an over expression of mature miR-128 from both HT1080 and U87-MG cells transduced with pAC3-miR-128-1, pAC3-miR-128-2, and pAC3-H1-shRNAmiR128 vectors, respectively, compared to untransduced cells (Figure 13). In both cell lines, cells transduced with pAC3-miR-128-1 and pAC3-H1-shRNAmiR128 vector expressed higher level of mature miR-128 than cells transduced with pAC3-miR-128-2 vector.

### F. Testing of Bmi-1 expression from cells transduced with miR-128 containing recombinant replication competent retroviral vectors to demonstrate target engagement of miR-12.

Bmi-1 expression has been observed to be up-regulated in a variety of human cancers including glioblastoma, and has been shown to be the target of miR-128. To confirm target engagement of miR-128, Bmi-1 expression from cells transduced with pAC3-miR-128-1, pAC3-miR-128-2 and pAC3-H1-shRNAmiR128 vector, respectively, was detected by qRT-PCR. Figure 14 shows that U87-MG cells transduced with pAC3-miR-128-1, pAC3-miR-128-2 and pAC3-H1-shRNAmiR128 vector, respectively, expressed lower level of Bmi-1 than untransduced cells, whereas in HT1080 cells no significant difference was observed between transduced and untransduced cells. The data support the concept that miR-128 plays an important functional role in the central nervous system.

### Example 12: Construction and testing of recombinant replication competent retroviral vector containing heterologous polynucleotide sequences of IRES, yCD2, human H1 promoter and human pre-miR128-1

### A.Construction

The replication competent retroviral vector, pAC3-yCD2-H1-shRNAmiR128 is derived from the backbone of pAC3-yCD2 described in one of the embodiments. The pAC3-yCD2 backbone in the pAC3-yCD2-H1-shRNAmiR128 vector is isolated by endonuclease digestion of the pAC3-yCD2 plasmid DNA with Not I. The polynucleotide DNA sequence of the human H1 promoter is obtained from the product information of pSilencer 3.1 H1 hygro expression vector (Ambion), and the polynucleotide DNA sequence of the short hairpin structured pre-miR-128-1 is obtained from the http://www.mirbase.org/. DNA sequence of pre-miR128-1 linked to the human H1 promoter (SEQ ID NO: 34) is synthesized with a Not I restriction enzyme site at both ends of the double-stranded DNA fragment for subsequent insertion at the corresponding site in Not I digested pAC3-yCD2 plasmid DNA described above. The resulting construct, pAC3-H1-shRNAmiR128, encodes 4 genes: the gag, the pol, and the env, and the yCD2, and the non-coding short hairpin structured pre-miR-128-1 sequence (Figure 11).

Vector stock is produced by transient transfection of the vector-encoding plasmid DNA into 293T cells using calcium phosphate method. Eighteen hours post transfection, the culture is replaced with fresh medium. Twenty-four hours post medium replacement, the supernatant containing the vector is collected and filtered through a 0.45 m filter and used immediately or stored in aliquots at -80°C for later use. Twenty micro-liter of the collected vector stocks is used to infect human prostate cancer cells, PC3. Twenty-four hours post infection, AZT is added to the cells to inhibit further viral replication. Forty-eight hours post infection, genomic DNA of infected PC3 cells is extracted for titer assay. The titer of the vector stocks is determined by qPCR with an inclusion of standards of known copy numbers.

### B. Testing of replication kinetics of the pAC3-yCD2-H1-shRNAmiR128 recombinant replication competent retroviral vectors in culture

In order to confirm that the incorporation of H1-pre-miR-128-1 in the present invention replicates normally, calculated volume of each vector stocks collected from transient transfection mentioned above is used to infect fresh human fibrosarcoma cells, HT1080 and human glioma cells, U87-MG, respectively, at a MOI of 0.1. Transduced cells are passaged at day 3, 6 and 9 post infection. At each time point, a portion of cells are collected for genomic DNA extraction for qPCR. Dilutions of genomic DNA are made to generate aliquots of genomic DNA with same concentration for equal amount of genomic in-put in qPCR. Replication kinetics of each vectors are generated by plotting inversed C(t) values vs. time points. Result show that the vector replicates at similar kinetics compared to control MLV virus.

### C. Testing of expression of mature miR-128 from cells transduced with the pAC3-yCD2-H1-shRNAmiR128 recombinant replication competent retroviral vector

To confirm the expression of miR-128 from cells transduced with pAC3-yCD2-H1-shRNAmiR128 recombinant replication competent retroviral vector, cells from day 9 post infection, at which the maximal infectivity is reached, are expanded and harvested to extract total RNA for detection of mature miRNA expression. Result from Taqman microRNA assay shows an over expression of mature miR-128 from both HT1080 and U87-MG cells transduced with the pAC3-yCD2-H1-shRNAmiR128compared to untransduced cells.

### D. Testing of Bmi-1 expression from cells transduced with pAC3-yCD2-H1-shRNAmiR128 recombinant replication competent retroviral vectors to demonstrate target engagement of miR-128

Bmi-1 expression has been observed to be up-regulated in a variety of human cancers including glioblastoma, and has been shown to be the target of miR-128. To confirm target engagement of miR-128, Bmi-1 expression from cells transduced with pAC3-yCD2-H1-shRNAmiR128 is detected by qRT-PCR. The result shows that U87-MG cells transduced with pAC3-yCD2-H1-shRNAmiR128 express lower level of Bmi-1 than untransduced cells, whereas in HT1080 cells no significant difference was observed between transduced and untransduced cells. The data support the concept that miR-128 plays an important functional role in the central nervous system.

### E. Testing of yCD2 expression from cells transduced with pAC3-yCD2-H1-shRNAmiR128 by immune-blot

To confirm the expression of yCD2 from cells transduced with pAC3-yCD2-H1-shRNAmiR128 recombinant replication competent retroviral vector, cells from day 9 post infection, at which the maximal infectivity is reached, are expanded and harvested to extract total protein for detection of yCD2 expression. The result from immune-blot shows normal expression yCD2 from both HT1080 and U87-MG cells transduced with the pAC3-yCD2-H1-shRNAmiR128 compared to pAC3-yCD2 transduced cells.

### Example 13: Testing of vector stability of miR-128 containing recombinant retroviral vectors in culture.

Multiple serial infection cycles of miR-128 containing recombinant retroviral vectors (pAC3-miR-128-1, pAC3-miR-128-2, pAC3-H1-shRNAmiR128 and pAC3-yCD2-H1-shRNAmiR128) is tested to assess the stability of the vectors. HT1080 and U87-MG cells are initially infected with vectors at a low MOI and are allowed to spread in culture. Vector stocks at each infection cycles are collected, filtered diluted to infect fresh cells. At the end of each infection cycles, cells are harvested and genomic DNA are extracted for assessment of transgene stability by standard PCR using primers that bind to the 3'of the env gene and 3' of the untranlated region in the vector upstream of the 3'LTR. The result shows that all vectors tested remain stable for at least over 8-20 cycles.

### Example 14: Anti-tumor efficacy studies with miRNA expressing vector in a mouse/human xenograft model.

### Objective

The objective of this study is to assess the effect of a novel MLV based replication-competent retroviral vectors carrying the miR128 sequence (AC3-miR128-1(V); AC3-miR128-2(V); AC3-miR128-3(V) on survival, when delivered via intracranial (IC) injection in nude mice bearing a human glioma xenograft, at three Toca 511 dose levels.

### Mice

Female athymic nude-Foxn1^nu (nude) mice (age ∼8 weeks) are purchased from Harlan (Indianapolis IN). Mice are acclimated for 7 days after arrival. Mice undergo surgical placement of an indwelling guide cannula with a 3.0 mm projection implanted into the right striatum, and fitted with a cap containing a 3.5 mm projection. The stereotaxic coordinates are AP= +0.5 mm, ML= -1.8 mm (from bregma).

### Cells

U-87 MG cells (ATCC, Manassas VA) are derived from a malignant glioma from a 44 year old Caucasian female. Cells are cultured in Dulbecco's modified Eagles medium with 10% fetal bovine serum, sodium pyruvate, and Glutamax (Hyclone, Logan UT, and Invitrogen, San Diego CA). Cells are resuspended in PBS (Hyclone, Logan UT) for implantation. U-87 MG cells (1E5 in 1 µL) are infused at 0.2 µL per minute (5 minutes, followed by a hold of 5 minutes) IC through an injection cannula with a 3.5 mm projection inserted through the guide cannula.

Vectors preparations are made by transient transfection (or from a producer cell line refer to provisional) and all have titers of approximately 5E6TU/ml. For initial studies vector is not further purified or concentrated. For follow on experiments to determine full dose response curves, high titer purified material is prepared with a titer of around 10E8/ml. Vector is administered IC in a volume of 5ul or less for a minimum total dose/mouse of approximately 2.5E4 TU/mouse.

### Tumor implantation and vector injection

Six groups of female athymic nude-Foxn1^nu mice (65 mice, 9-10 weeks of age) are implanted IC with U-87 tumor cells (Day 0) then dosed IC (day 4-7 depending on growth rate of the U87 cells) with vehicle (Groups 1), with control vector (AC3-GFP(V), Group2) or IC with AC3-miR128-1(V); AC3-miR128-2(V); AC3-miR128-3(V) (Groups 3-5). Group 6 mice were not implanted with tumor or vector.

### Data analyses

Survival analysis to day 60 is performed on 10 mice each from Groups 1-6 and plotted as a Kaplan Meyer plot. Survival curves are compared by the log-rank test. P values of <0.05 are considered statistically significant in all analyses, which are performed with Prism 5 statistical software (GraphPad Software) or equivalent.

Results from treatment with the vectors show a statistically significant survival advantage in this human glioma xenograft model compared to treatment with control vector or vehicle alone.

### Example 15: Anti-tumor efficacy studies with yCD2 and miRNA expressing vector in a mouse/human xenograft model.

The objective of this study is to assess the effect of a novel MLV based replication-competent retroviral vector expressing yCD2 and miR-128, designated AC3-yCD2-H1-shRNAmiR128 (V) on survival when delivered via intracranial (IC) injection in nude mice bearing a "stem-cell" like enriched human glioma xenograft, at three dose levels.

Female athymic nude-Foxn1^nu (nude) mice (age ∼8 weeks) are purchased from Harlan (Indianapolis IN). Mice are acclimated for 7 days after arrival. Mice undergo surgical placement of an indwelling guide cannula with a 3.0 mm projection implanted into the right striatum, and fitted with a cap containing a 3.5 mm projection. The stereotaxic coordinates are AP= +0.5 mm, ML= -1.8 mm (from bregma).

Early passages of primary human glioma (Dr. Carol Kruse, Burnham Biomedical Res Inst, San Diego, CA) are cultured in serum-free medium with EGF, bFGF and B27 supplement. Cells are resuspended in PBS (Hyclone, Logan UT) for implantation. Approximately 1000 cells in 1 µL are infused at 0.2 µL per minute (5 minutes, followed by a hold of 5 minutes) IC through an injection cannula with a 3.5 mm projection inserted through the guide cannula.

Vectors preparations are made by transient transfection (or from a producer cell line refer to provisional) and all have titers of approximately 5E6TU/ml. For initial studies vector is not further purified or concentrated. For follow on experiments to determine full dose response curves, high titer purified material is prepared with a titer of around 10E8/ml. Vector is administered IC in a volume of 5ul or less for a minimum total dose/mouse of approximately 2.5E4 TU/mouse.

### Tumor implantation and vector injection

Six groups of female athymic nude-Foxn1^nu mice (66 mice, 9-10 weeks of age) are implanted IC with "stem-like" cells (Day 0) then dosed IC at day 7-14 post tumor implantation depending on growth rate of the cells with Groups 1: vehicle; Group2: control vector AC3-GFP(V); Group 3: TOCA511; Group 4: AC3-yH1-shRNAmiR128(V); Group 5: AC3-yCD2-H1-shRNAmiR128(V); and Group 6: untreated mice that are not implanted with tumor or vector.

### Data analyses

Survival analysis to day 60 is performed on 10 mice each from Groups 1-5 and plotted as a Kaplan Meyer plot. Survival curves are compared by the log-rank test. P values of <0.05 are considered statistically significant in all analyses, which are performed with Prism 5 statistical software (GraphPad Software) or equivalent.

### Results

Results from treatment with the vectors show a statistically significant survival advantage in this human glioma xenograft model compared to treatment with control vector or vehicle alone.

### Example 16: Construction of vectors with miR target sequences

### A.Construction of replication competent retroviral vector expressing GFP and containing a single copy of 142-3p target sequence

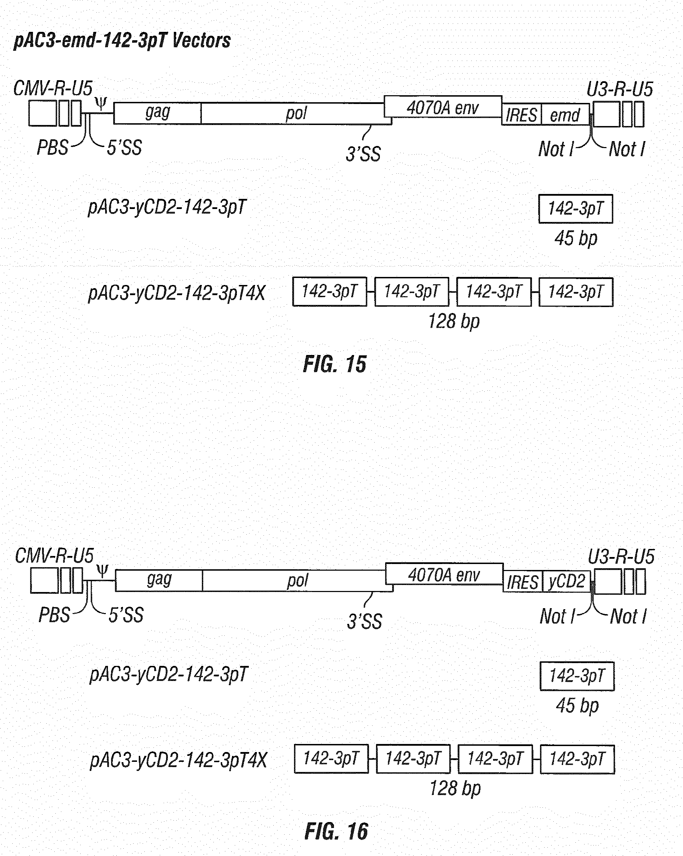

The replication competent retroviral vector, pAC3-emd-142-3pT, encoding a GFP was derived from the backbone of pAC3-emd described above (current existing patent). The pAC3-emd backbone in the pAC3-emd-142-3pT vector was isolated by endonuclease digestion of the pAC3-emd plasmid DNA with Not I. The perfect complementary target sequence of miR142-3pT was obtained from published literature (Brown et al., 2006 Nature Medicine 12:5 585-591). The target sequence of the miR-142-3p (SEQ ID NO: 35) was synthesized with a Not I restriction enzyme site present at each end of the double-stranded DNA fragment for subsequent insertion at the corresponding site in the pAC3-emd plasmid DNA. The orientation of the 142-3pT insert was confirmed by sequencing analysis. The resulting construct, pAC3-emd-142-3pT, encodes 4 genes: the gag, the pol, the env, and the emd, and the non-coding 142-3pT sequence (Figure 15).

Vector stock was produced by transient transfetion of the vector-encoding plasmid DNA into 293T cells using calcium phosphate method. Eighteen hours post transfection, the culture was replaced with fresh medium. Twenty-four hours post medium replacement, the supernatant containing the vector was collected and filtered through a 0.45 m filter and used immediately or stored in aliquots at -80°C for later use. Twenty micro-liter of the collected vector stocks was used to infect human prostate cancer cells, PC3. Twenty-four hours post infection, AZT was added to the cells to inhibit further viral replication. Forty-eight hours post infection, genomic DNA of infected PC3 cells was extracted for titer assay. The titer of the vector stocks was determined by qPCR with an inclusion of standards of known copy numbers.

### B. Construction of replication competent retroviral vector expressing yCD2 and containing a single copy of 142-3p target sequence

The replication competent retroviral vector, pAC3-yCD2-142-3pT, encoding a yCD2 gene was derived from the backbone of pAC3-yCD2 described above (current existing patent). The pAC3-yCD2 backbone in the pAC3-yCD2-142-3pT vector was isolated by endonuclease digestion of the pAC3-yCD2 plasmid DNA with Not I. The perfect complementary target sequence of miR142-3pT was obtained from published literature (Brown et al., 2006 Nature Medicine 12:5 585-591). The target sequence of the miR-142-3p (SEQ ID NO: 35) was synthesized with Not I restriction enzyme site present at each end of the double-stranded DNA fragment for subsequent insertion at the corresponding site in the pAC3-yCD2 plasmid DNA. The resulting construct, pAC3-yCD2-142-3pT, encodes 4 genes: the gag, the pol, the env, and the yCD2, and the non-coding 142-3pT sequence (Figure 16).

Vector stock was produced by transient transfetion of the vector-encoding plasmid DNA into 293T cells using calcium phosphate method. Eighteen hours post transfection, the culture was replaced with fresh medium. Twenty-four hours post medium replacement, the supernatant containing the vector was collected and filtered through a 0.45 m filter and used immediately or stored in aliquots at -80°C for later use. Twenty micro-liter of the collected vector stocks was used to infect human prostate cancer cells, PC3. Twenty-four hours post infection, AZT was added to the cells to inhibit further viral replication. Forty-eight hours post infection, genomic DNA of infected PC3 cells was extracted for titer assay. The titer of the vector stocks was determined by qPCR with an inclusion of standards of known copy numbers.

### C. Construction of replication competent retroviral vector expressing GFP and containing 4 copies of 142-3p target sequence

The replication competent retroviral vector, pAC3-emd-142-3pT4X, encoding a GFP was derived from the backbone of pAC3-emd described above (current existing patent). The pAC3-emd backbone in the pAC3-emd-142-3pT 4X vector was isolated by endonuclease digestion of the pAC3-emd plasmid DNA with Not I. Four tandem repeat of the perfect complementary target sequence of miR142-3pT4X was obtained from published literature (Brown et al., 2006 Nature Medicine 12:5 585-591). The target sequence of the miR-142-3p4X (SEQ ID NO: 36) was synthesized with a Not I restriction enzyme site present at each end of the double-stranded DNA fragment for subsequent insertion at the corresponding site in the pAC3-emd plasmid DNA. The orientation of the 142-3pT insert was confirmed by sequencing analysis. The resulting construct, pAC3-emd-142-3pT4X, encodes 4 genes: the gag, the pol, the env, and the emd, and the non-coding 142-3pT4X sequence (Figure 15).

Vector stock was produced by transient transfetion of the vector-encoding plasmid DNA into 293T cells using calcium phosphate method. Eighteen hours post transfection, the culture was replaced with fresh medium. Twenty-four hours post medium replacement, the supernatant containing the vector was collected and filtered through a 0.45 m filter and used immediately or stored in aliquots at -80°C for later use. Twenty micro-liter of the collected vector stocks was used to infect human prostate cancer cells, PC3. Twenty-four hours post infection, AZT was added to the cells to inhibit further viral replication. Forty-eight hours post infection, genomic DNA of infected PC3 cells was extracted for titer assay. The titer of the vector stocks was determined by qPCR with an inclusion of standards of known copy numbers.

### D.Construction of replication competent retroviral vector expressing yCD2 and containing 4 copies of 142-3p target sequence

The replication competent retroviral vector, pAC3-yCD2-142-3pT4X, encoding a GFP was derived from the backbone of pAC3-emd described above. The pAC3-emd backbone in the pAC3-yCD2-142-3pT 4Xvector was isolated by endonuclease digestion of the pAC3-yCD2 plasmid DNA with Not I. Four tandem repeat of the perfect complementary target sequence of miR142-3pT4X was obtained from published literature (Brown et al., 2006 Nature Medicine 12:5 585-591). The target sequence of the miR-142-3pT4X (SEQ ID NO: 36) was synthesized with a Not I restriction enzyme site present at each end of the double-stranded DNA fragment for subsequent insertion at the corresponding site in the pAC3-yCD2 plasmid DNA. The orientation of the 142-3pT4X insert was confirmed by sequencing analysis. The resulting construct, pAC3-emd-142-3pT4X, encodes 4 genes: the gag, the pol, the env, and the emd , and the non-coding 142-3pT4X sequence (Figure 16).

Vector stock was produced by transient transfection of the vector-encoding plasmid DNA into 293T cells using calcium phosphate method. Eighteen hours post transfection, the culture was replaced with fresh medium. Twenty-four hours post medium replacement, the supernatant containing the vector was collected and filtered through a 0.45 m filter and used immediately or stored in aliquots at -80°C for later use. Twenty micro-liter of the collected vector stocks was used to infect human prostate cancer cells, PC3. Twenty-four hours post infection, AZT was added to the cells to inhibit further viral replication. Forty-eight hours post infection, genomic DNA of infected PC3 cells was extracted for titer assay. The titer of the vector stocks was determined by qPCR with an inclusion of standards of known copy numbers.

### Example 17: Graphing of replication kinetics of recombinant retroviral vector by qPCR assay

Currently, there are at least two common to obtain replication kinetics of recombinant replication competent retroviral vector: (1) analysis of GFP expression with vectors expressing the GFP protein by flow cytometric analysis, and (2) reversed transcriptase assay by measuring the reverase transcriptase activity of the vector stock collected from cultured medium of transduced cells. The concept that titers assessed by DNA analysis of transduced cells provide the most reliable estimate of the functional titers as compared to titers obtained by RNA and transgene expression in which titers were over-estimated and under-estimated, respectively (Sastry et al., 2002 Gene Therapy 9, 1155-1162). The replication kinetics of viral spread correlates with the percent of a cell population being transduced in which an integrated proviral DNA of the viral genome can be detected by qPCR with primers specific to the viral sequence is being tested in culture. The present assay requires equal amount of gDNA in-put from all time points within the same vector tested and among various vectors if a comparison of replication kinetics is being tested. The replication kinetics graph was generated by plotting inversed C(t) values vs. time points. Figure 17A and Figure 17A show comparisons of replication kinetics of various recombinant replication competent retroviral vectors. The assay is sensitive to reveal the small difference in replication kinetics among various vectors.

### Example 18: Testing of replication kinetics

### 142-3pT containing recombinant retroviral vectors in non-hematopoietic human cell lines

In order to confirm that the incorporation of the 142-3pT sequence in the present invention replicates similar to their parental vectors, calculated volume of each vector stocks collected from transient transfection mentioned above was used to infect fresh human firbrosarcoma cells, HT1080 and human glioma cells, U87-MG, respectively, at a MOI of 0.1. Transduced cells were passaged at day 3, 6 and 9 post infection. At each time point, a portion of cells were collected for genomic DNA extraction for qPCR. Dilutions of genomic DNA were made to generate aliquots of genomic DNA with same concentration for equal amount of genomic input in qPCR.

Replication kinetics of each vectors were generated by plotting inversed C(t) values vs. time points. Figure 17A and 18A show that all vectors tested replicated at similar kinetics compared to their parental vectors (pAC3-emd and pAC3-yCD2). Replication kinetics of vectors expressing GFP protein (pAC3-emd, pAC3-emd-142-3pT and pAC3-emd-142-3pT4X) were also assessed by flow cytometric analysis. Figure 3B and 4B show that pAC3-emd-142-3pT and pAC3-emd-142-3pT4X vectors replicated at a similar kinetic as their parental vector, pAC3-emd.

### A. Testing of replication kinetics of 142-3pT containing recombinant retroviral vectors in human and mouse hematopoietic cells

The expression of mature miR-142-3p was first confirmed in a mouse T-lymphocytic cell line EL4, a human T-lymphocytic cell line SUP-T1 and a human monocytic cell line U937 by Taqman microRNA assay using the primer set specific for mouse and human miR-142-3p as the sequences of mature miR-142-3p of the two species are identical. The replication kinetics of recombinant retroviral vector expressing the GFP (pAC3-emd) was tested in all three cell lines with an initial infection at a MOI of 2. Figures 19 shows that the pAC3-emd vector replicated efficiently in human T-lymphocytes and monocytes (SUP-T1 and U937) as the viral spread reached 65% and 95% of cell population, respectively, by day 28 post infection. Vector spread in EL4 cells remained less than 5% during the time frame tested.

### Example 18: Testing of vector spread of 142-3pT containing recombinant retroviral vectors in human and mouse hematopoietic cells.

The functional effect of miR-142-3p in suppressing the GFP expression in the recombinant retroviral vector expressing GFP was tested by flow cytometric analysis. Calculated volume of pAC3-emd, pAC3-emd-142-3pT and pAC3-emd-142-3pT4X vector stock collected from transient transfection mentioned above was used to infect fresh EL4, SUP-T1 and U937 cells at a MOI of 2. A portion of cells were collected at day 6, 12 and 18 post infection for analysis of GFP expression by flow cytometric analysis. Figure 20A shows the GFP expression was suppressed to background level in EL4 cells transduced with pAC3-emd-142-3pT and pAC3-emd-142-3pT4X vector compared to their parental vector pAC3-emd. The GFP suppression remained persistent within the time frame tested. Figure 20B and Figure 20C show remarkable suppression of GFP expression in SUP-T1 and U937 cells transduced with pAC3-emd-142-3pT and pAC3-emd-142-3pT4X vector, respectively, compared to their parental vector pAC3-emd. The results confirm that the miR-142-3p expression in mouse and human hematopoietic cells effectively suppress the GFP expression in cells transduced with recombinant retroviral vectors containing the 142-3pT sequence. In U937 cells, the result suggested that the vector containing 4 copies of 142-3pT (pAC3-emd-142-3pT4X) may be more effective in suppressing GFP expression than the vector containing single copy of 142-3pT (pAC3-emd-142-3pT4X).

### Example 19: Testing of viral RNA genome

It is unclear whether the functional effect of miR-142-3pT in suppressing the GFP expression mentioned above is due direct suppression of GFP expression at the translational level or due to degradation of viral genome at post transcriptional level. A portion of cells at the end of the experimental time point is collected for total RNA extraction using standard molecular biology method. qRT-PCR using primers (e.g. pol primer set and env2 primer set) specific to cDNA derived from reverse transcribed viral RNAd is performed to assess viral load of transduced cells. The result shows that the viral load of cells transduced with pAC3-emd -142-3pT and pAC3-emd -142-3pT4X, respectively, is significantly lower than cells transduced with pAC3-emd vector. The result supports the concept that miR-142-3p in mouse and human hematopoietic cells effectively degrades the viral RNA genome at post transcriptional level, thereby, restricts the vector spread in mouse and human hematopoietic cells.

### Example 20: Testing of integrated proviral DNA by qPCR.

It is unclear whether the functional effect of miR-142-3pT in suppressing the GFP expression mentioned above is due direct suppression of GFP expression at the translational level or due to degradation of viral genome and thus integration of proviral DNA. A portion of cells at the end of the experimental time point is collected for genomic DNA extraction using standard molecular biology methods. qPCR using primers specific to integrated proviral DNA is performed to assess the copy number of integrated proviral DNA per cell. The result shows that the copy number per cells of cells transduced with pAC3-emd -142-3pT and pAC3-emd -142-3pT4X, respectively, is significantly lower than cells transduced with pAC3-emd vector. The result supports the concept that miR-142-3p in mouse and human hematopoietic cells effectively degrades the viral RNA genome at post transcriptional level, and thereby restricts the vector spread in mouse and human hematopoietic cells.

### Example 21: Testing of vector stability of 142-3pT containing recombinant retroviral vectors in culture.

Multiple serial infection cycles of 142-3pT containing recombinant retroviral vectors is tested to assess the stability of the vectors. HT1080 and U87-MG cells are initially infected with vectors at a low MOI and are allowed to spread in culture. Vector stocks at each infection cycles are collected, filtered diluted to infect fresh cells. At the end of each infection cycles, cells are harvested and genomic DNA are extracted for assessment of transgene stability by standard PCR using primers that bind to the 3'of the env gene and 3' of the untranlated region in the vector downstream of the heterologous polynucleotide sequence linked to the IRES. The result shows that vectors containing single copy of 142-3pT (pAC3-emd-142-3pT and pAC3-yCD2-142-3pT) remains stable for at least over 10-20 cycles, whereas vectors containing 4 tandem repeats of 142-3pT (pAC3-emd-142-3pT4X and pAC3-yCD2-142-3pT4X) show deletion of 142-3pT sequence in early infection cycles.

### Example 22: Testing of controlled vector spread of 142-3pT containing recombinant retroviral vectors in in vivo.

This experiment is conducted with the samne design as example 27 below. The functional effect of miR-142-3p in restricting vector spread via hematopoietic cells is tested *in vivo* by intravenous injection of the recombinant retroviral vectors (pAC3-emd, pAC3-emd-142-3pT and pAC3-emd-142-3pT4X) in 8-wk-old nude Balb/C mice with implanted U87 xenografts. For each vector, there are three groups of mice each represents a time point (e.g. 30 day, 60 day and 90 day post viral vector administration). A dose of 1E4 to 1E7 TU of each vector stock is administered by intravenous injection to all animals. Animals from each time point are sacrificed to harvest spleen, lymph nodes and bone marrow and tumor. GFP expression of subpopulation of cells (e.g. CD4+, CD8+ and etc.) are harvested and analyzed by flow cytometric analysis. In a duplicate experiment, animals from each time point are sacrificed and tissues (e.g. liver, kidney, spleen, tumor etc.) are collected for genomic DNA extraction. qPCR is performed to assess the presence of integrated proviral DNA in tissues collected. The result shows vector spread of vectors containing the 142-3pT is significantly reduced in hematopoietic tissues demonstrating the reduction of vector replication in these tisues. At the same time GFP and PCR signal is still observed in the tumor, showing that the miR target sequences have depressed spread in the lymphoid tissues, but still aloowed spread in the tumor tissue.

### Example 23. Extended Survival in a patient dog with spontaneous recurrent malignant glioma and treated with T5.0002 vector plus 5-FC

A male 35kg Boxer dog, presenting with recurrent anaplastic oligodenroglioma 3 months following complete surgical resection, was treated with T5.0002 virus, purified and formulated (see US 5792643; T. Rodriugez et al. J Gene Med 9:233 2007; US Application 61218063) in isotonic Tris/NaCl pH7.2 rendered isotonic with mannitol & sucrose, 1mg/ml HSA, 0.1mg/ml ascorbate) in combination with 5-FC. The tumor measured approximately 13 cm³, and caused major lateral ventricle compression and significant midline shift (See Figure 1) Due to the large size of the tumor, Toca 511 was infused through 2 separate catheters (400 µL and 480 pL), using Convection Enhanced Delivery (CED). The total Toca 511 dose administered was approximately 4.1 x10⁶ TU/g brain. ProHance® (gadoteridol) was added to Toca 511 prior to injection to allow visualization of delivery by MRI. The volume of distribution of the vector was estimated to be approximately 10-12% of the tumor volume.

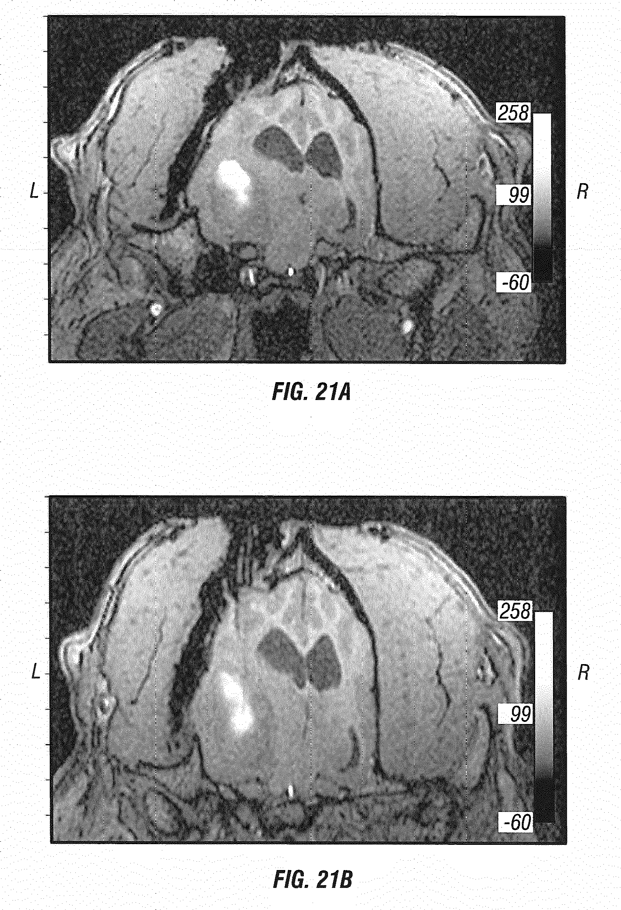

Figures 21A and 21B are still frames from the MRI images obtained from the patient dog during intratumoral CED infusion of Toca 511 and gadolinium. Note the large tumor on the left side of the image compressing both sides of the brain and shifting midline structures to the right. The white areas are the gadolinium-Toca 511 infusion. Figure 21B shows the placement of the two catheters into the tumor

Toca 511 was allowed to spread for 8 days. The dog was treated with 5-FC at a divided dose of 130 mg/kg/day, by mouth, three times daily with food for 5 days. The dose was increased to 160 mg/kg /day for 2 more days (7 days of 5-FC total). A follow-up MRI showed no change in tumor size and some possible changes to the internal area of the tumor. After 21 days of viral spread, a second cycle of 5-FC was initiated at the higher dose of 160 mg/kg/day (divided, three times a day with food). The drug was stopped after the fifth day of dosing due to the development of rash.

MRI performed at 2 weeks after the first course of 5-FC and 2 weeks after the second course of 5-FC (7 weeks after treatment began) has shown that the tumor volume has plateaued while the rate of tumor growth has declined. The patient became more alert and active, and remained clinically stable, 13 weeks after injection of vector. As of writing the dog remains alive. The estimated lifespan of the dog was no more than 3-4 weeks at the time of initial injection of the vector. Efficacy of the Toca 511/5-FC combination in this patient dog is shown by survival 3 to 4 times longer than that originally estimated by his attending veterinarian.

### Example 24 Construction of gamma intererferon vectors

### A. Construction and testing of a replication competent retroviral vector encoding the human IFN-gamma gene

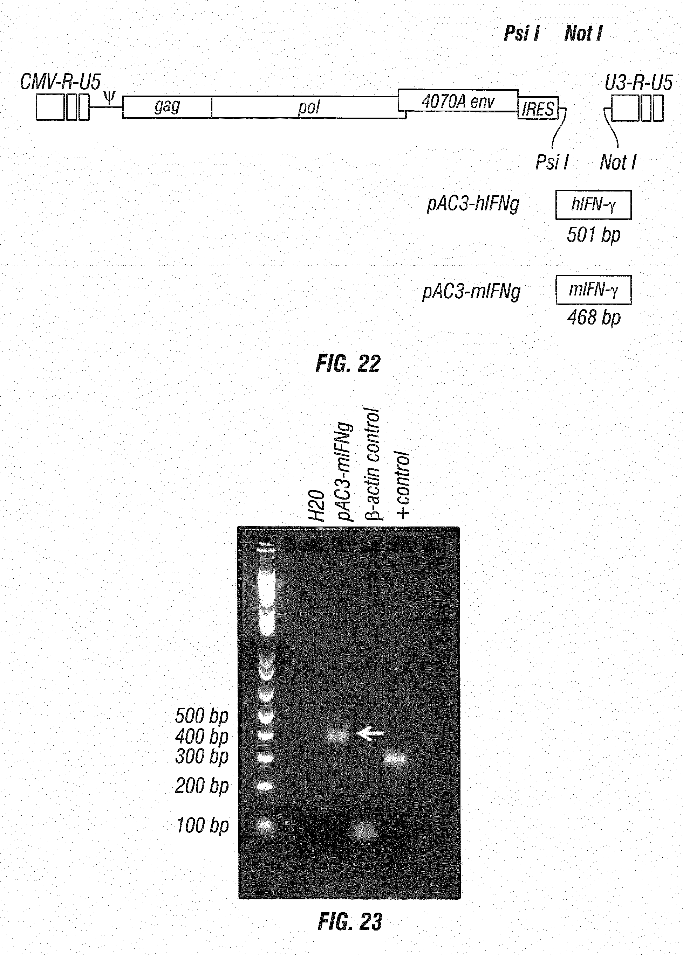

The replication competent retroviral vector, pAC3-hIFNg, encoding the human IFN-gamma gene, was derived from the backbone of pAC3-yCD2 described above. The pAC3 backbone in the pAC3-hIFNg vector was isolated by endonuclease digestion of the pAC3-yCD2 plasmid DNA with Psi I and Not I. The cDNA sequence of human IFN-gamma gene was identified and confirmed among three sequences obtained from different accession numbers (AM903379, BC070256, and NM000619). Sequence alignment showed identical sequence among the three. The open reading frame of the human IFN-gamma (SEQ ID NO: 38) was synthesized with Psi I and Not I restriction enzyme site present at each end of the DNA fragment for subsequent insertion at the corresponding site in the pAC3 backbone. The resulting construct, pAC3-hIFNg, encodes 4 genes: the gag, the pol, the env, and the human IFN-gamma. (Figure 22).

Vector stock was produced by transient transfetion of the vector-encoding plasmid DNA into HT1080 cells using FUGENE HD transfection Reagent. Forty-eight hours post transfection, the supernatant containing the vector was collected and filtered through a 0.45 m filter and used immediately or stored in aliquots at -80°C for later use. Specific volume of the undiluted vector stock was used to infect fresh 75% confluent HT1080. At day 4 and day 5 post infection, the supernatant containing the vector was collected and filtered through a 0.45 m filter and used immediately or stored in aliquots at -80°C for later use. Twenty micro-liter of the collected vector stocks was used to infect human prostate cancer cells, PC3. Twenty-four hours post infection, AZT was added to the cells to inhibit further viral replication. Forty-eight hours post infection, genomic DNA of infected PC3 cells was extracted for titer assay. The titer of the vector stocks was determined by qPCR with an inclusion of standards of known copy numbers.

The expression of human IFN-gamma was first tested at the RNA level. Total RNA was extracted from transduced HT1080 cells at 5 days post infection in the second infection using standard RNA extraction method. RT-PCR was performed to detect the expression of human IFN-. Fifty nano-gram of total RNA was used in the RT reaction to generate cDNA. One tenth of the volume from RT reaction was subsequently used for PCR using PCR primer set specific for human IFN-. Result from RT-PCR showed that human IFN-gamma is expressed in HT1080 cells infected with pAC3-hIFNg vector.

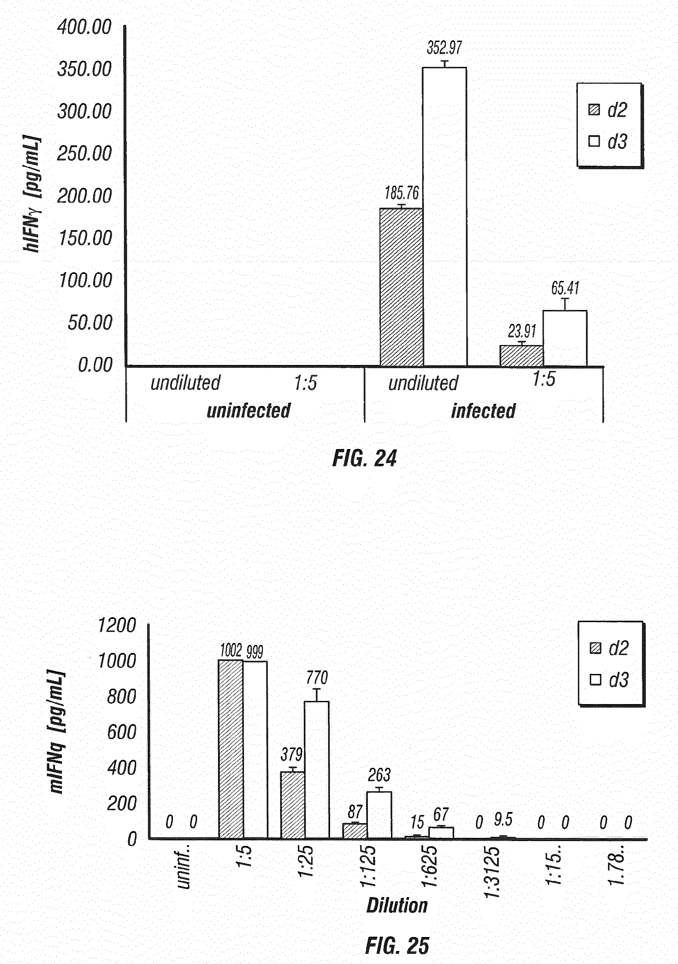

The expression of secreted human IFN- protein was tested by standard ELISA. Vector stock collected from day 4 and day 5 post infection was serially diluted in the ELISA assay in order to obtain a linear range between protein concentration and dilution factor. The result showed that human IFN- protein is secreted at a higher concentration by the HT1080 cells at day 5 post infection than by the cells at day 4 post infection (Fig 24). Cells at post d5 infection secreted approximately 325-355 pg/mL human IFN-gamma protein.

### B. B. Construction and testing of a replication competent retroviral vector encoding the Mouse IFN-gamma gene

The replication competent retroviral vector, pAC3-mIFNg, encoding the mouse IFN- gene was derived from the backbone of pAC3-yCD2 described above. The pAC3 backbone in the pAC3-mIFNg vector was isolated by endonuclease digestion of the pAC3-yCD2 plasmid DNA with Psi I and Not I. The cDNA sequence of mouse IFN-gamma gene was identified and confirmed among three sequences obtained from different accession numbers (BC119063, BC119065 and NM008337). Sequence alignment showed identical sequence among the three. The open reading frame of the mouse IFN-gamma (SEQ ID NO: 39) was synthesized with Psi I and Not I restriction enzyme site present at each end of the DNA fragment for insertion at the corresponding site in the pAC3 backbone. The resulting construct, pAC3-mIFNg,encodes 4 genes: the gag, the pol, the env, and the mouse IFN-gamma. (Figure 22).

Vector stock was produced by transient transfetion of the vector-encoding plasmid DNA into HT1080 cells using FUGENE HD transfection Reagent. Forty-eight hours post transfection, the supernatant containing the vector was collected and filtered through a 0.45 micron filter and used immediately or stored in aliquots at -80°C for later use. Specific volume of the undiluted vector stock was used to infect fresh 75% confluent HT1080. At day 4 and day 5 post infection, the supernatant containing the vector was collected and filtered through a 0.45 micron filter and used immediately or stored in aliquots at -80°C for later use. Twenty micro-liter of the collected vector stocks was used to infect human prostate cancer cells, PC3. Twenty-four hours post infection, AZT was added to the cells to inhibit further viral replication. Forty-eight hours post infection, genomic DNA of infected PC3 cells was extracted for titer assay. The titer of the vector stocks was determined by qPCR with an inclusion of standards of known copy numbers.

The expression of mouse IFN-gamma was first tested at the RNA level. Total RNA was extracted from transduced HT1080 cells at 5 days post infection in the second infection using standard RNA extraction method. RT-PCR was performed to detect the expression of mouse IFN-gamma. Fifty nano-gram of total RNA was used in the RT reaction to generate cDNA. One tenth of the volume from RT reaction was subsequently used for PCR using PCR primer set specific for mouse IFN-gamma (Fig 23). Result from RT-PCR showed that mouse IFN-gamma is expressed in HT1080 cells infected with pAC3-mIFNg vector.

The expression of secreted human IFN-gamma protein was tested by standard ELISA. Vector stock collected from day 4 and day 5 post infection was serially diluted in the ELISA assay in order to obtain a linear range between protein concentration and dilution factor. The result showed that mouse IFN- protein is secreted at a higher concentration by the HT1080 cells at day 5 post infection than by the cells at day 4 post infection (Fig 25). Cells at post d5 infection secreted approximately 33- 42 ng/mL mouse IFN-gama protein.

### Example 25 Example D: Anti-tumor efficacy studies with gamma interferon expressing vector in a mouse subcutaneous tumor model.

### Objective

The objective of this study is to assess the effect of a novel MLV based replication-competent retroviral vector carrying the murine gamma interferon sequence (pAC3-mIFNg) on tumor growth, when delivered via intratumoral (IT) injection in BALB/c mice bearing subcutaneous colon carcinoma (CT26.WT).

### Mice

Female BALB/c mice (age ∼8 weeks) are purchased from Jackson Laboratories (Bar Harbor, ME). Mice will be acclimated for 7 days after arrival before start of studies.

### Cells

CT26.WT cells (ATCC, Manassas VA) are an N-nitroso-N-methylurethane-(NNMU) induced, undifferentiated colon carcinoma cell line. Cells are cultured in Dulbecco's modified Eagles medium with 10% fetal bovine serum, sodium pyruvate, and Glutamax (Hyclone, Logan UT, and Invitrogen, San Diego CA). Cells are resuspended in PBS (Hyclone, Logan UT) for implantation. CT26.WT cells (2E5 in 100 pL) are injected into the right flank of BALB/c mice.

### Vector

Vectors preparations are made by transient transfection (or from a producer cell line after infection of a second cell line with the infectious virus from the initial transfection; US app 61218063) with titers of approximately 3E6TU/ml. For initial studies vector is not further purified or concentrated. For follow on experiments to determine full dose response curves, high titer purified material is prepared with a titer expected around 10E8/ml. To achieve high titer material, canine cell line CF2 are chosen for production as gamma interferon is poorly cross-species reactive and use of xenogeniec cell lines will prevent the inhibitory action of gamma interferon on the producing cells. The vector is purified and concentrated as described in the specification. Vector is administered IT in a volume of 100 µL and the total dose/mouse of approximately 3E3, 3E4 and 3E5 TU/mouse. Vector expressing gamma interferon is identified as Toca 621.

### Tumor implantation and vector injection

Nine groups of female BALB/c (99 mice, 9-10 weeks of age) are implanted subcutaneously with CT26.WT tumor cells (Day 0) and then dosed (day 4-7 depending on growth rate of the CT26 tumor; approximately 50-100mm³) with vehicle (Groups 1), with control vector [AC3-GFP(V), (Group2), IT Toca 621 vector injection (Groups 3-5), or intravenous Toca 621 vector injection (group 6-8). Group 9 mice have no tumor implanted and are intravenously injected with vector only.

### Data analyses

Tumor growth analysis is carried out to 2000mm³ or to 60 days based on which ever comes first. 10 mice from each group will be plotted for tumor size over time. Statistical significance are determined using analysis of variance (ANOVA). P values of <0.05 are considered statistically significant in all analyses, which are performed with Prism 5 statistical software (GraphPad Software) or equivalent. In-life observations are also taken to assess any adverse events to Toca 621 administration.

### Results

The results of measurement of tumor size over time show a statistically significant difference in the growth of tumors treated with the vector expressing gamma IFN over the tumors in animals that received control vector or vehicle

### Example 26 Example D: Anti-tumor efficacy studies with gamma interferon expressing vector in a mouse subcutaneous tumor model.

### Objective

The objective of this study is to assess the effect of a novel MLV based replication-competent retroviral vector carrying the murine gamma interferon sequence (pAC3-mIFNg) on tumor growth, when delivered via intratumoral (IT) injection in BALB/c mice bearing subcutaneous colon carcinoma (CT26.WT).

### Mice

Female BALB/c mice (age ∼8 weeks) are purchased from Jackson Laboratories (Bar Harbor, ME). Mice will be acclimated for 7 days after arrival before start of studies.

### Cells

CT26.WT cells (ATCC, Manassas VA) are an N-nitroso-N-methylurethane-(NNMU) induced, undifferentiated colon carcinoma cell line. Cells are cultured in Dulbecco's modified Eagles medium with 10% fetal bovine serum, sodium pyruvate, and Glutamax (Hyclone, Logan UT, and Invitrogen, San Diego CA). Cells are resuspended in PBS (Hyclone, Logan UT) for implantation. CT26.WT cells (2E5 in 100 pL) are injected into the right flank of BALB/c mice.

### Vector

Vectors preparations are made by transient transfection (or from a producer cell line after infection of a second cell line with the infectious virus from the initial transfection; US app 61218063) with titers of approximately 3E6TU/ml. For initial studies vector is not further purified or concentrated. For follow on experiments to determine full dose response curves, high titer purified material is prepared with a titer expected around 10E8/ml. To achieve high titer material, canine cell line CF2 are chosen for production as gamma interferon is poorly cross-species reactive and use of xenogeniec cell lines will prevent the inhibitory action of gamma interferon on the producing cells. The vector is purified and concentrated as described in the specification. Vector is administered IT in a volume of 100 µL and the total dose/mouse of approximately 3E3, 3E4 and 3E5 TU/mouse. Vector expressing gamma interferon is identified as Toca 621.

### Tumor implantation and vector injection

Nine groups of female BALB/c (99 mice, 9-10 weeks of age) are implanted subcutaneously with CT26.WT tumor cells (Day 0) and then dosed (day 4-7 depending on growth rate of the CT26 tumor; approximately 50-100mm³) with vehicle (Groups 1), with control vector [AC3-GFP(V), (Group2), IT Toca 621 vector injection (Groups 3-5), or intravenous Toca 621 vector injection (group 6-8). Group 9 mice have no tumor implanted and are intravenously injected with vector only.

### Data analyses

Tumor growth analysis is carried out to 2000mm³ or to 60 days based on which ever comes first. 10 mice from each group will be plotted for tumor size over time. Statistical significance will be determined using analysis of variance (ANOVA). P values of <0.05 are considered statistically significant in all analyses, which are performed with Prism 5 statistical software (GraphPad Software) or equivalent. In-life observations are also taken to assess any adverse events to Toca 621 administration.

### Results

The results of measurement of tumor size over time show a statistically significant difference in the growth of tumors treated with the vector expressing gamma IFN over the tumors in animals that received control vector or vehicle

### Example 27 Intravenous gene delivery using a replicative retroviral vector.

### Objective

The objective of this study was to assess the effectiveness of intravenous delivery of a novel MLV based replication-competent retroviral vector carrying the marker green fluorescent protein (AC3-GFP(V)) to U87 gliomas implanted in the brains of nude mice.

### Mice

Female athymic nude-Foxn1^nu (nude) mice (age ∼8 weeks) were purchased from Harlan (Indianapolis IN). Mice were acclimated for 7 days after arrival. Mice underwent surgical placement of an indwelling guide cannula with a 3.0 mm projection implanted into the right striatum, and fitted with a cap containing a 3.5 mm projection. The stereotaxic coordinates are AP= +0.5 mm, ML= -1.8 mm (from bregma).

### Cells

U-87 MG cells (ATCC, Manassas VA) are derived from a malignant glioma from a 44 year old Caucasian female. Cells were cultured in Dulbecco's modified Eagles medium with 10% fetal bovine serum, sodium pyruvate, and Glutamax (Hyclone, Logan UT, and Invitrogen, San Diego CA). Cells are resuspended in PBS (Hyclone, Logan UT) for implantation. U-87 MG cells (1E5 in 1 pL) were infused at 0.2 µL per minute (5 minutes, followed by a hold of 5 minutes) intracranially (IC) through an injection cannula with a 3.5 mm projection inserted through the guide cannula.

### Vector

Vector preparations were made by transient transfection and all had a titer of approximately 2.8E7TU/ml. Vector was administered intratumorally (IT) in a volume of 5ul or less for a minimum total dose/mouse of approximately 1.4E45 TU/mouse. Intravenous injections were done through the tail vein with 2.8E6/100uL.

### Tumor implantation and vector injection

Five groups of female athymic nude-Foxn1^nu mice (16 mice, 9-10 weeks of age) were implanted IC with U-87 tumor cells (Day 0) then dosed IT or IV (day 4-7 depending on growth rate of the U87 cells) with vehicle IV (Group 1), with vector IV (Group2), IT with a blood/brain barrier disruptor Vardenafil and vector (Group 3), IT with Vardenafil and vector (Groups 4), or IT with vector (group 5). 14 days after vector injection mice were sacrificed and tumors are isolated and analyzed for GFP expression.

### Data analyses

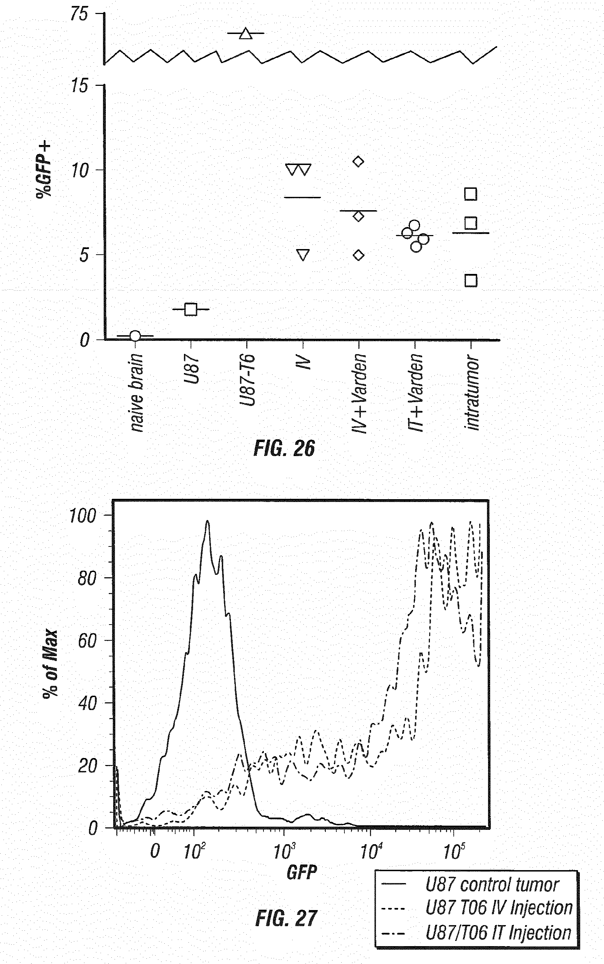

U87 cells from disrupted tumors isolated from the mice were analyzed by flow cytometry for the percentage GFP positive from groups 2-5 (figure 26). Histogram analysis was also done on groups 1,3, and 5 to measure the distribution of GFP signal in isolated U87 cells (figure 27).

### Results

Intravenous delivery of GFP was as equally effective as intratumor injection of U87 glioma cells intracranially implanted into nude mice.

### Example 28 Construction of replication competent retroviral vector encoding the human IL-2 gene

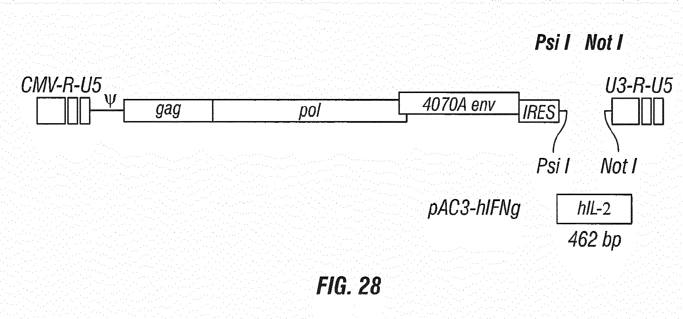

The replication competent retroviral vector, pAC3-hIL2, encoding the human IL2 gene, is derived from the backbone of pAC3-yCD2 vector. The pAC3 backbone in the pAC3-hIL2 vector-enconding plasmid DNA was isolated by endonuclease digestion of the pAC3-yCD2 plasmid DNA with Psi I and Not I. The cDNA sequence of human IFN-γ gene was identified and confirmed using sequences obtained from different accession numbers (BC066255 and BC066257). Sequence alignment of the two revealed identical sequence. The open reading frame of the human IFN-γ (SEQ ID NO: 40) was synthesized with Psi I and Not I restriction enzyme site present at each end of the DNA fragment for subsequent insertion at the corresponding site in the pAC3 backbone. The resulting construct, pAC3-hIL2, encodes 4 genes: the gag, the pol, the env, and the human IL2. (Figure 28).

Vector stock is produced by transient transfetion of the vector-encoding plasmid DNA into 293T cells using calcium phosphate method. Eighteen hours post transfection, the culture was replaced with fresh medium. Twenty-four hours post medium replacement, the supernatant containing the vector was collected and filtered through a 0.45 µm filter and used immediately or stored in aliquots at -80°C for later use. Twenty micro-liter of the collected vector stocks was used to infect human prostate cancer cells, PC3. Twenty-four hours post infection, AZT was added to the cells to inhibit further viral replication. Forty-eight hours post infection, genomic DNA of infected PC3 cells was extracted for titer assay. The titer of the vector stocks was determined by qPCR with an inclusion of standards of known copy numbers.

The expression of human IL-2 is first tested at the RNA level. Total RNA is extracted from transduced CF2TH and HT1080 cells at 5 days post infection using standard RNA extraction method. RT-PCR was performed to detect the expression of human IL-2. Fifty nano-gram of total RNA was used in the RT reaction to generate cDNA. One tenth of the volume from RT reaction was subsequently used for PCR using PCR primer set specific for human IL-2. Result from RT-PCR shows that human IL-2 is expressed in HT1080 cells transduced with pAC3-hIL2 vector.

The expression of secreted human IL-2 protein was tested by standard ELISA. Vector stock collected from day 5 post infection was serially diluted in the ELISA assay in order to obtain a linear range between protein concentration and dilution factor. The result showed that human IL-2 protein is secreted at a higher concentration by the CF2TH cells than HT1080 at day 5 post infection.

### Example 29 Anti-tumor efficacy studies with interleukin 2 expressing vector in a mouse subcutaneous tumor model.

### Objective

The objective of this study is to assess the effect of a novel MLV based replication-competent retroviral vector carrying the murine leukocytotrophic hormone interleukin 2 (IL-2) sequence (pAC3-mIL2) on tumor growth, when delivered via intratumoral (IT) injection in BALB/c mice bearing subcutaneous colon carcinoma (CT26.WT).

### Mice

Female BALB/c mice (age ∼8 weeks) are purchased from Jackson Laboratories (Bar Harbor, ME). Mice will be acclimated for 7 days after arrival before start of studies.

### Cells

CT26.WT cells (ATCC , Manassas VA) are an N-nitroso-N-methylurethane-(NNMU) induced, undifferentiated colon carcinoma cell line. Cells are cultured in Dulbecco's modified Eagles medium with 10% fetal bovine serum, sodium pyruvate, and Glutamax (Hyclone, Logan UT, and Invitrogen, San Diego CA). Cells are resuspended in PBS (Hyclone, Logan UT) for implantation. CT26.WT cells (2E5 in 100 pL) are injected into the right flank of BALB/c mice.

### Vector

Vectors preparations are made by transient transfection (or from a producer cell line; refer to provisional) with titers of approximately 6E6TU/ml. For initial studies vector is not further purified or concentrated. For follow on experiments to determine full dose response curves, high titer purified material is prepared with a titer expected around 10E8/ml. Vector is administered IT in a volume of 100 µL and the total dose/mouse of approximately 6E5 TU/mouse. Vector expressing gamma interferon is identified as Toca IL2.

### Tumor implantation and vector injection

Five groups of female BALB/c (55 mice, 9-10 weeks of age) are implanted subcutaneously with CT26.WT tumor cells (Day 0) and then dosed (day 4-7 depending on growth rate of the CT26 tumor; approximately 50-100mm³) with vehicle (Groups 1), with control vector [AC3-GFP(V), (Group2), IT Toca IL2 vector injection (Groups 3), or intravenous Toca IL2 vector injection (group 4). Group 5 mice have no tumor implanted and are intravenously injected with vector only.

### Data analyses

Tumor growth analysis is carried out to 2000mm³ or to 60 days based on which ever comes first. 10 mice from each group will be plotted for tumor size over time. Statistical significance will be determined using analysis of variance (ANOVA). P values of <0.05 are considered statistically significant in all analyses, which are performed with Prism 5 statistical software (GraphPad Software) or equivalent. In-life observations are also taken to assess any adverse events to IL-2 expression during treatment.

### Results

Delivery of IL-2 by replicating MLV reduces and in some instances clears tumors burden from the BALB/c CT26 mouse model..

A number of embodiments of the disclosure have been described. Nevertheless, it will be understood that various modifications may be made without departing from the spirit and scope of the disclosure. Accordingly, other embodiments are within the scope of the following claims.

The present disclosure provides the following clauses:
Clause 1. A recombinant replication competent retrovirus (RCR) comprising:
   a retroviral GAG protein;
   a retroviral POL protein;
   a retroviral envelope;
   a retroviral polynucleotide comprising Long-Terminal Repeat (LTR) sequences at the 3' end of the retroviral polynucleotide sequence, a promoter sequence at the 5' end of the retroviral polynucleotide, said promoter being suitable for expression in a mammalian cell, a gag nucleic acid domain, a pol nucleic acid domain and an env nucleic acid domain;
   a cassette comprising an internal ribosome entry site (IRES) operably linked to a heterologous polynucleotide, wherein the cassette is positioned 5' to the 3' LTR and 3' to the env nucleic acid domain encoding the retroviral envelope; and
   cis-acting sequences necessary for reverse transcription, packaging and integration in a target cell,
   wherein the RCR maintains higher replication competency after 6 passages compared to a vector comprising SEQ ID NO:21 (pACE).
Clause 2. The retrovirus of Clause 1, wherein the retroviral polynucleotide sequence is derived from murine leukemia virus (MLV), Moloney murine leukemia virus (MoMLV), Feline leukemia virus (FeLV)or Gibbon ape leukemia virus (GALV).
Clause 3. The retrovirus of Clause 1 or 2, wherein the MLV is an amphotropic MLV.
Clause 4. The retrovirus of Clause 1, wherein the retrovirus is a gammaretrovirus.
Clause 5. The retrovirus of Clause 1, wherein the target cell is a cell having a cell proliferative disorder.
Clause 6. The retrovirus of Clause 1, wherein the target cell is a neoplastic cell.
Clause 7. The retrovirus of Clause 5, wherein the cell proliferative disorder is selected from the group consisting of lung cancer, colon-rectum cancer, breast cancer, prostate cancer, urinary tract cancer, uterine cancer, brain cancer, head and neck cancer, pancreatic cancer, melanoma, stomach cancer and ovarian cancer, rheumatoid arthritis or other autoimmune disease.
Clause 8. The retrovirus of Clause 1, wherein the promoter sequence is associated with a growth regulatory gene.
Clause 9. The retrovirus of Clause 1, wherein the promoter sequence comprises a tissue-specific promoter sequence.
Clause 10. The retrovirus of Clause 9, wherein the tissue-specific promoter sequence comprises at least one androgen response element (ARE).
Clause 11. The retrovirus of Clause 10, wherein the androgen response element is derived from the probasin promoter.
Clause 12. The retrovirus of Clause 9, wherein the tissue-specific promoter sequence comprises a probasin promoter.
Clause 13. The retrovirus of Clause 1, wherein the promoter comprises a CMV promoter having a sequence as set forth in SEQ ID NO:19, 20 or 22 from nucleotide 1 to about nucleotide 582 and may include modification to one or more nucleic acid bases and which is capable of directing and initiating transcription.
Clause 14. The retrovirus of Clause 1, wherein the promoter comprises a sequence as set forth in SEQ ID NO:19, 20 or 22 from nucleotide 1 to about nucleotide 582.
Clause 15. The retrovirus of Clause 1, wherein the promoter comprises a CMV-R-U5 domain polynucleotide.
Clause 16. The retrovirus of Clause 15, wherein the CMV-R-U5 domain comprise the immediately early promoter from human cytomegalovirus linked to an MLV R-U5 region.
Clause 17. The retrovirus of Clause 16, wherein the CMV-R-U5 domain polynucleotide comprises a sequence as set forth in SEQ ID NO:19, 20 or 22 from about nucleotide 1 to about nucleotide 1202 or sequences that are at least 95% identical to a sequence as set forth in SEQ ID NO:19, 20 or 22, wherein the polynucleotide promotes transcription of a nucleic acid molecule operably linked thereto.
Clause 18. The retrovirus of Clause 1, wherein the gag polynucleotide is derived from a gammaretrovirus.
Clause 19. The retrovirus of Clause 18, wherein the gag nucleic acid domain comprises a sequence from about nucleotide number 1203 to about nucleotide 2819 of SEQ ID NO: 19 or 22 or a sequence having at least 95%, 98%, 99% or 99.8% identity thereto.
Clause 20. The retrovirus of Clause 1, wherein the pol domain of the polynucleotide is derived from a gammaretrovirus.
Clause 21. The retrovirus of Clause 20, wherein the pol domain comprises a sequence from about nucleotide number 2820 to about nucleotide 6358 of SEQ ID NO:19 or 22 or a sequence having at least 95%, 98%, 99% or 99.9% identity thereto.
Clause 22. The retrovirus of Clause 1, wherein the env domain comprises a sequence from about nucleotide number 6359 to about nucleotide 8323 of SEQ ID NO:19 or 22 or a sequence having at least 95%, 98%, 99% or 99.8% identity thereto.
Clause 23. The retrovirus of Clause 1, wherein the IRES is derived from an encephalomyocarditis virus.
Clause 24. The retrovirus of Clause 23, wherein the IRES comprises a sequence from about nucleotide number 8327 to about nucleotide 8876 of SEQ ID NO:19 or 22 or a sequence having at least 95%, 98%, or 99% identity thereto.
Clause 25. The retrovirus of Clause 1, wherein the heterologous nucleic acid comprises a polynucleotide having a sequence as set forth in SEQ ID NO:3, 5, 11, 13, 15 or 17.
Clause 26. The retrovirus of Clause 1, wherein the heterologous nucleic acid encodes a polypeptide comprising a sequence as set forth in SEQ ID NO:4.
Clause 27. The retrovirus of Clause 1, wherein the heterologous nucleic acid is human codon optimized and encodes a polypeptide as set forth in SEQ ID NO:4.
Clause 28. The retrovirus of Clause 1, wherein the heterologous nuecleic acid comprises a sequence as set forth in SEQ ID NO: 19 or 22 from about nucleotide number 8877 to about 9353.
Clause 29. The retrovirus of Clause 28, wherein the 3' LTR is derived from a gammaretrovirus.
Clause 30. The retrovirus of Clause 29, wherein the 3' LTR comprises a U3-R-U5 domain.
Clause 31. The retrovirus of Clause 29, wherein the 3' LTR comprises a sequence as set forth in SEQ ID NO:19 or 22 from about nucleotide 9405 to about 9998 or a sequence that is at least 95%, 98% or 99.5% identical thereto.
Clause 32. The retrovirus of Clause 1, wherein the retroviral polynucleotide comprises a sequence as set forth in SEQ ID NO:19, 20 or 22.
Clause 33. The retrovirus of Clause 1, wherein the heterologous nucleic acid sequence encodes a biological response modifier. Clause 34. The retrovirus according to Clause 33, wherein the biological response modifier comprises an immunopotentiating cytokine.
Clause 35. The retrovirus according to Clause 34, wherein the immunopotentiating cytokine is selected from the group consisting of interleukins 1 through 15, interferon, tumor necrosis factor (TNF), and granulocyte-macrophage-colony stimulating factor (GM-CSF).
Clause 36. The retrovirus according to Clause 34, wherein the immunopotentiating cytokine is interferon.
Clause 37. The retrovirus according to Clause 36, wherein the interferon is gamma interferon.
Clause 38. The retrovirus according to Clause 1, wherein the heterologous nucleic acid encodes a polypeptide that converts a nontoxic prodrug in to a toxic drug.
Clause 39. The retrovirus according to Clause 38, wherein the polypeptide that converts a nontoxic prodrug in to a toxic drug is thymidine kinase, purine nucleoside phosphorylase (PNP), or cytosine deaminase.
Clause 40. The retrovirus according to Clause 1, wherein the heterologous nucleic acid sequence encodes targeting moiety.
Clause 41. The retrovirus according to Clause 40, wherein the targeting moiety comprises a cancer antigen.
Clause 42. The retrovirus according to Clause 1, wherein the heterologous nucleic acid sequence encodes a binding domain.
Clause 43. The retrovirus according to Clause 42, wherein the binding domain comprises a receptor domain, an antibody, or antibody fragment.
Clause 44. The retrovirus according to Clause 1, wherein the heterologous nucleic acid sequence comprises an inhibitory polynucleotide.
Clause 45. The retrovirus according to Clause 44, wherein the inhibitory polynucleotide comprises an RNAi or siRNA sequence.
Clause 46. An isolated polynucleotide comprising from 5' to 3':
   a CMV-R-U5 fusion of the immediate early promoter from human cytomegalovirus to an MLV R-U5 region;
   a PBS, primer binding site for reverse transcriptase;
   a 5' splice site;
   ψ packaging signal;
   a gag coding sequence for MLV group specific antigen;
   a pol coding sequence for MLV polymerase polyprotein;
   a 3' splice site;
   a 4070A env coding sequence for envelope protein of MLV strain 4070A;
   an internal ribosome entry site (IRES) from
   encephalomyocarditis virus;
   wherein the 4070A env coding sequence and the IRES comprise a sequence from about nucleotide number 6359 to about nucleotide 8876 of SEQ ID NO:19 or 22 or a sequence having at least 95%, 98%, or 99% identity thereto,
   a heterologous nucleic acid sequence;
   a polypurine tract; and
   a U3-R-U5 MLV long terminal repeat.
Clause 47. The polynucleotide of Clause 46 wherein the retroviral polynucleotide comprises a sequence as set forth in SEQ ID NO:19, 20 or 22.
Clause 48. The polynucleotide of Clause 46, wherein the retroviral polynucleotide sequence is derived from murine leukemia virus (MLV), Moloney murine leukemia virus (MoMLV), Feline leukemia virus (FeLV)or Gibbon ape leukemia virus (GALV).
Clause 49. The polynucleotide of Clause 48, wherein the MoMLV is an amphotropic MoMLV.
Clause 50. A method of treating a subject with a cell proliferative disorder comprising contacting the subject with a polynucleotide of Clause 1 or 46 under conditions such that the polynucleotide is expressed and contacting the subject with 5-fluorocytosine.
Clause 51. The method of Clause 50, wherein the polynucleotide is integrated into cell of the subject.
Clause 52. The method of Clause 50, wherein the polynucleotide is delivered by a retroviral vector.
Clause 53. The method of Clause 52, wherein the retroviral vector comprises a sequence as set forth in SEQ ID NO:19, 20 or 22.
Clause 54. The method of Clause 50, wherein the cell proliferative disorder is glioblasoma multiforme.
Clause 55. The method of Clause 50, wherein the cell proliferative disorder is selected from the group consisting of lung cancer, colon-rectum cancer, breast cancer, prostate cancer, urinary tract cancer, uterine cancer, brain cancer, head and neck cancer, pancreatic cancer, melanoma, stomach cancer and ovarian cancer.
Clause 56. A method of treating a cell proliferative disorder in a subject comprising contacting the subject with a retrovirus of Clause 1, wherein the heterologous nucleic acid sequence encodes a therapeutic protein that inhibits proliferation of a neoplastic cell.
Clause 57. The method of Clause 56, wherein the therapeutic protein comprises a polypeptide that converts a non-cytotoxic drug to a cytotoxic drug.
Clause 58. The method of Clause 57, wherein the polypeptide has cytosine deaminase activity.
Clause 59. The method of Clause 58, wherein the polypeptide comprises a sequence as set forth in SEQ ID NO:4, 12, 14, 16, or 18.
Clause 60. The method of Clause 57, wherein the non-cytotoxic drug is 5-fluorocytosine.
Clause 61. The method of Clause 56, wherein the retroviral polynucleotide comprises a sequence as set forth in SEQ ID NO:19, 20 or 22.
Clause 62. A method of treating a cell proliferative disorder comprising administering a retrovirus comprising a polynucleotide of Clause 46 to a subject having a cell proliferative disorder under conditions such that the retrovirus infects cells with the disorder and contacting the subject with an anti-cancer agent or chemotherapeutic agent.
Clause 63. The method of Clause 62, wherein the anti-cancer agent is selected from the group consisting of bevacizumab, pegaptanib, ranibizumab, sorafenib, sunitinib, AE-941, VEGF Trap, pazopanib, vandetanib, vatalanib, cediranib, fenretinide, squalamine, INGN-241, oral tetrathiomolybdate, tetrathiomolybdate, Panzem NCD, 2-methoxyestradiol, AEE-788, AG-013958, bevasiranib sodium, AMG-706, axitinib, BIBF-1120, CDP-791, CP-547632, PI-88, SU-14813, SU-6668, XL-647, XL-999, IMC-1121B, ABT-869, BAY-57-9352, BAY-73-4506, BMS-582664, CEP-7055, CHIR-265, CT-322, CX-3542, E-7080, ENMD-1198, OSI-930, PTC-299, Sirna-027, TKI-258, Veglin, XL-184, or ZK-304709.
Clause 64. A method of treating a cell proliferative disorder in a subject comprising administering a retrovirus of any one of Clauses 40-43 and contacting the subject with a cognate comprising a cytotoxic agent.
Clause 65. An isolated polynucleotide comprising:
   a sequence encoding a polypeptide comprising the extracellular (ECD) and transmembrane (TM) domains of HER2 linked to a coding sequence for an optimized cytosine deaminase of SEQ ID NO:4.
Clause 66. An isolated polynucleotide encoding a polypeptide comprising the Herceptin binding domain IV of the extracellular (ECDIV) and transmembrane (TM) domains of HER2 linked to an optimized cytosine deaminase comprising SEQ ID NO:4.
Clause 67. An isolated polynucleotide encoding a polypeptide comprising the signal peptide (S) of HER 2 operably fused to the Herceptin binding domain IV of the extracellular (ECDIV) and transmembrane (TM) domains of HER2 linked to an optimized cytosine deaminase comprising SEQ ID NO:4.
Clause 68. A recombinant replication competent retrovirus comprising a polynucleotide of Clause 65, 66 or 67 operably linked to an IRES and 3' to the ENV sequence.
Clause 69. A recombinant retrovirus of Clause 1, wherein the heterologous polypeptide is HER2 or truncated HER2 (HER2 ECDTM) or HER2 SECDIVTM.
Clause 70. The recombinant retrovirus of Clause 1, administered at a dose of 10⁻³ to 10⁻⁷ TU/g brain weight.
Clause 71. The method of any one of Clauses 50-54, wherein the retrovirus is administered from about 10⁻³ to 10⁻⁷ TU/g brain weight.
Clause 72. The method of Clause 71, wherein the retrovirus is administered from about 10⁻⁴ to 10⁻⁶ TU/g brain weight.
Clause 73. A recombinant replication competent retrovirus (RCR) comprising:
   a retroviral GAG protein;
   a retroviral POL protein;
   a retroviral envelope;
   a retroviral polynucleotide comprising Long-Terminal Repeat (LTR) sequences at the 3' end of the retroviral polynucleotide sequence, a promoter sequence at the 5' end of the retroviral polynucleotide, said promoter being suitable for expression in a mammalian cell, a gag nucleic acid domain, a pol nucleic acid domain and an env nucleic acid domain;
   a cassette comprising an primary precursor miRNA (pri-miRNA) for an miRNA or siRNA sequence.
   cis-acting sequences necessary for reverse transcription, packaging and integration in a target cell,
Clause 74. A recombinant replication competent retrovirus (RCR) comprising:
   a retroviral GAG protein;
   a retroviral POL protein;
   a retroviral envelope;
   a retroviral polynucleotide comprising Long-Terminal Repeat (LTR) sequences at the 3' end of the retroviral polynucleotide sequence, a promoter sequence at the 5' end of the retroviral polynucleotide, said promoter being suitable for expression in a mammalian cell, a gag nucleic acid domain, a pol nucleic acid domain and an env nucleic acid domain;
   a cassette comprising a precursor miRNA (pre-miRNA) for an miRNA or siRNA sequence.
   cis-acting sequences necessary for reverse transcription, packaging and integration in a target cell.
Clause 75. A recombinant replication competent retrovirus (RCR) comprising:
   a retroviral GAG protein;
   a retroviral POL protein;
   a retroviral envelope;
   a retroviral polynucleotide comprising Long-Terminal Repeat (LTR) sequences at the 3' end of the retroviral polynucleotide sequence, a promoter sequence at the 5' end of the retroviral polynucleotide, said promoter being suitable for expression in a mammalian cell, a gag nucleic acid domain, a pol nucleic acid domain and an env nucleic acid domain;
   a cassette comprising a polIII promoter and an siRNA or shRNA,
   cis-acting sequences necessary for reverse transcription, packaging and integration in a target cell,
   where the vector is at least 90% stable for at least 6 passages in cells.
Clause 76. The vector of any one of Clauses 73, 74 or 75, wherein Dependent claims for miR 128-1 and miR 128-2.
Clause 77. The vector of any one of Clauses 73, 74 or 75, wherein the vector further comprises an IRES cassette operably linked to a heterologous polynucleotide.
Clause 78. The vector of Clause 77, wherein the IRES cassette is located immediately 3' to the env gene.
Clause 79. A recombinant replication competent retrovirus (RCR) comprising:
   a retroviral GAG protein;
   a retroviral POL protein;
   a retroviral envelope;
   a retroviral polynucleotide comprising Long-Terminal Repeat (LTR) sequences at the 3' end of the retroviral polynucleotide sequence, a promoter sequence at the 5' end of the retroviral polynucleotide, said promoter being suitable for expression in a mammalian cell, a gag nucleic acid domain, a pol nucleic acid domain and an env nucleic acid domain;
   a cassette comprising a target sequence for a naturally occurring miRNA; and
   cis-acting sequences necessary for reverse transcription, packaging and integration in a target cell.

## Claims

1. A recombinant replication competent gamma-retrovirus comprising:
a gamma-retroviral GAG protein;
a gamma-retroviral POL protein;
a gamma-retroviral envelope; and
a gamma-retroviral polynucleotide comprising from 5' to 3': R-U5-gag-pol-env-(a heterologous polynucleotide selected from a binding domain or an siRNA)-U3-R,
wherein the "R" and the "U5" are the repeat and 5' untranslated region, respectively, of a retroviral Long-Terminal Repeat (LTR), and the "U3" is the 3' untranslated region of the LTR,
wherein "gag" comprises the coding sequence for the GAG protein, and
wherein "pol" comprises the coding sequence for the POL protein.

2. The recombinant replication competent gamma-retrovirus of claim 1, wherein the binding domain comprises an antibody binding domain.

3. The recombinant replication competent gamma-retrovirus of claim 1 or claim 2, wherein the binding domain is a single chain antibody domain.

4. The recombinant replication competent gamma-retrovirus of any of the foregoing claims, wherein the binding domain is preceded by an internal ribosome entry site (IRES) or a promoter.

5. The recombinant replication competent gamma-retrovirus of claim 1, wherein the siRNA is preceded by a promoter comprising a pollll transcription unit.

6. The recombinant replication competent gamma-retrovirus of claim 5, wherein the promoter is a U6 or H1 promoter.

7. The recombinant replication competent gamma-retrovirus of claim 1, 5 or 6, wherein the siRNA comprises a miR128-1 or miR128-2 sequence.
